(19) Europäisches Patentamt
European Patent Office
Office européen des brevets

(11) EP 1 600 440 A1

(12) # EUROPEAN PATENT APPLICATION
published in accordance with Art. 158(3) EPC

(43) Date of publication:
**30.11.2005 Bulletin 2005/48**

(21) Application number: **04717836.3**

(22) Date of filing: **05.03.2004**

(51) Int Cl.[7]: **C07D 209/26**

(86) International application number:
**PCT/JP2004/002813**

(87) International publication number:
**WO 2004/078719 (16.09.2004 Gazette 2004/38)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **06.03.2003 JP 2003059459**

(71) Applicant: **ONO PHARMACEUTICAL CO., LTD.
Osaka-shi, Osaka 541-8526 (JP)**

(72) Inventors:
- **IWAHASHI, M., Ono Pharmaceutical Co., Ltd. Mishima-gun, Osaka 6188585 (JP)**
- **NAGANAWA, A., Ono Pharmaceutical Co., Ltd. Mishima-gun, Osaka 6188585 (JP)**
- **NISHIYAMA, T., Ono Pharmaceutical Co., Ltd. Mishima-gun, Osaka 618-8585 (JP)**
- **NAGASE, T., Ono Pharmaceutical Co., Ltd. Mishima-gun, Osaka 618-8585 (JP)**
- **KOBAYASHI, K., Ono Pharmaceutical Co., Ltd. Mishima-gun, Osaka 618-8585 (JP)**
- **NAMBU, F., Ono Pharmaceutical Co., Ltd. Mishima-gun, Osaka 618-8585 (JP)**

(74) Representative: **Henkel, Feiler & Hänzel
Möhlstrasse 37
81675 München (DE)**

(54) **INDOLE DERIVATIVE COMPOUNDS AND DRUGS CONTAINING THE COMPOUNDS AS THE ACTIVE INGREDIENT**

(57) An indole derivative compound represented by formula (I)

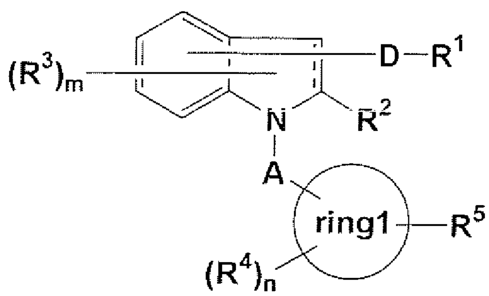

( I )

(wherein the symbols in the formula are as mentioned in the specification) and a salt thereof Since the compounds represented by formula (I) binds to $PGD_2$ receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, diseases accompanied by itch (such as atopic dermatitis, urticaria), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, chronic articular rheumatism, pleuritis, ulcerative colitis and irritable bowel syndrome.

EP 1 600 440 A1

**Description**

Technical Field

The present invention relates to an indole derivative compound. More particularly, the present invention relates to:

(1) an indole derivative compound represented by formula (1)

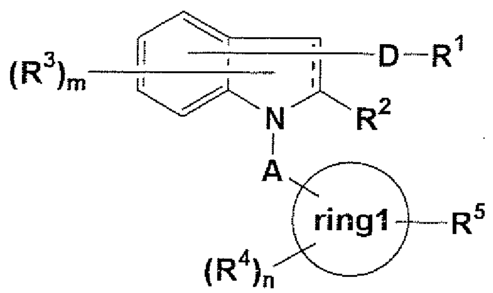

( I )

(in the formula, all symbols have the same meanings as those which will be mentioned later), a salt thereof, an N-oxide thereof, a solvate thereof or a prodrug thereof,
(2) a process for producing the same and
(3) a pharmaceutical agent containing the same as an active ingredient.

Background Art

Prostaglandin $D_2$ (abbreviated as $PGD_2$) has been known as a metabolite in an arachidonic acid cascade and is considered to be one of chemical transmitters participating in allergic diseases such as allergic rhinitis, bronchial asthma and allergic conjunctivitis. It has been known that $PGD_2$ is produced in and liberated from mast cells, macrophage or Th2 cell, *etc.* and that the liberated $PGD_2$ shows an activity of constriction of bronchus, promotion of hemal permeability, dilation or constriction of vessels, promotion of secretion of mucilage, inhibition of aggregation of platelets, chemotaxis of eosinophil, basophil or lymphocyte, and enhancement of cytokine production from lymphocyte. It has been also reported that $PGD_2$ induces airway constriction and nasal obstruction symptoms *in vivo* as well and an increase in $PGD_2$ concentration in pathological lesion of patients suffering from systemic mastocylosis, nasal allergy, bronchial asthma, atopic dermatitis, urticaria, *etc.* (*N. Engl. J. Med.* 1989; 303: 1400-4, *Am. Rev. Respir. Dis.* 1983; 128: 597-602, *J. Allergy Clin. Immunol.* 1991; 88: 33-42, *Arch. Otolaryngol. Head Neck Surg.* 1987; 113: 179-83, *J. Allergy Clin. Immunol.* 1988; 82: 869-77, *J. Immunol.* 1991; 146: 671-6, *J. Allergy Clin. Immunol.* 1989; 83: 905-12, *N. Eng. J. Med.* 1986; 315: 800-4, *Am. Rev. Respir. Dis.* 1990; 142, 126-32, *J. Allergy Clin. Immunol.* 1991; 87: 540-8, *J. Allery Clin. Immunol.* 1986; 78: 458-61). It has been also reported that $PGD_2$ participates in nerve activity, particularly in sleeping, thermoregulation, hormone secretion and pain. It has been also reported that it participates in aggregation of platelets, glycogen metabolism and adjustment of ocular tension.

$PGD_2$ exerts its function when binds to a chemoattractant receptor - homologous molecule expressed on Th2 cells (CRTH2) which is one of its receptors. CRTH2 receptor antagonists binds to the receptors and inhibits effect of $PGD_2$. CRTH2 receptor antagonists have been believed to be useful for prevention and/or treatment of diseases such as allergic disease (*e.g.,* allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (*e.g.*, atopic dermatitis, urticaria, allergic conjunctivitis, allergic rhinitis and contact dermatitis), diseases (*e.g.,* cataract, retinal detachment, inflammation, infection and sleep disorder) which is generated secondarily as a result of behavior accompanied by itch (*e.g.,* scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis, irritable bowel syndrome, *etc*. It also participates in sleep and aggregation of platelets and is believed to be useful for those diseases as well.

$PGD_2$ binds to prostanoid DP receptor (DP receptor) as well as CRTH2 receptor, and it is known that various kinds of biological activity is shown. Because $PGD_2$ is internal ligand of DP receptor and CRTH2 receptor, CRTH2

receptor antagonist binds and antagonizes to DP receptor. Therefore, it is expected that CRTH2 receptor antagonist is useful for prevention and/or treatment of various kinds of allergic reaction (disease) and inflammatory reaction (disease) which caused by $PGD_2$.

For example, as the disease, allergic rhinitis, allergic conjunctivitis, atopic dermatitis, bronchial asthma and food allergy, systemic mastacytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (*e.g.,* atopic dermatitis, urticaria, allergic conjunctivitis, allergic rhinitis and contact dermatitis), diseases (*e.g.,* cataract, retinal detachment, inflammation, infection and sleep disorder) which is generated secondarily as a result of behavior accompanied by itch (*e.g.*, scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuratis, osteoarthritis, Crohn's disease, ulcerative colitis, irritable bowel syndrome, *etc.* are given.

As a compound having the activity of antagonizing CRTH2 receptor, only compound represented by a following formula (A) is shown (JP-A-2002-98702, page 29, figure 15).

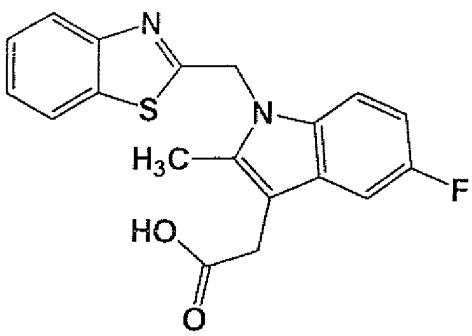

(A)

In addition, as the compound having the activity of antagonizing DP receptor, for example, indole derivative compound represented by formula (B);

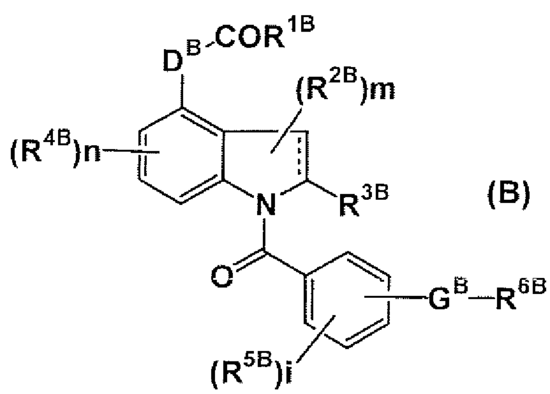

(B)

(wherein $R^{1B}$ represents hydroxy, $R^{2B}$ represents a hydrogen atom or C1-6 alkyl, $R^{3B}$ represents a hydrogen atom or C1-6 alkyl, $R^{4B}$ and $R^{5B}$ each independently represents a hydrogen atozn, C1-6 alkyl, C1-6 alkoxy, a halogen atom or trihalomethyl, $D^B$ represents a single bond or C1-6 alkylene, in $-G^B-R^{6B}$, 1) $G^B$ represents C1-6 alkylene which may be substituted with 1 to 2 oxygen atom(s) and/or sulfur atom(s), C2-6 alkenylene which may be substituted with 1 to 2 oxygen atom(s) and/or sulfur atom(s), $R^{6B}$ represents a C3-15 saturated or unsaturated carbocyclic ring, or a 4- to 15-membered heterocyclic ring containing 1 to 5 nitrogen atom(s), sulfur atom(s) and/or oxygen atom(s), or 2) $G^B$ and $R^{6B}$ are taken together to represent C1-15 alkyl which may be substituted with 1 to 5 oxygen atom(s) and/or sulfur atom(s)) or non-toxic salt thereof is disclosed (The description of substituent extracted only necessary part.) (WO01/66520, page 3).

Moreover, for example, 2-(1-(4-benzyloxybenzoyl)-2-methyl-5-methoxyindol-3-yl)acetic acid methyl ester, 2-(1-(4-phenylbenzoyl)-2-methyl-5-methoxyindol-3-yl)acetic acid methyl ester, *etc.* are disclosed as an synthetic intermediate of antiinflammatory, but it is not described about effect with respect to CRTH2 receptor at all. (for example, GB997638, page 15)

In prostaglandin receptors, there are many receptors including subtypes and each of them has a different pharmacological action. Now, if novel compounds which specifically binds to a DP receptor, *i.e.* CRTH2 receptor and/or DP receptor, and binds weakly to other $PGD_2$ receptors are able to be found, they can be pharmaceuticals having little side effect since no other functions are not exerted. Therefore, there has been a demand for finding such pharmaceuticals.

Disclosure of the Invention

The inventors of the present invention have carried out intensive studies for finding compounds which specifically binds to $PGD_2$ receptors and exerts antagonistic activity and, as a result, they have found that indole derivatives represented by formula (I) achieve the problem to accomplish the present invention.

Thus, the present invention relates to:

(1) An indole derivative compound represented by formula (I)

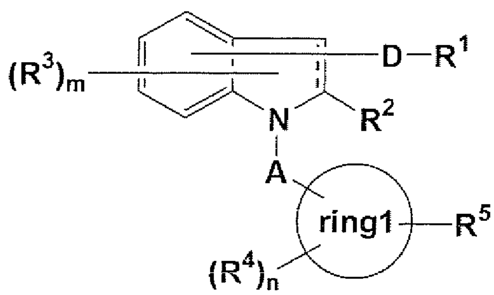

(I)

wherein $R^1$ represents (1) $-COR^6$ or (2) $-CH_2OR^7$;

$R^6$ represents (1) hydroxy, (2) C1-6 alkoxy, (3) $-NR^8R^9$, (4) C1-6 alkoxy substituted with phenyl or (5) C2-6 alkenyloxy;

$R^7$ represents (1) a hydrogen atom or (2) C2-6 acyl;

$R^8$ and $R^9$ each independently represents (1) a hydrogen atom, (2) C1-6 alkyl or (3) - $SO_2R^{10}$;

$R^{10}$ represents (1) C1-6 alkyl, (2) carbocycle-1 or (3) heterocycle-1;

D represents (1) a single bond, (2) C1-6 alkylene, (3) C2-6 alkenylene or (4) -O-(C1-6 alkylene)-;

$R^2$ represents (1) C1-6 alkyl, (2) C1-6 alkoxy, (3) a halogen atom, (4) trihalomethyl, (5) cyano, (6) hydroxy or (7) a hydrogen atom;

$R^3$ and $R^4$ each independently represents (1) a hydrogen atom, (2) C1-6 alkyl, (3) C1-6 alkoxy, (4) C1-6 alkyl substituted with C1-6 alkoxy, (5) a halogen atom, (6) nitro, (7) - $NR^{11}R^{12}$, (8) trihalomethyl, (9) cyano, (10) hydroxy or (11) trihalomethoxy;

$R^{11}$ and $R^{12}$ each independently represents a hydrogen atom or C1-6 alkyl;

m represents an integer of 1 to 3 or 4;

n represents an integer of 1 to 4;

$R^5$ represents $R^{5-1}$, $R^{5-2}$, $R^{5-3}$, $R^{5-4}$, $R^{5-5}$ or $R^{5-6}$;

$R^{5-1}$ represents

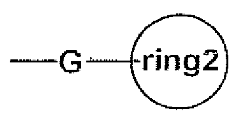

;

$R^{5-2}$ represents (1) C1-15 alkyl may be substituted with 1-5 of an oxygen atom and/or a sulfur atom, in which the alkyl may be substituted with 1 to 12 substituent(s) selected from C1-6 alkoxy, a halogen atom, hydroxy, cyano, oxo and $NR^{13}R^{14}$, in which $R^{13}$ and $R^{14}$ each independently represents a hydrogen atom, C1-6 alkyl, C2-6 alkenyl, phenyl, benzoyl, naphthyl, phenyl substituted with C1-6 alkyl, or C1-6 alkyl substituted with phenyl or cyano, (2) C2-15 alkenyl may be substituted with 1-5 of an oxygen atom and/or a sulfur atom, in which the alkenyl may be substituted with 1 to 12 substituent(s) selected from C1-6 alkoxy, a halogen atom, hydroxy, cyano, oxo and

$NR^{13}R^{14}$, in which $R^{13}$ and $R^{14}$ have the same meanings as described above, or (3) C2-1 alkynyl may be substituted with 1-5 of an oxygen atom and/or a sulfur atom, in which the alkynyl may be substituted with 1 to 12 substituent (s) selected from C1-6 alkoxy, a halogen atom, hydroxy, cyano, oxo and $NR^{13}R^{14}$, in which $R^{13}$ and $R^{14}$ have the same meanings as described above, except a group represented by $R^{5-3}$ and $R^{5-5}$ described below;

$R^{5-3}$ represents (1) C1-6 alkyl substituted with C1-6 alkoxy or (2) C1-6 alkoxy substituted with C1-6 alkoxy;

$R^{5-4}$ represents (1) C1-15 alkyl which is substituted with one nitrogen atom and may be further substituted with 1 to 4 of a nitrogen atom, an oxygen atom and/or a sulfur atom, in which the alkyl may be substituted with 1 to 12 substituent(s) selected from C1-6 alkoxy, a halogen atom, hydroxy, cyano, oxo and $NR^{15}R^{16}$, in which $R^{15}$ and $R^{16}$ each independently represents a hydrogen atom, C1-6 alkyl, C2-6 alkenyl, phenyl, benzoyl, naphthyl, phenyl substituted with C1-6 alkyl, or C1-6 alkyl substituted with phenyl or cyano, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4, (2) C2-15 alkenyl which is substituted with one nitrogen atom and may be further substituted with 1 to 4 of a nitrogen atom, an oxygen atom and/or a sulfur atom, in which the alkenyl may be substituted with 1 to 12 substituent(s) selected from C1-6 alkoxy, a halogen atom, hydroxy, cyano, oxo and $NR^{15}R^{16}$, in which $R^{15}$ and $R^{16}$ have the same meanings as described above, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4, or (3) C2-15 alkynyl which is substituted with one nitrogen atom and may be further substituted with 1 to 4 of a nitrogen atom, an oxygen atom and/or a sulfur atom, in which the alkynyl may be substituted with 1 to 12 substituent(s) selected from C1-6 alkoxy, a halogen atom, hydroxy, cyano, oxo and $NR^{15}R^{16}$, in which $R^{15}$ and $R^{16}$ have the same meanings as described above, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4;

$R^{5-5}$ represents (1) C1-15 alkyl, (2) C1-15 alkoxy, (3) carboxyl, (4) C1-4 alkoxycarbonyl, (5) trihalomethyl or (6) C1-4 alkylthio;

$R^{5-6}$ represents (1) a halogen atom, (2) amino, (3) nitro, (4) cyano or (5) hydroxy;

G represents $G^1$ or $G^2$;

$G^1$ represents (1) a single bond, (2) C1-6 alkylene may be substituted with 1 to 2 oxygen atom and/or sulfur atom, in which the alkylene may be substituted with hydroxy or C1-4 alkoxy, (3) C2-6 alkenylene may be substituted with 1 to 2 oxygen atom and/or sulfur atom, in which the alkenylene may be substituted with hydroxy or C1-4 alkoxy, (4) $-CONR^{17}-$, (5) $-NR^{18}CO-$, (6) $-SO_2NR^{19}-$, (7) $-NR^{20}SO_2-$ or (8) -N=N-;

$G^2$ represents (1) C1-6 alkylene which is substituted with one nitrogen atom and may be further substituted with 1 to 2 of a nitrogen atom, an oxygen atom and/or a sulfur atom, in which the alkylene may be substituted with hydroxy or C1-4 alkoxy, and the substituted nitrogen atom may be substituted with (a) C1-6 alkyl, (b) C1-6 alkyl substituted with C1-6 alkoxy, (c) carbocycle-5, (d) heterocycle-5, (e) C1-6 alkyl substituted with carbocycle-5 or (f) C1-6 alkyl substituted with heterocycle-5, or (2) C2-6 alkenylene which is substituted with one nitrogen atom and may be further substituted with 1 to 2 of a nitrogen atom, an oxygen atom and/or a sulfur atom, in which the alkenylene may be substituted with hydroxy or C1-4 alkoxy, and the substituted nitrogen atom may be substituted with (a) C1-6 alkyl, (b) C1-6 alkyl substituted with C1-6 alkoxy, (c) carbocycle-5, (d) heterocycle-5, (e) C1-6 alkyl substituted with carbocycle-5 or (f) C1-6 alkyl substituted with heterocycle-5;

$R^{17}$, $R^{18}$, $R^{19}$ and $R^{20}$ each independently represents a hydrogen atom or C1-6 alkyl;

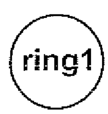

represents (1) carbocycle-2 or (2) heterocycle-2;

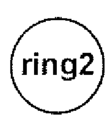

represents (1) carbocycle-3 or (2) heterocycle-3;

carbocycle-1, carbocycle-2, carbocycle-3, carbocycle-4 and carbocycle-5 each independently represents

C3-15 mono-, bi- or tricyclic carboaryl which may be partially or fully saturated;

heterocycle-1, heterocycle-2, heterocycle-3, heterocycle-4 and heterocycle-5 each independently represents 3-15 membered mono-, bi- or tricyclic heteroaryl containing 1 to 5 of hetero atom which is selected from an oxygen atom, a nitrogen atom and a sulfur atom, which may be partially or fully saturated;

carbocycle-1, carbocycle-2, carbocycle-3, carbocycle-4, carbocycle-5, heterocycle-1, heterocycle-2, heterocycle-3, heterocycle-4 and heterocycle-5 each independently may be substituted with 1 to 5 of substituent(s) selected from (1) C1-6 alkyl, (2) C1-10 alkoxy, (3) C1-6 alkyl substituted with C1-6 alkoxy, (4) a halogen atom, (5) hydroxy, (6) trihalomethyl, (7) nitro, (8) $-NR^{21}R^{22}$, (9) phenyl, (10) phenoxy, (11) oxo, (12) C2-6 acyl, (13) cyano or (14) $-SO_2R^{23}$;

$R^{21}$ and $R^{22}$ each independently represents a hydrogen atom or C1-6 alkyl;

$R^{23}$ represents C1-6 alkyl;

A represents (1) carbonyl, (2) $-S(O)_p-$, (3)$G^1$ or (4)$G^2$;

p represents 0 or an integer of 1 to 2;

----- represents (1) a single bond or (2) a double bond;

except for compounds of (1) and (2);

(1) 2-(1-(4-benzyloxybenzoyl)-2-methyl-5-methoxyindol-3-yl)acetic acid methyl ester,
(2) 2-(1-(4-phenylbenzoyl)-2-methyl-5-methoxyindol-3-yl)acetic acid methyl ester),

a salt thereof, an N-oxide thereof, a solvate thereof or a prodrug thereof,

(2) a process for producing the same and

(3) a pharmaceutical comprising the same as an active ingredient.

In the present specification, C1-4 alkyl includes such as methyl, ethyl, propyl, isopropyl, butyl, isobutyl, *sec*-butyl, *tert*-butyl, and the like.

In the present specification, C1-6 alkyl includes such as methyl, ethyl, propyl, isopropyl, butyl, isobutyl, *sec*-butyl, *tert*-butyl, pentyl, isopentyl, neopentyl, hexyl, and the like.

In the present specification, C1-15 alkyl includes such as methyl, ethyl, propyl, isopropyl, butyl, isobutyl, *sec*-butyl, *tert*-butyl, pentyl, isopentyl, neopentyl, hexyl, heptyl, octyl, nonyl, decyl, undecyl, dodecyl, tridecyl, tetradecyl, pentadecyl, and the like.

In the present specification, C2-6 alkenyl includes linear or branched C2-6 alkenyl such as vinyl, allyl, isopropenyl, 2-methallyl, 3-methallyl, 3-butenyl, pentenyl, hexenyl, and the like.

In the present specification, C2-15 alkenyl includes linear or branched C2-15 alkenyl such as vinyl, allyl, isopropenyl, 2-methallyl, 3-methallyl, 3-butenyl, pentenyl, hexenyl, heptenyl, octenyl, nonenyl, decenyl, undecenyl, dodecenyl, tridecenyl, tetradecenyl, pentadecenyl, and the like.

In the present specification, C2-15 alkynyl includes linear or branched C2-15 alkynyl such as ethynyl, propynyl, butynyl, pentynyl, hexynyl, heptynyl, octynyl, nonynyl, decynyl, undecynyl, dodecynyl, tridecynyl, tetradecynyl, pentadecynyl, and the like,

In the present specification, C2-6 alkenyloxy includes linear or branched C2-6 alkenyloxy such as vinyloxy, allyloxy, isopropenyloxy, 2-methallyloxy, 3-methallyloxy, 3-butenyloxy, pentenyloxy, hexenyloxy, and the like.

In the present specification, C1-2 alkoxy includes such as methoxy and ethoxy.

In the present specification, C1-4 alkoxy includes linear or branched C1-4 alkoxy such as methoxy, ethoxy, propoxy, isopropoxy, butoxy, isobutoxy, *sec*-butoxy, *tert*-butoxy, and the like.

In the present specification, C1 -6 alkoxy includes linear or branched C1-6 alkoxy such as methoxy, ethoxy, propoxy, isopropoxy, butoxy, isobutoxy, *sec*-butoxy, *tert*-butoxy, pentyloxy, isopentyloxy, neopentyloxy, hexyloxy, isohexyloxy, and the like.

In the present specification, C1-10 alkoxy includes linear or branched C1-10 alkoxy such as methoxy, ethoxy, propoxy, isopropoxy, butoxy, isobutoxy, *sec*-butoxy, *tert* butoxy, pentyloxy, isopentyloxy, neopentyloxy, hexyloxy, isohexyloxy, heptyloxy, octyloxy, nonyloxy, decyloxy, and the like.

In the present specification, C1-15 alkoxy includes linear or branched C1-15 alkoxy such as methoxy, ethoxy, propoxy, isopropoxy, butoxy, isobutoxy, *sec*-butoxy, *tert*-butoxy, pentyloxy, isopentyloxy, neopentyloxy, hexyloxy, isohexyloxy, heptyloxy, octyloxy, nonyloxy, decyloxy, undecyloxy, dodecyloxy, tridecyloxy, tetradecyloxy, pentadecyloxy, and the like.

In the present specification, a halogen atom includes such as a fluorine, chlorine, bromine and iodine atom.

In the present specification, examples of the trihalomethyl are methyl which are substituted with three halogen atoms.

In the present specification, examples of the trihalomethoxy are methoxy which are substituted with three halogen atoms.

In the present specification, C1-4 alkoxycarbonyl includes linear or branched C1-4 alkoxycarbonyl such as methoxycarbonyl, ethoxycarbonyl, propoxycarbonyl, isopropoxycarbonyl, butoxycarbonyl, isobutoxycarbonyl, *sec*-butoxycarbonyl, *tert*-butoxycarbonyl, and the like.

In the present specification, C1-2 alkylthio includes such as methylthio, ethylthio, and the like.

In the present specification, C1-4 alkylthio includes such as methylthio, ethylthio, propylthio, isopropylthio, butylthio, isobutylthio, *sec*-butylthio, *tert*-butylthio, pentylthio, isopentylthio, neopentylthio, hexylthio, and the like.

In the present specification, C5-14 alkylthio includes such as pentylthio, isopentylthio, neopentylthio, hexylthio, heptylthio, octylthio, nonylthio, decylthio, undecylthio, dodecylthio, tridecylthio, tetradecylthio, pentadecylthio, and the like.

In the present specification, C1-6 alkylene includes such as methylene, ethylene, propylene, isopropylene, butylene, isobutylene, pentylene, hexylene, and the like.

In the present specification, C2-6 alkenylene includes such as vinylene, propenylene, 1- or 2-butenylene, butadienylene, pentenylene, hexenylene, and the like.

In the present specification, C2-6 acyl includes linear or branched C2-6 acyl such as ethanoyl, propanoyl, butanoyl, 2-methylpropanoyl, pentanoyl, 2-methylbutanoyl, 3-methylbutanoyl, hexanoyl, 2-methylpentanoyl, 3-methylpentanoyl, 4-methylpentanoyl, 2-ethylbutanoyl, 2,3-dimethylbutanoyl, and the like.

In the present specification, C3-15 mono-, bi- or tricyclic carbocyclic aryl that may be saturated partially or fully includes bicyclic carbocyclic ring having spiro bond or bicyclic bridged carbocyclic ring; for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, tetrahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2. 1]hept-2-ene, bicyclo[3.1.1]heptane, bicyclo[3.1. 1]hept-2-ene, bicyclo[2.2.2]octane, bicyclo[2.2.2]oct-2-ene, adamantane and noradamantane.

In the present specification, among 3-15 membered mono-, bi- or tricyclic heteroaryl containing 1 to 5 of hetero atom which is selected from an oxygen atom, a nitrogen atom and a sulfur atom, which may be partially or fully saturated, 3-15 membered mono-, bi- or tricyclic heteroaryl containing 1 to 5 of hetero atom which is selected from an oxygen atom, a nitrogen atom and a sulfur atom is, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, and perimidine.

In the present specification, among 3-15 membered mono-, bi- or tricyclic heteroaryl containing 1 to 5 of hetero atom which is selected from an oxygen atom, a nitrogen atom and a sulfur atom, which may be partially or fully saturated, 3-15 membered mono-, bi- or tricyclic heteroaryl containing 1 to 5 of hetero atom which is selected from an oxygen atom, a nitrogen atom and a sulfur atom which is partially or fully saturated is, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrathiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane and benzodithiane.

Unless otherwise specifically mentioned, all isomers are included in the present specification. For example, alkyl, alkoxy and alkylene include linear and branched ones. Moreover, all of isomers due to double bond, ring and fused ring (E-, Z-, cis- and trans-substances), isomers due to presence of asymmetric carbon, *etc.* (R-, S-, ($\alpha$- and $\beta$-substances, enantiomer and diastereomer), optically active substances having optical rotation (D-, L-, d- and 1-substances), polar substances by chromatographic separation (high-polar substance and low-polar substance), equilibrium compounds, rotational isomers, a mixture thereof in any proportion and a racemic mixture are included in the present invention.

Unless otherwise specifically mentioned in the present specification, a symbol 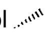 means a bond to the opposite side of the paper (*i.e.*, $\alpha$-configuration), 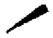 means a bond to this side of the paper (*i.e.*, $\beta$-configuration), a 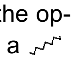 means a $\alpha$-configuration, $\beta$-configuration, or mixture of $\alpha$- and $\beta$-configurations and 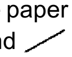 means a mixture of $\alpha$- and $\beta$-configurations as will be obvious for persons skilled in the art.

The compounds of the present invention are converted to pharmaceutically acceptable salts by known methods. With regard to the pharmaceutically acceptable salts, those which are non-toxic and soluble in water are preferred. Examples of appropriate salts are salt with alkaline metal (such as potassium, sodium and lithium), salt with alkaline earth metal (such as calcium and magnesium), ammonium salt (such as tetramethylammonium salt and tetrabutylammonium salt), salt with organic amine (such as triethylamine, methylamine, dimethylamine, cyclopentylamine, benzylamine, phenethylamine, piperidine, monoethanolamine, diethanolamine, tris(hydroxymethyl) methylamine, lysine, arginine and N-methyl-D-glucamine) and acid addition salt (such as inorganic acid salt (*e.g.,* hydrochloride, hydrobromide, hydroiodide, sulfate, phosphate and nitrate) and organic acid salt (*e.g.,* acetate, trifluoroacetate, lactate, tartrate, oxalate, fumarate, maleate, benzoate, citrate, methanesulfonate, ethanesulfonate, benzenesulfonate, toluenesulfonate, isothionate, glucuranate and gluconate)).

The salt of the compound of the present invention also includes solvates and also solvates with the above-mentioned alkaline (earth) metal salt, ammonium salt, organic amine salt and acid addition salt.

The solvate is preferably non-toxic and water-soluble. Examples of an appropriate solvate are solvates with water and with alcoholic solvent (such as ethanol).

In formula (I), $R^1$ is preferably $-COR^6$ or $-CH_2OR^7$, more preferably $-COR^6$.

In formula (I), $R^6$ is preferably hydroxy or C1-6 alkoxy, more preferably hydroxy.

In formula (I), $R^7$ is preferably a hydrogen atom or C2-6 acyl, more preferably a hydrogen atom.

In formula (I), D is preferably a single bond or C1-6 alkylene, more preferably C1-6 alkylene, and most preferably methylene or ethylene.

In formula (I), $R^2$ is preferably C1-6 alkyl or a hydrogen atom, more preferably C1-6 alkyl, and most preferably methyl.

In formula (I), $R^3$ is preferably a hydrogen atom, 1-6 alkyl, C1-6 alkoxy, a halogen atom, or trihalomethyl, more preferably a hydrogen atom, 1-6 alkyl, C1-6 alkoxy or a halogen atom, and most preferably a hydrogen atom, C1-6 alkoxy or a halogen atom.

In formula (I), $R^4$ is preferably a hydrogen atom, 1-6 alkyl, C1-6 alkoxy, a halogen atom, or trihalomethyl, more preferably a hydrogen atom, 1-6 alkyl, C1-6 alkoxy or a halogen atom, and most preferably a hydrogen atom, C1-6 alkyl or a halogen atom.

In formula (I), m is preferably an integer of 1 to 3, more preferably an integer of 1 to 2, and most preferably 1.

In formula (I), n is preferably an integer of 1 to 3, more preferably an integer of 1 to 2, and most preferably 1.

In formula (I), $R^5$ is preferably $R^{5-1}$, $R^{5-2}$, $R^{5-3}$, $R^{5-4}$, $R^{5-5}$ or $R^{5-6}$, more preferably $R^{5-1}$, $R^{5-2}$, $R^{5-3}$ or $R^{5-4}$, and most preferably $R^{5-1}$ or $R^{5-3}$.

In formula (I), $R^{5-2}$ is preferably C1-15 alkyl may be substituted with 1-5 of an oxygen atom and/or a sulfur atom or C2-15 alkenyl may be substituted with 1-5 of an oxygen atom and/or a sulfur atom, more preferably C1-15 alkyl may be substituted with 1-5 of an oxygen atom and/or a sulfur atom, and most preferably C5-14 alkylthio, C1-6 alkyl substituted with C1-4 alkylthio, (C1-4 alkylthio)-C1-4 alkoxy, (C1-4 alkoxy)-C1-4 alkylthio, (C1-4 alkylthio)-C1-4

alkylthio, (C1-4 alkoxy)-(C1-2 alkoxy)-C1-4 alkyl, (C1-4 alkylthio)-(C1-2 alkoxy)-C1-4 alkyl, (C1-4 alkoxy)-(C1-2 alkylthio)-C1-4 alkyl, (C1-4 alkylthio)-(C1-2 alkylthio)-C1-4 alkyl, (C1-4 alkoxy)-(C1-2 alkoxy)-C1-4 alkoxy, (C1-4 alkylthio) -(C1-2 alkoxy)-C1-4 alkoxy, (C1-4 alkoxy)-(C1-2 alkylthio)-C1-4 alkoxy, (C1-4 alkylthio)-(C1-2 alkylthio)-C1-4 alkoxy, (C1-4 alkoxy)-(C1-2 alkoxy)-C1-4 alkylthio, (C1-4 alkylthio)-(C1-2 alkoxy)-C1-4 alkylthio, (C1-4 alkoxy)-(C1-2 alkylthio) -C1-4 alkylthio, (CI-4 alkylthio)-(C1-2 alkylthio)-C1-4 alkylthio, (C1-4 alkoxy)-(C1-2 alkoxy)-(C1-2 alkoxy)-C1-4 alkyl, (C1-4 alkylthio)-(C1-2 alkoxy)-(C1-2 alkoxy)-C1-4 alkyl, (C1-4 alkoxy)-(C1-2 alkylthio)-(C1-2 alkoxy)-C1-4 alkyl, (C1-4 alkylthio)-(C1-2 alkylthio)-(C1-2 alkoxy)-C1-4 alkyl, (C1-4 alkoxy)-(C1-2 alkoxy)-(C1-2 alkylthio)-C1-4 alkyl, (C1-4 alkylthio)-(C1-2 alkoxy)-(C1-2 alkylthio)-C1-4 alkyl, (C1-4 alkoxy)-(C1-2 alkylthio)-(C1-2 alkylthio)-C1-4 alkyl or (C1-4 alkylthio)-(C1-2 alkylthio)-(C1-2 alkylthio)-C1-4 alkyl.

In formula (I), $R^{5-3}$ is preferably C1-6 alkyl substituted with C1-6 alkoxy or C1-6 alkoxy substituted with C1-6 alkoxy.

In formula (I), $R^{5-4}$ is preferably C1-15 alkyl which is substituted with one nitrogen atom and may be further substituted with 1 to 4 of a nitrogen atom, an oxygen atom and/or a sulfur atom or C2-15 alkenyl which is substituted with one nitrogen atom and may be further substituted with 1 to 4 of a nitrogen atom, an oxygen atom and/or a sulfur atom, and more preferably C1-15 alkyl which is substituted with one nitrogen atom and may be further substituted with 1 to 4 of a nitrogen atom, an oxygen atom and/or a sulfur atom.

In formula (I), G is preferably $G^1$ or $G^2$, more preferably $G^1$.

In formula (I), $G^1$ is preferably C1-6 alkylene may be substituted with 1 to 2 oxygen atom and/or sulfur atom or C2-6 alkenylene may be substituted with 1 to 2 oxygen atom and/or sulfur atom, and more preferably C1-6 alkylene may be substituted with 1 to 2 oxygen atom and/or sulfur atom.

In formula (I), $G^2$ is preferably C1-6 alkylene which is substituted with one nitrogen atom and may be further substituted with I to 2 of a nitrogen atom, an oxygen atom and/or a sulfur atom, and more preferably C1-6 alkylene which is substituted with one nitrogen atom.

In formula (I), (ring1) is preferably carbocycle-2 and heterocycle-2, more preferably heterocycle-2, and most preferably 3-10 membered mono-, or bicyclic heteroaryl containing 1 to 3 of hetero atom which is selected from an oxygen atom, a nitrogen atom and a sulfur atom, which may be partially or fully saturated.

In formula (I), (ring2) is preferably carbocycle-3 and heterocycle-3, more preferably heterocycle-3, and more preferably 3-10 membered mono-, or bicyclic heteroaryl containing 1 to 3 of hetero atom which is selected from an oxygen atom, a nitrogen atom and a sulfur atom, which may be partially or fully saturated.

In formula (I), A is preferably carbonyl or $-S(O)_p-$, more preferably carbonyl or $-SO_2-$ and most preferably carbonyl.

In formula (I), p is preferably 1 and 2, more preferably 2.

In formula (I), ----- is preferably a double bond.

With regard to the compound represented by formula (I), a preferred compound is a compound represented by formula (I-A-1):

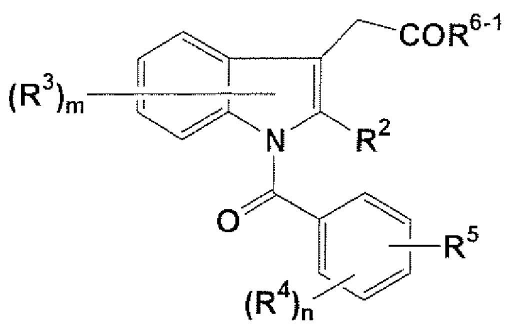

(I-A-1)

(wherein $R^{6-1}$ represents hydroxy or C1-6 alkoxy, and other symbols have the same meanings as described above), a compound represented by formula (I-A-2):

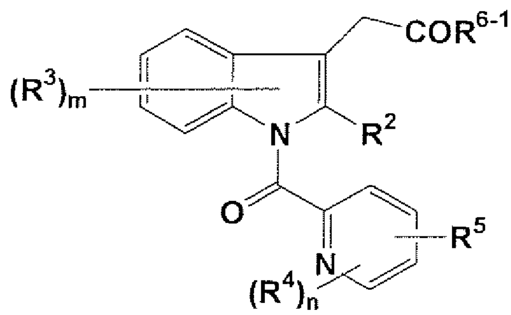

(I-A-2)

(wherein all symbols have the same meanings as described above), a compound represented by formula (I-A-3):

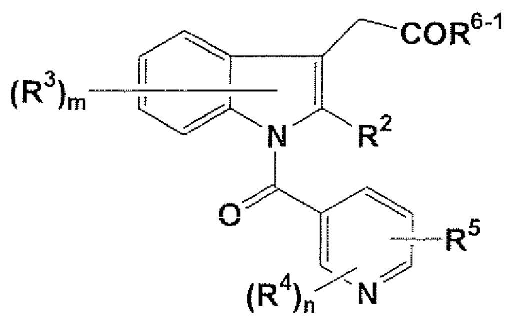

(I-A-3)

(wherein all symbols have the same meanings as described above), a compound represented by formula (I-A-4):

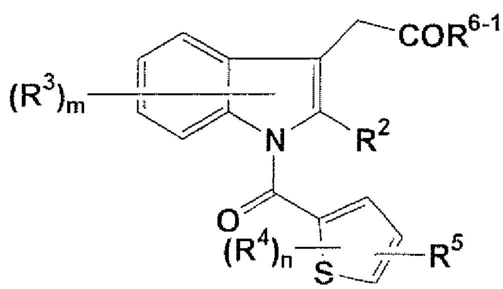

(I-A-4)

(wherein all symbols have the same meanings as described above), a compound represented by formula (I-A-5):

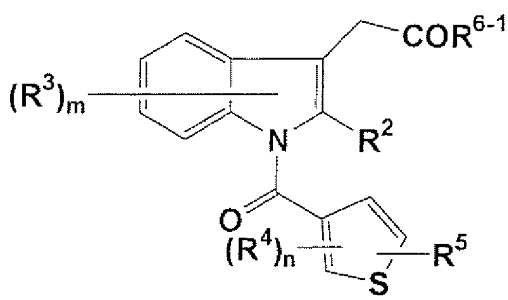

(I-A-5)

(wherein all symbols have the same meanings as described above), a compound represented by formula (I-A-6):

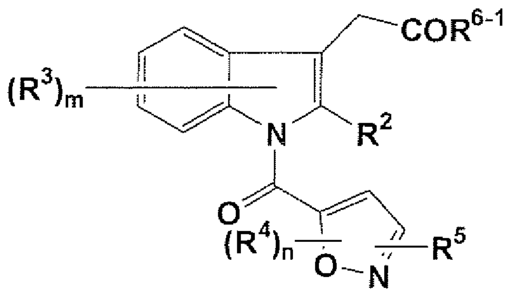

(I-A-6)

(wherein all symbols have the same meanings as described above), a compound represented by formula (I-A-7):

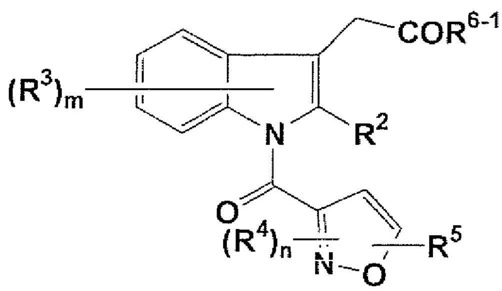

(I-A-7)

(wherein all symbols have the same meanings as described above), a compound represented by formula (I-A-8):

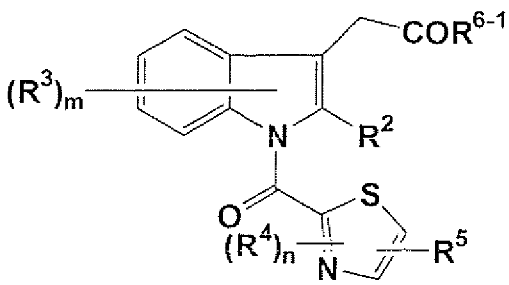

(I-A-8)

(wherein all symbols have the same meanings as described above), a compound represented by formula (I-A-9):

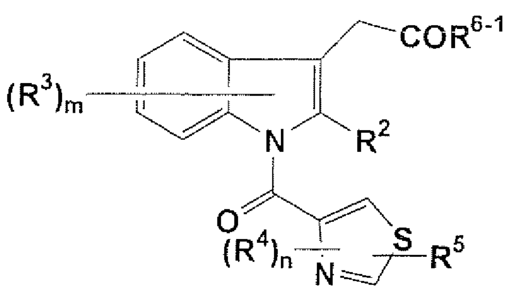

(I-A-9)

(wherein all symbols have the same meanings as described above), a compound represented by formula (I-A-10):

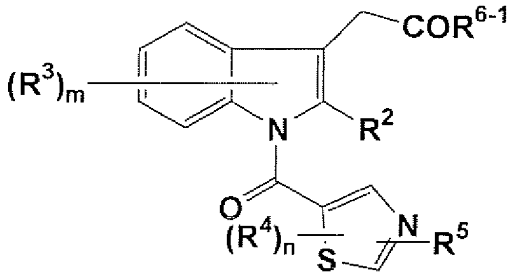

(I-A-10)

(wherein all symbols have the same meanings as described above), a compound represented by formula (I-A-11):

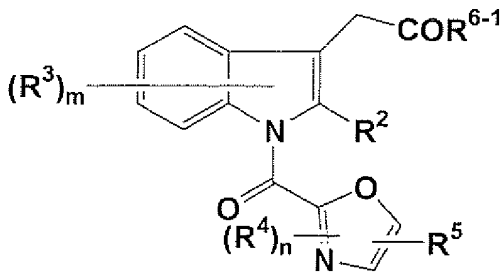

(I-A-11)

(wherein all symbols have the same meanings as described above), a compound represented by formula (I-A-12):

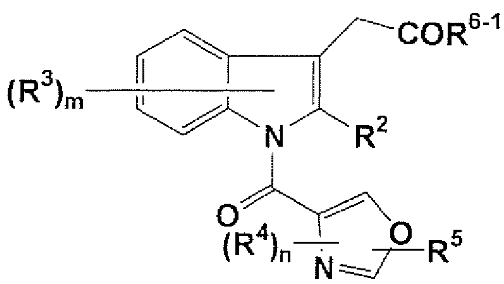

(I-A-12)

(wherein all symbols have the same meanings as described above), a compound represented by formula (I-A-13):

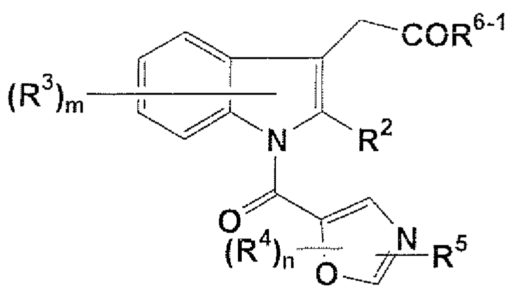

(I-A-13)

(wherein all symbols have the same meanings as described above), a compound represented by formula (I-B-1):

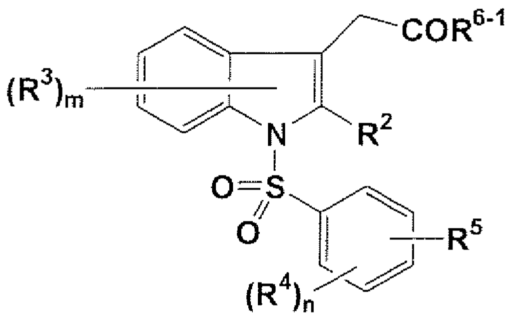

(I－B－1)

(wherein all symbols have the same meanings as described above), a compound represented by formula (I-B-2):

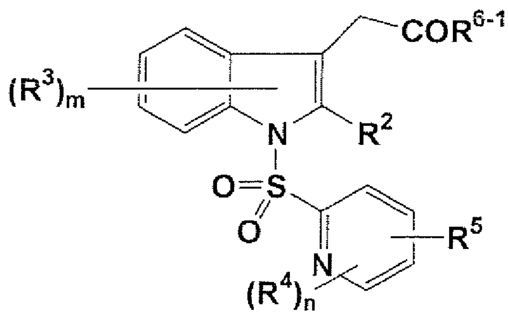

(I－B－2)

(wherein all symbols have the same meanings as described above), a compound represented by formula (I-B-3):

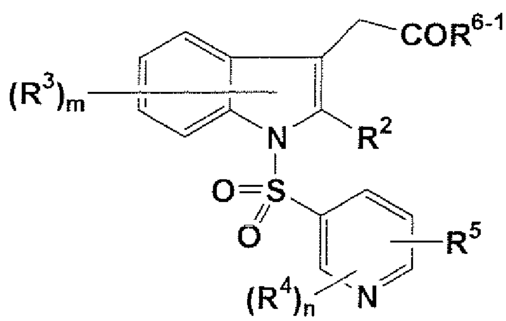

(I－B－3)

(wherein all symbols have the same meanings as described above), a compound represented by formula (I-B-4):

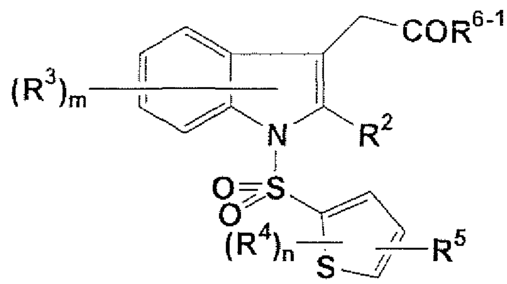

(I－B－4)

(wherein all symbols have the same meanings as described above), a compound represented by formula (I-B-5):

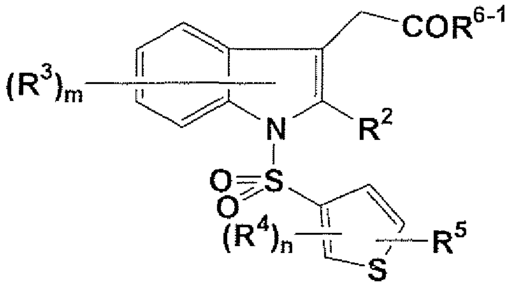

(I-B-5)

(wherein all symbols have the same meanings as described above), a compound represented by formula (I-B-6):

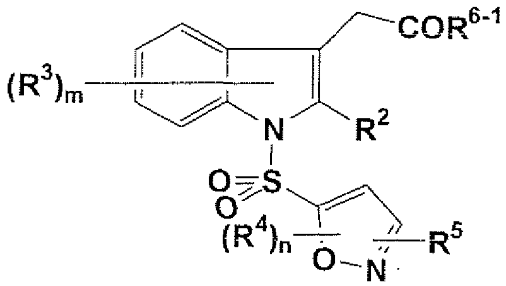

(I-B-6)

(wherein all symbols have the same meanings as described above), a compound represented by formula (I-B-7):

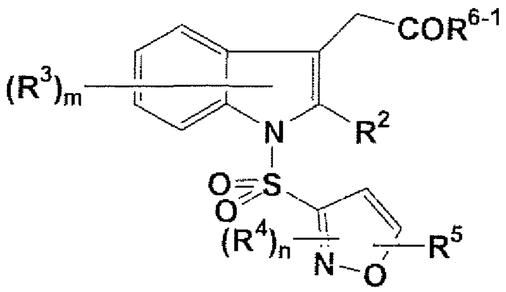

(I-B-7)

(wherein all symbols have the same meanings as described above), a compound represented by formula (I-B-8):

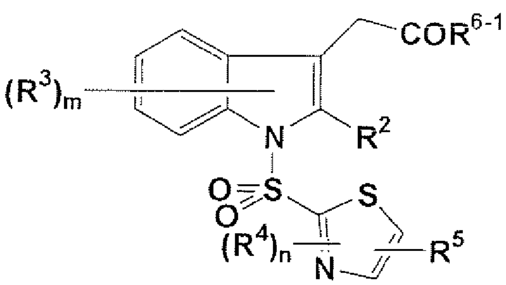

(I-B-8)

(wherein all symbols have the same meanings as described above), a compound represented by formula (I-B-9):

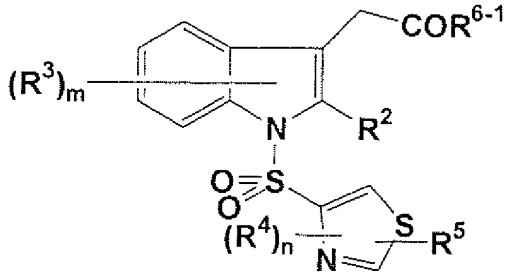

(I-B-9)

(wherein all symbols have the same meanings as described above), a compound represented by formula (I-B-10):

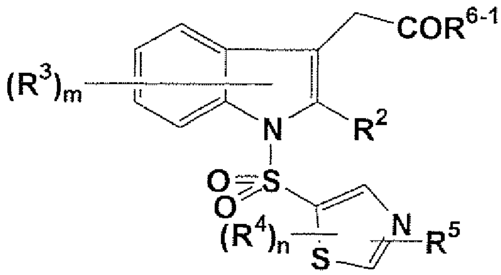

(I-B-10)

(wherein all symbols have the same meanings as described above), a compound represented by formula (I-B-11):

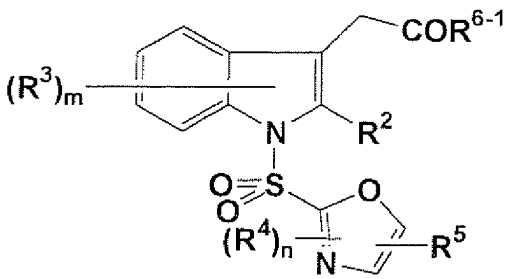

(I-B-11)

(wherein all symbols have the same meanings as described above), a compound represented by formula (I-B-12):

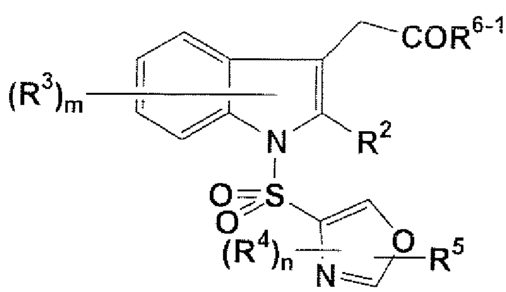

(I-B-12)

(wherein all symbols have the same meanings as described above), or a compound represented by formula (I-B-13):

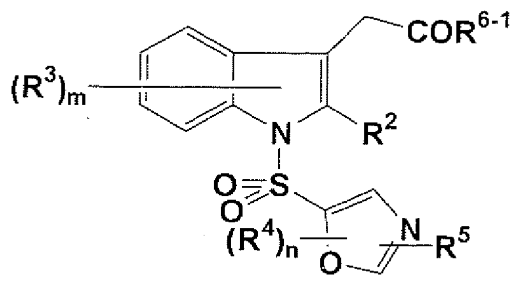

(I－B－13)

(wherein all symbols have the same meanings as described above).

Specific compounds of the present invention are the compounds mentioned in Examples, the compounds mentioned in Table 1 to Table 60, (1-{2-[2-(2-ethoxyethoxy)ethoxy]benzoyl}-5-isopropyl-2-methyl-1H-indol-3-yl)acetic acid, {1-[4-(1,3-benzodioxol-2-ylmethoxy)-2,6-dimethylbenzoyl]-6-ethyl-2-methyl-1H-indol-5-yl}acetic acid, 3-[2-methyl-1-({2-[(1-methyl-1,2,3,4-tetrahydroquinolin-3-yl)methoxy]-1H-indol-5-yl}carbonyl)-1H-indol-4-yl]propanoic acid, (2,5,6-trimethyl-1-{[2-(pyrazin-2-ylmethoxy)-1H-indol-5-yl]carbonyl}-1H-indol-3-yl)acetic acid, {4-fluoro-2-methyl-1-[(5-{2-[2-(propylsulfanyl)ethoxy]ethoxy}-1,3,4-thiadiazol-2-yl)carbonyl]-1H-indol-6-yl}acetic acid, [1-({3,5-dimethyl-4-[3-(1,3-thiazol-2-ylsulfanyl)propoxy]phenyl}sulfinyl)-2,7-dimethyl-1H-indol-5-yl]acetic acid, [1-({2-chloro-4-[(2,3-dihydro-1,4-benzodioxin-2-ylmethyl)sulfanyl]-5-fluorophenyl}sulfonyl)-3-methyl-1H-indol-5-yl]acetic acid, 5-fluoro-2-methyl-1-{[6-(quinolin-3-ylmethoxy)-5,6,7,8-tetrahydronaphthalen-2-yl]carbonyl}-1H-indole-3-carboxylic acid, [1-({4-[2-(6-chloropyridin-2-yl)ethoxy]cyclohexyl}carbonyl)-2-methyl-1H-indol-7-yl]acetic acid, [1-({5-[(6-chloro-2,3-dihydro-1,4-benzodioxin-2-yl)methoxy]-1,3,4-thiadiazol-2-yl}carbonyl)-4-fluoro-2-methyl-1H-indol-6-yl]acetic acid, {1-[6-(1,3-dihydro-2-benzofuran-1-ylmethoxy)-2-naphthoyl]-2-methyl-1H-indol-5-yl}acetic acid, {1-[(5-{2-[(2-ethoxyethyl)(methyl)amino]ethoxy}piperazin-2-yl)carbonyl]-5-fluoro-2-methyl-1H-indol-7-yl}acetic acid, {2-methyl-1-[(5-phenylpyrazin-2-yl)carbonyl]-1H-indol-6-yl}acetic acid, {5-butyl-1-[(5-hydroxy-1,3,4-oxadiazol-2-yl)carbonyl]-2-methyl-1H-indol-7-yl}acetic acid, 3-[1-({4-[2-(isoxazol-3-ylmethoxy)ethoxy]-1,3-thiazol-2-yl}carbonyl)-6-methoxy-2-methyl-1H-indol-4-yl]propanoic acid, {2-ethyl-7-methyl-1-[2,3,5,6-tetramethyl-4-(3-pyrazin-2-ylpropyl)benzoyl]-1H-indol-5-yl} acetic acid, (4-chloro-1-{2,5-difluoro-4-[(tetrahydro-2H-pyran-2-ylmethy)sulfanyl]benzoyl}-1H-indol-5-yl)acetic acid or 1-[(5-hydroxypyridin-3-yl)carbonyl]-2-methyl-1H-indole-3-carboxylic acid, a salt thereof, an N-oxide thereof, a solvate thereof or a prodrug thereof.

With regard to the specific compounds, a preferred compound is the compound mentioned in Examples or the compound mentioned in Table 1 to Table 60, more preferred compound is the compound mentioned in Examples.

In the tables, all symbols have the same meanings as described above.

Table 1

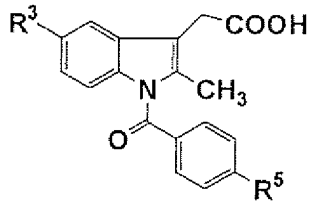

(I-A-1-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 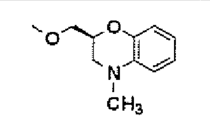 | 51<br>52<br>53<br>54<br>55 | H<br>F<br>Cl<br>$CH_3$<br>$CH_3O$ | 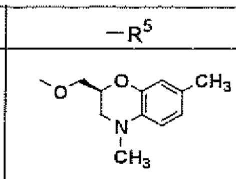 | 101<br>102<br>103<br>104<br>105 | H<br>F<br>Cl<br>$CH_3$<br>$CH_3O$ | 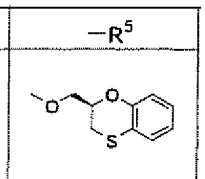 |
| 6<br>7<br>8<br>9<br>10 | H<br>F<br>Cl<br>$CH_3$<br>$CH_3O$ | 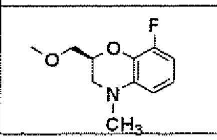 | 56<br>57<br>58<br>59<br>60 | H<br>F<br>Cl<br>$CH_3$<br>$CH_3O$ | 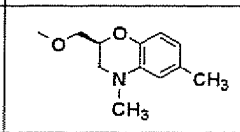 | 106<br>107<br>108<br>109<br>110 | H<br>F<br>Cl<br>$CH_3$<br>$CH_3O$ | 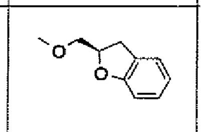 |
| 11<br>12<br>13<br>14<br>15 | H<br>F<br>Cl<br>$CH_3$<br>$CH_3O$ | 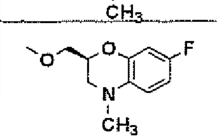 | 61<br>62<br>63<br>64<br>65 | H<br>F<br>Cl<br>$CH_3$<br>$CH_3O$ | 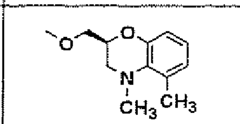 | 111<br>112<br>113<br>114<br>115 | H<br>F<br>Cl<br>$CH_3$<br>$CH_3O$ | 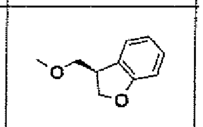 |
| 16<br>17<br>18<br>19<br>20 | H<br>F<br>Cl<br>$CH_3$<br>$CH_3O$ | 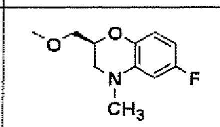 | 66<br>67<br>68<br>69<br>70 | H<br>F<br>Cl<br>$CH_3$<br>$CH_3O$ | 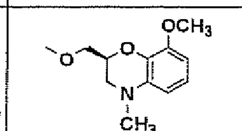 | 116<br>117<br>118<br>119<br>120 | H<br>F<br>Cl<br>$CH_3$<br>$CH_3O$ | 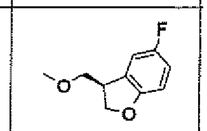 |
| 21<br>22<br>23<br>24<br>25 | H<br>F<br>Cl<br>$CH_3$<br>$CH_3O$ | 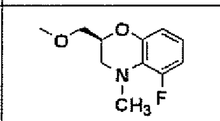 | 71<br>72<br>73<br>74<br>75 | H<br>F<br>Cl<br>$CH_3$<br>$CH_3O$ | 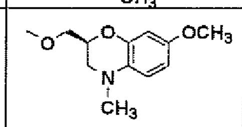 | 121<br>122<br>123<br>124<br>125 | H<br>F<br>Cl<br>$CH_3$<br>$CH_3O$ | 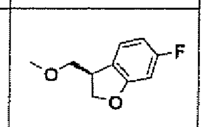 |
| 26<br>27<br>28<br>29<br>30 | H<br>F<br>Cl<br>$CH_3$<br>$CH_3O$ | 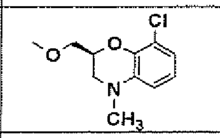 | 76<br>77<br>78<br>79<br>80 | H<br>F<br>Cl<br>$CH_3$<br>$CH_3O$ | 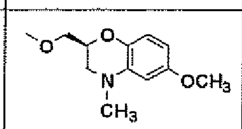 | 126<br>127<br>128<br>129<br>130 | H<br>F<br>Cl<br>$CH_3$<br>$CH_3O$ | 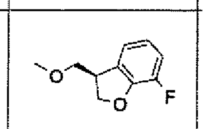 |
| 31<br>32<br>33<br>34<br>35 | H<br>F<br>Cl<br>$CH_3$<br>$CH_3O$ | 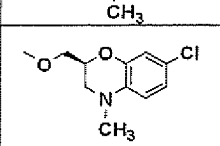 | 81<br>82<br>83<br>84<br>85 | H<br>F<br>Cl<br>$CH_3$<br>$CH_3O$ | 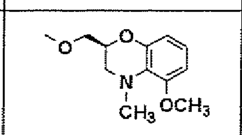 | 131<br>132<br>133<br>134<br>135 | H<br>F<br>Cl<br>$CH_3$<br>$CH_3O$ | 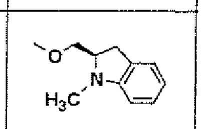 |
| 36<br>37<br>38<br>39<br>40 | H<br>F<br>Cl<br>$CH_3$<br>$CH_3O$ | 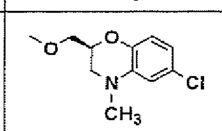 | 86<br>87<br>88<br>89<br>90 | H<br>F<br>Cl<br>$CH_3$<br>$CH_3O$ | 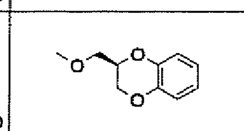 | 136<br>137<br>138<br>139<br>140 | H<br>F<br>Cl<br>$CH_3$<br>$CH_3O$ | 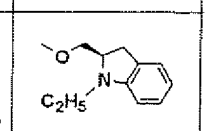 |
| 41<br>42<br>43<br>44<br>45 | H<br>F<br>Cl<br>$CH_3$<br>$CH_3O$ | 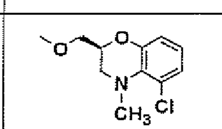 | 91<br>92<br>93<br>94<br>95 | H<br>F<br>Cl<br>$CH_3$<br>$CH_3O$ | 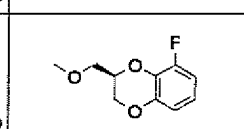 | 141<br>142<br>143<br>144<br>145 | H<br>F<br>Cl<br>$CH_3$<br>$CH_3O$ | 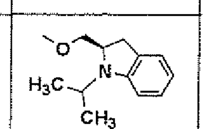 |
| 46<br>47<br>48<br>49<br>50 | H<br>F<br>Cl<br>$CH_3$<br>$CH_3O$ | 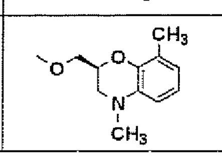 | 96<br>97<br>98<br>99<br>100 | H<br>F<br>Cl<br>$CH_3$<br>$CH_3O$ | 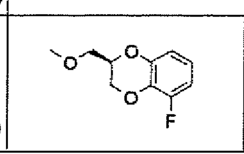 | 146<br>147<br>148<br>149<br>150 | H<br>F<br>Cl<br>$CH_3$<br>$CH_3O$ | 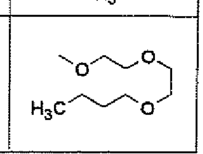 |

Table 2

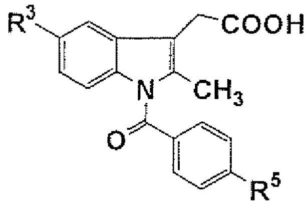

(I-A-1-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 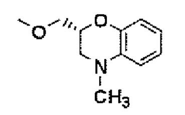 | 51<br>52<br>53<br>54<br>55 | H<br>F<br>Cl<br>$CH_3$<br>$CH_3O$ | 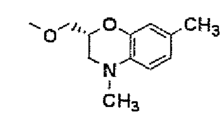 | 101<br>102<br>103<br>104<br>105 | H<br>F<br>Cl<br>$CH_3$<br>$CH_3O$ | 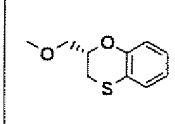 |
| 6<br>7<br>8<br>9<br>10 | H<br>F<br>Cl<br>$CH_3$<br>$CH_3O$ | 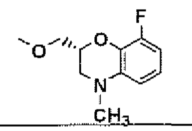 | 56<br>57<br>58<br>59<br>60 | H<br>F<br>Cl<br>$CH_3$<br>$CH_3O$ | 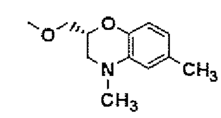 | 106<br>107<br>108<br>109<br>110 | H<br>F<br>Cl<br>$CH_3$<br>$CH_3O$ | 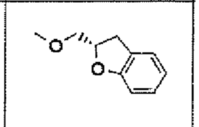 |
| 11<br>12<br>13<br>14<br>15 | H<br>F<br>Cl<br>$CH_3$<br>$CH_3O$ | 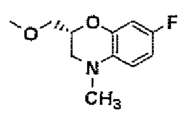 | 61<br>62<br>63<br>64<br>65 | H<br>F<br>Cl<br>$CH_3$<br>$CH_3O$ | 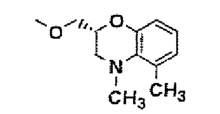 | 111<br>112<br>113<br>114<br>115 | H<br>F<br>Cl<br>$CH_3$<br>$CH_3O$ | 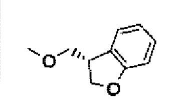 |
| 16<br>17<br>18<br>19<br>20 | H<br>F<br>Cl<br>$CH_3$<br>$CH_3O$ | 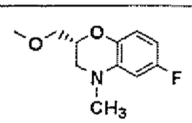 | 66<br>67<br>68<br>69<br>70 | H<br>F<br>Cl<br>$CH_3$<br>$CH_3O$ | 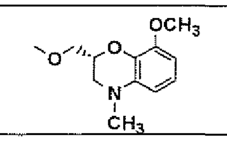 | 116<br>117<br>118<br>119<br>120 | H<br>F<br>Cl<br>$CH_3$<br>$CH_3O$ | 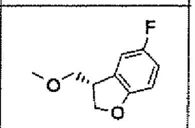 |
| 21<br>22<br>23<br>24<br>25 | H<br>F<br>Cl<br>$CH_3$<br>$CH_3O$ | 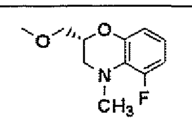 | 71<br>72<br>73<br>74<br>75 | H<br>F<br>Cl<br>$CH_3$<br>$CH_3O$ | 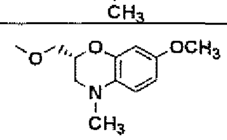 | 121<br>122<br>123<br>124<br>125 | H<br>F<br>Cl<br>$CH_3$<br>$CH_3O$ | 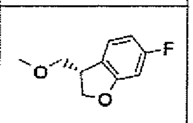 |
| 26<br>27<br>28<br>29<br>30 | H<br>F<br>Cl<br>$CH_3$<br>$CH_3O$ | 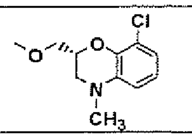 | 76<br>77<br>78<br>79<br>80 | H<br>F<br>Cl<br>$CH_3$<br>$CH_3O$ | 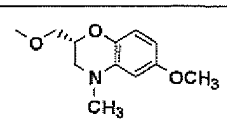 | 126<br>127<br>128<br>129<br>130 | H<br>F<br>Cl<br>$CH_3$<br>$CH_3O$ | 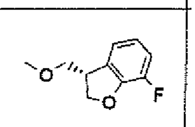 |
| 31<br>32<br>33<br>34<br>35 | H<br>F<br>Cl<br>$CH_3$<br>$CH_3O$ | 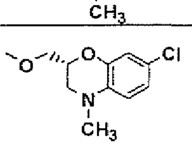 | 81<br>82<br>83<br>84<br>85 | H<br>F<br>Cl<br>$CH_3$<br>$CH_3O$ | 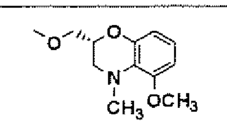 | 131<br>132<br>133<br>134<br>135 | H<br>F<br>Cl<br>$CH_3$<br>$CH_3O$ | 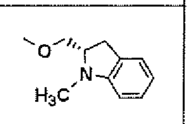 |
| 36<br>37<br>38<br>39<br>40 | H<br>F<br>Cl<br>$CH_3$<br>$CH_3O$ | 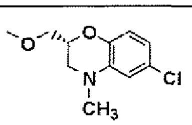 | 86<br>87<br>88<br>89<br>90 | H<br>F<br>Cl<br>$CH_3$<br>$CH_3O$ | 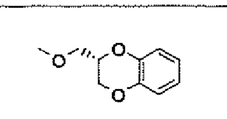 | 136<br>137<br>138<br>139<br>140 | H<br>F<br>Cl<br>$CH_3$<br>$CH_3O$ | 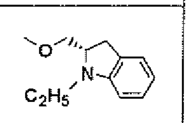 |
| 41<br>42<br>43<br>44<br>45 | H<br>F<br>Cl<br>$CH_3$<br>$CH_3O$ | 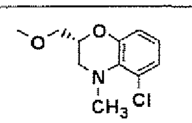 | 91<br>92<br>93<br>94<br>95 | H<br>F<br>Cl<br>$CH_3$<br>$CH_3O$ | 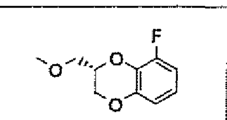 | 141<br>142<br>143<br>144<br>145 | H<br>F<br>Cl<br>$CH_3$<br>$CH_3O$ | 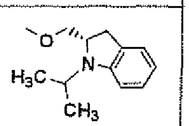 |
| 46<br>47<br>48<br>49<br>50 | H<br>F<br>Cl<br>$CH_3$<br>$CH_3O$ | 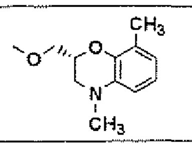 | 96<br>97<br>98<br>99<br>100 | H<br>F<br>Cl<br>$CH_3$<br>$CH_3O$ | 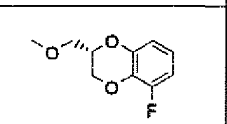 | 146<br>147<br>148<br>149<br>150 | H<br>F<br>Cl<br>$CH_3$<br>$CH_3O$ | 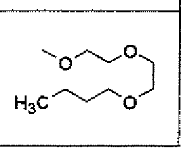 |

Table 3

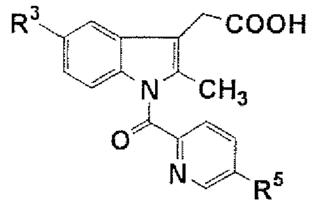

(I-A-2-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 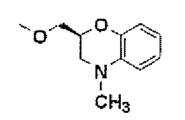 | 51<br>52<br>53<br>54<br>55 | H<br>F<br>Cl<br>$CH_3$<br>$CH_3O$ | 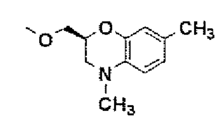 | 101<br>102<br>103<br>104<br>105 | H<br>F<br>Cl<br>$CH_3$<br>$CH_3O$ | 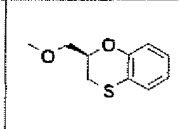 |
| 6<br>7<br>8<br>9<br>10 | H<br>F<br>Cl<br>$CH_3$<br>$CH_3O$ | 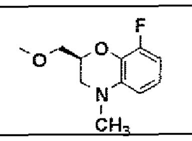 | 56<br>57<br>58<br>59<br>60 | H<br>F<br>Cl<br>$CH_3$<br>$CH_3O$ | 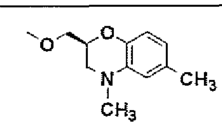 | 106<br>107<br>108<br>109<br>110 | H<br>F<br>Cl<br>$CH_3$<br>$CH_3O$ | 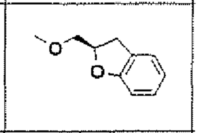 |
| 11<br>12<br>13<br>14<br>15 | H<br>F<br>Cl<br>$CH_3$<br>$CH_3O$ | 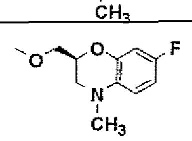 | 61<br>62<br>63<br>64<br>65 | H<br>F<br>Cl<br>$CH_3$<br>$CH_3O$ | 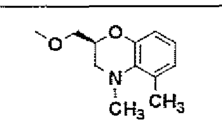 | 111<br>112<br>113<br>114<br>115 | H<br>F<br>Cl<br>$CH_3$<br>$CH_3O$ | 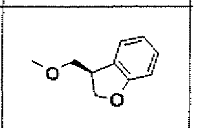 |
| 16<br>17<br>18<br>19<br>20 | H<br>F<br>Cl<br>$CH_3$<br>$CH_3O$ | 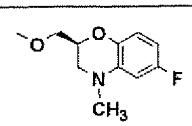 | 66<br>67<br>68<br>69<br>70 | H<br>F<br>Cl<br>$CH_3$<br>$CH_3O$ | 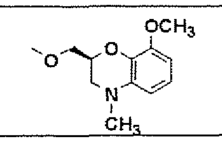 | 116<br>117<br>118<br>119<br>120 | H<br>F<br>Cl<br>$CH_3$<br>$CH_3O$ | 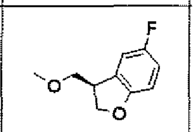 |
| 21<br>22<br>23<br>24<br>25 | H<br>F<br>Cl<br>$CH_3$<br>$CH_3O$ | 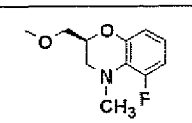 | 71<br>72<br>73<br>74<br>75 | H<br>F<br>Cl<br>$CH_3$<br>$CH_3O$ | 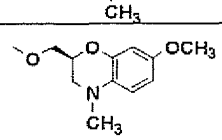 | 121<br>122<br>123<br>124<br>125 | H<br>F<br>Cl<br>$CH_3$<br>$CH_3O$ | 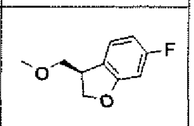 |
| 26<br>27<br>28<br>29<br>30 | H<br>F<br>Cl<br>$CH_3$<br>$CH_3O$ | 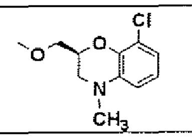 | 76<br>77<br>78<br>79<br>80 | H<br>F<br>Cl<br>$CH_3$<br>$CH_3O$ | 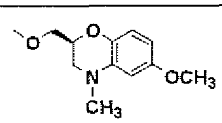 | 126<br>127<br>128<br>129<br>130 | H<br>F<br>Cl<br>$CH_3$<br>$CH_3O$ | 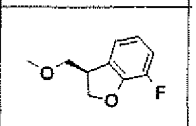 |
| 31<br>32<br>33<br>34<br>35 | H<br>F<br>Cl<br>$CH_3$<br>$CH_3O$ | 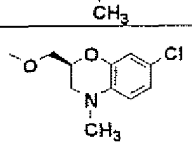 | 81<br>82<br>83<br>84<br>85 | H<br>F<br>Cl<br>$CH_3$<br>$CH_3O$ | 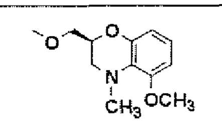 | 131<br>132<br>133<br>134<br>135 | H<br>F<br>Cl<br>$CH_3$<br>$CH_3O$ | 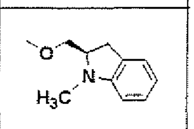 |
| 36<br>37<br>38<br>39<br>40 | H<br>F<br>Cl<br>$CH_3$<br>$CH_3O$ | 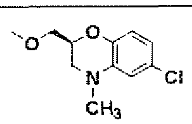 | 86<br>87<br>88<br>89<br>90 | H<br>F<br>Cl<br>$CH_3$<br>$CH_3O$ | 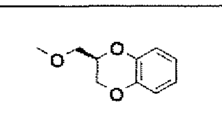 | 136<br>137<br>138<br>139<br>140 | H<br>F<br>Cl<br>$CH_3$<br>$CH_3O$ | 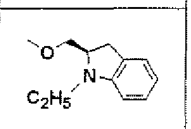 |
| 41<br>42<br>43<br>44<br>45 | H<br>F<br>Cl<br>$CH_3$<br>$CH_3O$ | 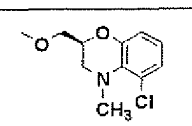 | 91<br>92<br>93<br>94<br>95 | H<br>F<br>Cl<br>$CH_3$<br>$CH_3O$ | 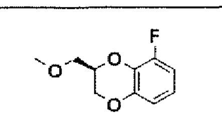 | 141<br>142<br>143<br>144<br>145 | H<br>F<br>Cl<br>$CH_3$<br>$CH_3O$ | 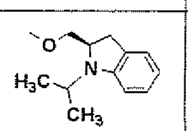 |
| 46<br>47<br>48<br>49<br>50 | H<br>F<br>Cl<br>$CH_3$<br>$CH_3O$ | 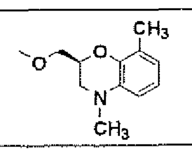 | 96<br>97<br>98<br>99<br>100 | H<br>F<br>Cl<br>$CH_3$<br>$CH_3O$ | 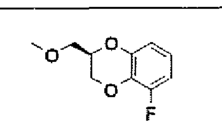 | 146<br>147<br>148<br>149<br>150 | H<br>F<br>Cl<br>$CH_3$<br>$CH_3O$ | 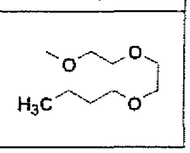 |

Table 4

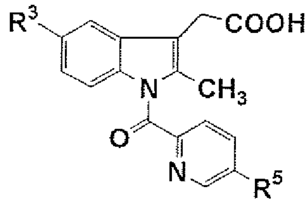

(I-A-2-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 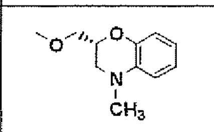 | 51<br>52<br>53<br>54<br>55 | H<br>F<br>Cl<br>$CH_3$<br>$CH_3O$ | 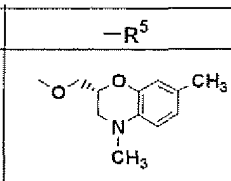 | 101<br>102<br>103<br>104<br>105 | H<br>F<br>Cl<br>$CH_3$<br>$CH_3O$ | 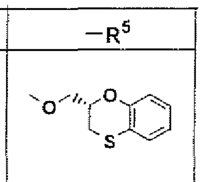 |
| 6<br>7<br>8<br>9<br>10 | H<br>F<br>Cl<br>$CH_3$<br>$CH_3O$ | 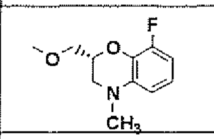 | 56<br>57<br>58<br>59<br>60 | H<br>F<br>Cl<br>$CH_3$<br>$CH_3O$ | 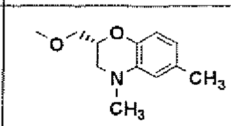 | 106<br>107<br>108<br>109<br>110 | H<br>F<br>Cl<br>$CH_3$<br>$CH_3O$ | 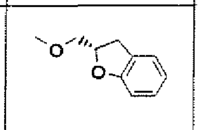 |
| 11<br>12<br>13<br>14<br>15 | H<br>F<br>Cl<br>$CH_3$<br>$CH_3O$ | 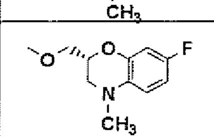 | 61<br>62<br>63<br>64<br>65 | H<br>F<br>Cl<br>$CH_3$<br>$CH_3O$ | 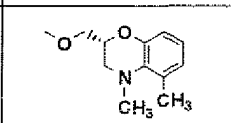 | 111<br>112<br>113<br>114<br>115 | H<br>F<br>Cl<br>$CH_3$<br>$CH_3O$ | 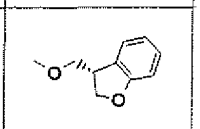 |
| 16<br>17<br>18<br>19<br>20 | H<br>F<br>Cl<br>$CH_3$<br>$CH_3O$ | 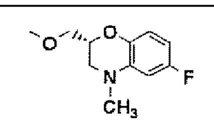 | 66<br>67<br>68<br>69<br>70 | H<br>F<br>Cl<br>$CH_3$<br>$CH_3O$ | 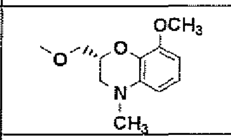 | 116<br>117<br>118<br>119<br>120 | H<br>F<br>Cl<br>$CH_3$<br>$CH_3O$ | 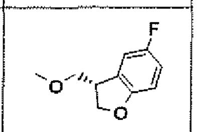 |
| 21<br>22<br>23<br>24<br>25 | H<br>F<br>Cl<br>$CH_3$<br>$CH_3O$ | 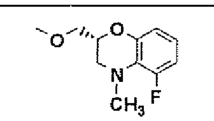 | 71<br>72<br>73<br>74<br>75 | H<br>F<br>Cl<br>$CH_3$<br>$CH_3O$ | 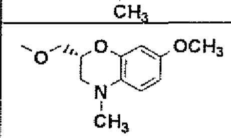 | 121<br>122<br>123<br>124<br>125 | H<br>F<br>Cl<br>$CH_3$<br>$CH_3O$ | 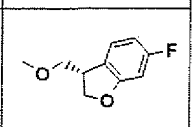 |
| 26<br>27<br>28<br>29<br>30 | H<br>F<br>Cl<br>$CH_3$<br>$CH_3O$ | 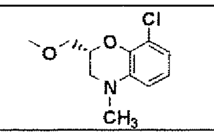 | 76<br>77<br>78<br>79<br>80 | H<br>F<br>Cl<br>$CH_3$<br>$CH_3O$ | 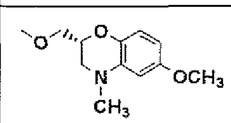 | 126<br>127<br>128<br>129<br>130 | H<br>F<br>Cl<br>$CH_3$<br>$CH_3O$ | 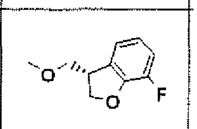 |
| 31<br>32<br>33<br>34<br>35 | H<br>F<br>Cl<br>$CH_3$<br>$CH_3O$ | 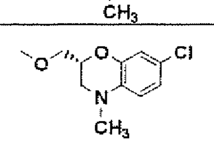 | 81<br>82<br>83<br>84<br>85 | H<br>F<br>Cl<br>$CH_3$<br>$CH_3O$ | 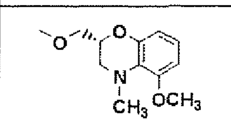 | 131<br>132<br>133<br>134<br>135 | H<br>F<br>Cl<br>$CH_3$<br>$CH_3O$ | 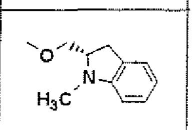 |
| 36<br>37<br>38<br>39<br>40 | H<br>F<br>Cl<br>$CH_3$<br>$CH_3O$ | 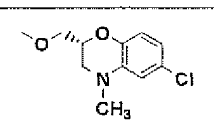 | 86<br>87<br>88<br>89<br>90 | H<br>F<br>Cl<br>$CH_3$<br>$CH_3O$ | 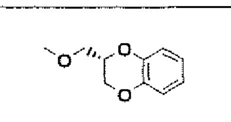 | 136<br>137<br>138<br>139<br>140 | H<br>F<br>Cl<br>$CH_3$<br>$CH_3O$ | 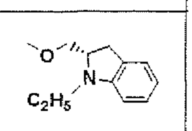 |
| 41<br>42<br>43<br>44<br>45 | H<br>F<br>Cl<br>$CH_3$<br>$CH_3O$ | 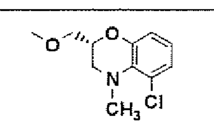 | 91<br>92<br>93<br>94<br>95 | H<br>F<br>Cl<br>$CH_3$<br>$CH_3O$ | 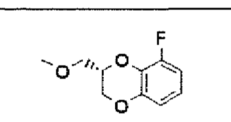 | 141<br>142<br>143<br>144<br>145 | H<br>F<br>Cl<br>$CH_3$<br>$CH_3O$ | 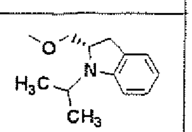 |
| 46<br>47<br>48<br>49<br>50 | H<br>F<br>Cl<br>$CH_3$<br>$CH_3O$ | 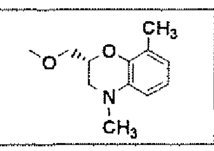 | 96<br>97<br>98<br>99<br>100 | H<br>F<br>Cl<br>$CH_3$<br>$CH_3O$ | 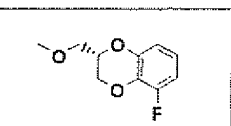 | 146<br>147<br>148<br>149<br>150 | H<br>F<br>Cl<br>$CH_3$<br>$CH_3O$ | 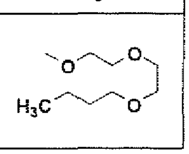 |

Table 5

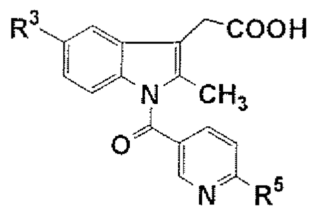

(I-A-3-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 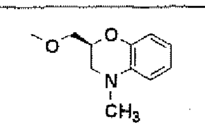 | 51<br>52<br>53<br>54<br>55 | H<br>F<br>Cl<br>$CH_3$<br>$CH_3O$ | 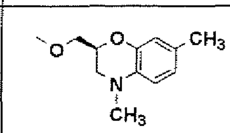 | 101<br>102<br>103<br>104<br>105 | H<br>F<br>Cl<br>$CH_3$<br>$CH_3O$ | 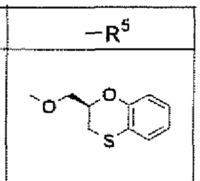 |
| 6<br>7<br>8<br>9<br>10 | H<br>F<br>Cl<br>$CH_3$<br>$CH_3O$ | 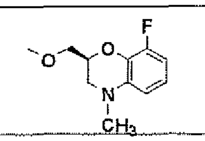 | 56<br>57<br>58<br>59<br>60 | H<br>F<br>Cl<br>$CH_3$<br>$CH_3O$ | 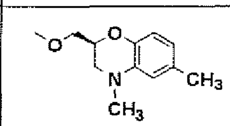 | 106<br>107<br>108<br>109<br>110 | H<br>F<br>Cl<br>$CH_3$<br>$CH_3O$ | 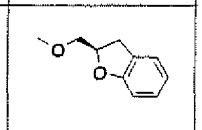 |
| 11<br>12<br>13<br>14<br>15 | H<br>F<br>Cl<br>$CH_3$<br>$CH_3O$ | 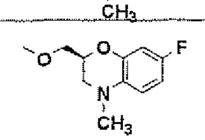 | 61<br>62<br>63<br>64<br>65 | H<br>F<br>Cl<br>$CH_3$<br>$CH_3O$ | 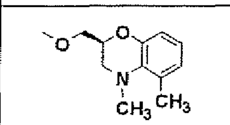 | 111<br>112<br>113<br>114<br>115 | H<br>F<br>Cl<br>$CH_3$<br>$CH_3O$ | 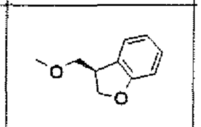 |
| 16<br>17<br>18<br>19<br>20 | H<br>F<br>Cl<br>$CH_3$<br>$CH_3O$ | 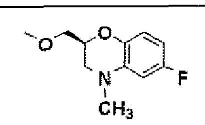 | 66<br>67<br>68<br>69<br>70 | H<br>F<br>Cl<br>$CH_3$<br>$CH_3O$ | 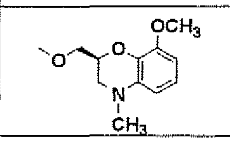 | 116<br>117<br>118<br>119<br>120 | H<br>F<br>Cl<br>$CH_3$<br>$CH_3O$ | 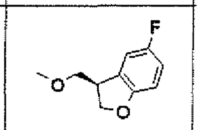 |
| 21<br>22<br>23<br>24<br>25 | H<br>F<br>Cl<br>$CH_3$<br>$CH_3O$ | 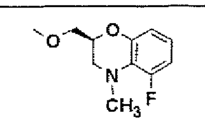 | 71<br>72<br>73<br>74<br>75 | H<br>F<br>Cl<br>$CH_3$<br>$CH_3O$ | 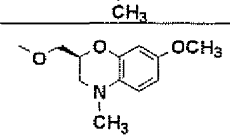 | 121<br>122<br>123<br>124<br>125 | H<br>F<br>Cl<br>$CH_3$<br>$CH_3O$ | 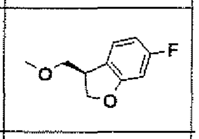 |
| 26<br>27<br>28<br>29<br>30 | H<br>F<br>Cl<br>$CH_3$<br>$CH_3O$ | 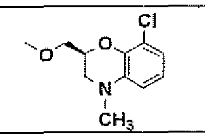 | 76<br>77<br>78<br>79<br>80 | H<br>F<br>Cl<br>$CH_3$<br>$CH_3O$ | 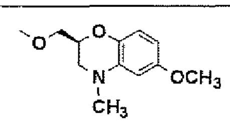 | 126<br>127<br>128<br>129<br>130 | H<br>F<br>Cl<br>$CH_3$<br>$CH_3O$ | 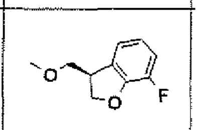 |
| 31<br>32<br>33<br>34<br>35 | H<br>F<br>Cl<br>$CH_3$<br>$CH_3O$ | 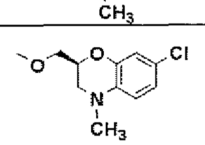 | 81<br>82<br>83<br>84<br>85 | H<br>F<br>Cl<br>$CH_3$<br>$CH_3O$ | 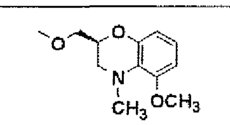 | 131<br>132<br>133<br>134<br>135 | H<br>F<br>Cl<br>$CH_3$<br>$CH_3O$ | 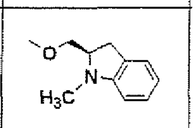 |
| 36<br>37<br>38<br>39<br>40 | H<br>F<br>Cl<br>$CH_3$<br>$CH_3O$ | 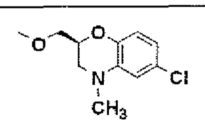 | 86<br>87<br>88<br>89<br>90 | H<br>F<br>Cl<br>$CH_3$<br>$CH_3O$ | 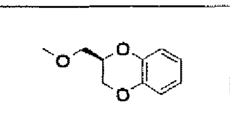 | 136<br>137<br>138<br>139<br>140 | H<br>F<br>Cl<br>$CH_3$<br>$CH_3O$ | 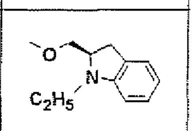 |
| 41<br>42<br>43<br>44<br>45 | H<br>F<br>Cl<br>$CH_3$<br>$CH_3O$ | 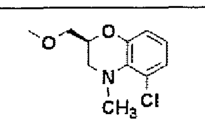 | 91<br>92<br>93<br>94<br>95 | H<br>F<br>Cl<br>$CH_3$<br>$CH_3O$ | 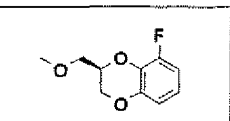 | 141<br>142<br>143<br>144<br>145 | H<br>F<br>Cl<br>$CH_3$<br>$CH_3O$ | 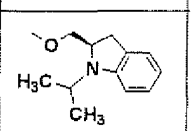 |
| 46<br>47<br>48<br>49<br>50 | H<br>F<br>Cl<br>$CH_3$<br>$CH_3O$ | 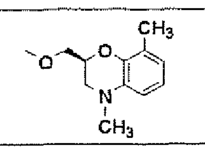 | 96<br>97<br>98<br>99<br>100 | H<br>F<br>Cl<br>$CH_3$<br>$CH_3O$ | 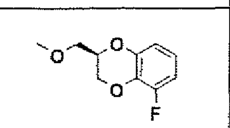 | 146<br>147<br>148<br>149<br>150 | H<br>F<br>Cl<br>$CH_3$<br>$CH_3O$ | 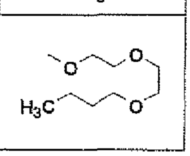 |

Table 6

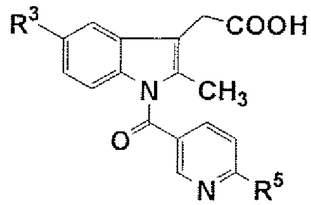

(I-A-3-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 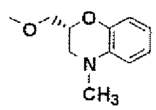 | 51<br>52<br>53<br>54<br>55 | H<br>F<br>Cl<br>$CH_3$<br>$CH_3O$ | 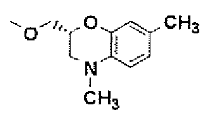 | 101<br>102<br>103<br>104<br>105 | H<br>F<br>Cl<br>$CH_3$<br>$CH_3O$ | 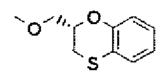 |
| 6<br>7<br>8<br>9<br>10 | H<br>F<br>Cl<br>$CH_3$<br>$CH_3O$ | 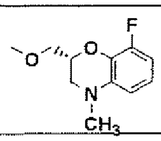 | 56<br>57<br>58<br>59<br>60 | H<br>F<br>Cl<br>$CH_3$<br>$CH_3O$ | 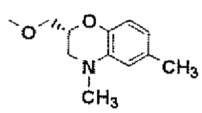 | 106<br>107<br>108<br>109<br>110 | H<br>F<br>Cl<br>$CH_3$<br>$CH_3O$ | 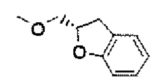 |
| 11<br>12<br>13<br>14<br>15 | H<br>F<br>Cl<br>$CH_3$<br>$CH_3O$ | 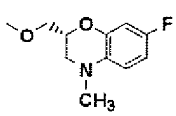 | 61<br>62<br>63<br>64<br>65 | H<br>F<br>Cl<br>$CH_3$<br>$CH_3O$ | 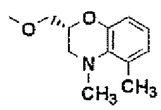 | 111<br>112<br>113<br>114<br>115 | H<br>F<br>Cl<br>$CH_3$<br>$CH_3O$ | 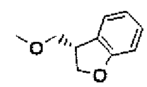 |
| 16<br>17<br>18<br>19<br>20 | H<br>F<br>Cl<br>$CH_3$<br>$CH_3O$ | 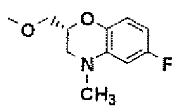 | 66<br>67<br>68<br>69<br>70 | H<br>F<br>Cl<br>$CH_3$<br>$CH_3O$ | 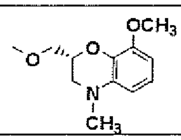 | 116<br>117<br>118<br>119<br>120 | H<br>F<br>Cl<br>$CH_3$<br>$CH_3O$ | 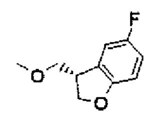 |
| 21<br>22<br>23<br>24<br>25 | H<br>F<br>Cl<br>$CH_3$<br>$CH_3O$ | 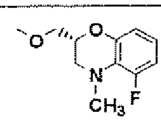 | 71<br>72<br>73<br>74<br>75 | H<br>F<br>Cl<br>$CH_3$<br>$CH_3O$ | 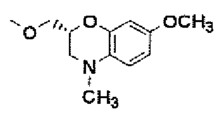 | 121<br>122<br>123<br>124<br>125 | H<br>F<br>Cl<br>$CH_3$<br>$CH_3O$ | 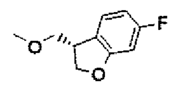 |
| 26<br>27<br>28<br>29<br>30 | H<br>F<br>Cl<br>$CH_3$<br>$CH_3O$ | 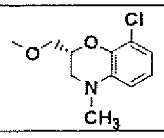 | 76<br>77<br>78<br>79<br>80 | H<br>F<br>Cl<br>$CH_3$<br>$CH_3O$ | 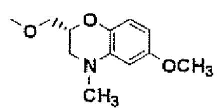 | 126<br>127<br>128<br>129<br>130 | H<br>F<br>Cl<br>$CH_3$<br>$CH_3O$ | 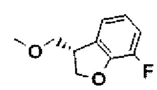 |
| 31<br>32<br>33<br>34<br>35 | H<br>F<br>Cl<br>$CH_3$<br>$CH_3O$ | 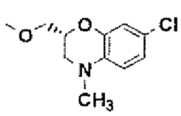 | 81<br>82<br>83<br>84<br>85 | H<br>F<br>Cl<br>$CH_3$<br>$CH_3O$ | 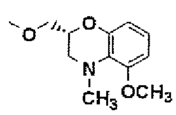 | 131<br>132<br>133<br>134<br>135 | H<br>F<br>Cl<br>$CH_3$<br>$CH_3O$ | 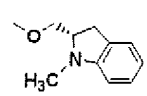 |
| 36<br>37<br>38<br>39<br>40 | H<br>F<br>Cl<br>$CH_3$<br>$CH_3O$ | 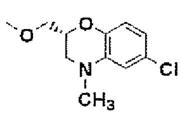 | 86<br>87<br>88<br>89<br>90 | H<br>F<br>Cl<br>$CH_3$<br>$CH_3O$ | 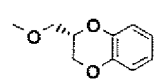 | 136<br>137<br>138<br>139<br>140 | H<br>F<br>Cl<br>$CH_3$<br>$CH_3O$ | 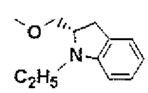 |
| 41<br>42<br>43<br>44<br>45 | H<br>F<br>Cl<br>$CH_3$<br>$CH_3O$ | 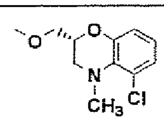 | 91<br>92<br>93<br>94<br>95 | H<br>F<br>Cl<br>$CH_3$<br>$CH_3O$ | 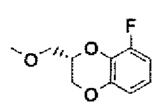 | 141<br>142<br>143<br>144<br>145 | H<br>F<br>Cl<br>$CH_3$<br>$CH_3O$ | 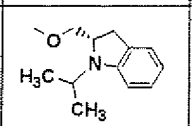 |
| 46<br>47<br>48<br>49<br>50 | H<br>F<br>Cl<br>$CH_3$<br>$CH_3O$ | 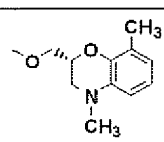 | 96<br>97<br>98<br>99<br>100 | H<br>F<br>Cl<br>$CH_3$<br>$CH_3O$ | 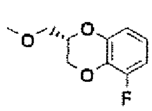 | 146<br>147<br>148<br>149<br>150 | H<br>F<br>Cl<br>$CH_3$<br>$CH_3O$ | 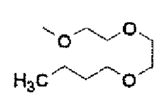 |

Table 7

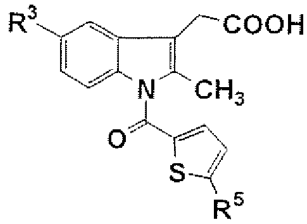

(I－A－4－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 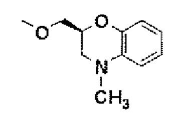 | 51<br>52<br>53<br>54<br>55 | H<br>F<br>Cl<br>$CH_3$<br>$CH_3O$ | 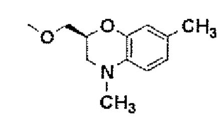 | 101<br>102<br>103<br>104<br>105 | H<br>F<br>Cl<br>$CH_3$<br>$CH_3O$ | 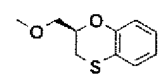 |
| 6<br>7<br>8<br>9<br>10 | H<br>F<br>Cl<br>$CH_3$<br>$CH_3O$ | 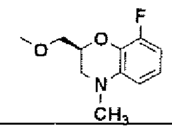 | 56<br>57<br>58<br>59<br>60 | H<br>F<br>Cl<br>$CH_3$<br>$CH_3O$ | 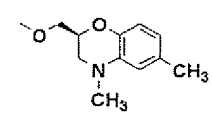 | 106<br>107<br>108<br>109<br>110 | H<br>F<br>Cl<br>$CH_3$<br>$CH_3O$ | 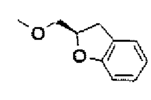 |
| 11<br>12<br>13<br>14<br>15 | H<br>F<br>Cl<br>$CH_3$<br>$CH_3O$ | 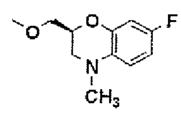 | 61<br>62<br>63<br>64<br>65 | H<br>F<br>Cl<br>$CH_3$<br>$CH_3O$ | 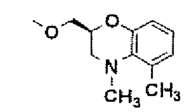 | 111<br>112<br>113<br>114<br>115 | H<br>F<br>Cl<br>$CH_3$<br>$CH_3O$ | 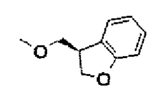 |
| 16<br>17<br>18<br>19<br>20 | H<br>F<br>Cl<br>$CH_3$<br>$CH_3O$ | 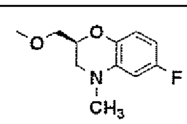 | 66<br>67<br>68<br>69<br>70 | H<br>F<br>Cl<br>$CH_3$<br>$CH_3O$ | 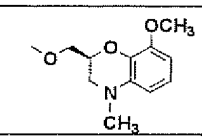 | 116<br>117<br>118<br>119<br>120 | H<br>F<br>Cl<br>$CH_3$<br>$CH_3O$ | 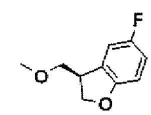 |
| 21<br>22<br>23<br>24<br>25 | H<br>F<br>Cl<br>$CH_3$<br>$CH_3O$ | 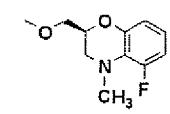 | 71<br>72<br>73<br>74<br>75 | H<br>F<br>Cl<br>$CH_3$<br>$CH_3O$ | 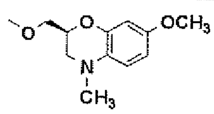 | 121<br>122<br>123<br>124<br>125 | H<br>F<br>Cl<br>$CH_3$<br>$CH_3O$ | 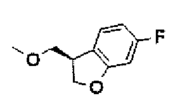 |
| 26<br>27<br>28<br>29<br>30 | H<br>F<br>Cl<br>$CH_3$<br>$CH_3O$ | 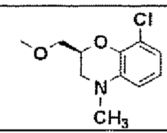 | 76<br>77<br>78<br>79<br>80 | H<br>F<br>Cl<br>$CH_3$<br>$CH_3O$ | 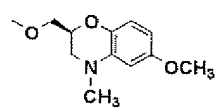 | 126<br>127<br>128<br>129<br>130 | H<br>F<br>Cl<br>$CH_3$<br>$CH_3O$ | 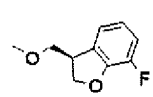 |
| 31<br>32<br>33<br>34<br>35 | H<br>F<br>Cl<br>$CH_3$<br>$CH_3O$ | 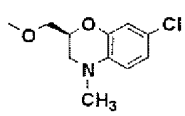 | 81<br>82<br>83<br>84<br>85 | H<br>F<br>Cl<br>$CH_3$<br>$CH_3O$ | 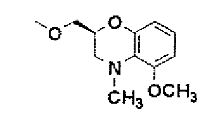 | 131<br>132<br>133<br>134<br>135 | H<br>F<br>Cl<br>$CH_3$<br>$CH_3O$ | 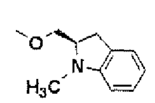 |
| 36<br>37<br>38<br>39<br>40 | H<br>F<br>Cl<br>$CH_3$<br>$CH_3O$ | 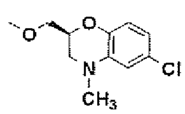 | 86<br>87<br>88<br>89<br>90 | H<br>F<br>Cl<br>$CH_3$<br>$CH_3O$ | 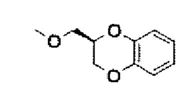 | 136<br>137<br>138<br>139<br>140 | H<br>F<br>Cl<br>$CH_3$<br>$CH_3O$ | 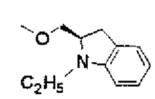 |
| 41<br>42<br>43<br>44<br>45 | H<br>F<br>Cl<br>$CH_3$<br>$CH_3O$ | 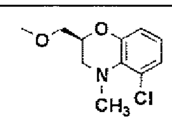 | 91<br>92<br>93<br>94<br>95 | H<br>F<br>Cl<br>$CH_3$<br>$CH_3O$ | 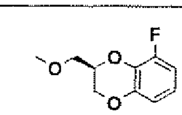 | 141<br>142<br>143<br>144<br>145 | H<br>F<br>Cl<br>$CH_3$<br>$CH_3O$ | 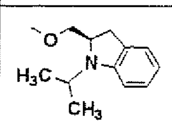 |
| 46<br>47<br>48<br>49<br>50 | H<br>F<br>Cl<br>$CH_3$<br>$CH_3O$ | 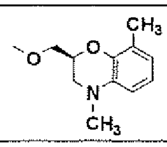 | 96<br>97<br>98<br>99<br>100 | H<br>F<br>Cl<br>$CH_3$<br>$CH_3O$ | 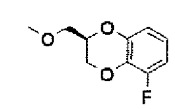 | 146<br>147<br>148<br>149<br>150 | H<br>F<br>Cl<br>$CH_3$<br>$CH_3O$ | 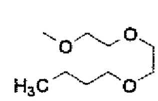 |

Table 8

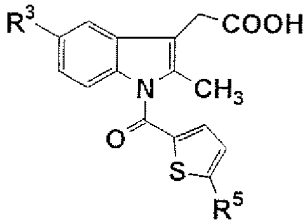

(I－A－4－1)

| No. | $R^3$ | $-R^5$ | No. | $R^3$ | $-R^5$ | No. | $R^3$ | $-R^5$ |
|---|---|---|---|---|---|---|---|---|
| 1 | H | 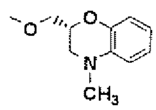 | 51 | H | 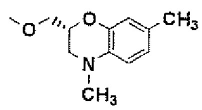 | 101 | H | 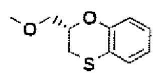 |
| 2 | F | | 52 | F | | 102 | F | |
| 3 | Cl | | 53 | Cl | | 103 | Cl | |
| 4 | $CH_3$ | | 54 | $CH_3$ | | 104 | $CH_3$ | |
| 5 | $CH_3O$ | | 55 | $CH_3O$ | | 105 | $CH_3O$ | |
| 6 | H | 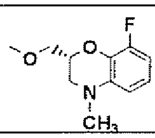 | 56 | H | 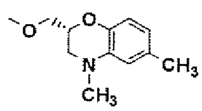 | 106 | H | 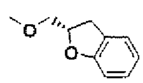 |
| 7 | F | | 57 | F | | 107 | F | |
| 8 | Cl | | 58 | Cl | | 108 | Cl | |
| 9 | $CH_3$ | | 59 | $CH_3$ | | 109 | $CH_3$ | |
| 10 | $CH_3O$ | | 60 | $CH_3O$ | | 110 | $CH_3O$ | |
| 11 | H | 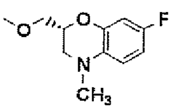 | 61 | H | 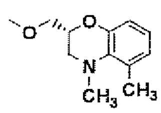 | 111 | H | 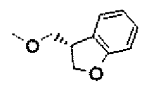 |
| 12 | F | | 62 | F | | 112 | F | |
| 13 | Cl | | 63 | Cl | | 113 | Cl | |
| 14 | $CH_3$ | | 64 | $CH_3$ | | 114 | $CH_3$ | |
| 15 | $CH_3O$ | | 65 | $CH_3O$ | | 115 | $CH_3O$ | |
| 16 | H | 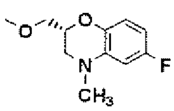 | 66 | H | 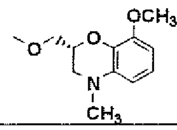 | 116 | H | 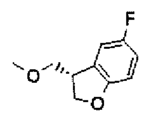 |
| 17 | F | | 67 | F | | 117 | F | |
| 18 | Cl | | 68 | Cl | | 118 | Cl | |
| 19 | $CH_3$ | | 69 | $CH_3$ | | 119 | $CH_3$ | |
| 20 | $CH_3O$ | | 70 | $CH_3O$ | | 120 | $CH_3O$ | |
| 21 | H | 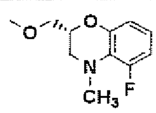 | 71 | H | 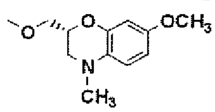 | 121 | H | 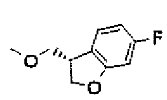 |
| 22 | F | | 72 | F | | 122 | F | |
| 23 | Cl | | 73 | Cl | | 123 | Cl | |
| 24 | $CH_3$ | | 74 | $CH_3$ | | 124 | $CH_3$ | |
| 25 | $CH_3O$ | | 75 | $CH_3O$ | | 125 | $CH_3O$ | |
| 26 | H | 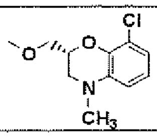 | 76 | H | 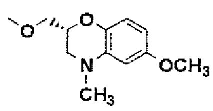 | 126 | H | 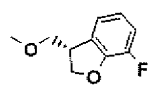 |
| 27 | F | | 77 | F | | 127 | F | |
| 28 | Cl | | 78 | Cl | | 128 | Cl | |
| 29 | $CH_3$ | | 79 | $CH_3$ | | 129 | $CH_3$ | |
| 30 | $CH_3O$ | | 80 | $CH_3O$ | | 130 | $CH_3O$ | |
| 31 | H | 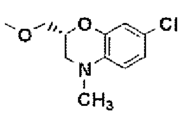 | 81 | H | 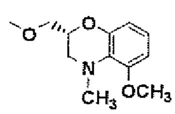 | 131 | H | 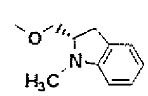 |
| 32 | F | | 82 | F | | 132 | F | |
| 33 | Cl | | 83 | Cl | | 133 | Cl | |
| 34 | $CH_3$ | | 84 | $CH_3$ | | 134 | $CH_3$ | |
| 35 | $CH_3O$ | | 85 | $CH_3O$ | | 135 | $CH_3O$ | |
| 36 | H | 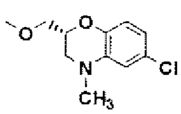 | 86 | H | 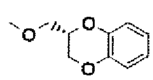 | 136 | H | 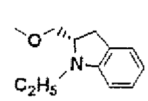 |
| 37 | F | | 87 | F | | 137 | F | |
| 38 | Cl | | 88 | Cl | | 138 | Cl | |
| 39 | $CH_3$ | | 89 | $CH_3$ | | 139 | $CH_3$ | |
| 40 | $CH_3O$ | | 90 | $CH_3O$ | | 140 | $CH_3O$ | |
| 41 | H | 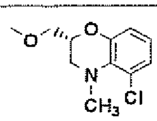 | 91 | H | 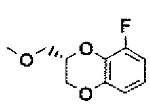 | 141 | H | 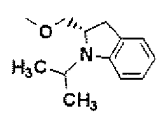 |
| 42 | F | | 92 | F | | 142 | F | |
| 43 | Cl | | 93 | Cl | | 143 | Cl | |
| 44 | $CH_3$ | | 94 | $CH_3$ | | 144 | $CH_3$ | |
| 45 | $CH_3O$ | | 95 | $CH_3O$ | | 145 | $CH_3O$ | |
| 46 | H | 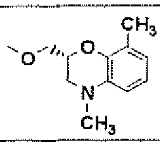 | 96 | H | 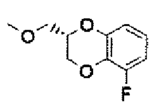 | 146 | H | 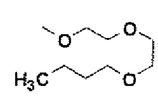 |
| 47 | F | | 97 | F | | 147 | F | |
| 48 | Cl | | 98 | Cl | | 148 | Cl | |
| 49 | $CH_3$ | | 99 | $CH_3$ | | 149 | $CH_3$ | |
| 50 | $CH_3O$ | | 100 | $CH_3O$ | | 150 | $CH_3O$ | |

Table 9

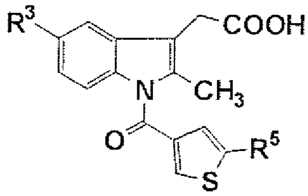

(I-A-5-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 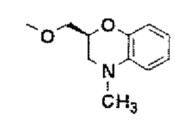 | 51<br>52<br>53<br>54<br>55 | H<br>F<br>Cl<br>$CH_3$<br>$CH_3O$ | 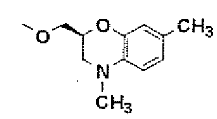 | 101<br>102<br>103<br>104<br>105 | H<br>F<br>Cl<br>$CH_3$<br>$CH_3O$ | 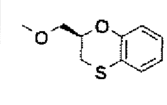 |
| 6<br>7<br>8<br>9<br>10 | H<br>F<br>Cl<br>$CH_3$<br>$CH_3O$ | 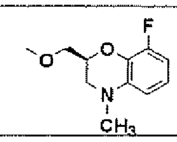 | 56<br>57<br>58<br>59<br>60 | H<br>F<br>Cl<br>$CH_3$<br>$CH_3O$ | 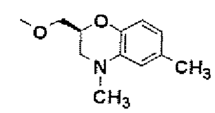 | 106<br>107<br>108<br>109<br>110 | H<br>F<br>Cl<br>$CH_3$<br>$CH_3O$ | 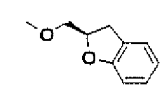 |
| 11<br>12<br>13<br>14<br>15 | H<br>F<br>Cl<br>$CH_3$<br>$CH_3O$ | 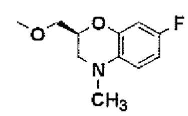 | 61<br>62<br>63<br>64<br>65 | H<br>F<br>Cl<br>$CH_3$<br>$CH_3O$ | 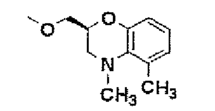 | 111<br>112<br>113<br>114<br>115 | H<br>F<br>Cl<br>$CH_3$<br>$CH_3O$ | 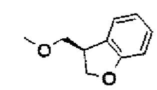 |
| 16<br>17<br>18<br>19<br>20 | H<br>F<br>Cl<br>$CH_3$<br>$CH_3O$ | 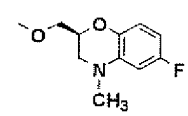 | 66<br>67<br>68<br>69<br>70 | H<br>F<br>Cl<br>$CH_3$<br>$CH_3O$ | 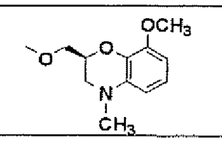 | 116<br>117<br>118<br>119<br>120 | H<br>F<br>Cl<br>$CH_3$<br>$CH_3O$ | 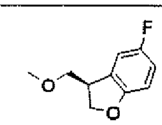 |
| 21<br>22<br>23<br>24<br>25 | H<br>F<br>Cl<br>$CH_3$<br>$CH_3O$ | 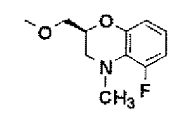 | 71<br>72<br>73<br>74<br>75 | H<br>F<br>Cl<br>$CH_3$<br>$CH_3O$ | 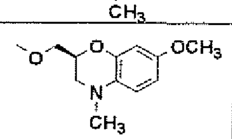 | 121<br>122<br>123<br>124<br>125 | H<br>F<br>Cl<br>$CH_3$<br>$CH_3O$ | 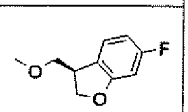 |
| 26<br>27<br>28<br>29<br>30 | H<br>F<br>Cl<br>$CH_3$<br>$CH_3O$ | 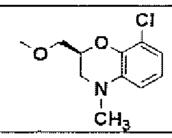 | 76<br>77<br>78<br>79<br>80 | H<br>F<br>Cl<br>$CH_3$<br>$CH_3O$ | 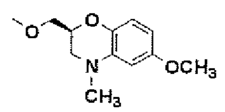 | 126<br>127<br>128<br>129<br>130 | H<br>F<br>Cl<br>$CH_3$<br>$CH_3O$ | 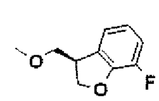 |
| 31<br>32<br>33<br>34<br>35 | H<br>F<br>Cl<br>$CH_3$<br>$CH_3O$ | 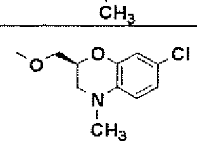 | 81<br>82<br>83<br>84<br>85 | H<br>F<br>Cl<br>$CH_3$<br>$CH_3O$ | 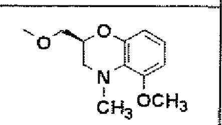 | 131<br>132<br>133<br>134<br>135 | H<br>F<br>Cl<br>$CH_3$<br>$CH_3O$ | 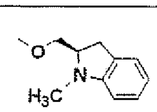 |
| 36<br>37<br>38<br>39<br>40 | H<br>F<br>Cl<br>$CH_3$<br>$CH_3O$ | 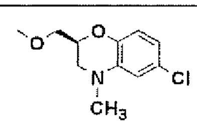 | 86<br>87<br>88<br>89<br>90 | H<br>F<br>Cl<br>$CH_3$<br>$CH_3O$ | 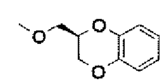 | 136<br>137<br>138<br>139<br>140 | H<br>F<br>Cl<br>$CH_3$<br>$CH_3O$ | 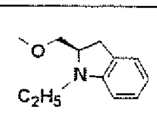 |
| 41<br>42<br>43<br>44<br>45 | H<br>F<br>Cl<br>$CH_3$<br>$CH_3O$ | 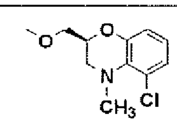 | 91<br>92<br>93<br>94<br>95 | H<br>F<br>Cl<br>$CH_3$<br>$CH_3O$ | 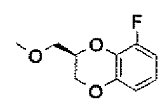 | 141<br>142<br>143<br>144<br>145 | H<br>F<br>Cl<br>$CH_3$<br>$CH_3O$ | 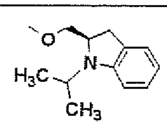 |
| 46<br>47<br>48<br>49<br>50 | H<br>F<br>Cl<br>$CH_3$<br>$CH_3O$ | 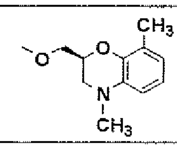 | 96<br>97<br>98<br>99<br>100 | H<br>F<br>Cl<br>$CH_3$<br>$CH_3O$ | 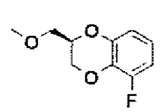 | 146<br>147<br>148<br>149<br>150 | H<br>F<br>Cl<br>$CH_3$<br>$CH_3O$ | 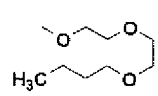 |

Table 10

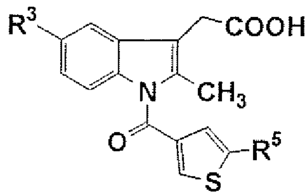

(I－A－5－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 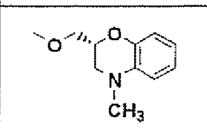 | 51<br>52<br>53<br>54<br>55 | H<br>F<br>Cl<br>$CH_3$<br>$CH_3O$ | 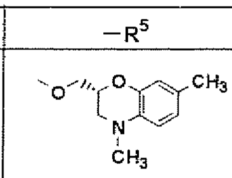 | 101<br>102<br>103<br>104<br>105 | H<br>F<br>Cl<br>$CH_3$<br>$CH_3O$ | 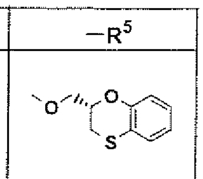 |
| 6<br>7<br>8<br>9<br>10 | H<br>F<br>Cl<br>$CH_3$<br>$CH_3O$ | 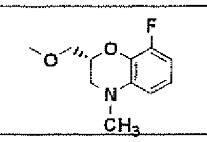 | 56<br>57<br>58<br>59<br>60 | H<br>F<br>Cl<br>$CH_3$<br>$CH_3O$ | 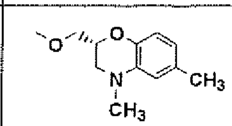 | 106<br>107<br>108<br>109<br>110 | H<br>F<br>Cl<br>$CH_3$<br>$CH_3O$ | 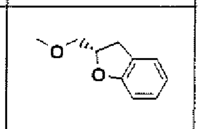 |
| 11<br>12<br>13<br>14<br>15 | H<br>F<br>Cl<br>$CH_3$<br>$CH_3O$ | 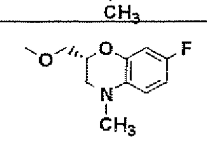 | 61<br>62<br>63<br>64<br>65 | H<br>F<br>Cl<br>$CH_3$<br>$CH_3O$ | 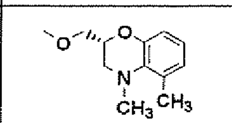 | 111<br>112<br>113<br>114<br>115 | H<br>F<br>Cl<br>$CH_3$<br>$CH_3O$ | 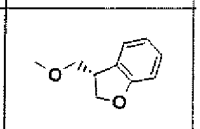 |
| 16<br>17<br>18<br>19<br>20 | H<br>F<br>Cl<br>$CH_3$<br>$CH_3O$ | 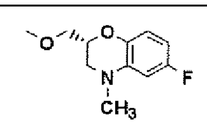 | 66<br>67<br>68<br>69<br>70 | H<br>F<br>Cl<br>$CH_3$<br>$CH_3O$ | 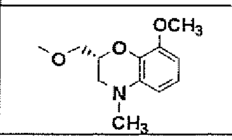 | 116<br>117<br>118<br>119<br>120 | H<br>F<br>Cl<br>$CH_3$<br>$CH_3O$ | 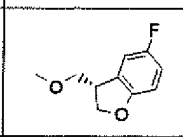 |
| 21<br>22<br>23<br>24<br>25 | H<br>F<br>Cl<br>$CH_3$<br>$CH_3O$ | 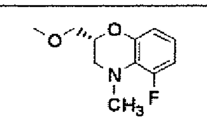 | 71<br>72<br>73<br>74<br>75 | H<br>F<br>Cl<br>$CH_3$<br>$CH_3O$ | 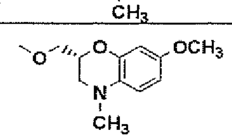 | 121<br>122<br>123<br>124<br>125 | H<br>F<br>Cl<br>$CH_3$<br>$CH_3O$ | 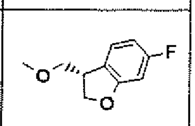 |
| 26<br>27<br>28<br>29<br>30 | H<br>F<br>Cl<br>$CH_3$<br>$CH_3O$ | 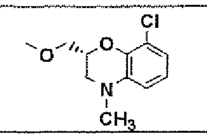 | 76<br>77<br>78<br>79<br>80 | H<br>F<br>Cl<br>$CH_3$<br>$CH_3O$ | 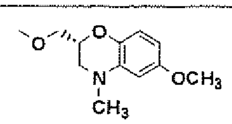 | 126<br>127<br>128<br>129<br>130 | H<br>F<br>Cl<br>$CH_3$<br>$CH_3O$ | 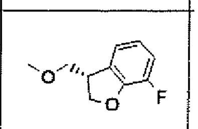 |
| 31<br>32<br>33<br>34<br>35 | H<br>F<br>Cl<br>$CH_3$<br>$CH_3O$ | 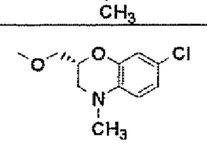 | 81<br>82<br>83<br>84<br>85 | H<br>F<br>Cl<br>$CH_3$<br>$CH_3O$ | 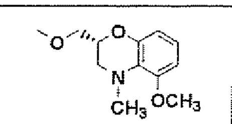 | 131<br>132<br>133<br>134<br>135 | H<br>F<br>Cl<br>$CH_3$<br>$CH_3O$ | 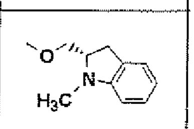 |
| 36<br>37<br>38<br>39<br>40 | H<br>F<br>Cl<br>$CH_3$<br>$CH_3O$ | 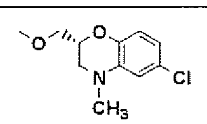 | 86<br>87<br>88<br>89<br>90 | H<br>F<br>Cl<br>$CH_3$<br>$CH_3O$ | 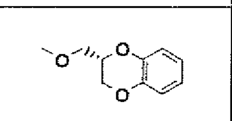 | 136<br>137<br>138<br>139<br>140 | H<br>F<br>Cl<br>$CH_3$<br>$CH_3O$ | 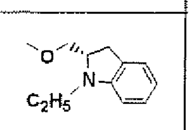 |
| 41<br>42<br>43<br>44<br>45 | H<br>F<br>Cl<br>$CH_3$<br>$CH_3O$ | 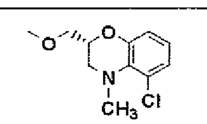 | 91<br>92<br>93<br>94<br>95 | H<br>F<br>Cl<br>$CH_3$<br>$CH_3O$ | 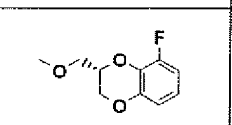 | 141<br>142<br>143<br>144<br>145 | H<br>F<br>Cl<br>$CH_3$<br>$CH_3O$ | 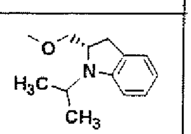 |
| 46<br>47<br>48<br>49<br>50 | H<br>F<br>Cl<br>$CH_3$<br>$CH_3O$ | 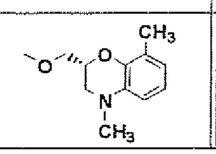 | 96<br>97<br>98<br>99<br>100 | H<br>F<br>Cl<br>$CH_3$<br>$CH_3O$ | 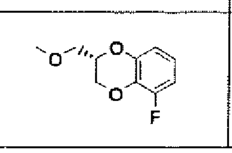 | 146<br>147<br>148<br>149<br>150 | H<br>F<br>Cl<br>$CH_3$<br>$CH_3O$ | 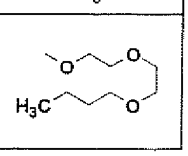 |

Table 11

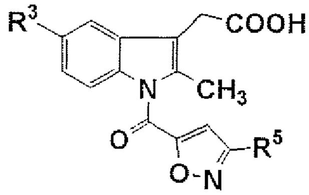

(I-A-6-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 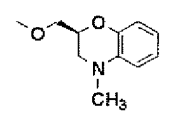 | 51<br>52<br>53<br>54<br>55 | H<br>F<br>Cl<br>$CH_3$<br>$CH_3O$ | 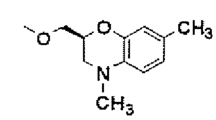 | 101<br>102<br>103<br>104<br>105 | H<br>F<br>Cl<br>$CH_3$<br>$CH_3O$ | 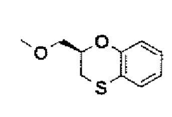 |
| 6<br>7<br>8<br>9<br>10 | H<br>F<br>Cl<br>$CH_3$<br>$CH_3O$ | 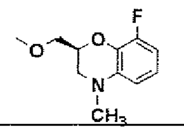 | 56<br>57<br>58<br>59<br>60 | H<br>F<br>Cl<br>$CH_3$<br>$CH_3O$ | 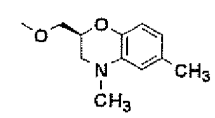 | 106<br>107<br>108<br>109<br>110 | H<br>F<br>Cl<br>$CH_3$<br>$CH_3O$ | 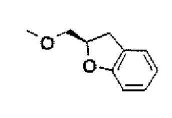 |
| 11<br>12<br>13<br>14<br>15 | H<br>F<br>Cl<br>$CH_3$<br>$CH_3O$ | 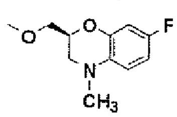 | 61<br>62<br>63<br>64<br>65 | H<br>F<br>Cl<br>$CH_3$<br>$CH_3O$ | 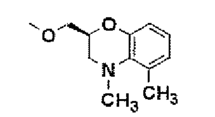 | 111<br>112<br>113<br>114<br>115 | H<br>F<br>Cl<br>$CH_3$<br>$CH_3O$ | 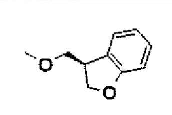 |
| 16<br>17<br>18<br>19<br>20 | H<br>F<br>Cl<br>$CH_3$<br>$CH_3O$ | 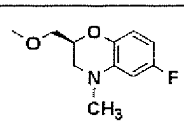 | 66<br>67<br>68<br>69<br>70 | H<br>F<br>Cl<br>$CH_3$<br>$CH_3O$ | 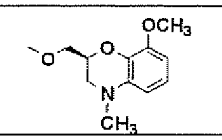 | 116<br>117<br>118<br>119<br>120 | H<br>F<br>Cl<br>$CH_3$<br>$CH_3O$ | 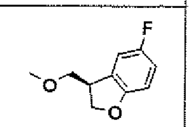 |
| 21<br>22<br>23<br>24<br>25 | H<br>F<br>Cl<br>$CH_3$<br>$CH_3O$ | 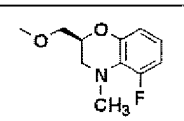 | 71<br>72<br>73<br>74<br>75 | H<br>F<br>Cl<br>$CH_3$<br>$CH_3O$ | 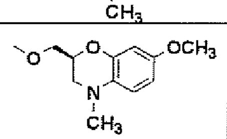 | 121<br>122<br>123<br>124<br>125 | H<br>F<br>Cl<br>$CH_3$<br>$CH_3O$ | 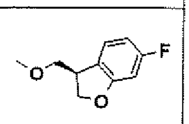 |
| 26<br>27<br>28<br>29<br>30 | H<br>F<br>Cl<br>$CH_3$<br>$CH_3O$ | 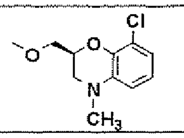 | 76<br>77<br>78<br>79<br>80 | H<br>F<br>Cl<br>$CH_3$<br>$CH_3O$ | 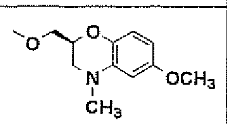 | 126<br>127<br>128<br>129<br>130 | H<br>F<br>Cl<br>$CH_3$<br>$CH_3O$ | 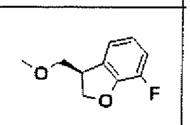 |
| 31<br>32<br>33<br>34<br>35 | H<br>F<br>Cl<br>$CH_3$<br>$CH_3O$ | 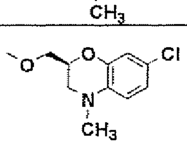 | 81<br>82<br>83<br>84<br>85 | H<br>F<br>Cl<br>$CH_3$<br>$CH_3O$ | 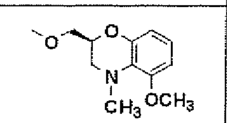 | 131<br>132<br>133<br>134<br>135 | H<br>F<br>Cl<br>$CH_3$<br>$CH_3O$ | 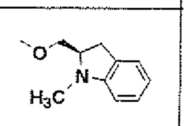 |
| 36<br>37<br>38<br>39<br>40 | H<br>F<br>Cl<br>$CH_3$<br>$CH_3O$ | 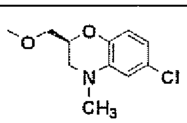 | 86<br>87<br>88<br>89<br>90 | H<br>F<br>Cl<br>$CH_3$<br>$CH_3O$ | 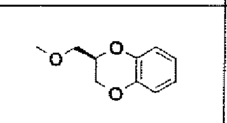 | 136<br>137<br>138<br>139<br>140 | H<br>F<br>Cl<br>$CH_3$<br>$CH_3O$ | 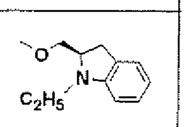 |
| 41<br>42<br>43<br>44<br>45 | H<br>F<br>Cl<br>$CH_3$<br>$CH_3O$ | 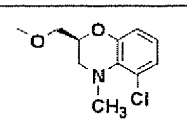 | 91<br>92<br>93<br>94<br>95 | H<br>F<br>Cl<br>$CH_3$<br>$CH_3O$ | 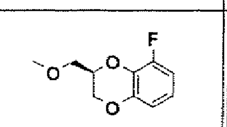 | 141<br>142<br>143<br>144<br>145 | H<br>F<br>Cl<br>$CH_3$<br>$CH_3O$ | 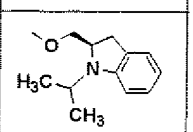 |
| 46<br>47<br>48<br>49<br>50 | H<br>F<br>Cl<br>$CH_3$<br>$CH_3O$ | 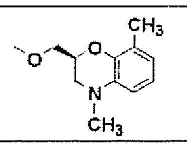 | 96<br>97<br>98<br>99<br>100 | H<br>F<br>Cl<br>$CH_3$<br>$CH_3O$ | 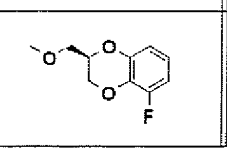 | 146<br>147<br>148<br>149<br>150 | H<br>F<br>Cl<br>$CH_3$<br>$CH_3O$ | 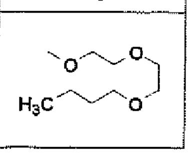 |

Table 12

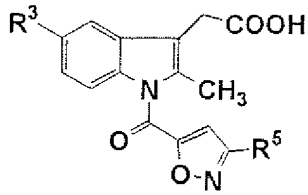

(I-A-6-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 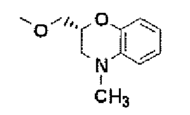 | 51<br>52<br>53<br>54<br>55 | H<br>F<br>Cl<br>$CH_3$<br>$CH_3O$ | 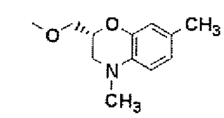 | 101<br>102<br>103<br>104<br>105 | H<br>F<br>Cl<br>$CH_3$<br>$CH_3O$ | 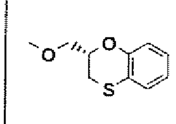 |
| 6<br>7<br>8<br>9<br>10 | H<br>F<br>Cl<br>$CH_3$<br>$CH_3O$ | 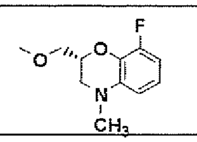 | 56<br>57<br>58<br>59<br>60 | H<br>F<br>Cl<br>$CH_3$<br>$CH_3O$ | 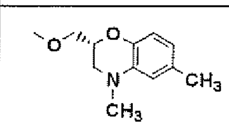 | 106<br>107<br>108<br>109<br>110 | H<br>F<br>Cl<br>$CH_3$<br>$CH_3O$ | 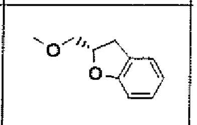 |
| 11<br>12<br>13<br>14<br>15 | H<br>F<br>Cl<br>$CH_3$<br>$CH_3O$ | 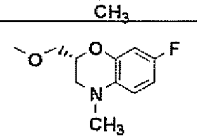 | 61<br>62<br>63<br>64<br>65 | H<br>F<br>Cl<br>$CH_3$<br>$CH_3O$ | 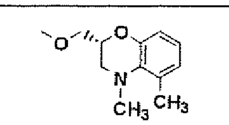 | 111<br>112<br>113<br>114<br>115 | H<br>F<br>Cl<br>$CH_3$<br>$CH_3O$ | 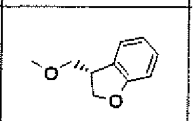 |
| 16<br>17<br>18<br>19<br>20 | H<br>F<br>Cl<br>$CH_3$<br>$CH_3O$ | 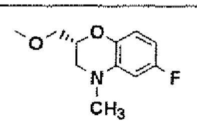 | 66<br>67<br>68<br>69<br>70 | H<br>F<br>Cl<br>$CH_3$<br>$CH_3O$ | 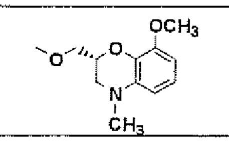 | 116<br>117<br>118<br>119<br>120 | H<br>F<br>Cl<br>$CH_3$<br>$CH_3O$ | 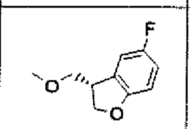 |
| 21<br>22<br>23<br>24<br>25 | H<br>F<br>Cl<br>$CH_3$<br>$CH_3O$ | 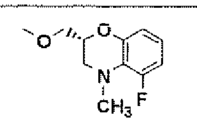 | 71<br>72<br>73<br>74<br>75 | H<br>F<br>Cl<br>$CH_3$<br>$CH_3O$ | 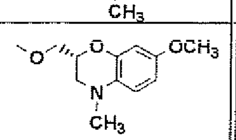 | 121<br>122<br>123<br>124<br>125 | H<br>F<br>Cl<br>$CH_3$<br>$CH_3O$ | 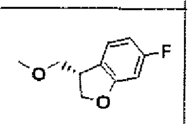 |
| 26<br>27<br>28<br>29<br>30 | H<br>F<br>Cl<br>$CH_3$<br>$CH_3O$ | 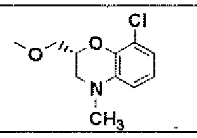 | 76<br>77<br>78<br>79<br>80 | H<br>F<br>Cl<br>$CH_3$<br>$CH_3O$ | 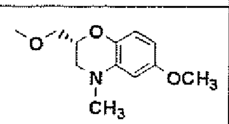 | 126<br>127<br>128<br>129<br>130 | H<br>F<br>Cl<br>$CH_3$<br>$CH_3O$ | 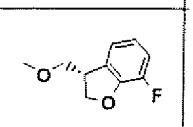 |
| 31<br>32<br>33<br>34<br>35 | H<br>F<br>Cl<br>$CH_3$<br>$CH_3O$ | 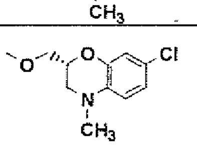 | 81<br>82<br>83<br>84<br>85 | H<br>F<br>Cl<br>$CH_3$<br>$CH_3O$ | 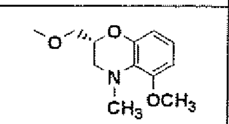 | 131<br>132<br>133<br>134<br>135 | H<br>F<br>Cl<br>$CH_3$<br>$CH_3O$ | 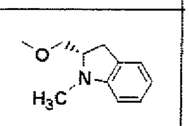 |
| 36<br>37<br>38<br>39<br>40 | H<br>F<br>Cl<br>$CH_3$<br>$CH_3O$ | 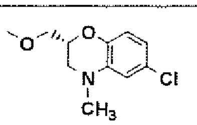 | 86<br>87<br>88<br>89<br>90 | H<br>F<br>Cl<br>$CH_3$<br>$CH_3O$ | 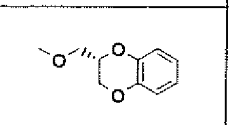 | 136<br>137<br>138<br>139<br>140 | H<br>F<br>Cl<br>$CH_3$<br>$CH_3O$ | 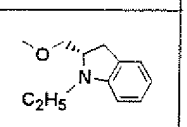 |
| 41<br>42<br>43<br>44<br>45 | H<br>F<br>Cl<br>$CH_3$<br>$CH_3O$ | 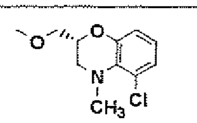 | 91<br>92<br>93<br>94<br>95 | H<br>F<br>Cl<br>$CH_3$<br>$CH_3O$ | 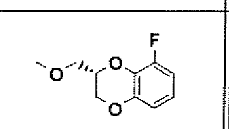 | 141<br>142<br>143<br>144<br>145 | H<br>F<br>Cl<br>$CH_3$<br>$CH_3O$ | 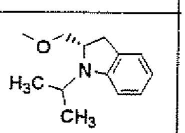 |
| 46<br>47<br>48<br>49<br>50 | H<br>F<br>Cl<br>$CH_3$<br>$CH_3O$ | 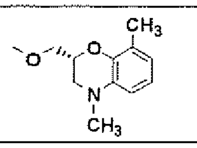 | 96<br>97<br>98<br>99<br>100 | H<br>F<br>Cl<br>$CH_3$<br>$CH_3O$ | 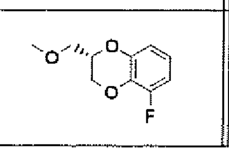 | 146<br>147<br>148<br>149<br>150 | H<br>F<br>Cl<br>$CH_3$<br>$CH_3O$ | 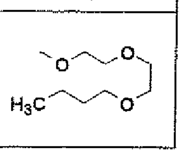 |

Table 13

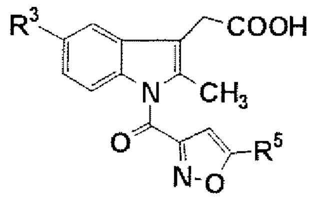

(1-A-7-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 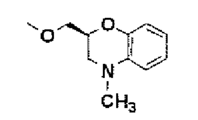 | 51<br>52<br>53<br>54<br>55 | H<br>F<br>Cl<br>$CH_3$<br>$CH_3O$ | 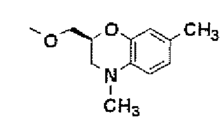 | 101<br>102<br>103<br>104<br>105 | H<br>F<br>Cl<br>$CH_3$<br>$CH_3O$ | 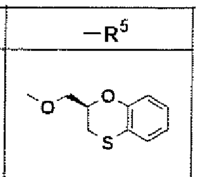 |
| 6<br>7<br>8<br>9<br>10 | H<br>F<br>Cl<br>$CH_3$<br>$CH_3O$ | 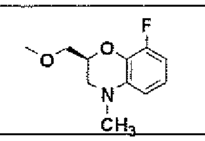 | 56<br>57<br>58<br>59<br>60 | H<br>F<br>Cl<br>$CH_3$<br>$CH_3O$ | 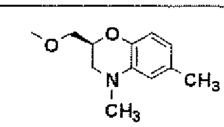 | 106<br>107<br>108<br>109<br>110 | H<br>F<br>Cl<br>$CH_3$<br>$CH_3O$ | 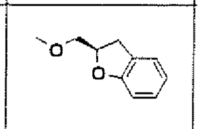 |
| 11<br>12<br>13<br>14<br>15 | H<br>F<br>Cl<br>$CH_3$<br>$CH_3O$ | 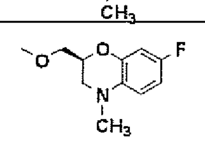 | 61<br>62<br>63<br>64<br>65 | H<br>F<br>Cl<br>$CH_3$<br>$CH_3O$ | 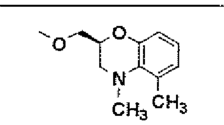 | 111<br>112<br>113<br>114<br>115 | H<br>F<br>Cl<br>$CH_3$<br>$CH_3O$ | 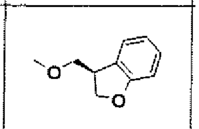 |
| 16<br>17<br>18<br>19<br>20 | H<br>F<br>Cl<br>$CH_3$<br>$CH_3O$ | 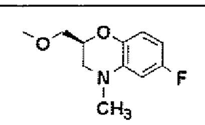 | 66<br>67<br>68<br>69<br>70 | H<br>F<br>Cl<br>$CH_3$<br>$CH_3O$ | 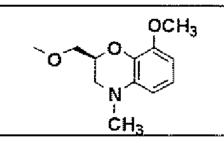 | 116<br>117<br>118<br>119<br>120 | H<br>F<br>Cl<br>$CH_3$<br>$CH_3O$ | 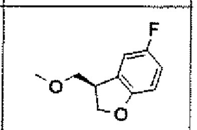 |
| 21<br>22<br>23<br>24<br>25 | H<br>F<br>Cl<br>$CH_3$<br>$CH_3O$ | 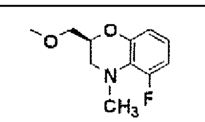 | 71<br>72<br>73<br>74<br>75 | H<br>F<br>Cl<br>$CH_3$<br>$CH_3O$ | 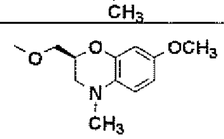 | 121<br>122<br>123<br>124<br>125 | H<br>F<br>Cl<br>$CH_3$<br>$CH_3O$ | 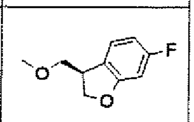 |
| 26<br>27<br>28<br>29<br>30 | H<br>F<br>Cl<br>$CH_3$<br>$CH_3O$ | 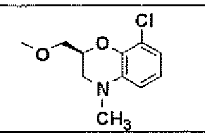 | 76<br>77<br>78<br>79<br>80 | H<br>F<br>Cl<br>$CH_3$<br>$CH_3O$ | 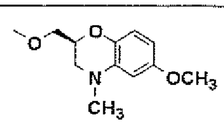 | 126<br>127<br>128<br>129<br>130 | H<br>F<br>Cl<br>$CH_3$<br>$CH_3O$ | 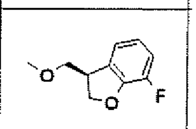 |
| 31<br>32<br>33<br>34<br>35 | H<br>F<br>Cl<br>$CH_3$<br>$CH_3O$ | 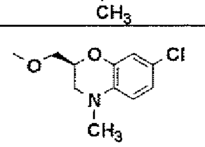 | 81<br>82<br>83<br>84<br>85 | H<br>F<br>Cl<br>$CH_3$<br>$CH_3O$ | 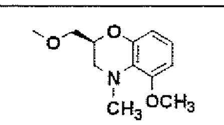 | 131<br>132<br>133<br>134<br>135 | H<br>F<br>Cl<br>$CH_3$<br>$CH_3O$ | 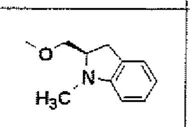 |
| 36<br>37<br>38<br>39<br>40 | H<br>F<br>Cl<br>$CH_3$<br>$CH_3O$ | 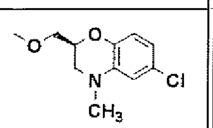 | 86<br>87<br>88<br>89<br>90 | H<br>F<br>Cl<br>$CH_3$<br>$CH_3O$ | 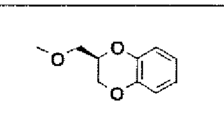 | 136<br>137<br>138<br>139<br>140 | H<br>F<br>Cl<br>$CH_3$<br>$CH_3O$ | 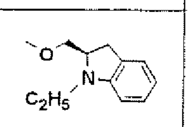 |
| 41<br>42<br>43<br>44<br>45 | H<br>F<br>Cl<br>$CH_3$<br>$CH_3O$ | 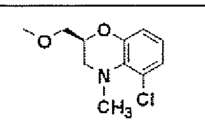 | 91<br>92<br>93<br>94<br>95 | H<br>F<br>Cl<br>$CH_3$<br>$CH_3O$ | 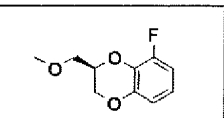 | 141<br>142<br>143<br>144<br>145 | H<br>F<br>Cl<br>$CH_3$<br>$CH_3O$ | 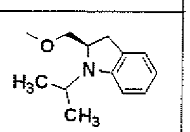 |
| 46<br>47<br>48<br>49<br>50 | H<br>F<br>Cl<br>$CH_3$<br>$CH_3O$ | 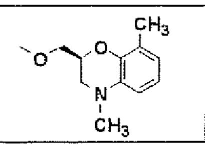 | 96<br>97<br>98<br>99<br>100 | H<br>F<br>Cl<br>$CH_3$<br>$CH_3O$ | 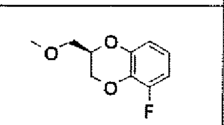 | 146<br>147<br>148<br>149<br>150 | H<br>F<br>Cl<br>$CH_3$<br>$CH_3O$ | 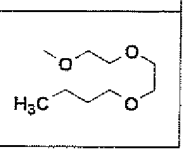 |

Table 14

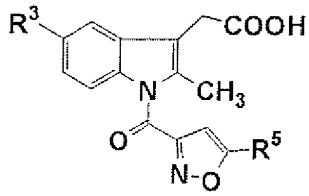

(I－A－7－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 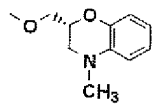 | 51<br>52<br>53<br>54<br>55 | H<br>F<br>Cl<br>$CH_3$<br>$CH_3O$ | 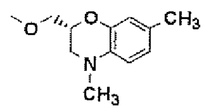 | 101<br>102<br>103<br>104<br>105 | H<br>F<br>Cl<br>$CH_3$<br>$CH_3O$ | 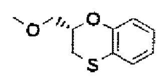 |
| 6<br>7<br>8<br>9<br>10 | H<br>F<br>Cl<br>$CH_3$<br>$CH_3O$ | 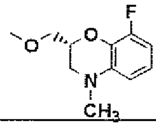 | 56<br>57<br>58<br>59<br>60 | H<br>F<br>Cl<br>$CH_3$<br>$CH_3O$ | 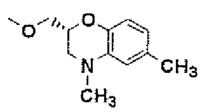 | 106<br>107<br>108<br>109<br>110 | H<br>F<br>Cl<br>$CH_3$<br>$CH_3O$ | 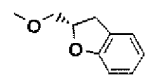 |
| 11<br>12<br>13<br>14<br>15 | H<br>F<br>Cl<br>$CH_3$<br>$CH_3O$ | 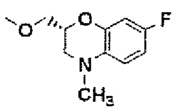 | 61<br>62<br>63<br>64<br>65 | H<br>F<br>Cl<br>$CH_3$<br>$CH_3O$ | 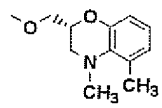 | 111<br>112<br>113<br>114<br>115 | H<br>F<br>Cl<br>$CH_3$<br>$CH_3O$ | 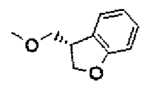 |
| 16<br>17<br>18<br>19<br>20 | H<br>F<br>Cl<br>$CH_3$<br>$CH_3O$ | 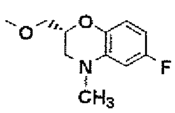 | 66<br>67<br>68<br>69<br>70 | H<br>F<br>Cl<br>$CH_3$<br>$CH_3O$ | 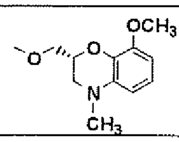 | 116<br>117<br>118<br>119<br>120 | H<br>F<br>Cl<br>$CH_3$<br>$CH_3O$ | 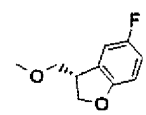 |
| 21<br>22<br>23<br>24<br>25 | H<br>F<br>Cl<br>$CH_3$<br>$CH_3O$ | 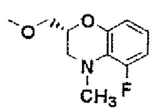 | 71<br>72<br>73<br>74<br>75 | H<br>F<br>Cl<br>$CH_3$<br>$CH_3O$ | 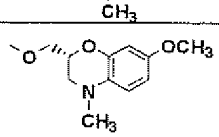 | 121<br>122<br>123<br>124<br>125 | H<br>F<br>Cl<br>$CH_3$<br>$CH_3O$ | 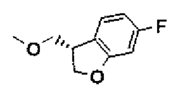 |
| 26<br>27<br>28<br>29<br>30 | H<br>F<br>Cl<br>$CH_3$<br>$CH_3O$ | 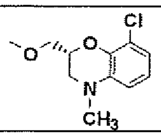 | 76<br>77<br>78<br>79<br>80 | H<br>F<br>Cl<br>$CH_3$<br>$CH_3O$ | 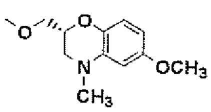 | 126<br>127<br>128<br>129<br>130 | H<br>F<br>Cl<br>$CH_3$<br>$CH_3O$ | 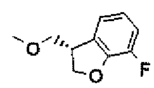 |
| 31<br>32<br>33<br>34<br>35 | H<br>F<br>Cl<br>$CH_3$<br>$CH_3O$ | 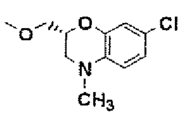 | 81<br>82<br>83<br>84<br>85 | H<br>F<br>Cl<br>$CH_3$<br>$CH_3O$ | 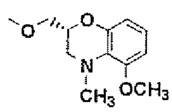 | 131<br>132<br>133<br>134<br>135 | H<br>F<br>Cl<br>$CH_3$<br>$CH_3O$ | 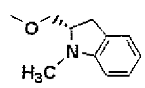 |
| 36<br>37<br>38<br>39<br>40 | H<br>F<br>Cl<br>$CH_3$<br>$CH_3O$ | 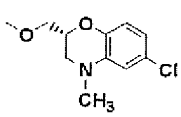 | 86<br>87<br>88<br>89<br>90 | H<br>F<br>Cl<br>$CH_3$<br>$CH_3O$ | 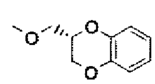 | 136<br>137<br>138<br>139<br>140 | H<br>F<br>Cl<br>$CH_3$<br>$CH_3O$ | 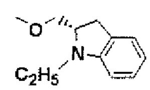 |
| 41<br>42<br>43<br>44<br>45 | H<br>F<br>Cl<br>$CH_3$<br>$CH_3O$ | 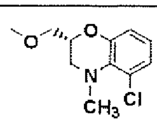 | 91<br>92<br>93<br>94<br>95 | H<br>F<br>Cl<br>$CH_3$<br>$CH_3O$ | 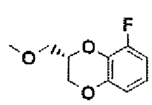 | 141<br>142<br>143<br>144<br>145 | H<br>F<br>Cl<br>$CH_3$<br>$CH_3O$ | 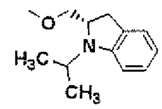 |
| 46<br>47<br>48<br>49<br>50 | H<br>F<br>Cl<br>$CH_3$<br>$CH_3O$ | 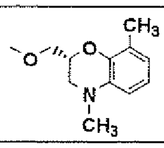 | 96<br>97<br>98<br>99<br>100 | H<br>F<br>Cl<br>$CH_3$<br>$CH_3O$ | 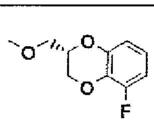 | 146<br>147<br>148<br>149<br>150 | H<br>F<br>Cl<br>$CH_3$<br>$CH_3O$ | 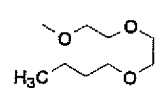 |

Table 15

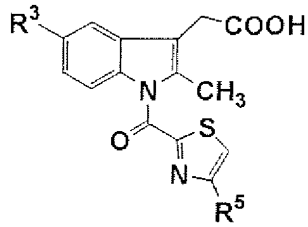

(I－A－8－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 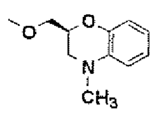 | 51<br>52<br>53<br>54<br>55 | H<br>F<br>Cl<br>$CH_3$<br>$CH_3O$ | 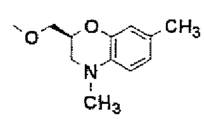 | 101<br>102<br>103<br>104<br>105 | H<br>F<br>Cl<br>$CH_3$<br>$CH_3O$ | 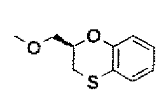 |
| 6<br>7<br>8<br>9<br>10 | H<br>F<br>Cl<br>$CH_3$<br>$CH_3O$ | 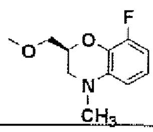 | 56<br>57<br>58<br>59<br>60 | H<br>F<br>Cl<br>$CH_3$<br>$CH_3O$ | 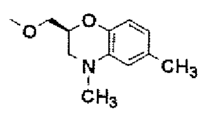 | 106<br>107<br>108<br>109<br>110 | H<br>F<br>Cl<br>$CH_3$<br>$CH_3O$ | 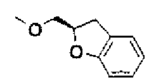 |
| 11<br>12<br>13<br>14<br>15 | H<br>F<br>Cl<br>$CH_3$<br>$CH_3O$ | 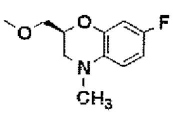 | 61<br>62<br>63<br>64<br>65 | H<br>F<br>Cl<br>$CH_3$<br>$CH_3O$ | 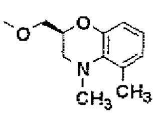 | 111<br>112<br>113<br>114<br>115 | H<br>F<br>Cl<br>$CH_3$<br>$CH_3O$ | 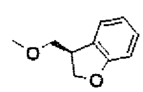 |
| 16<br>17<br>18<br>19<br>20 | H<br>F<br>Cl<br>$CH_3$<br>$CH_3O$ | 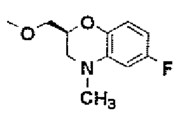 | 66<br>67<br>68<br>69<br>70 | H<br>F<br>Cl<br>$CH_3$<br>$CH_3O$ | 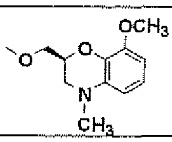 | 116<br>117<br>118<br>119<br>120 | H<br>F<br>Cl<br>$CH_3$<br>$CH_3O$ | 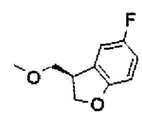 |
| 21<br>22<br>23<br>24<br>25 | H<br>F<br>Cl<br>$CH_3$<br>$CH_3O$ | 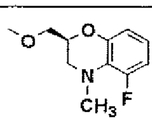 | 71<br>72<br>73<br>74<br>75 | H<br>F<br>Cl<br>$CH_3$<br>$CH_3O$ | 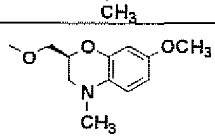 | 121<br>122<br>123<br>124<br>125 | H<br>F<br>Cl<br>$CH_3$<br>$CH_3O$ | 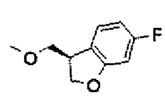 |
| 26<br>27<br>28<br>29<br>30 | H<br>F<br>Cl<br>$CH_3$<br>$CH_3O$ | 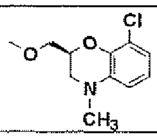 | 76<br>77<br>78<br>79<br>80 | H<br>F<br>Cl<br>$CH_3$<br>$CH_3O$ | 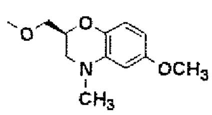 | 126<br>127<br>128<br>129<br>130 | H<br>F<br>Cl<br>$CH_3$<br>$CH_3O$ | 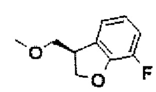 |
| 31<br>32<br>33<br>34<br>35 | H<br>F<br>Cl<br>$CH_3$<br>$CH_3O$ | 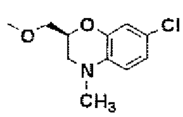 | 81<br>82<br>83<br>84<br>85 | H<br>F<br>Cl<br>$CH_3$<br>$CH_3O$ | 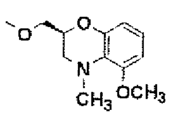 | 131<br>132<br>133<br>134<br>135 | H<br>F<br>Cl<br>$CH_3$<br>$CH_3O$ | 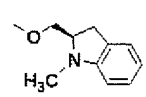 |
| 36<br>37<br>38<br>39<br>40 | H<br>F<br>Cl<br>$CH_3$<br>$CH_3O$ | 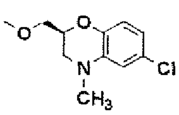 | 86<br>87<br>88<br>89<br>90 | H<br>F<br>Cl<br>$CH_3$<br>$CH_3O$ | 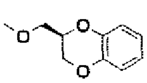 | 136<br>137<br>138<br>139<br>140 | H<br>F<br>Cl<br>$CH_3$<br>$CH_3O$ | 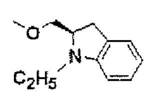 |
| 41<br>42<br>43<br>44<br>45 | H<br>F<br>Cl<br>$CH_3$<br>$CH_3O$ | 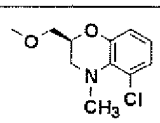 | 91<br>92<br>93<br>94<br>95 | H<br>F<br>Cl<br>$CH_3$<br>$CH_3O$ | 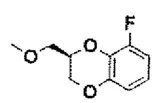 | 141<br>142<br>143<br>144<br>145 | H<br>F<br>Cl<br>$CH_3$<br>$CH_3O$ | 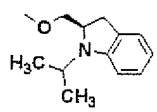 |
| 46<br>47<br>48<br>49<br>50 | H<br>F<br>Cl<br>$CH_3$<br>$CH_3O$ | 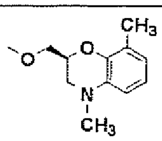 | 96<br>97<br>98<br>99<br>100 | H<br>F<br>Cl<br>$CH_3$<br>$CH_3O$ | 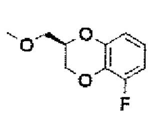 | 146<br>147<br>148<br>149<br>150 | H<br>F<br>Cl<br>$CH_3$<br>$CH_3O$ | 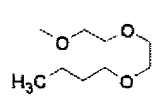 |

Table 16

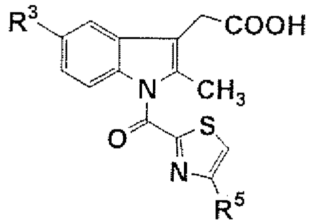

(I－A－8－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 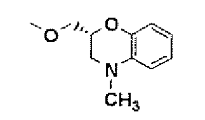 | 51<br>52<br>53<br>54<br>55 | H<br>F<br>Cl<br>$CH_3$<br>$CH_3O$ | 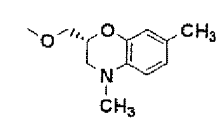 | 101<br>102<br>103<br>104<br>105 | H<br>F<br>Cl<br>$CH_3$<br>$CH_3O$ | 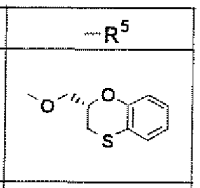 |
| 6<br>7<br>8<br>9<br>10 | H<br>F<br>Cl<br>$CH_3$<br>$CH_3O$ | 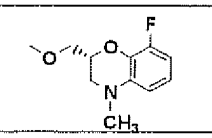 | 56<br>57<br>58<br>59<br>60 | H<br>F<br>Cl<br>$CH_3$<br>$CH_3O$ | 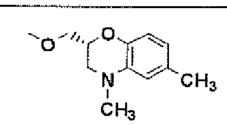 | 106<br>107<br>108<br>109<br>110 | H<br>F<br>Cl<br>$CH_3$<br>$CH_3O$ | 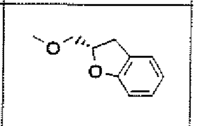 |
| 11<br>12<br>13<br>14<br>15 | H<br>F<br>Cl<br>$CH_3$<br>$CH_3O$ | 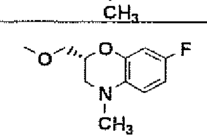 | 61<br>62<br>63<br>64<br>65 | H<br>F<br>Cl<br>$CH_3$<br>$CH_3O$ | 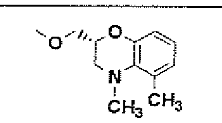 | 111<br>112<br>113<br>114<br>115 | H<br>F<br>Cl<br>$CH_3$<br>$CH_3O$ | 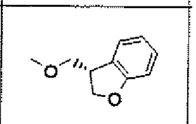 |
| 16<br>17<br>18<br>19<br>20 | H<br>F<br>Cl<br>$CH_3$<br>$CH_3O$ | 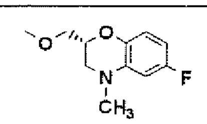 | 66<br>67<br>68<br>69<br>70 | H<br>F<br>Cl<br>$CH_3$<br>$CH_3O$ | 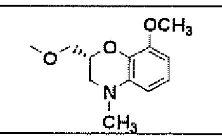 | 116<br>117<br>118<br>119<br>120 | H<br>F<br>Cl<br>$CH_3$<br>$CH_3O$ | 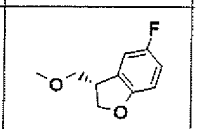 |
| 21<br>22<br>23<br>24<br>25 | H<br>F<br>Cl<br>$CH_3$<br>$CH_3O$ | 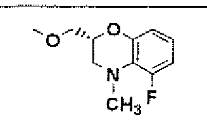 | 71<br>72<br>73<br>74<br>75 | H<br>F<br>Cl<br>$CH_3$<br>$CH_3O$ | 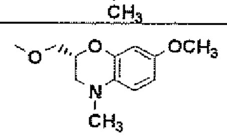 | 121<br>122<br>123<br>124<br>125 | H<br>F<br>Cl<br>$CH_3$<br>$CH_3O$ | 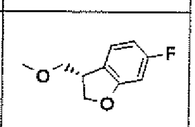 |
| 26<br>27<br>28<br>29<br>30 | H<br>F<br>Cl<br>$CH_3$<br>$CH_3O$ | 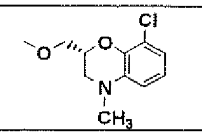 | 76<br>77<br>78<br>79<br>80 | H<br>F<br>Cl<br>$CH_3$<br>$CH_3O$ | 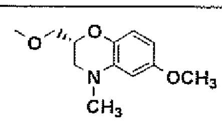 | 126<br>127<br>128<br>129<br>130 | H<br>F<br>Cl<br>$CH_3$<br>$CH_3O$ | 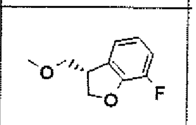 |
| 31<br>32<br>33<br>34<br>35 | H<br>F<br>Cl<br>$CH_3$<br>$CH_3O$ | 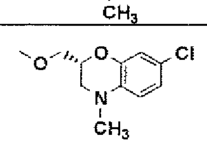 | 81<br>82<br>83<br>84<br>85 | H<br>F<br>Cl<br>$CH_3$<br>$CH_3O$ | 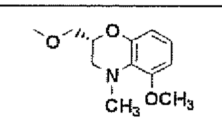 | 131<br>132<br>133<br>134<br>135 | H<br>F<br>Cl<br>$CH_3$<br>$CH_3O$ | 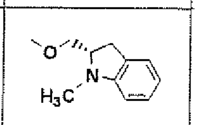 |
| 36<br>37<br>38<br>39<br>40 | H<br>F<br>Cl<br>$CH_3$<br>$CH_3O$ | 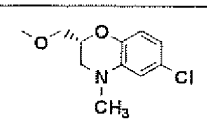 | 86<br>87<br>88<br>89<br>90 | H<br>F<br>Cl<br>$CH_3$<br>$CH_3O$ | 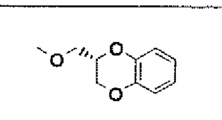 | 136<br>137<br>138<br>139<br>140 | H<br>F<br>Cl<br>$CH_3$<br>$CH_3O$ | 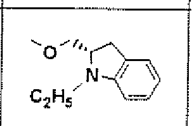 |
| 41<br>42<br>43<br>44<br>45 | H<br>F<br>Cl<br>$CH_3$<br>$CH_3O$ | 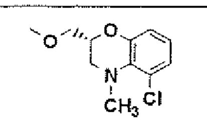 | 91<br>92<br>93<br>94<br>95 | H<br>F<br>Cl<br>$CH_3$<br>$CH_3O$ | 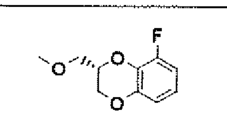 | 141<br>142<br>143<br>144<br>145 | H<br>F<br>Cl<br>$CH_3$<br>$CH_3O$ | 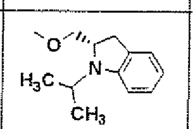 |
| 46<br>47<br>48<br>49<br>50 | H<br>F<br>Cl<br>$CH_3$<br>$CH_3O$ | 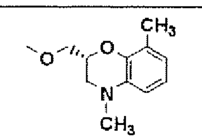 | 96<br>97<br>98<br>99<br>100 | H<br>F<br>Cl<br>$CH_3$<br>$CH_3O$ | 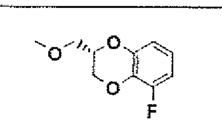 | 146<br>147<br>148<br>149<br>150 | H<br>F<br>Cl<br>$CH_3$<br>$CH_3O$ | 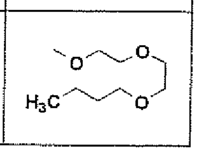 |

Table 17

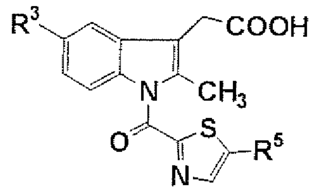

(1-A-8-2)

| No. R$^3$ | | $-R^5$ | No. R$^3$ | | $-R^5$ | No. R$^3$ | | $-R^5$ |
|---|---|---|---|---|---|---|---|---|
| 1<br>2<br>3<br>4<br>5 | H<br>F<br>Cl<br>$CH_3$<br>$CH_3O$ | 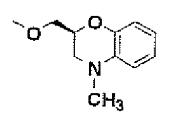 | 51<br>52<br>53<br>54<br>55 | H<br>F<br>Cl<br>$CH_3$<br>$CH_3O$ | 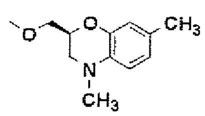 | 101<br>102<br>103<br>104<br>105 | H<br>F<br>Cl<br>$CH_3$<br>$CH_3O$ | 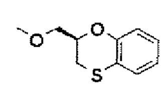 |
| 6<br>7<br>8<br>9<br>10 | H<br>F<br>Cl<br>$CH_3$<br>$CH_3O$ | 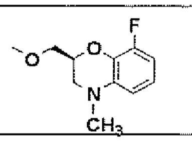 | 56<br>57<br>58<br>59<br>60 | H<br>F<br>Cl<br>$CH_3$<br>$CH_3O$ | 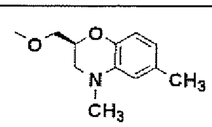 | 106<br>107<br>108<br>109<br>110 | H<br>F<br>Cl<br>$CH_3$<br>$CH_3O$ | 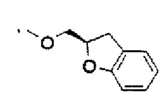 |
| 11<br>12<br>13<br>14<br>15 | H<br>F<br>Cl<br>$CH_3$<br>$CH_3O$ | 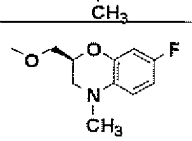 | 61<br>62<br>63<br>64<br>65 | H<br>F<br>Cl<br>$CH_3$<br>$CH_3O$ | 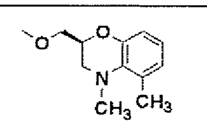 | 111<br>112<br>113<br>114<br>115 | H<br>F<br>Cl<br>$CH_3$<br>$CH_3O$ | 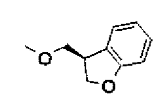 |
| 16<br>17<br>18<br>19<br>20 | H<br>F<br>Cl<br>$CH_3$<br>$CH_3O$ | 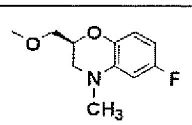 | 66<br>67<br>68<br>69<br>70 | H<br>F<br>Cl<br>$CH_3$<br>$CH_3O$ | 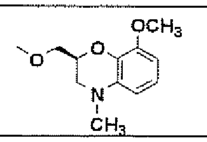 | 116<br>117<br>118<br>119<br>120 | H<br>F<br>Cl<br>$CH_3$<br>$CH_3O$ | 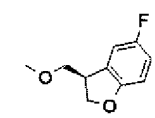 |
| 21<br>22<br>23<br>24<br>25 | H<br>F<br>Cl<br>$CH_3$<br>$CH_3O$ | 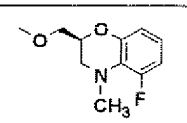 | 71<br>72<br>73<br>74<br>75 | H<br>F<br>Cl<br>$CH_3$<br>$CH_3O$ | 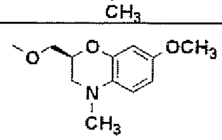 | 121<br>122<br>123<br>124<br>125 | H<br>F<br>Cl<br>$CH_3$<br>$CH_3O$ | 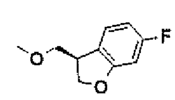 |
| 26<br>27<br>28<br>29<br>30 | H<br>F<br>Cl<br>$CH_3$<br>$CH_3O$ | 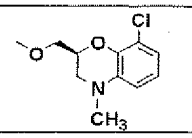 | 76<br>77<br>78<br>79<br>80 | H<br>F<br>Cl<br>$CH_3$<br>$CH_3O$ | 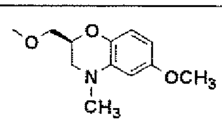 | 126<br>127<br>128<br>129<br>130 | H<br>F<br>Cl<br>$CH_3$<br>$CH_3O$ | 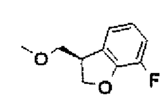 |
| 31<br>32<br>33<br>34<br>35 | H<br>F<br>Cl<br>$CH_3$<br>$CH_3O$ | 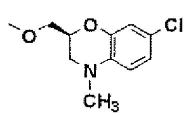 | 81<br>82<br>83<br>84<br>85 | H<br>F<br>Cl<br>$CH_3$<br>$CH_3O$ | 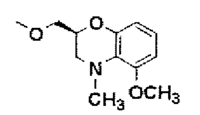 | 131<br>132<br>133<br>134<br>135 | H<br>F<br>Cl<br>$CH_3$<br>$CH_3O$ | 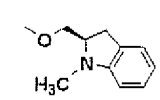 |
| 36<br>37<br>38<br>39<br>40 | H<br>F<br>Cl<br>$CH_3$<br>$CH_3O$ | 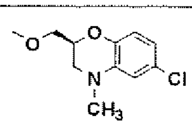 | 86<br>87<br>88<br>89<br>90 | H<br>F<br>Cl<br>$CH_3$<br>$CH_3O$ | 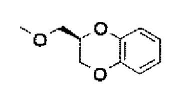 | 136<br>137<br>138<br>139<br>140 | H<br>F<br>Cl<br>$CH_3$<br>$CH_3O$ | 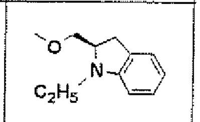 |
| 41<br>42<br>43<br>44<br>45 | H<br>F<br>Cl<br>$CH_3$<br>$CH_3O$ | 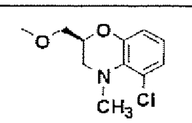 | 91<br>92<br>93<br>94<br>95 | H<br>F<br>Cl<br>$CH_3$<br>$CH_3O$ | 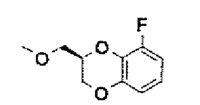 | 141<br>142<br>143<br>144<br>145 | H<br>F<br>Cl<br>$CH_3$<br>$CH_3O$ | 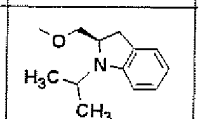 |
| 46<br>47<br>48<br>49<br>50 | H<br>F<br>Cl<br>$CH_3$<br>$CH_3O$ | 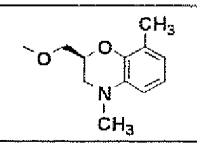 | 96<br>97<br>98<br>99<br>100 | H<br>F<br>Cl<br>$CH_3$<br>$CH_3O$ | 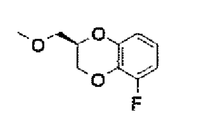 | 146<br>147<br>148<br>149<br>150 | H<br>F<br>Cl<br>$CH_3$<br>$CH_3O$ | 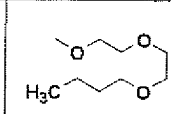 |

Table 18

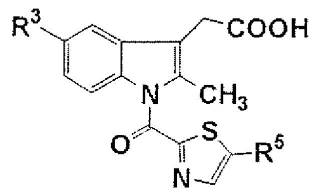

(I-A-8-2)

| No. | $R^3$ | $-R^5$ | No. | $R^3$ | $-R^5$ | No. | $R^3$ | $-R^5$ |
|---|---|---|---|---|---|---|---|---|
| 1 | H | 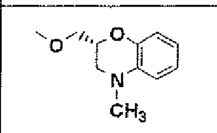 | 51 | H | 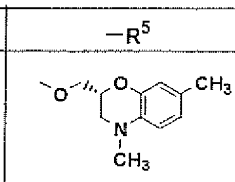 | 101 | H | 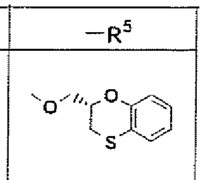 |
| 2 | F | | 52 | F | | 102 | F | |
| 3 | Cl | | 53 | Cl | | 103 | Cl | |
| 4 | $CH_3$ | | 54 | $CH_3$ | | 104 | $CH_3$ | |
| 5 | $CH_3O$ | | 55 | $CH_3O$ | | 105 | $CH_3O$ | |
| 6 | H | 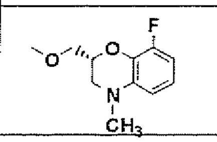 | 56 | H | 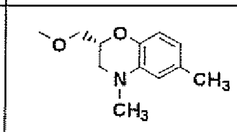 | 106 | H | 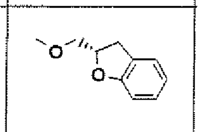 |
| 7 | F | | 57 | F | | 107 | F | |
| 8 | Cl | | 58 | Cl | | 108 | Cl | |
| 9 | $CH_3$ | | 59 | $CH_3$ | | 109 | $CH_3$ | |
| 10 | $CH_3O$ | | 60 | $CH_3O$ | | 110 | $CH_3O$ | |
| 11 | H | 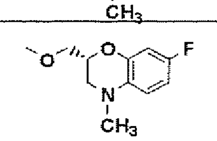 | 61 | H | 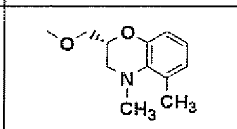 | 111 | H | 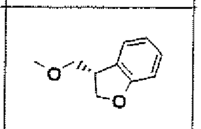 |
| 12 | F | | 62 | F | | 112 | F | |
| 13 | Cl | | 63 | Cl | | 113 | Cl | |
| 14 | $CH_3$ | | 64 | $CH_3$ | | 114 | $CH_3$ | |
| 15 | $CH_3O$ | | 65 | $CH_3O$ | | 115 | $CH_3O$ | |
| 16 | H | 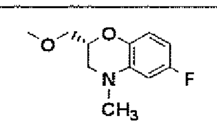 | 66 | H | 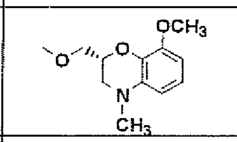 | 116 | H | 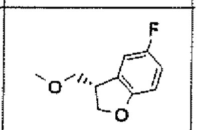 |
| 17 | F | | 67 | F | | 117 | F | |
| 18 | Cl | | 68 | Cl | | 118 | Cl | |
| 19 | $CH_3$ | | 69 | $CH_3$ | | 119 | $CH_3$ | |
| 20 | $CH_3O$ | | 70 | $CH_3O$ | | 120 | $CH_3O$ | |
| 21 | H | 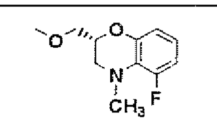 | 71 | H | 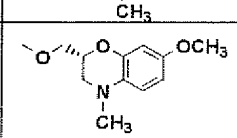 | 121 | H | 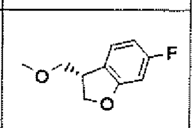 |
| 22 | F | | 72 | F | | 122 | F | |
| 23 | Cl | | 73 | Cl | | 123 | Cl | |
| 24 | $CH_3$ | | 74 | $CH_3$ | | 124 | $CH_3$ | |
| 25 | $CH_3O$ | | 75 | $CH_3O$ | | 125 | $CH_3O$ | |
| 26 | H | 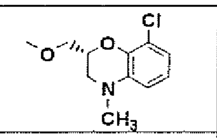 | 76 | H | 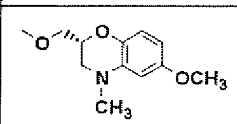 | 126 | H | 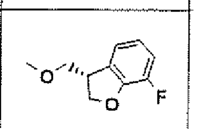 |
| 27 | F | | 77 | F | | 127 | F | |
| 28 | Cl | | 78 | Cl | | 128 | Cl | |
| 29 | $CH_3$ | | 79 | $CH_3$ | | 129 | $CH_3$ | |
| 30 | $CH_3O$ | | 80 | $CH_3O$ | | 130 | $CH_3O$ | |
| 31 | H | 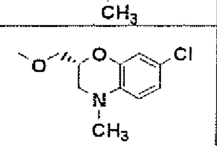 | 81 | H | 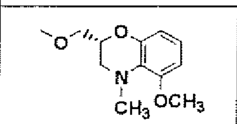 | 131 | H | 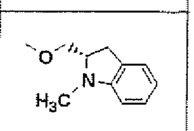 |
| 32 | F | | 82 | F | | 132 | F | |
| 33 | Cl | | 83 | Cl | | 133 | Cl | |
| 34 | $CH_3$ | | 84 | $CH_3$ | | 134 | $CH_3$ | |
| 35 | $CH_3O$ | | 85 | $CH_3O$ | | 135 | $CH_3O$ | |
| 36 | H | 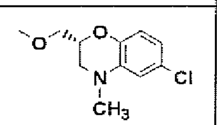 | 86 | H | 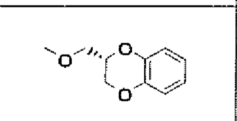 | 136 | H | 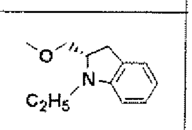 |
| 37 | F | | 87 | F | | 137 | F | |
| 38 | Cl | | 88 | Cl | | 138 | Cl | |
| 39 | $CH_3$ | | 89 | $CH_3$ | | 139 | $CH_3$ | |
| 40 | $CH_3O$ | | 90 | $CH_3O$ | | 140 | $CH_3O$ | |
| 41 | H | 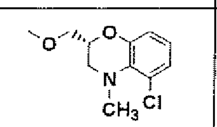 | 91 | H | 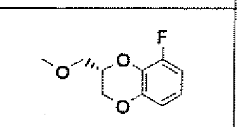 | 141 | H | 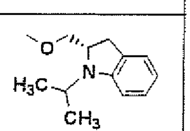 |
| 42 | F | | 92 | F | | 142 | F | |
| 43 | Cl | | 93 | Cl | | 143 | Cl | |
| 44 | $CH_3$ | | 94 | $CH_3$ | | 144 | $CH_3$ | |
| 45 | $CH_3O$ | | 95 | $CH_3O$ | | 145 | $CH_3O$ | |
| 46 | H | 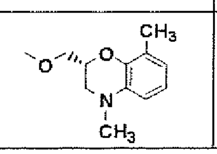 | 96 | H | 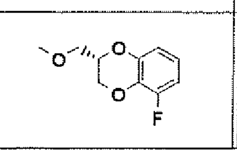 | 146 | H | 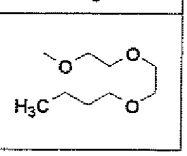 |
| 47 | F | | 97 | F | | 147 | F | |
| 48 | Cl | | 98 | Cl | | 148 | Cl | |
| 49 | $CH_3$ | | 99 | $CH_3$ | | 149 | $CH_3$ | |
| 50 | $CH_3O$ | | 100 | $CH_3O$ | | 150 | $CH_3O$ | |

Table 19

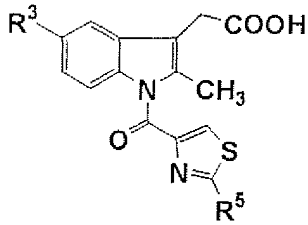

(I-A-9-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 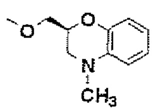 | 51<br>52<br>53<br>54<br>55 | H<br>F<br>Cl<br>$CH_3$<br>$CH_3O$ | 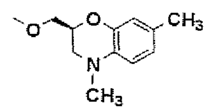 | 101<br>102<br>103<br>104<br>105 | H<br>F<br>Cl<br>$CH_3$<br>$CH_3O$ | 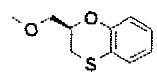 |
| 6<br>7<br>8<br>9<br>10 | H<br>F<br>Cl<br>$CH_3$<br>$CH_3O$ | 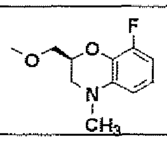 | 56<br>57<br>58<br>59<br>60 | H<br>F<br>Cl<br>$CH_3$<br>$CH_3O$ | 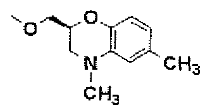 | 106<br>107<br>108<br>109<br>110 | H<br>F<br>Cl<br>$CH_3$<br>$CH_3O$ | 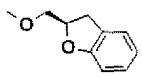 |
| 11<br>12<br>13<br>14<br>15 | H<br>F<br>Cl<br>$CH_3$<br>$CH_3O$ | 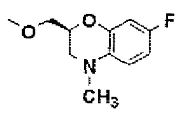 | 61<br>62<br>63<br>64<br>65 | H<br>F<br>Cl<br>$CH_3$<br>$CH_3O$ | 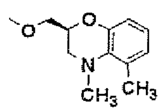 | 111<br>112<br>113<br>114<br>115 | H<br>F<br>Cl<br>$CH_3$<br>$CH_3O$ | 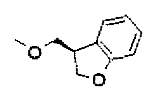 |
| 16<br>17<br>18<br>19<br>20 | H<br>F<br>Cl<br>$CH_3$<br>$CH_3O$ | 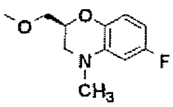 | 66<br>67<br>68<br>69<br>70 | H<br>F<br>Cl<br>$CH_3$<br>$CH_3O$ | 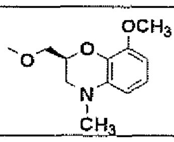 | 116<br>117<br>118<br>119<br>120 | H<br>F<br>Cl<br>$CH_3$<br>$CH_3O$ | 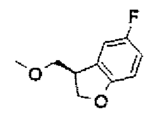 |
| 21<br>22<br>23<br>24<br>25 | H<br>F<br>Cl<br>$CH_3$<br>$CH_3O$ | 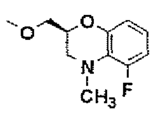 | 71<br>72<br>73<br>74<br>75 | H<br>F<br>Cl<br>$CH_3$<br>$CH_3O$ | 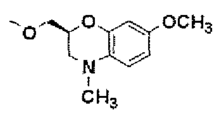 | 121<br>122<br>123<br>124<br>125 | H<br>F<br>Cl<br>$CH_3$<br>$CH_3O$ | 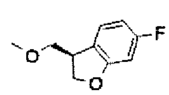 |
| 26<br>27<br>28<br>29<br>30 | H<br>F<br>Cl<br>$CH_3$<br>$CH_3O$ | 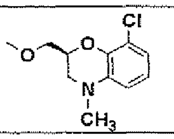 | 76<br>77<br>78<br>79<br>80 | H<br>F<br>Cl<br>$CH_3$<br>$CH_3O$ | 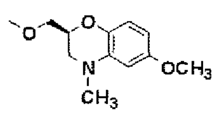 | 126<br>127<br>128<br>129<br>130 | H<br>F<br>Cl<br>$CH_3$<br>$CH_3O$ | 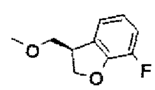 |
| 31<br>32<br>33<br>34<br>35 | H<br>F<br>Cl<br>$CH_3$<br>$CH_3O$ | 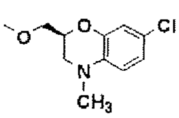 | 81<br>82<br>83<br>84<br>85 | H<br>F<br>Cl<br>$CH_3$<br>$CH_3O$ | 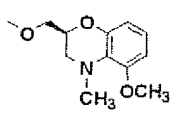 | 131<br>132<br>133<br>134<br>135 | H<br>F<br>Cl<br>$CH_3$<br>$CH_3O$ | 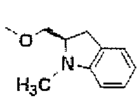 |
| 36<br>37<br>38<br>39<br>40 | H<br>F<br>Cl<br>$CH_3$<br>$CH_3O$ | 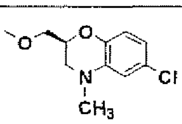 | 86<br>87<br>88<br>89<br>90 | H<br>F<br>Cl<br>$CH_3$<br>$CH_3O$ | 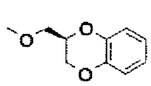 | 136<br>137<br>138<br>139<br>140 | H<br>F<br>Cl<br>$CH_3$<br>$CH_3O$ | 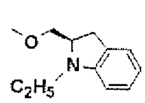 |
| 41<br>42<br>43<br>44<br>45 | H<br>F<br>Cl<br>$CH_3$<br>$CH_3O$ | 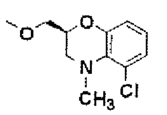 | 91<br>92<br>93<br>94<br>95 | H<br>F<br>Cl<br>$CH_3$<br>$CH_3O$ | 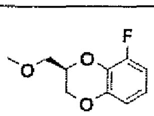 | 141<br>142<br>143<br>144<br>145 | H<br>F<br>Cl<br>$CH_3$<br>$CH_3O$ | 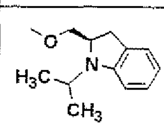 |
| 46<br>47<br>48<br>49<br>50 | H<br>F<br>Cl<br>$CH_3$<br>$CH_3O$ | 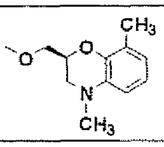 | 96<br>97<br>98<br>99<br>100 | H<br>F<br>Cl<br>$CH_3$<br>$CH_3O$ | 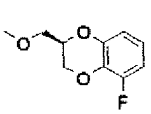 | 146<br>147<br>148<br>149<br>150 | H<br>F<br>Cl<br>$CH_3$<br>$CH_3O$ | 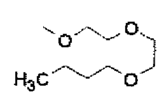 |

Table 20

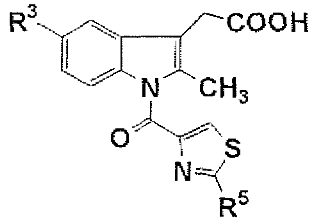

(I-A-9-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 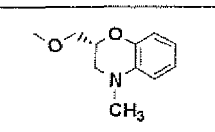 | 51<br>52<br>53<br>54<br>55 | H<br>F<br>Cl<br>$CH_3$<br>$CH_3O$ | 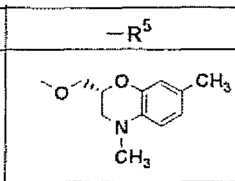 | 101<br>102<br>103<br>104<br>105 | H<br>F<br>Cl<br>$CH_3$<br>$CH_3O$ | 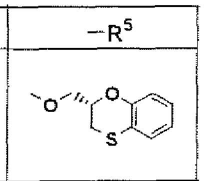 |
| 6<br>7<br>8<br>9<br>10 | H<br>F<br>Cl<br>$CH_3$<br>$CH_3O$ | 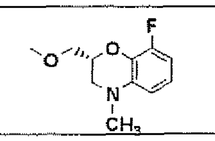 | 56<br>57<br>58<br>59<br>60 | H<br>F<br>Cl<br>$CH_3$<br>$CH_3O$ | 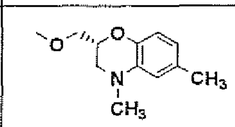 | 106<br>107<br>108<br>109<br>110 | H<br>F<br>Cl<br>$CH_3$<br>$CH_3O$ | 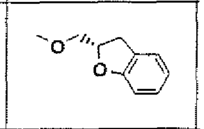 |
| 11<br>12<br>13<br>14<br>15 | H<br>F<br>Cl<br>$CH_3$<br>$CH_3O$ | 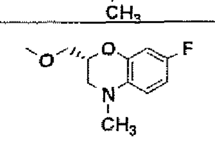 | 61<br>62<br>63<br>64<br>65 | H<br>F<br>Cl<br>$CH_3$<br>$CH_3O$ | 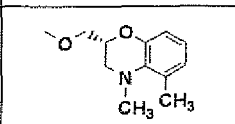 | 111<br>112<br>113<br>114<br>115 | H<br>F<br>Cl<br>$CH_3$<br>$CH_3O$ | 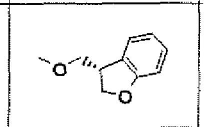 |
| 16<br>17<br>18<br>19<br>20 | H<br>F<br>Cl<br>$CH_3$<br>$CH_3O$ | 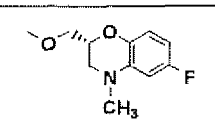 | 66<br>67<br>68<br>69<br>70 | H<br>F<br>Cl<br>$CH_3$<br>$CH_3O$ | 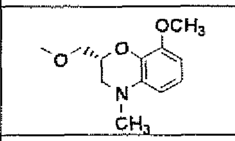 | 116<br>117<br>118<br>119<br>120 | H<br>F<br>Cl<br>$CH_3$<br>$CH_3O$ | 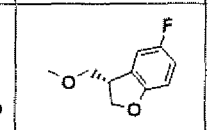 |
| 21<br>22<br>23<br>24<br>25 | H<br>F<br>Cl<br>$CH_3$<br>$CH_3O$ | 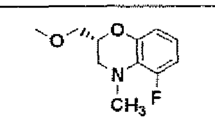 | 71<br>72<br>73<br>74<br>75 | H<br>F<br>Cl<br>$CH_3$<br>$CH_3O$ | 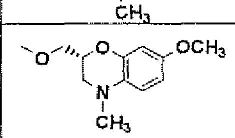 | 121<br>122<br>123<br>124<br>125 | H<br>F<br>Cl<br>$CH_3$<br>$CH_3O$ | 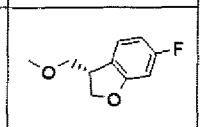 |
| 26<br>27<br>28<br>29<br>30 | H<br>F<br>Cl<br>$CH_3$<br>$CH_3O$ | 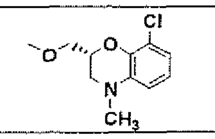 | 76<br>77<br>78<br>79<br>80 | H<br>F<br>Cl<br>$CH_3$<br>$CH_3O$ | 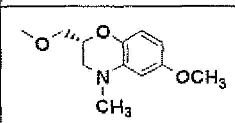 | 126<br>127<br>128<br>129<br>130 | H<br>F<br>Cl<br>$CH_3$<br>$CH_3O$ | 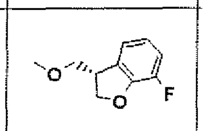 |
| 31<br>32<br>33<br>34<br>35 | H<br>F<br>Cl<br>$CH_3$<br>$CH_3O$ | 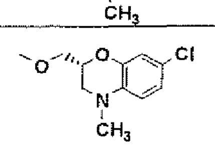 | 81<br>82<br>83<br>84<br>85 | H<br>F<br>Cl<br>$CH_3$<br>$CH_3O$ | 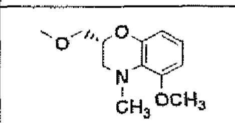 | 131<br>132<br>133<br>134<br>135 | H<br>F<br>Cl<br>$CH_3$<br>$CH_3O$ | 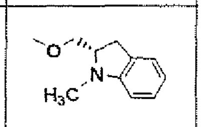 |
| 36<br>37<br>38<br>39<br>40 | H<br>F<br>Cl<br>$CH_3$<br>$CH_3O$ | 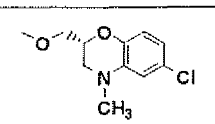 | 86<br>87<br>88<br>89<br>90 | H<br>F<br>Cl<br>$CH_3$<br>$CH_3O$ | 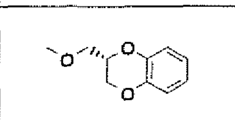 | 136<br>137<br>138<br>139<br>140 | H<br>F<br>Cl<br>$CH_3$<br>$CH_3O$ | 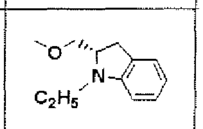 |
| 41<br>42<br>43<br>44<br>45 | H<br>F<br>Cl<br>$CH_3$<br>$CH_3O$ | 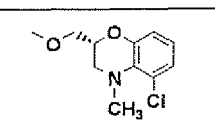 | 91<br>92<br>93<br>94<br>95 | H<br>F<br>Cl<br>$CH_3$<br>$CH_3O$ | 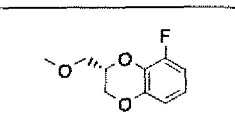 | 141<br>142<br>143<br>144<br>145 | H<br>F<br>Cl<br>$CH_3$<br>$CH_3O$ | 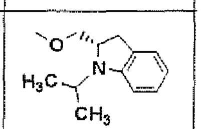 |
| 46<br>47<br>48<br>49<br>50 | H<br>F<br>Cl<br>$CH_3$<br>$CH_3O$ | 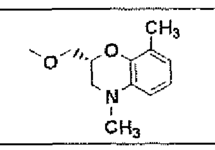 | 96<br>97<br>98<br>99<br>100 | H<br>F<br>Cl<br>$CH_3$<br>$CH_3O$ | 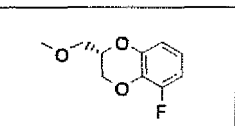 | 146<br>147<br>148<br>149<br>150 | H<br>F<br>Cl<br>$CH_3$<br>$CH_3O$ | 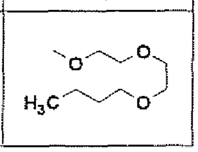 |

Table 21

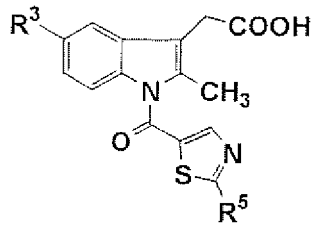

(I－A－1 0－1)

| No. | $R^3$ | $-R^5$ | No. | $R^3$ | $-R^5$ | No. | $R^3$ | $-R^5$ |
|---|---|---|---|---|---|---|---|---|
| 1 | H | 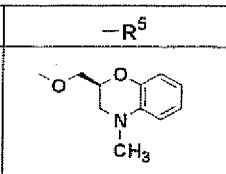 | 51 | H | 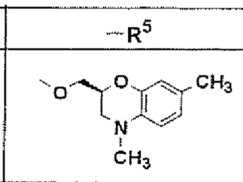 | 101 | H | 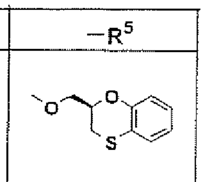 |
| 2 | F | | 52 | F | | 102 | F | |
| 3 | Cl | | 53 | Cl | | 103 | Cl | |
| 4 | $CH_3$ | | 54 | $CH_3$ | | 104 | $CH_3$ | |
| 5 | $CH_3O$ | | 55 | $CH_3O$ | | 105 | $CH_3O$ | |
| 6 | H | 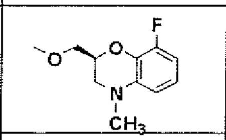 | 56 | H | 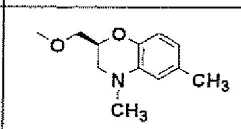 | 106 | H | 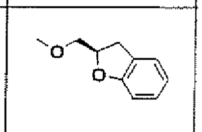 |
| 7 | F | | 57 | F | | 107 | F | |
| 8 | Cl | | 58 | Cl | | 108 | Cl | |
| 9 | $CH_3$ | | 59 | $CH_3$ | | 109 | $CH_3$ | |
| 10 | $CH_3O$ | | 60 | $CH_3O$ | | 110 | $CH_3O$ | |
| 11 | H | 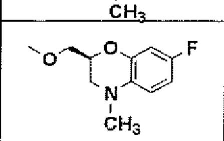 | 61 | H | 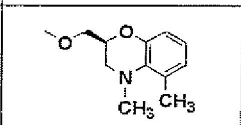 | 111 | H | 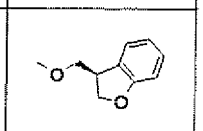 |
| 12 | F | | 62 | F | | 112 | F | |
| 13 | Cl | | 63 | Cl | | 113 | Cl | |
| 14 | $CH_3$ | | 64 | $CH_3$ | | 114 | $CH_3$ | |
| 15 | $CH_3O$ | | 65 | $CH_3O$ | | 115 | $CH_3O$ | |
| 16 | H | 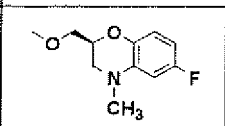 | 66 | H | 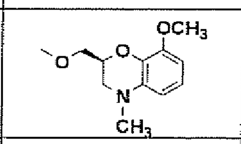 | 116 | H | 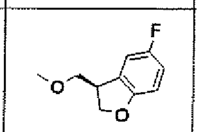 |
| 17 | F | | 67 | F | | 117 | F | |
| 18 | Cl | | 68 | Cl | | 118 | Cl | |
| 19 | $CH_3$ | | 69 | $CH_3$ | | 119 | $CH_3$ | |
| 20 | $CH_3O$ | | 70 | $CH_3O$ | | 120 | $CH_3O$ | |
| 21 | H | 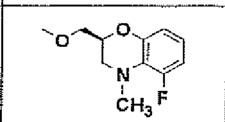 | 71 | H | 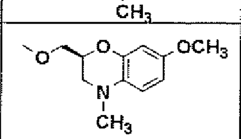 | 121 | H | 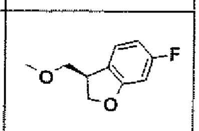 |
| 22 | F | | 72 | F | | 122 | F | |
| 23 | Cl | | 73 | Cl | | 123 | Cl | |
| 24 | $CH_3$ | | 74 | $CH_3$ | | 124 | $CH_3$ | |
| 25 | $CH_3O$ | | 75 | $CH_3O$ | | 125 | $CH_3O$ | |
| 26 | H | 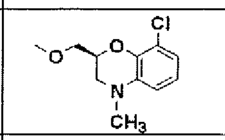 | 76 | H | 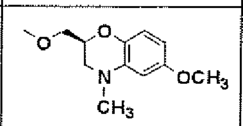 | 126 | H | 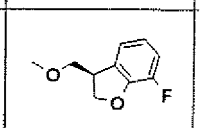 |
| 27 | F | | 77 | F | | 127 | F | |
| 28 | Cl | | 78 | Cl | | 128 | Cl | |
| 29 | $CH_3$ | | 79 | $CH_3$ | | 129 | $CH_3$ | |
| 30 | $CH_3O$ | | 80 | $CH_3O$ | | 130 | $CH_3O$ | |
| 31 | H | 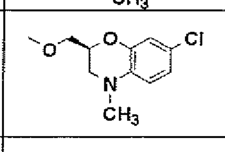 | 81 | H | 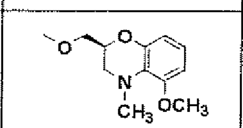 | 131 | H | 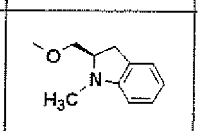 |
| 32 | F | | 82 | F | | 132 | F | |
| 33 | Cl | | 83 | Cl | | 133 | Cl | |
| 34 | $CH_3$ | | 84 | $CH_3$ | | 134 | $CH_3$ | |
| 35 | $CH_3O$ | | 85 | $CH_3O$ | | 135 | $CH_3O$ | |
| 36 | H | 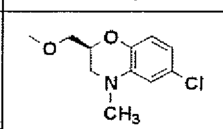 | 86 | H | 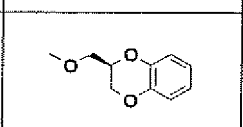 | 136 | H | 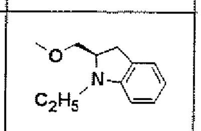 |
| 37 | F | | 87 | F | | 137 | F | |
| 38 | Cl | | 88 | Cl | | 138 | Cl | |
| 39 | $CH_3$ | | 89 | $CH_3$ | | 139 | $CH_3$ | |
| 40 | $CH_3O$ | | 90 | $CH_3O$ | | 140 | $CH_3O$ | |
| 41 | H | 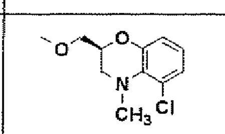 | 91 | H | 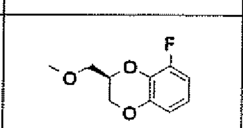 | 141 | H | 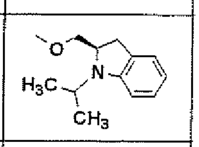 |
| 42 | F | | 92 | F | | 142 | F | |
| 43 | Cl | | 93 | Cl | | 143 | Cl | |
| 44 | $CH_3$ | | 94 | $CH_3$ | | 144 | $CH_3$ | |
| 45 | $CH_3O$ | | 95 | $CH_3O$ | | 145 | $CH_3O$ | |
| 46 | H | 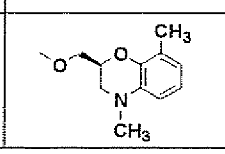 | 96 | H | 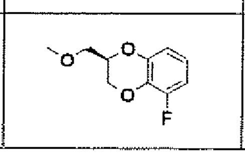 | 146 | H | 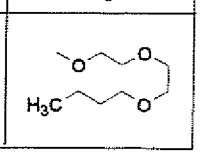 |
| 47 | F | | 97 | F | | 147 | F | |
| 48 | Cl | | 98 | Cl | | 148 | Cl | |
| 49 | $CH_3$ | | 99 | $CH_3$ | | 149 | $CH_3$ | |
| 50 | $CH_3O$ | | 100 | $CH_3O$ | | 150 | $CH_3O$ | |

Table 22

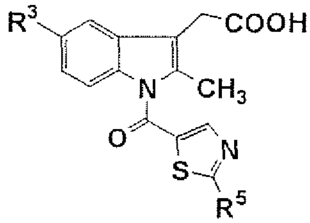

(I－A－10－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 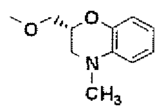 | 51<br>52<br>53<br>54<br>55 | H<br>F<br>Cl<br>$CH_3$<br>$CH_3O$ | 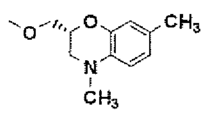 | 101<br>102<br>103<br>104<br>105 | H<br>F<br>Cl<br>$CH_3$<br>$CH_3O$ | 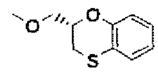 |
| 6<br>7<br>8<br>9<br>10 | H<br>F<br>Cl<br>$CH_3$<br>$CH_3O$ | 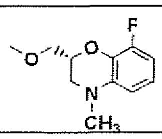 | 56<br>57<br>58<br>59<br>60 | H<br>F<br>Cl<br>$CH_3$<br>$CH_3O$ | 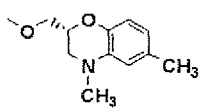 | 106<br>107<br>108<br>109<br>110 | H<br>F<br>Cl<br>$CH_3$<br>$CH_3O$ | 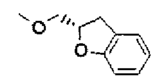 |
| 11<br>12<br>13<br>14<br>15 | H<br>F<br>Cl<br>$CH_3$<br>$CH_3O$ | 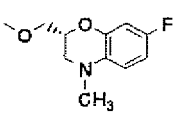 | 61<br>62<br>63<br>64<br>65 | H<br>F<br>Cl<br>$CH_3$<br>$CH_3O$ | 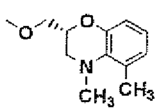 | 111<br>112<br>113<br>114<br>115 | H<br>F<br>Cl<br>$CH_3$<br>$CH_3O$ | 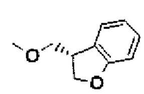 |
| 16<br>17<br>18<br>19<br>20 | H<br>F<br>Cl<br>$CH_3$<br>$CH_3O$ | 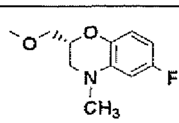 | 66<br>67<br>68<br>69<br>70 | H<br>F<br>Cl<br>$CH_3$<br>$CH_3O$ | 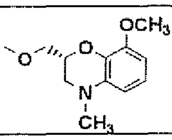 | 116<br>117<br>118<br>119<br>120 | H<br>F<br>Cl<br>$CH_3$<br>$CH_3O$ | 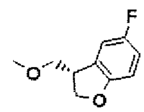 |
| 21<br>22<br>23<br>24<br>25 | H<br>F<br>Cl<br>$CH_3$<br>$CH_3O$ | 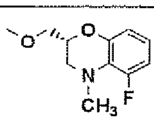 | 71<br>72<br>73<br>74<br>75 | H<br>F<br>Cl<br>$CH_3$<br>$CH_3O$ | 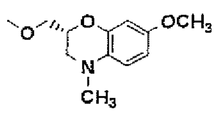 | 121<br>122<br>123<br>124<br>125 | H<br>F<br>Cl<br>$CH_3$<br>$CH_3O$ | 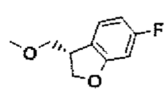 |
| 26<br>27<br>28<br>29<br>30 | H<br>F<br>Cl<br>$CH_3$<br>$CH_3O$ | 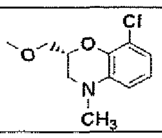 | 76<br>77<br>78<br>79<br>80 | H<br>F<br>Cl<br>$CH_3$<br>$CH_3O$ | 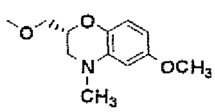 | 126<br>127<br>128<br>129<br>130 | H<br>F<br>Cl<br>$CH_3$<br>$CH_3O$ | 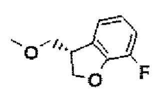 |
| 31<br>32<br>33<br>34<br>35 | H<br>F<br>Cl<br>$CH_3$<br>$CH_3O$ | 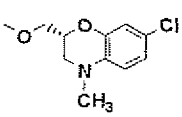 | 81<br>82<br>83<br>84<br>85 | H<br>F<br>Cl<br>$CH_3$<br>$CH_3O$ | 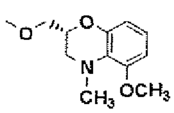 | 131<br>132<br>133<br>134<br>135 | H<br>F<br>Cl<br>$CH_3$<br>$CH_3O$ | 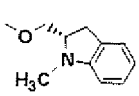 |
| 36<br>37<br>38<br>39<br>40 | H<br>F<br>Cl<br>$CH_3$<br>$CH_3O$ | 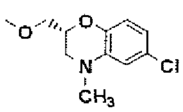 | 86<br>87<br>88<br>89<br>90 | H<br>F<br>Cl<br>$CH_3$<br>$CH_3O$ | 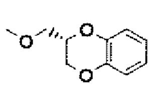 | 136<br>137<br>138<br>139<br>140 | H<br>F<br>Cl<br>$CH_3$<br>$CH_3O$ | 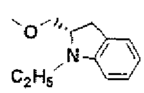 |
| 41<br>42<br>43<br>44<br>45 | H<br>F<br>Cl<br>$CH_3$<br>$CH_3O$ | 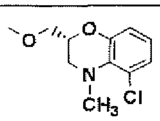 | 91<br>92<br>93<br>94<br>95 | H<br>F<br>Cl<br>$CH_3$<br>$CH_3O$ | 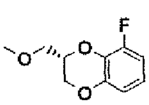 | 141<br>142<br>143<br>144<br>145 | H<br>F<br>Cl<br>$CH_3$<br>$CH_3O$ | 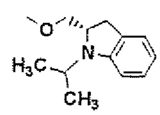 |
| 46<br>47<br>48<br>49<br>50 | H<br>F<br>Cl<br>$CH_3$<br>$CH_3O$ | 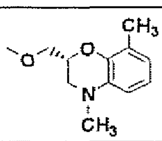 | 96<br>97<br>98<br>99<br>100 | H<br>F<br>Cl<br>$CH_3$<br>$CH_3O$ | 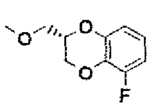 | 146<br>147<br>148<br>149<br>150 | H<br>F<br>Cl<br>$CH_3$<br>$CH_3O$ | 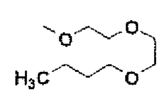 |

Table 23

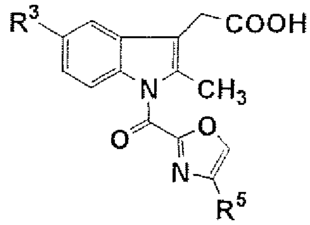

(I-A-11-1)

| No. | $R^3$ | $-R^5$ | No. | $R^3$ | $-R^5$ | No. | $R^3$ | $-R^5$ |
|---|---|---|---|---|---|---|---|---|
| 1 | H | 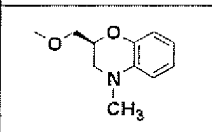 | 51 | H | 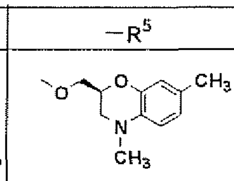 | 101 | H | 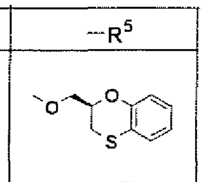 |
| 2 | F | | 52 | F | | 102 | F | |
| 3 | Cl | | 53 | Cl | | 103 | Cl | |
| 4 | $CH_3$ | | 54 | $CH_3$ | | 104 | $CH_3$ | |
| 5 | $CH_3O$ | | 55 | $CH_3O$ | | 105 | $CH_3O$ | |
| 6 | H | 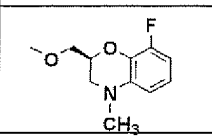 | 56 | H | 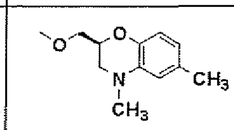 | 106 | H | 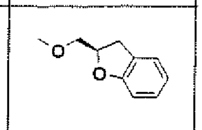 |
| 7 | F | | 57 | F | | 107 | F | |
| 8 | Cl | | 58 | Cl | | 108 | Cl | |
| 9 | $CH_3$ | | 59 | $CH_3$ | | 109 | $CH_3$ | |
| 10 | $CH_3O$ | | 60 | $CH_3O$ | | 110 | $CH_3O$ | |
| 11 | H | 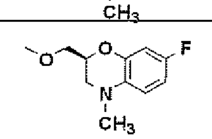 | 61 | H | 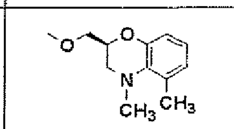 | 111 | H | 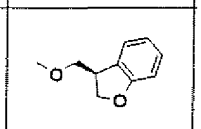 |
| 12 | F | | 62 | F | | 112 | F | |
| 13 | Cl | | 63 | Cl | | 113 | Cl | |
| 14 | $CH_3$ | | 64 | $CH_3$ | | 114 | $CH_3$ | |
| 15 | $CH_3O$ | | 65 | $CH_3O$ | | 115 | $CH_3O$ | |
| 16 | H | 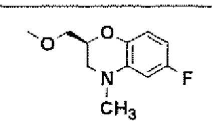 | 66 | H | 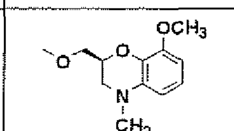 | 116 | H | 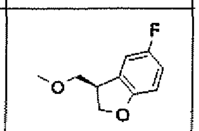 |
| 17 | F | | 67 | F | | 117 | F | |
| 18 | Cl | | 68 | Cl | | 118 | Cl | |
| 19 | $CH_3$ | | 69 | $CH_3$ | | 119 | $CH_3$ | |
| 20 | $CH_3O$ | | 70 | $CH_3O$ | | 120 | $CH_3O$ | |
| 21 | H | 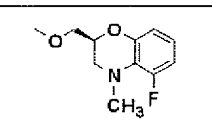 | 71 | H | 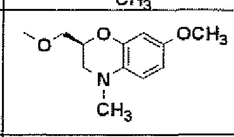 | 121 | H | 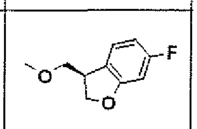 |
| 22 | F | | 72 | F | | 122 | F | |
| 23 | Cl | | 73 | Cl | | 123 | Cl | |
| 24 | $CH_3$ | | 74 | $CH_3$ | | 124 | $CH_3$ | |
| 25 | $CH_3O$ | | 75 | $CH_3O$ | | 125 | $CH_3O$ | |
| 26 | H | 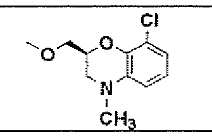 | 76 | H | 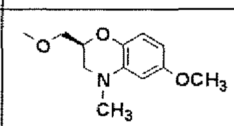 | 126 | H | 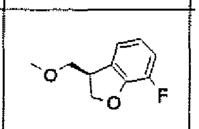 |
| 27 | F | | 77 | F | | 127 | F | |
| 28 | Cl | | 78 | Cl | | 128 | Cl | |
| 29 | $CH_3$ | | 79 | $CH_3$ | | 129 | $CH_3$ | |
| 30 | $CH_3O$ | | 80 | $CH_3O$ | | 130 | $CH_3O$ | |
| 31 | H | 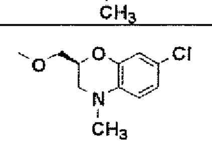 | 81 | H | 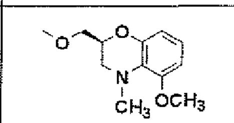 | 131 | H | 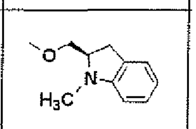 |
| 32 | F | | 82 | F | | 132 | F | |
| 33 | Cl | | 83 | Cl | | 133 | Cl | |
| 34 | $CH_3$ | | 84 | $CH_3$ | | 134 | $CH_3$ | |
| 35 | $CH_3O$ | | 85 | $CH_3O$ | | 135 | $CH_3O$ | |
| 36 | H | 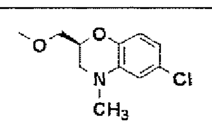 | 86 | H | 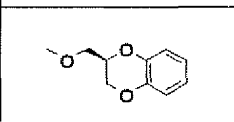 | 136 | H | 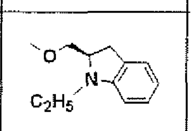 |
| 37 | F | | 87 | F | | 137 | F | |
| 38 | Cl | | 88 | Cl | | 138 | Cl | |
| 39 | $CH_3$ | | 89 | $CH_3$ | | 139 | $CH_3$ | |
| 40 | $CH_3O$ | | 90 | $CH_3O$ | | 140 | $CH_3O$ | |
| 41 | H | 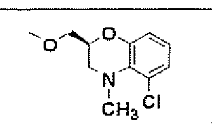 | 91 | H | 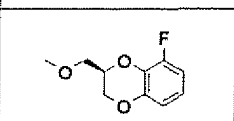 | 141 | H | 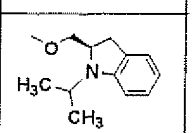 |
| 42 | F | | 92 | F | | 142 | F | |
| 43 | Cl | | 93 | Cl | | 143 | Cl | |
| 44 | $CH_3$ | | 94 | $CH_3$ | | 144 | $CH_3$ | |
| 45 | $CH_3O$ | | 95 | $CH_3O$ | | 145 | $CH_3O$ | |
| 46 | H | 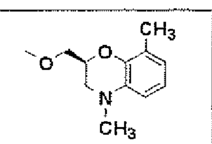 | 96 | H | 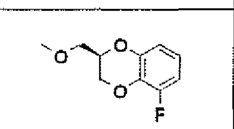 | 146 | H | 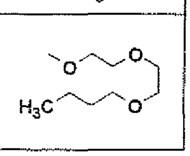 |
| 47 | F | | 97 | F | | 147 | F | |
| 48 | Cl | | 98 | Cl | | 148 | Cl | |
| 49 | $CH_3$ | | 99 | $CH_3$ | | 149 | $CH_3$ | |
| 50 | $CH_3O$ | | 100 | $CH_3O$ | | 150 | $CH_3O$ | |

Table 24

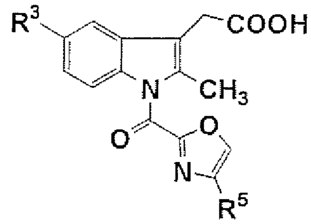

(I-A-11-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 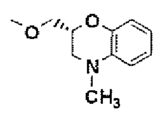 | 51<br>52<br>53<br>54<br>55 | H<br>F<br>Cl<br>$CH_3$<br>$CH_3O$ | 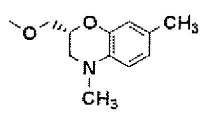 | 101<br>102<br>103<br>104<br>105 | H<br>F<br>Cl<br>$CH_3$<br>$CH_3O$ | 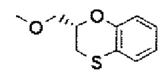 |
| 6<br>7<br>8<br>9<br>10 | H<br>F<br>Cl<br>$CH_3$<br>$CH_3O$ | 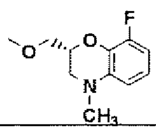 | 56<br>57<br>58<br>59<br>60 | H<br>F<br>Cl<br>$CH_3$<br>$CH_3O$ | 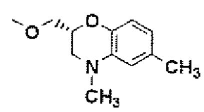 | 106<br>107<br>108<br>109<br>110 | H<br>F<br>Cl<br>$CH_3$<br>$CH_3O$ | 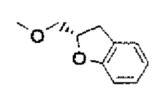 |
| 11<br>12<br>13<br>14<br>15 | H<br>F<br>Cl<br>$CH_3$<br>$CH_3O$ | 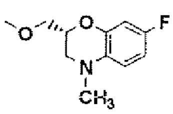 | 61<br>62<br>63<br>64<br>65 | H<br>F<br>Cl<br>$CH_3$<br>$CH_3O$ | 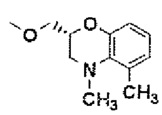 | 111<br>112<br>113<br>114<br>115 | H<br>F<br>Cl<br>$CH_3$<br>$CH_3O$ | 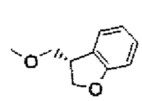 |
| 16<br>17<br>18<br>19<br>20 | H<br>F<br>Cl<br>$CH_3$<br>$CH_3O$ | 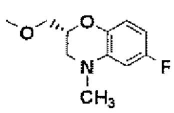 | 66<br>67<br>68<br>69<br>70 | H<br>F<br>Cl<br>$CH_3$<br>$CH_3O$ | 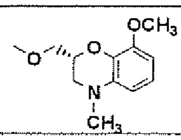 | 116<br>117<br>118<br>119<br>120 | H<br>F<br>Cl<br>$CH_3$<br>$CH_3O$ | 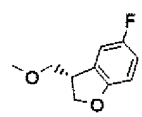 |
| 21<br>22<br>23<br>24<br>25 | H<br>F<br>Cl<br>$CH_3$<br>$CH_3O$ | 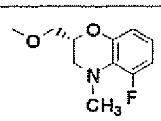 | 71<br>72<br>73<br>74<br>75 | H<br>F<br>Cl<br>$CH_3$<br>$CH_3O$ | 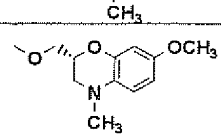 | 121<br>122<br>123<br>124<br>125 | H<br>F<br>Cl<br>$CH_3$<br>$CH_3O$ | 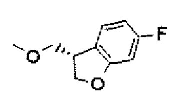 |
| 26<br>27<br>28<br>29<br>30 | H<br>F<br>Cl<br>$CH_3$<br>$CH_3O$ | 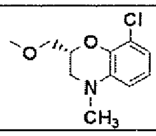 | 76<br>77<br>78<br>79<br>80 | H<br>F<br>Cl<br>$CH_3$<br>$CH_3O$ | 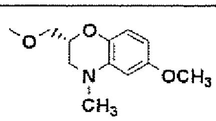 | 126<br>127<br>128<br>129<br>130 | H<br>F<br>Cl<br>$CH_3$<br>$CH_3O$ | 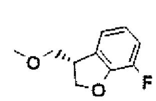 |
| 31<br>32<br>33<br>34<br>35 | H<br>F<br>Cl<br>$CH_3$<br>$CH_3O$ | 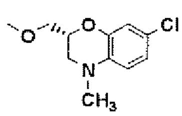 | 81<br>82<br>83<br>84<br>85 | H<br>F<br>Cl<br>$CH_3$<br>$CH_3O$ | 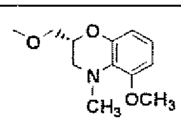 | 131<br>132<br>133<br>134<br>135 | H<br>F<br>Cl<br>$CH_3$<br>$CH_3O$ | 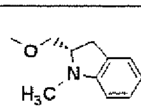 |
| 36<br>37<br>38<br>39<br>40 | H<br>F<br>Cl<br>$CH_3$<br>$CH_3O$ | 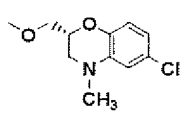 | 86<br>87<br>88<br>89<br>90 | H<br>F<br>Cl<br>$CH_3$<br>$CH_3O$ | 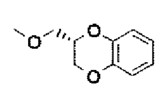 | 136<br>137<br>138<br>139<br>140 | H<br>F<br>Cl<br>$CH_3$<br>$CH_3O$ | 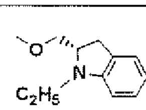 |
| 41<br>42<br>43<br>44<br>45 | H<br>F<br>Cl<br>$CH_3$<br>$CH_3O$ | 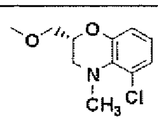 | 91<br>92<br>93<br>94<br>95 | H<br>F<br>Cl<br>$CH_3$<br>$CH_3O$ | 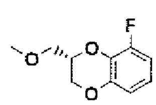 | 141<br>142<br>143<br>144<br>145 | H<br>F<br>Cl<br>$CH_3$<br>$CH_3O$ | 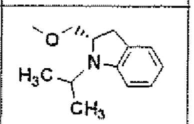 |
| 46<br>47<br>48<br>49<br>50 | H<br>F<br>Cl<br>$CH_3$<br>$CH_3O$ | 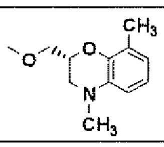 | 96<br>97<br>98<br>99<br>100 | H<br>F<br>Cl<br>$CH_3$<br>$CH_3O$ | 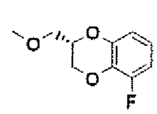 | 146<br>147<br>148<br>149<br>150 | H<br>F<br>Cl<br>$CH_3$<br>$CH_3O$ | 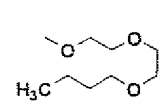 |

Table 25

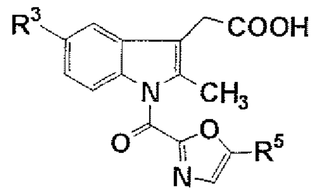

(I-A-11-2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 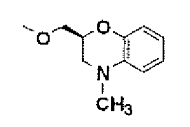 | 51<br>52<br>53<br>54<br>55 | H<br>F<br>Cl<br>$CH_3$<br>$CH_3O$ | 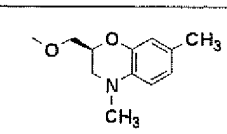 | 101<br>102<br>103<br>104<br>105 | H<br>F<br>Cl<br>$CH_3$<br>$CH_3O$ | 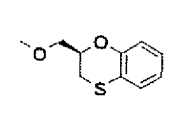 |
| 6<br>7<br>8<br>9<br>10 | H<br>F<br>Cl<br>$CH_3$<br>$CH_3O$ | 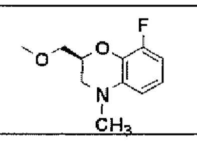 | 56<br>57<br>58<br>59<br>60 | H<br>F<br>Cl<br>$CH_3$<br>$CH_3O$ | 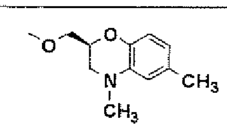 | 106<br>107<br>108<br>109<br>110 | H<br>F<br>Cl<br>$CH_3$<br>$CH_3O$ | 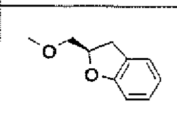 |
| 11<br>12<br>13<br>14<br>15 | H<br>F<br>Cl<br>$CH_3$<br>$CH_3O$ | 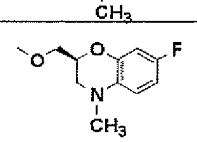 | 61<br>62<br>63<br>64<br>65 | H<br>F<br>Cl<br>$CH_3$<br>$CH_3O$ | 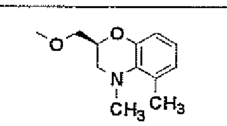 | 111<br>112<br>113<br>114<br>115 | H<br>F<br>Cl<br>$CH_3$<br>$CH_3O$ | 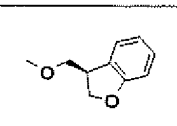 |
| 16<br>17<br>18<br>19<br>20 | H<br>F<br>Cl<br>$CH_3$<br>$CH_3O$ | 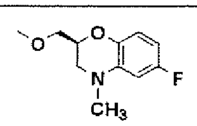 | 66<br>67<br>68<br>69<br>70 | H<br>F<br>Cl<br>$CH_3$<br>$CH_3O$ | 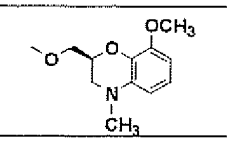 | 116<br>117<br>118<br>119<br>120 | H<br>F<br>Cl<br>$CH_3$<br>$CH_3O$ | 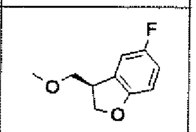 |
| 21<br>22<br>23<br>24<br>25 | H<br>F<br>Cl<br>$CH_3$<br>$CH_3O$ | 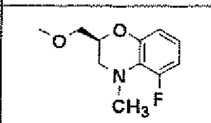 | 71<br>72<br>73<br>74<br>75 | H<br>F<br>Cl<br>$CH_3$<br>$CH_3O$ | 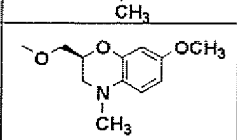 | 121<br>122<br>123<br>124<br>125 | H<br>F<br>Cl<br>$CH_3$<br>$CH_3O$ | 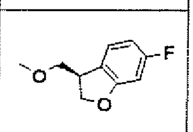 |
| 26<br>27<br>28<br>29<br>30 | H<br>F<br>Cl<br>$CH_3$<br>$CH_3O$ | 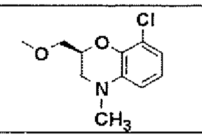 | 76<br>77<br>78<br>79<br>80 | H<br>F<br>Cl<br>$CH_3$<br>$CH_3O$ | 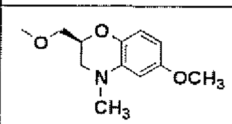 | 126<br>127<br>128<br>129<br>130 | H<br>F<br>Cl<br>$CH_3$<br>$CH_3O$ | 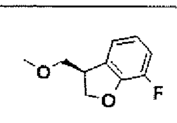 |
| 31<br>32<br>33<br>34<br>35 | H<br>F<br>Cl<br>$CH_3$<br>$CH_3O$ | 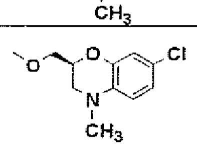 | 81<br>82<br>83<br>84<br>85 | H<br>F<br>Cl<br>$CH_3$<br>$CH_3O$ | 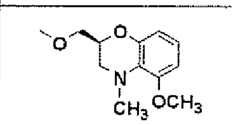 | 131<br>132<br>133<br>134<br>135 | H<br>F<br>Cl<br>$CH_3$<br>$CH_3O$ | 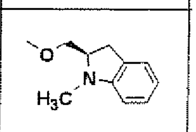 |
| 36<br>37<br>38<br>39<br>40 | H<br>F<br>Cl<br>$CH_3$<br>$CH_3O$ | 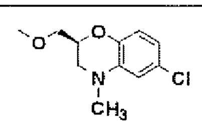 | 86<br>87<br>88<br>89<br>90 | H<br>F<br>Cl<br>$CH_3$<br>$CH_3O$ | 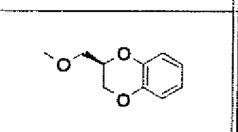 | 136<br>137<br>138<br>139<br>140 | H<br>F<br>Cl<br>$CH_3$<br>$CH_3O$ | 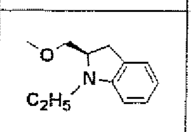 |
| 41<br>42<br>43<br>44<br>45 | H<br>F<br>Cl<br>$CH_3$<br>$CH_3O$ | 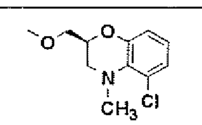 | 91<br>92<br>93<br>94<br>95 | H<br>F<br>Cl<br>$CH_3$<br>$CH_3O$ | 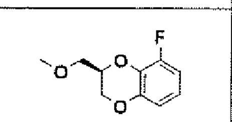 | 141<br>142<br>143<br>144<br>145 | H<br>F<br>Cl<br>$CH_3$<br>$CH_3O$ | 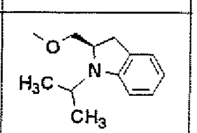 |
| 46<br>47<br>48<br>49<br>50 | H<br>F<br>Cl<br>$CH_3$<br>$CH_3O$ | 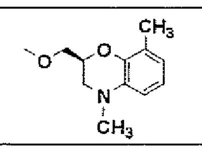 | 96<br>97<br>98<br>99<br>100 | H<br>F<br>Cl<br>$CH_3$<br>$CH_3O$ | 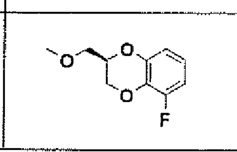 | 146<br>147<br>148<br>149<br>150 | H<br>F<br>Cl<br>$CH_3$<br>$CH_3O$ | 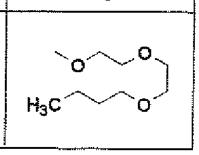 |

Table 26

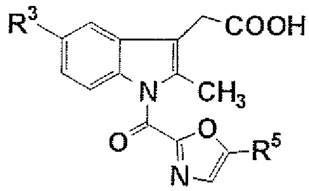

(I-A-II-2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 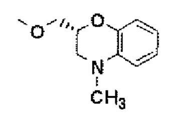 | 51<br>52<br>53<br>54<br>55 | H<br>F<br>Cl<br>$CH_3$<br>$CH_3O$ | 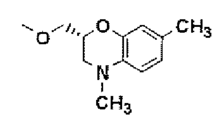 | 101<br>102<br>103<br>104<br>105 | H<br>F<br>Cl<br>$CH_3$<br>$CH_3O$ | 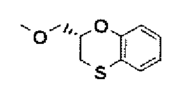 |
| 6<br>7<br>8<br>9<br>10 | H<br>F<br>Cl<br>$CH_3$<br>$CH_3O$ | 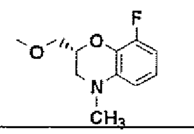 | 56<br>57<br>58<br>59<br>60 | H<br>F<br>Cl<br>$CH_3$<br>$CH_3O$ | 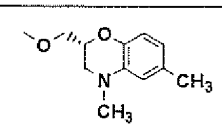 | 106<br>107<br>108<br>109<br>110 | H<br>F<br>Cl<br>$CH_3$<br>$CH_3O$ | 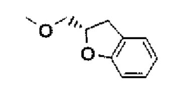 |
| 11<br>12<br>13<br>14<br>15 | H<br>F<br>Cl<br>$CH_3$<br>$CH_3O$ | 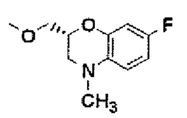 | 61<br>62<br>63<br>64<br>65 | H<br>F<br>Cl<br>$CH_3$<br>$CH_3O$ | 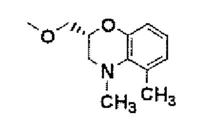 | 111<br>112<br>113<br>114<br>115 | H<br>F<br>Cl<br>$CH_3$<br>$CH_3O$ | 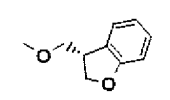 |
| 16<br>17<br>18<br>19<br>20 | H<br>F<br>Cl<br>$CH_3$<br>$CH_3O$ | 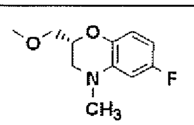 | 66<br>67<br>68<br>69<br>70 | H<br>F<br>Cl<br>$CH_3$<br>$CH_3O$ | 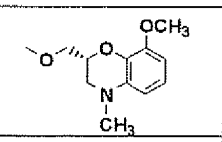 | 116<br>117<br>118<br>119<br>120 | H<br>F<br>Cl<br>$CH_3$<br>$CH_3O$ | 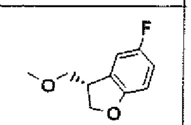 |
| 21<br>22<br>23<br>24<br>25 | H<br>F<br>Cl<br>$CH_3$<br>$CH_3O$ | 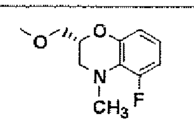 | 71<br>72<br>73<br>74<br>75 | H<br>F<br>Cl<br>$CH_3$<br>$CH_3O$ | 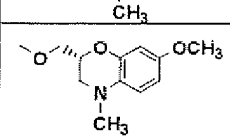 | 121<br>122<br>123<br>124<br>125 | H<br>F<br>Cl<br>$CH_3$<br>$CH_3O$ | 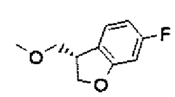 |
| 26<br>27<br>28<br>29<br>30 | H<br>F<br>Cl<br>$CH_3$<br>$CH_3O$ | 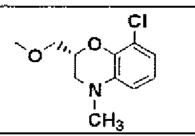 | 76<br>77<br>78<br>79<br>80 | H<br>F<br>Cl<br>$CH_3$<br>$CH_3O$ | 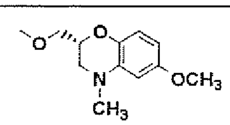 | 126<br>127<br>128<br>129<br>130 | H<br>F<br>Cl<br>$CH_3$<br>$CH_3O$ | 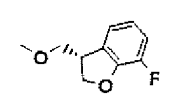 |
| 31<br>32<br>33<br>34<br>35 | H<br>F<br>Cl<br>$CH_3$<br>$CH_3O$ | 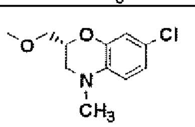 | 81<br>82<br>83<br>84<br>85 | H<br>F<br>Cl<br>$CH_3$<br>$CH_3O$ | 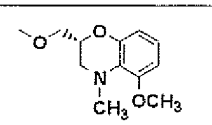 | 131<br>132<br>133<br>134<br>135 | H<br>F<br>Cl<br>$CH_3$<br>$CH_3O$ | 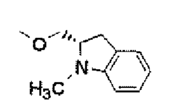 |
| 36<br>37<br>38<br>39<br>40 | H<br>F<br>Cl<br>$CH_3$<br>$CH_3O$ | 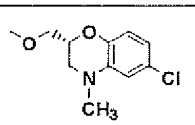 | 86<br>87<br>88<br>89<br>90 | H<br>F<br>Cl<br>$CH_3$<br>$CH_3O$ | 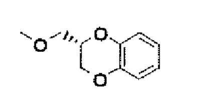 | 136<br>137<br>138<br>139<br>140 | H<br>F<br>Cl<br>$CH_3$<br>$CH_3O$ | 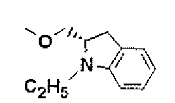 |
| 41<br>42<br>43<br>44<br>45 | H<br>F<br>Cl<br>$CH_3$<br>$CH_3O$ | 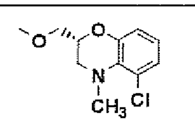 | 91<br>92<br>93<br>94<br>95 | H<br>F<br>Cl<br>$CH_3$<br>$CH_3O$ | 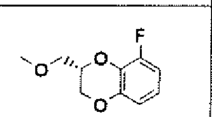 | 141<br>142<br>143<br>144<br>145 | H<br>F<br>Cl<br>$CH_3$<br>$CH_3O$ | 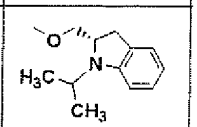 |
| 46<br>47<br>48<br>49<br>50 | H<br>F<br>Cl<br>$CH_3$<br>$CH_3O$ | 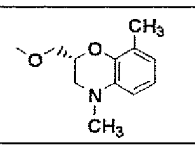 | 96<br>97<br>98<br>99<br>100 | H<br>F<br>Cl<br>$CH_3$<br>$CH_3O$ | 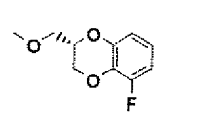 | 146<br>147<br>148<br>149<br>150 | H<br>F<br>Cl<br>$CH_3$<br>$CH_3O$ | 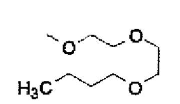 |

Table 27

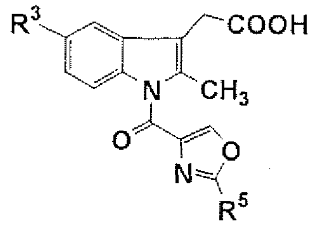

(I－A－1 2－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 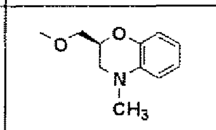 | 51<br>52<br>53<br>54<br>55 | H<br>F<br>Cl<br>$CH_3$<br>$CH_3O$ | 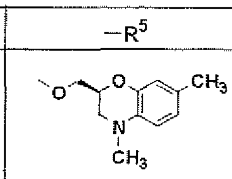 | 101<br>102<br>103<br>104<br>105 | H<br>F<br>Cl<br>$CH_3$<br>$CH_3O$ | 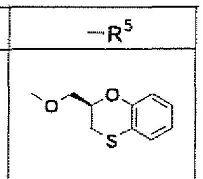 |
| 6<br>7<br>8<br>9<br>10 | H<br>F<br>Cl<br>$CH_3$<br>$CH_3O$ | 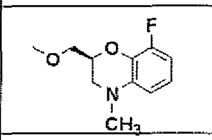 | 56<br>57<br>58<br>59<br>60 | H<br>F<br>Cl<br>$CH_3$<br>$CH_3O$ | 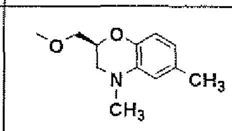 | 106<br>107<br>108<br>109<br>110 | H<br>F<br>Cl<br>$CH_3$<br>$CH_3O$ | 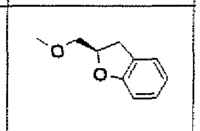 |
| 11<br>12<br>13<br>14<br>15 | H<br>F<br>Cl<br>$CH_3$<br>$CH_3O$ | 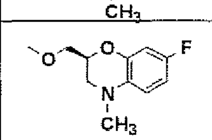 | 61<br>62<br>63<br>64<br>65 | H<br>F<br>Cl<br>$CH_3$<br>$CH_3O$ | 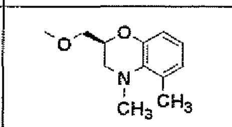 | 111<br>112<br>113<br>114<br>115 | H<br>F<br>Cl<br>$CH_3$<br>$CH_3O$ | 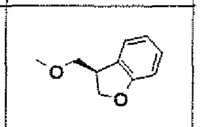 |
| 16<br>17<br>18<br>19<br>20 | H<br>F<br>Cl<br>$CH_3$<br>$CH_3O$ | 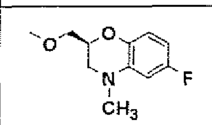 | 66<br>67<br>68<br>69<br>70 | H<br>F<br>Cl<br>$CH_3$<br>$CH_3O$ | 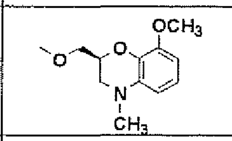 | 116<br>117<br>118<br>119<br>120 | H<br>F<br>Cl<br>$CH_3$<br>$CH_3O$ | 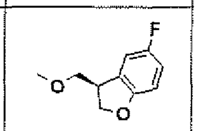 |
| 21<br>22<br>23<br>24<br>25 | H<br>F<br>Cl<br>$CH_3$<br>$CH_3O$ | 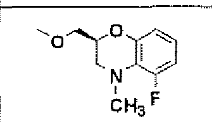 | 71<br>72<br>73<br>74<br>75 | H<br>F<br>Cl<br>$CH_3$<br>$CH_3O$ | 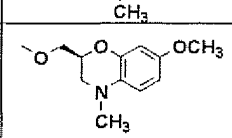 | 121<br>122<br>123<br>124<br>125 | H<br>F<br>Cl<br>$CH_3$<br>$CH_3O$ | 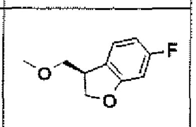 |
| 26<br>27<br>28<br>29<br>30 | H<br>F<br>Cl<br>$CH_3$<br>$CH_3O$ | 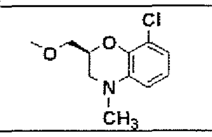 | 76<br>77<br>78<br>79<br>80 | H<br>F<br>Cl<br>$CH_3$<br>$CH_3O$ | 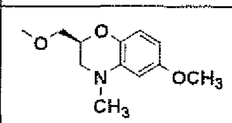 | 126<br>127<br>128<br>129<br>130 | H<br>F<br>Cl<br>$CH_3$<br>$CH_3O$ | 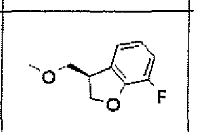 |
| 31<br>32<br>33<br>34<br>35 | H<br>F<br>Cl<br>$CH_3$<br>$CH_3O$ | 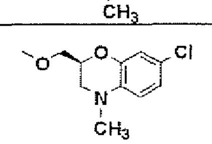 | 81<br>82<br>83<br>84<br>85 | H<br>F<br>Cl<br>$CH_3$<br>$CH_3O$ | 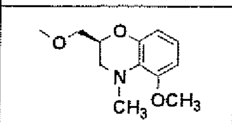 | 131<br>132<br>133<br>134<br>135 | H<br>F<br>Cl<br>$CH_3$<br>$CH_3O$ | 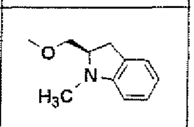 |
| 36<br>37<br>38<br>39<br>40 | H<br>F<br>Cl<br>$CH_3$<br>$CH_3O$ | 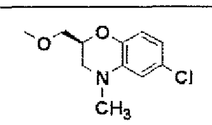 | 86<br>87<br>88<br>89<br>90 | H<br>F<br>Cl<br>$CH_3$<br>$CH_3O$ | 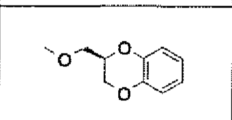 | 136<br>137<br>138<br>139<br>140 | H<br>F<br>Cl<br>$CH_3$<br>$CH_3O$ | 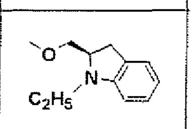 |
| 41<br>42<br>43<br>44<br>45 | H<br>F<br>Cl<br>$CH_3$<br>$CH_3O$ | 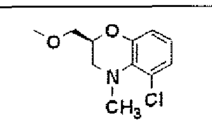 | 91<br>92<br>93<br>94<br>95 | H<br>F<br>Cl<br>$CH_3$<br>$CH_3O$ | 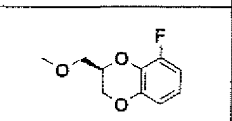 | 141<br>142<br>143<br>144<br>145 | H<br>F<br>Cl<br>$CH_3$<br>$CH_3O$ | 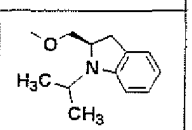 |
| 46<br>47<br>48<br>49<br>50 | H<br>F<br>Cl<br>$CH_3$<br>$CH_3O$ | 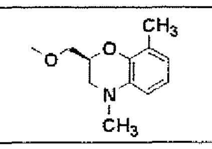 | 96<br>97<br>98<br>99<br>100 | H<br>F<br>Cl<br>$CH_3$<br>$CH_3O$ | 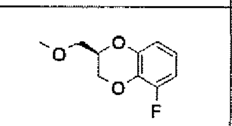 | 146<br>147<br>148<br>149<br>150 | H<br>F<br>Cl<br>$CH_3$<br>$CH_3O$ | 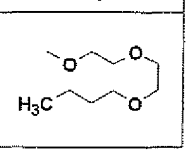 |

Table 28

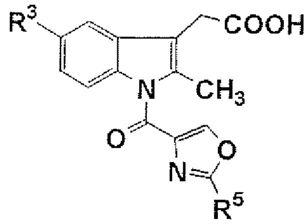

(1－A－12－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 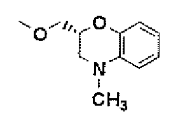 | 51<br>52<br>53<br>54<br>55 | H<br>F<br>Cl<br>$CH_3$<br>$CH_3O$ | 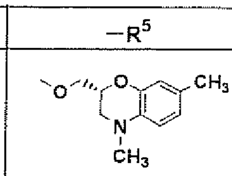 | 101<br>102<br>103<br>104<br>105 | H<br>F<br>Cl<br>$CH_3$<br>$CH_3O$ | 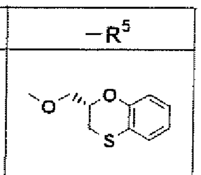 |
| 6<br>7<br>8<br>9<br>10 | H<br>F<br>Cl<br>$CH_3$<br>$CH_3O$ | 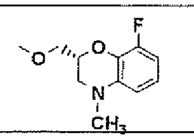 | 56<br>57<br>58<br>59<br>60 | H<br>F<br>Cl<br>$CH_3$<br>$CH_3O$ | 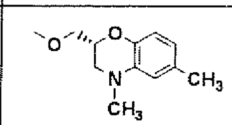 | 106<br>107<br>108<br>109<br>110 | H<br>F<br>Cl<br>$CH_3$<br>$CH_3O$ | 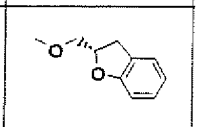 |
| 11<br>12<br>13<br>14<br>15 | H<br>F<br>Cl<br>$CH_3$<br>$CH_3O$ | 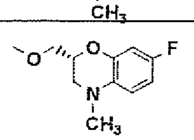 | 61<br>62<br>63<br>64<br>65 | H<br>F<br>Cl<br>$CH_3$<br>$CH_3O$ | 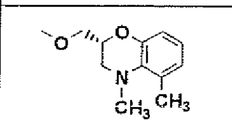 | 111<br>112<br>113<br>114<br>115 | H<br>F<br>Cl<br>$CH_3$<br>$CH_3O$ | 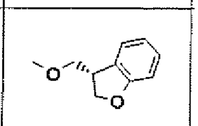 |
| 16<br>17<br>18<br>19<br>20 | H<br>F<br>Cl<br>$CH_3$<br>$CH_3O$ | 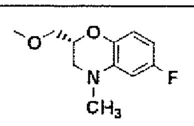 | 66<br>67<br>68<br>69<br>70 | H<br>F<br>Cl<br>$CH_3$<br>$CH_3O$ | 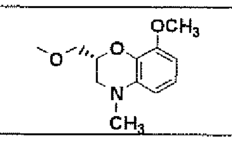 | 116<br>117<br>118<br>119<br>120 | H<br>F<br>Cl<br>$CH_3$<br>$CH_3O$ | 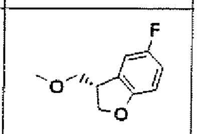 |
| 21<br>22<br>23<br>24<br>25 | H<br>F<br>Cl<br>$CH_3$<br>$CH_3O$ | 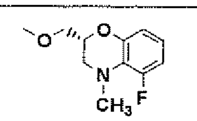 | 71<br>72<br>73<br>74<br>75 | H<br>F<br>Cl<br>$CH_3$<br>$CH_3O$ | 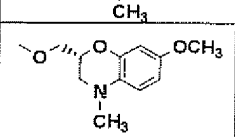 | 121<br>122<br>123<br>124<br>125 | H<br>F<br>Cl<br>$CH_3$<br>$CH_3O$ | 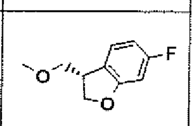 |
| 26<br>27<br>28<br>29<br>30 | H<br>F<br>Cl<br>$CH_3$<br>$CH_3O$ | 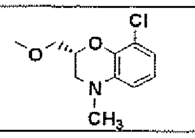 | 76<br>77<br>78<br>79<br>80 | H<br>F<br>Cl<br>$CH_3$<br>$CH_3O$ | 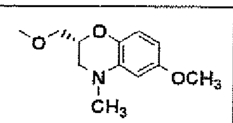 | 126<br>127<br>128<br>129<br>130 | H<br>F<br>Cl<br>$CH_3$<br>$CH_3O$ | 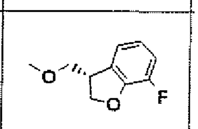 |
| 31<br>32<br>33<br>34<br>35 | H<br>F<br>Cl<br>$CH_3$<br>$CH_3O$ | 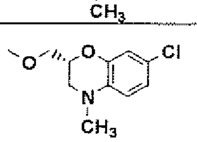 | 81<br>82<br>83<br>84<br>85 | H<br>F<br>Cl<br>$CH_3$<br>$CH_3O$ | 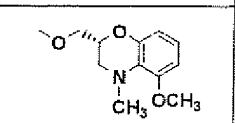 | 131<br>132<br>133<br>134<br>135 | H<br>F<br>Cl<br>$CH_3$<br>$CH_3O$ | 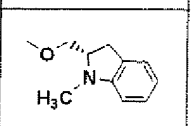 |
| 36<br>37<br>38<br>39<br>40 | H<br>F<br>Cl<br>$CH_3$<br>$CH_3O$ | 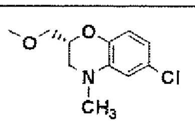 | 86<br>87<br>88<br>89<br>90 | H<br>F<br>Cl<br>$CH_3$<br>$CH_3O$ | 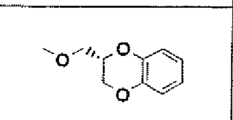 | 136<br>137<br>138<br>139<br>140 | H<br>F<br>Cl<br>$CH_3$<br>$CH_3O$ | 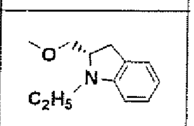 |
| 41<br>42<br>43<br>44<br>45 | H<br>F<br>Cl<br>$CH_3$<br>$CH_3O$ | 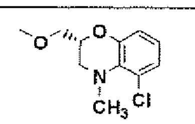 | 91<br>92<br>93<br>94<br>95 | H<br>F<br>Cl<br>$CH_3$<br>$CH_3O$ | 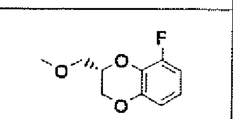 | 141<br>142<br>143<br>144<br>145 | H<br>F<br>Cl<br>$CH_3$<br>$CH_3O$ | 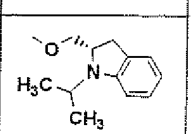 |
| 46<br>47<br>48<br>49<br>50 | H<br>F<br>Cl<br>$CH_3$<br>$CH_3O$ | 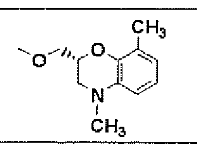 | 96<br>97<br>98<br>99<br>100 | H<br>F<br>Cl<br>$CH_3$<br>$CH_3O$ | 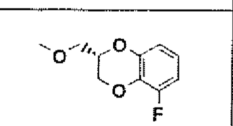 | 146<br>147<br>148<br>149<br>150 | H<br>F<br>Cl<br>$CH_3$<br>$CH_3O$ | 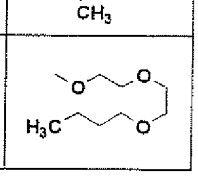 |

Table 29

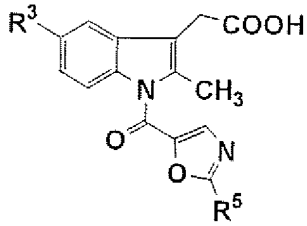

(1-A-13-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 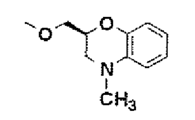 | 51<br>52<br>53<br>54<br>55 | H<br>F<br>Cl<br>$CH_3$<br>$CH_3O$ | 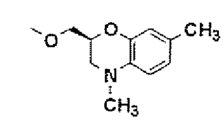 | 101<br>102<br>103<br>104<br>105 | H<br>F<br>Cl<br>$CH_3$<br>$CH_3O$ | 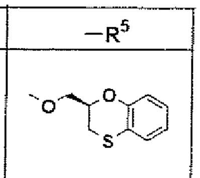 |
| 6<br>7<br>8<br>9<br>10 | H<br>F<br>Cl<br>$CH_3$<br>$CH_3O$ | 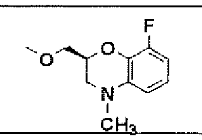 | 56<br>57<br>58<br>59<br>60 | H<br>F<br>Cl<br>$CH_3$<br>$CH_3O$ | 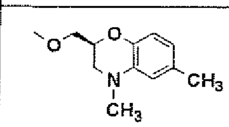 | 106<br>107<br>108<br>109<br>110 | H<br>F<br>Cl<br>$CH_3$<br>$CH_3O$ | 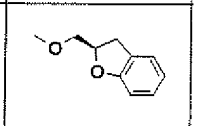 |
| 11<br>12<br>13<br>14<br>15 | H<br>F<br>Cl<br>$CH_3$<br>$CH_3O$ | 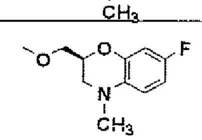 | 61<br>62<br>63<br>64<br>65 | H<br>F<br>Cl<br>$CH_3$<br>$CH_3O$ | 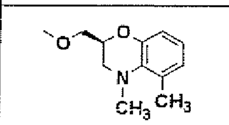 | 111<br>112<br>113<br>114<br>115 | H<br>F<br>Cl<br>$CH_3$<br>$CH_3O$ | 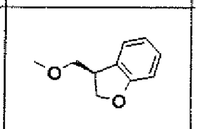 |
| 16<br>17<br>18<br>19<br>20 | H<br>F<br>Cl<br>$CH_3$<br>$CH_3O$ | 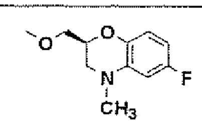 | 66<br>67<br>68<br>69<br>70 | H<br>F<br>Cl<br>$CH_3$<br>$CH_3O$ | 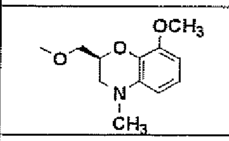 | 116<br>117<br>118<br>119<br>120 | H<br>F<br>Cl<br>$CH_3$<br>$CH_3O$ | 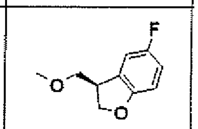 |
| 21<br>22<br>23<br>24<br>25 | H<br>F<br>Cl<br>$CH_3$<br>$CH_3O$ | 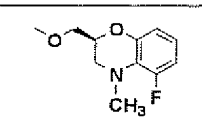 | 71<br>72<br>73<br>74<br>75 | H<br>F<br>Cl<br>$CH_3$<br>$CH_3O$ | 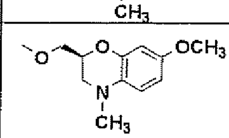 | 121<br>122<br>123<br>124<br>125 | H<br>F<br>Cl<br>$CH_3$<br>$CH_3O$ | 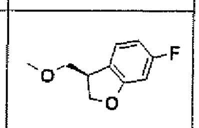 |
| 26<br>27<br>28<br>29<br>30 | H<br>F<br>Cl<br>$CH_3$<br>$CH_3O$ | 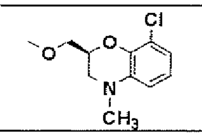 | 76<br>77<br>78<br>79<br>80 | H<br>F<br>Cl<br>$CH_3$<br>$CH_3O$ | 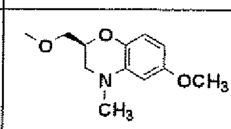 | 126<br>127<br>128<br>129<br>130 | H<br>F<br>Cl<br>$CH_3$<br>$CH_3O$ | 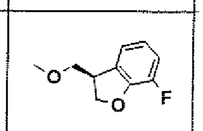 |
| 31<br>32<br>33<br>34<br>35 | H<br>F<br>Cl<br>$CH_3$<br>$CH_3O$ | 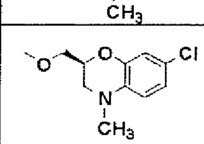 | 81<br>82<br>83<br>84<br>85 | H<br>F<br>Cl<br>$CH_3$<br>$CH_3O$ | 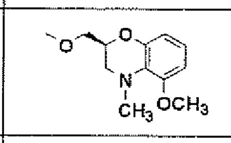 | 131<br>132<br>133<br>134<br>135 | H<br>F<br>Cl<br>$CH_3$<br>$CH_3O$ | 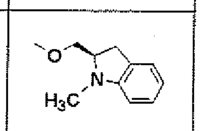 |
| 36<br>37<br>38<br>39<br>40 | H<br>F<br>Cl<br>$CH_3$<br>$CH_3O$ | 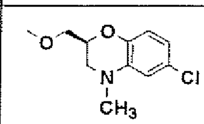 | 86<br>87<br>88<br>89<br>90 | H<br>F<br>Cl<br>$CH_3$<br>$CH_3O$ | 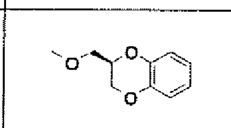 | 136<br>137<br>138<br>139<br>140 | H<br>F<br>Cl<br>$CH_3$<br>$CH_3O$ | 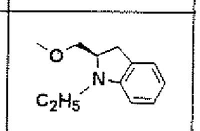 |
| 41<br>42<br>43<br>44<br>45 | H<br>F<br>Cl<br>$CH_3$<br>$CH_3O$ | 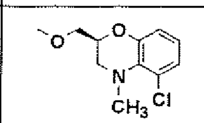 | 91<br>92<br>93<br>94<br>95 | H<br>F<br>Cl<br>$CH_3$<br>$CH_3O$ | 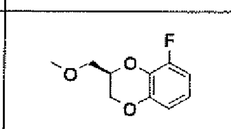 | 141<br>142<br>143<br>144<br>145 | H<br>F<br>Cl<br>$CH_3$<br>$CH_3O$ | 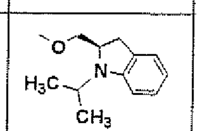 |
| 46<br>47<br>48<br>49<br>50 | H<br>F<br>Cl<br>$CH_3$<br>$CH_3O$ | 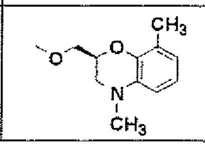 | 96<br>97<br>98<br>99<br>100 | H<br>F<br>Cl<br>$CH_3$<br>$CH_3O$ | 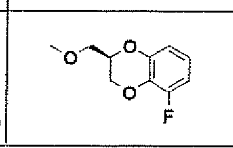 | 146<br>147<br>148<br>149<br>150 | H<br>F<br>Cl<br>$CH_3$<br>$CH_3O$ | 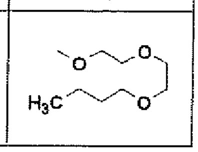 |

Table 30

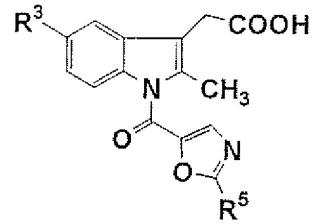

(I-A-13-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 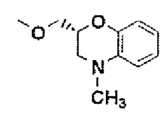 | 51<br>52<br>53<br>54<br>55 | H<br>F<br>Cl<br>$CH_3$<br>$CH_3O$ | 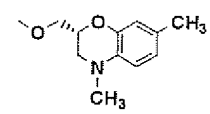 | 101<br>102<br>103<br>104<br>105 | H<br>F<br>Cl<br>$CH_3$<br>$CH_3O$ | 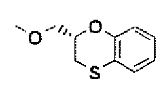 |
| 6<br>7<br>8<br>9<br>10 | H<br>F<br>Cl<br>$CH_3$<br>$CH_3O$ | 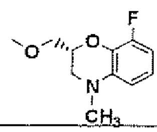 | 56<br>57<br>58<br>59<br>60 | H<br>F<br>Cl<br>$CH_3$<br>$CH_3O$ | 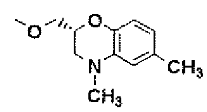 | 106<br>107<br>108<br>109<br>110 | H<br>F<br>Cl<br>$CH_3$<br>$CH_3O$ | 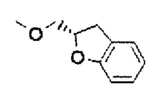 |
| 11<br>12<br>13<br>14<br>15 | H<br>F<br>Cl<br>$CH_3$<br>$CH_3O$ | 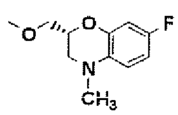 | 61<br>62<br>63<br>64<br>65 | H<br>F<br>Cl<br>$CH_3$<br>$CH_3O$ | 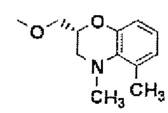 | 111<br>112<br>113<br>114<br>115 | H<br>F<br>Cl<br>$CH_3$<br>$CH_3O$ | 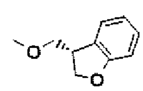 |
| 16<br>17<br>18<br>19<br>20 | H<br>F<br>Cl<br>$CH_3$<br>$CH_3O$ | 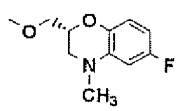 | 66<br>67<br>68<br>69<br>70 | H<br>F<br>Cl<br>$CH_3$<br>$CH_3O$ | 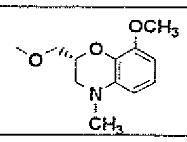 | 116<br>117<br>118<br>119<br>120 | H<br>F<br>Cl<br>$CH_3$<br>$CH_3O$ | 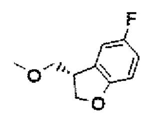 |
| 21<br>22<br>23<br>24<br>25 | H<br>F<br>Cl<br>$CH_3$<br>$CH_3O$ | 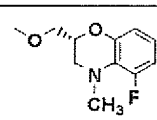 | 71<br>72<br>73<br>74<br>75 | H<br>F<br>Cl<br>$CH_3$<br>$CH_3O$ | 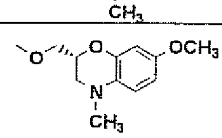 | 121<br>122<br>123<br>124<br>125 | H<br>F<br>Cl<br>$CH_3$<br>$CH_3O$ | 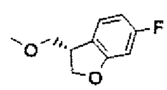 |
| 26<br>27<br>28<br>29<br>30 | H<br>F<br>Cl<br>$CH_3$<br>$CH_3O$ | 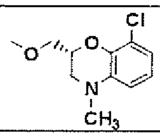 | 76<br>77<br>78<br>79<br>80 | H<br>F<br>Cl<br>$CH_3$<br>$CH_3O$ | 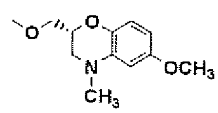 | 126<br>127<br>128<br>129<br>130 | H<br>F<br>Cl<br>$CH_3$<br>$CH_3O$ | 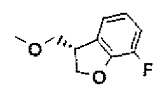 |
| 31<br>32<br>33<br>34<br>35 | H<br>F<br>Cl<br>$CH_3$<br>$CH_3O$ | 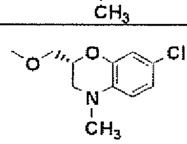 | 81<br>82<br>83<br>84<br>85 | H<br>F<br>Cl<br>$CH_3$<br>$CH_3O$ | 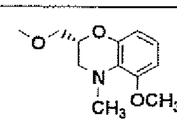 | 131<br>132<br>133<br>134<br>135 | H<br>F<br>Cl<br>$CH_3$<br>$CH_3O$ | 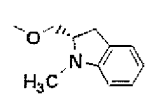 |
| 36<br>37<br>38<br>39<br>40 | H<br>F<br>Cl<br>$CH_3$<br>$CH_3O$ | 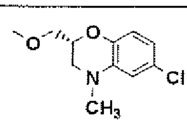 | 86<br>87<br>88<br>89<br>90 | H<br>F<br>Cl<br>$CH_3$<br>$CH_3O$ | 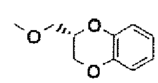 | 136<br>137<br>138<br>139<br>140 | H<br>F<br>Cl<br>$CH_3$<br>$CH_3O$ | 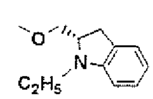 |
| 41<br>42<br>43<br>44<br>45 | H<br>F<br>Cl<br>$CH_3$<br>$CH_3O$ | 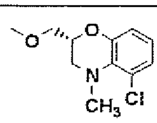 | 91<br>92<br>93<br>94<br>95 | H<br>F<br>Cl<br>$CH_3$<br>$CH_3O$ | 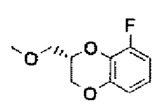 | 141<br>142<br>143<br>144<br>145 | H<br>F<br>Cl<br>$CH_3$<br>$CH_3O$ | 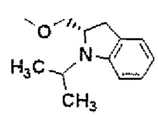 |
| 46<br>47<br>48<br>49<br>50 | H<br>F<br>Cl<br>$CH_3$<br>$CH_3O$ | 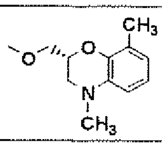 | 96<br>97<br>98<br>99<br>100 | H<br>F<br>Cl<br>$CH_3$<br>$CH_3O$ | 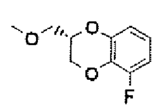 | 146<br>147<br>148<br>149<br>150 | H<br>F<br>Cl<br>$CH_3$<br>$CH_3O$ | 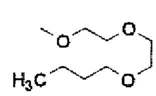 |

Table 31

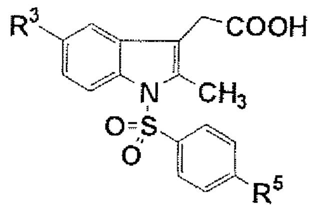

(I－B－1－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 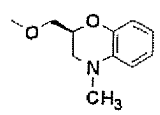 | 51<br>52<br>53<br>54<br>55 | H<br>F<br>Cl<br>$CH_3$<br>$CH_3O$ | 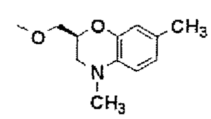 | 101<br>102<br>103<br>104<br>105 | H<br>F<br>Cl<br>$CH_3$<br>$CH_3O$ | 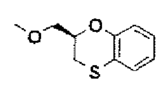 |
| 6<br>7<br>8<br>9<br>10 | H<br>F<br>Cl<br>$CH_3$<br>$CH_3O$ | 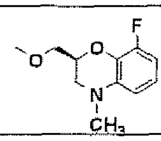 | 56<br>57<br>58<br>59<br>60 | H<br>F<br>Cl<br>$CH_3$<br>$CH_3O$ | 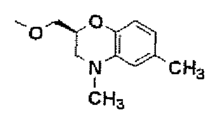 | 106<br>107<br>108<br>109<br>110 | H<br>F<br>Cl<br>$CH_3$<br>$CH_3O$ | 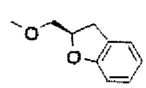 |
| 11<br>12<br>13<br>14<br>15 | H<br>F<br>Cl<br>$CH_3$<br>$CH_3O$ | 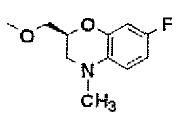 | 61<br>62<br>63<br>64<br>65 | H<br>F<br>Cl<br>$CH_3$<br>$CH_3O$ | 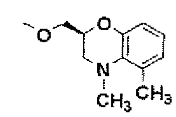 | 111<br>112<br>113<br>114<br>115 | H<br>F<br>Cl<br>$CH_3$<br>$CH_3O$ | 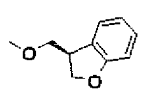 |
| 16<br>17<br>18<br>19<br>20 | H<br>F<br>Cl<br>$CH_3$<br>$CH_3O$ | 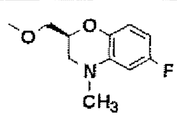 | 66<br>67<br>68<br>69<br>70 | H<br>F<br>Cl<br>$CH_3$<br>$CH_3O$ | 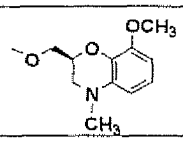 | 116<br>117<br>118<br>119<br>120 | H<br>F<br>Cl<br>$CH_3$<br>$CH_3O$ | 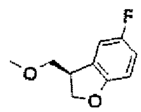 |
| 21<br>22<br>23<br>24<br>25 | H<br>F<br>Cl<br>$CH_3$<br>$CH_3O$ | 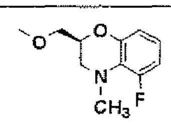 | 71<br>72<br>73<br>74<br>75 | H<br>F<br>Cl<br>$CH_3$<br>$CH_3O$ | 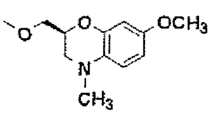 | 121<br>122<br>123<br>124<br>125 | H<br>F<br>Cl<br>$CH_3$<br>$CH_3O$ | 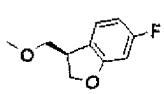 |
| 26<br>27<br>28<br>29<br>30 | H<br>F<br>Cl<br>$CH_3$<br>$CH_3O$ | 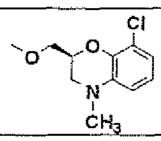 | 76<br>77<br>78<br>79<br>80 | H<br>F<br>Cl<br>$CH_3$<br>$CH_3O$ | 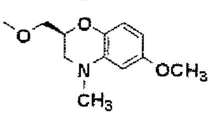 | 126<br>127<br>128<br>129<br>130 | H<br>F<br>Cl<br>$CH_3$<br>$CH_3O$ | 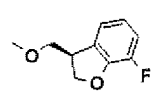 |
| 31<br>32<br>33<br>34<br>35 | H<br>F<br>Cl<br>$CH_3$<br>$CH_3O$ | 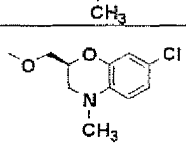 | 81<br>82<br>83<br>84<br>85 | H<br>F<br>Cl<br>$CH_3$<br>$CH_3O$ | 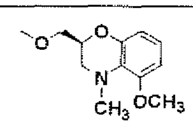 | 131<br>132<br>133<br>134<br>135 | H<br>F<br>Cl<br>$CH_3$<br>$CH_3O$ | 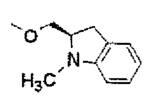 |
| 36<br>37<br>38<br>39<br>40 | H<br>F<br>Cl<br>$CH_3$<br>$CH_3O$ | 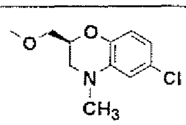 | 86<br>87<br>88<br>89<br>90 | H<br>F<br>Cl<br>$CH_3$<br>$CH_3O$ | 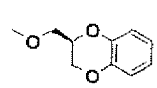 | 136<br>137<br>138<br>139<br>140 | H<br>F<br>Cl<br>$CH_3$<br>$CH_3O$ | 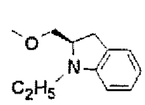 |
| 41<br>42<br>43<br>44<br>45 | H<br>F<br>Cl<br>$CH_3$<br>$CH_3O$ | 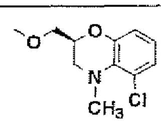 | 91<br>92<br>93<br>94<br>95 | H<br>F<br>Cl<br>$CH_3$<br>$CH_3O$ | 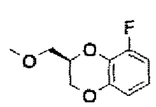 | 141<br>142<br>143<br>144<br>145 | H<br>F<br>Cl<br>$CH_3$<br>$CH_3O$ | 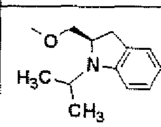 |
| 46<br>47<br>48<br>49<br>50 | H<br>F<br>Cl<br>$CH_3$<br>$CH_3O$ | 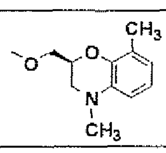 | 96<br>97<br>98<br>99<br>100 | H<br>F<br>Cl<br>$CH_3$<br>$CH_3O$ | 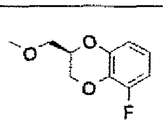 | 146<br>147<br>148<br>149<br>150 | H<br>F<br>Cl<br>$CH_3$<br>$CH_3O$ | 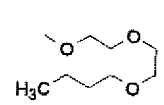 |

Table 32

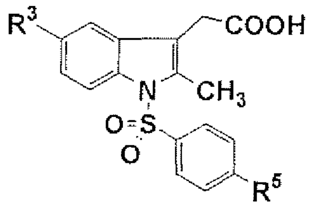

(1 - B - 1 - 1)

| No. R3 | $-R^5$ | No. R3 | $-R^5$ | No. R3 | $-R^5$ |
|---|---|---|---|---|---|
| 1 H<br>2 F<br>3 Cl<br>4 $CH_3$<br>5 $CH_3O$ | 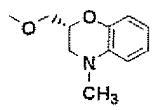 | 51 H<br>52 F<br>53 Cl<br>54 $CH_3$<br>55 $CH_3O$ | 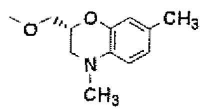 | 101 H<br>102 F<br>103 Cl<br>104 $CH_3$<br>105 $CH_3O$ | 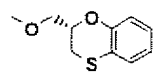 |
| 6 H<br>7 F<br>8 Cl<br>9 $CH_3$<br>10 $CH_3O$ | 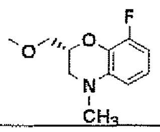 | 56 H<br>57 F<br>58 Cl<br>59 $CH_3$<br>60 $CH_3O$ | 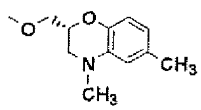 | 106 H<br>107 F<br>108 Cl<br>109 $CH_3$<br>110 $CH_3O$ | 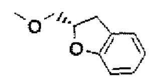 |
| 11 H<br>12 F<br>13 Cl<br>14 $CH_3$<br>15 $CH_3O$ | 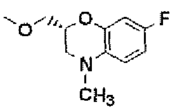 | 61 H<br>62 F<br>63 Cl<br>64 $CH_3$<br>65 $CH_3O$ | 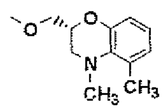 | 111 H<br>112 F<br>113 Cl<br>114 $CH_3$<br>115 $CH_3O$ | 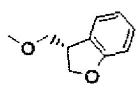 |
| 16 H<br>17 F<br>18 Cl<br>19 $CH_3$<br>20 $CH_3O$ | 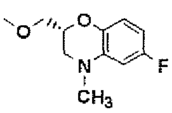 | 66 H<br>67 F<br>68 Cl<br>69 $CH_3$<br>70 $CH_3O$ | 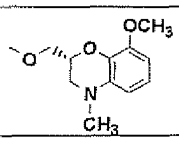 | 116 H<br>117 F<br>118 Cl<br>119 $CH_3$<br>120 $CH_3O$ | 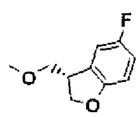 |
| 21 H<br>22 F<br>23 Cl<br>24 $CH_3$<br>25 $CH_3O$ | 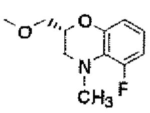 | 71 H<br>72 F<br>73 Cl<br>74 $CH_3$<br>75 $CH_3O$ | 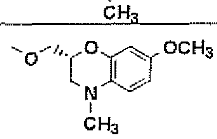 | 121 H<br>122 F<br>123 Cl<br>124 $CH_3$<br>125 $CH_3O$ | 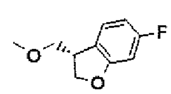 |
| 26 H<br>27 F<br>28 Cl<br>29 $CH_3$<br>30 $CH_3O$ | 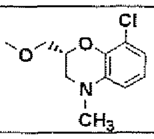 | 76 H<br>77 F<br>78 Cl<br>79 $CH_3$<br>80 $CH_3O$ | 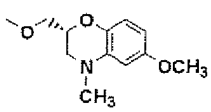 | 126 H<br>127 F<br>128 Cl<br>129 $CH_3$<br>130 $CH_3O$ | 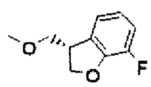 |
| 31 H<br>32 F<br>33 Cl<br>34 $CH_3$<br>35 $CH_3O$ | 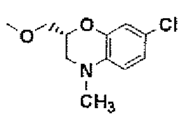 | 81 H<br>82 F<br>83 Cl<br>84 $CH_3$<br>85 $CH_3O$ | 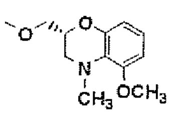 | 131 H<br>132 F<br>133 Cl<br>134 $CH_3$<br>135 $CH_3O$ | 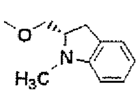 |
| 36 H<br>37 F<br>38 Cl<br>39 $CH_3$<br>40 $CH_3O$ | 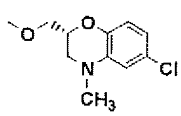 | 86 H<br>87 F<br>88 Cl<br>89 $CH_3$<br>90 $CH_3O$ | 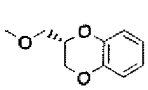 | 136 H<br>137 F<br>138 Cl<br>139 $CH_3$<br>140 $CH_3O$ | 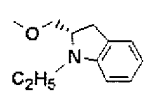 |
| 41 H<br>42 F<br>43 Cl<br>44 $CH_3$<br>45 $CH_3O$ | 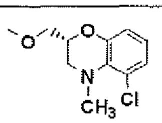 | 91 H<br>92 F<br>93 Cl<br>94 $CH_3$<br>95 $CH_3O$ | 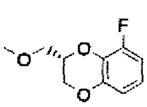 | 141 H<br>142 F<br>143 Cl<br>144 $CH_3$<br>145 $CH_3O$ | 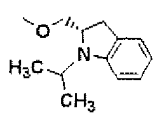 |
| 46 H<br>47 F<br>48 Cl<br>49 $CH_3$<br>50 $CH_3O$ | 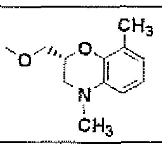 | 96 H<br>97 F<br>98 Cl<br>99 $CH_3$<br>100 $CH_3O$ | 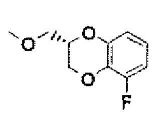 | 146 H<br>147 F<br>148 Cl<br>149 $CH_3$<br>150 $CH_3O$ | 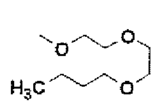 |

Table 33

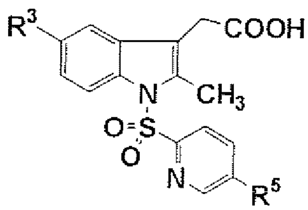

(I-B-2-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 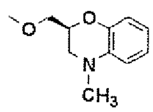 | 51<br>52<br>53<br>54<br>55 | H<br>F<br>Cl<br>$CH_3$<br>$CH_3O$ | 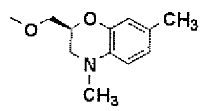 | 101<br>102<br>103<br>104<br>105 | H<br>F<br>Cl<br>$CH_3$<br>$CH_3O$ | 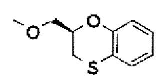 |
| 6<br>7<br>8<br>9<br>10 | H<br>F<br>Cl<br>$CH_3$<br>$CH_3O$ | 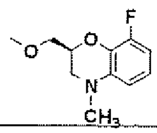 | 56<br>57<br>58<br>59<br>60 | H<br>F<br>Cl<br>$CH_3$<br>$CH_3O$ | 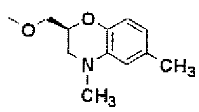 | 106<br>107<br>108<br>109<br>110 | H<br>F<br>Cl<br>$CH_3$<br>$CH_3O$ | 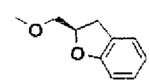 |
| 11<br>12<br>13<br>14<br>15 | H<br>F<br>Cl<br>$CH_3$<br>$CH_3O$ | 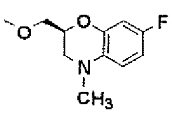 | 61<br>62<br>63<br>64<br>65 | H<br>F<br>Cl<br>$CH_3$<br>$CH_3O$ | 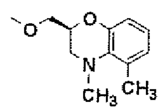 | 111<br>112<br>113<br>114<br>115 | H<br>F<br>Cl<br>$CH_3$<br>$CH_3O$ | 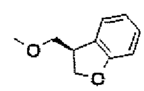 |
| 16<br>17<br>18<br>19<br>20 | H<br>F<br>Cl<br>$CH_3$<br>$CH_3O$ | 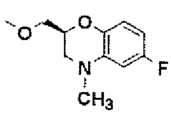 | 66<br>67<br>68<br>69<br>70 | H<br>F<br>Cl<br>$CH_3$<br>$CH_3O$ | 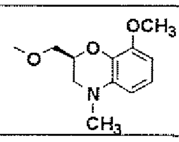 | 116<br>117<br>118<br>119<br>120 | H<br>F<br>Cl<br>$CH_3$<br>$CH_3O$ | 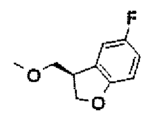 |
| 21<br>22<br>23<br>24<br>25 | H<br>F<br>Cl<br>$CH_3$<br>$CH_3O$ | 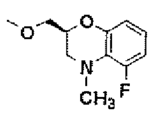 | 71<br>72<br>73<br>74<br>75 | H<br>F<br>Cl<br>$CH_3$<br>$CH_3O$ | 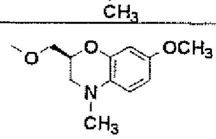 | 121<br>122<br>123<br>124<br>125 | H<br>F<br>Cl<br>$CH_3$<br>$CH_3O$ | 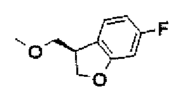 |
| 26<br>27<br>28<br>29<br>30 | H<br>F<br>Cl<br>$CH_3$<br>$CH_3O$ | 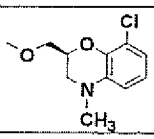 | 76<br>77<br>78<br>79<br>80 | H<br>F<br>Cl<br>$CH_3$<br>$CH_3O$ | 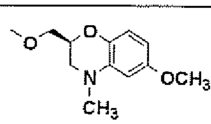 | 126<br>127<br>128<br>129<br>130 | H<br>F<br>Cl<br>$CH_3$<br>$CH_3O$ | 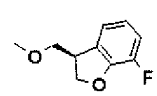 |
| 31<br>32<br>33<br>34<br>35 | H<br>F<br>Cl<br>$CH_3$<br>$CH_3O$ | 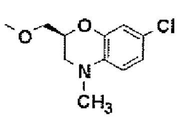 | 81<br>82<br>83<br>84<br>85 | H<br>F<br>Cl<br>$CH_3$<br>$CH_3O$ | 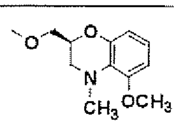 | 131<br>132<br>133<br>134<br>135 | H<br>F<br>Cl<br>$CH_3$<br>$CH_3O$ | 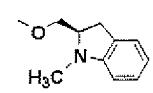 |
| 36<br>37<br>38<br>39<br>40 | H<br>F<br>Cl<br>$CH_3$<br>$CH_3O$ | 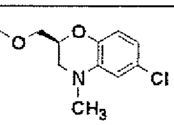 | 86<br>87<br>88<br>89<br>90 | H<br>F<br>Cl<br>$CH_3$<br>$CH_3O$ | 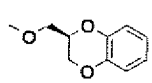 | 136<br>137<br>138<br>139<br>140 | H<br>F<br>Cl<br>$CH_3$<br>$CH_3O$ | 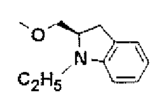 |
| 41<br>42<br>43<br>44<br>45 | H<br>F<br>Cl<br>$CH_3$<br>$CH_3O$ | 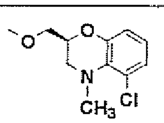 | 91<br>92<br>93<br>94<br>95 | H<br>F<br>Cl<br>$CH_3$<br>$CH_3O$ | 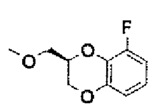 | 141<br>142<br>143<br>144<br>145 | H<br>F<br>Cl<br>$CH_3$<br>$CH_3O$ | 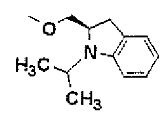 |
| 46<br>47<br>48<br>49<br>50 | H<br>F<br>Cl<br>$CH_3$<br>$CH_3O$ | 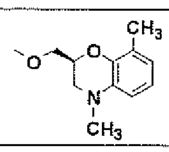 | 96<br>97<br>98<br>99<br>100 | H<br>F<br>Cl<br>$CH_3$<br>$CH_3O$ | 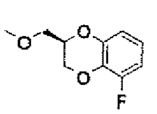 | 146<br>147<br>148<br>149<br>150 | H<br>F<br>Cl<br>$CH_3$<br>$CH_3O$ | 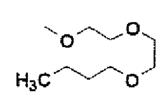 |

Table 34

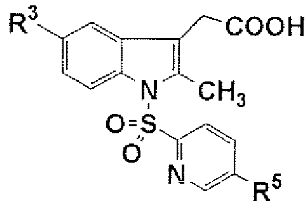

(I-B-2-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 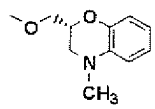 | 51<br>52<br>53<br>54<br>55 | H<br>F<br>Cl<br>$CH_3$<br>$CH_3O$ | 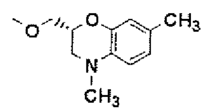 | 101<br>102<br>103<br>104<br>105 | H<br>F<br>Cl<br>$CH_3$<br>$CH_3O$ | 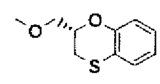 |
| 6<br>7<br>8<br>9<br>10 | H<br>F<br>Cl<br>$CH_3$<br>$CH_3O$ | 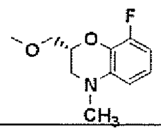 | 56<br>57<br>58<br>59<br>60 | H<br>F<br>Cl<br>$CH_3$<br>$CH_3O$ | 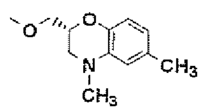 | 106<br>107<br>108<br>109<br>110 | H<br>F<br>Cl<br>$CH_3$<br>$CH_3O$ | 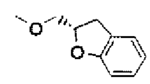 |
| 11<br>12<br>13<br>14<br>15 | H<br>F<br>Cl<br>$CH_3$<br>$CH_3O$ | 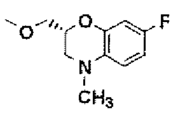 | 61<br>62<br>63<br>64<br>65 | H<br>F<br>Cl<br>$CH_3$<br>$CH_3O$ | 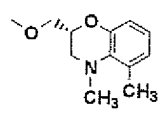 | 111<br>112<br>113<br>114<br>115 | H<br>F<br>Cl<br>$CH_3$<br>$CH_3O$ | 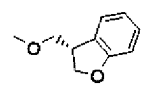 |
| 16<br>17<br>18<br>19<br>20 | H<br>F<br>Cl<br>$CH_3$<br>$CH_3O$ | 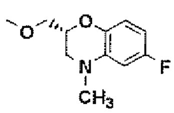 | 66<br>67<br>68<br>69<br>70 | H<br>F<br>Cl<br>$CH_3$<br>$CH_3O$ | 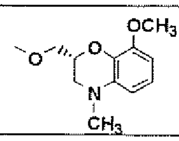 | 116<br>117<br>118<br>119<br>120 | H<br>F<br>Cl<br>$CH_3$<br>$CH_3O$ | 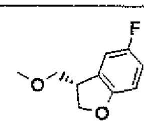 |
| 21<br>22<br>23<br>24<br>25 | H<br>F<br>Cl<br>$CH_3$<br>$CH_3O$ | 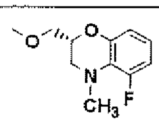 | 71<br>72<br>73<br>74<br>75 | H<br>F<br>Cl<br>$CH_3$<br>$CH_3O$ | 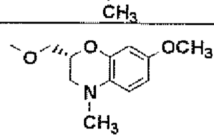 | 121<br>122<br>123<br>124<br>125 | H<br>F<br>Cl<br>$CH_3$<br>$CH_3O$ | 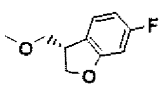 |
| 26<br>27<br>28<br>29<br>30 | H<br>F<br>Cl<br>$CH_3$<br>$CH_3O$ | 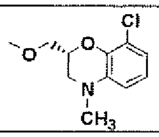 | 76<br>77<br>78<br>79<br>80 | H<br>F<br>Cl<br>$CH_3$<br>$CH_3O$ | 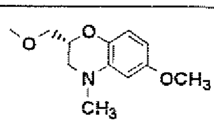 | 126<br>127<br>128<br>129<br>130 | H<br>F<br>Cl<br>$CH_3$<br>$CH_3O$ | 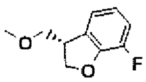 |
| 31<br>32<br>33<br>34<br>35 | H<br>F<br>Cl<br>$CH_3$<br>$CH_3O$ | 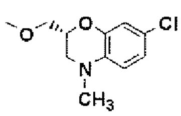 | 81<br>82<br>83<br>84<br>85 | H<br>F<br>Cl<br>$CH_3$<br>$CH_3O$ | 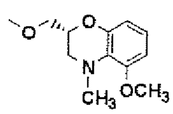 | 131<br>132<br>133<br>134<br>135 | H<br>F<br>Cl<br>$CH_3$<br>$CH_3O$ | 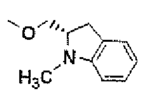 |
| 36<br>37<br>38<br>39<br>40 | H<br>F<br>Cl<br>$CH_3$<br>$CH_3O$ | 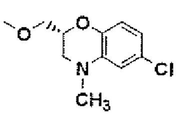 | 86<br>87<br>88<br>89<br>90 | H<br>F<br>Cl<br>$CH_3$<br>$CH_3O$ | 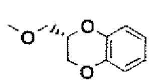 | 136<br>137<br>138<br>139<br>140 | H<br>F<br>Cl<br>$CH_3$<br>$CH_3O$ | 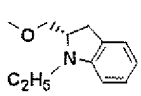 |
| 41<br>42<br>43<br>44<br>45 | H<br>F<br>Cl<br>$CH_3$<br>$CH_3O$ | 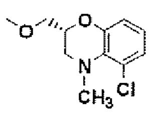 | 91<br>92<br>93<br>94<br>95 | H<br>F<br>Cl<br>$CH_3$<br>$CH_3O$ | 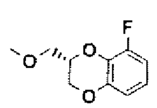 | 141<br>142<br>143<br>144<br>145 | H<br>F<br>Cl<br>$CH_3$<br>$CH_3O$ | 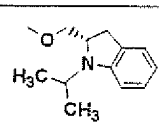 |
| 46<br>47<br>48<br>49<br>50 | H<br>F<br>Cl<br>$CH_3$<br>$CH_3O$ | 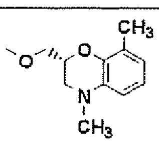 | 96<br>97<br>98<br>99<br>100 | H<br>F<br>Cl<br>$CH_3$<br>$CH_3O$ | 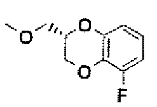 | 146<br>147<br>148<br>149<br>150 | H<br>F<br>Cl<br>$CH_3$<br>$CH_3O$ | 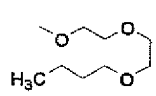 |

Table 35

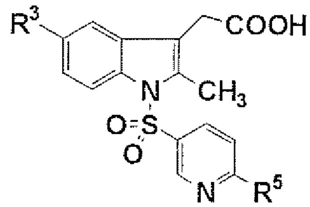

(I-B-3-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 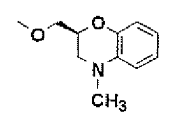 | 51<br>52<br>53<br>54<br>55 | H<br>F<br>Cl<br>$CH_3$<br>$CH_3O$ | 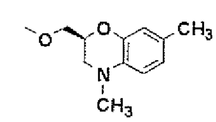 | 101<br>102<br>103<br>104<br>105 | H<br>F<br>Cl<br>$CH_3$<br>$CH_3O$ | 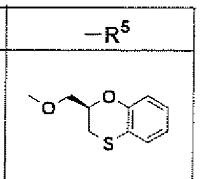 |
| 6<br>7<br>8<br>9<br>10 | H<br>F<br>Cl<br>$CH_3$<br>$CH_3O$ | 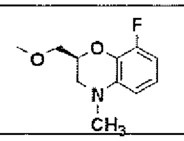 | 56<br>57<br>58<br>59<br>60 | H<br>F<br>Cl<br>$CH_3$<br>$CH_3O$ | 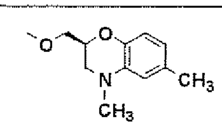 | 106<br>107<br>108<br>109<br>110 | H<br>F<br>Cl<br>$CH_3$<br>$CH_3O$ | 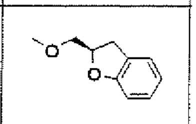 |
| 11<br>12<br>13<br>14<br>15 | H<br>F<br>Cl<br>$CH_3$<br>$CH_3O$ | 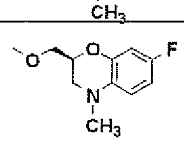 | 61<br>62<br>63<br>64<br>65 | H<br>F<br>Cl<br>$CH_3$<br>$CH_3O$ | 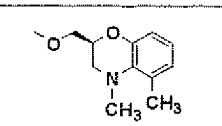 | 111<br>112<br>113<br>114<br>115 | H<br>F<br>Cl<br>$CH_3$<br>$CH_3O$ | 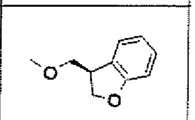 |
| 16<br>17<br>18<br>19<br>20 | H<br>F<br>Cl<br>$CH_3$<br>$CH_3O$ | 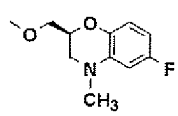 | 66<br>67<br>68<br>69<br>70 | H<br>F<br>Cl<br>$CH_3$<br>$CH_3O$ | 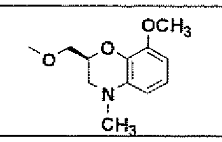 | 116<br>117<br>118<br>119<br>120 | H<br>F<br>Cl<br>$CH_3$<br>$CH_3O$ | 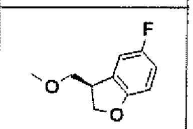 |
| 21<br>22<br>23<br>24<br>25 | H<br>F<br>Cl<br>$CH_3$<br>$CH_3O$ | 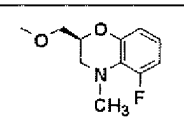 | 71<br>72<br>73<br>74<br>75 | H<br>F<br>Cl<br>$CH_3$<br>$CH_3O$ | 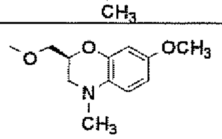 | 121<br>122<br>123<br>124<br>125 | H<br>F<br>Cl<br>$CH_3$<br>$CH_3O$ | 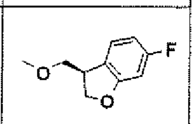 |
| 26<br>27<br>28<br>29<br>30 | H<br>F<br>Cl<br>$CH_3$<br>$CH_3O$ | 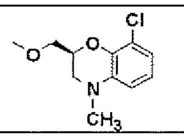 | 76<br>77<br>78<br>79<br>80 | H<br>F<br>Cl<br>$CH_3$<br>$CH_3O$ | 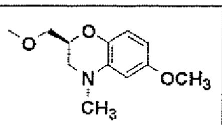 | 126<br>127<br>128<br>129<br>130 | H<br>F<br>Cl<br>$CH_3$<br>$CH_3O$ | 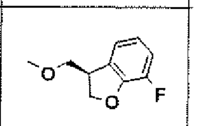 |
| 31<br>32<br>33<br>34<br>35 | H<br>F<br>Cl<br>$CH_3$<br>$CH_3O$ | 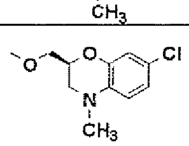 | 81<br>82<br>83<br>84<br>85 | H<br>F<br>Cl<br>$CH_3$<br>$CH_3O$ | 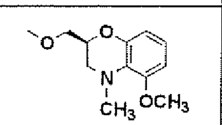 | 131<br>132<br>133<br>134<br>135 | H<br>F<br>Cl<br>$CH_3$<br>$CH_3O$ | 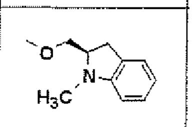 |
| 36<br>37<br>38<br>39<br>40 | H<br>F<br>Cl<br>$CH_3$<br>$CH_3O$ | 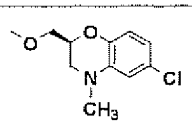 | 86<br>87<br>88<br>89<br>90 | H<br>F<br>Cl<br>$CH_3$<br>$CH_3O$ | 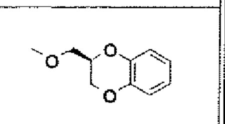 | 136<br>137<br>138<br>139<br>140 | H<br>F<br>Cl<br>$CH_3$<br>$CH_3O$ | 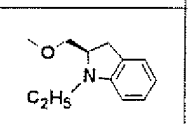 |
| 41<br>42<br>43<br>44<br>45 | H<br>F<br>Cl<br>$CH_3$<br>$CH_3O$ | 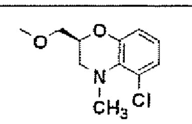 | 91<br>92<br>93<br>94<br>95 | H<br>F<br>Cl<br>$CH_3$<br>$CH_3O$ | 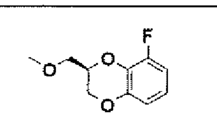 | 141<br>142<br>143<br>144<br>145 | H<br>F<br>Cl<br>$CH_3$<br>$CH_3O$ | 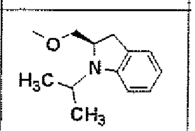 |
| 46<br>47<br>48<br>49<br>50 | H<br>F<br>Cl<br>$CH_3$<br>$CH_3O$ | 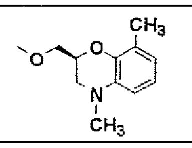 | 96<br>97<br>98<br>99<br>100 | H<br>F<br>Cl<br>$CH_3$<br>$CH_3O$ | 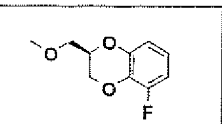 | 146<br>147<br>148<br>149<br>150 | H<br>F<br>Cl<br>$CH_3$<br>$CH_3O$ | 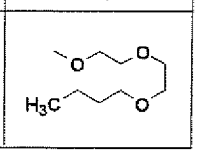 |

Table 36

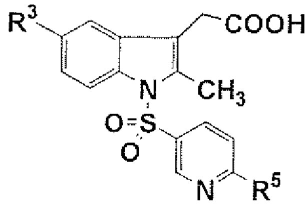

(I-B-3-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 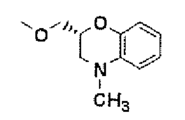 | 51<br>52<br>53<br>54<br>55 | H<br>F<br>Cl<br>$CH_3$<br>$CH_3O$ | 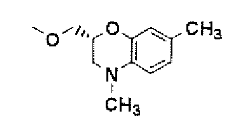 | 101<br>102<br>103<br>104<br>105 | H<br>F<br>Cl<br>$CH_3$<br>$CH_3O$ | 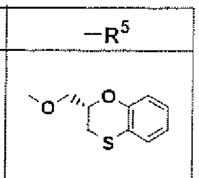 |
| 6<br>7<br>8<br>9<br>10 | H<br>F<br>Cl<br>$CH_3$<br>$CH_3O$ | 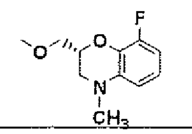 | 56<br>57<br>58<br>59<br>60 | H<br>F<br>Cl<br>$CH_3$<br>$CH_3O$ | 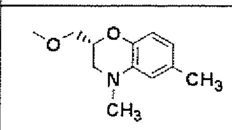 | 106<br>107<br>108<br>109<br>110 | H<br>F<br>Cl<br>$CH_3$<br>$CH_3O$ | 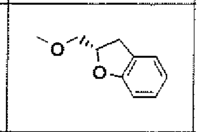 |
| 11<br>12<br>13<br>14<br>15 | H<br>F<br>Cl<br>$CH_3$<br>$CH_3O$ | 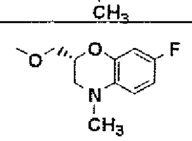 | 61<br>62<br>63<br>64<br>65 | H<br>F<br>Cl<br>$CH_3$<br>$CH_3O$ | 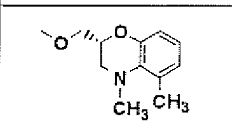 | 111<br>112<br>113<br>114<br>115 | H<br>F<br>Cl<br>$CH_3$<br>$CH_3O$ | 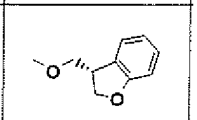 |
| 16<br>17<br>18<br>19<br>20 | H<br>F<br>Cl<br>$CH_3$<br>$CH_3O$ | 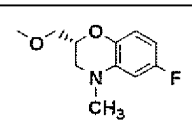 | 66<br>67<br>68<br>69<br>70 | H<br>F<br>Cl<br>$CH_3$<br>$CH_3O$ | 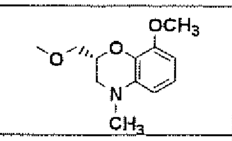 | 116<br>117<br>118<br>119<br>120 | H<br>F<br>Cl<br>$CH_3$<br>$CH_3O$ | 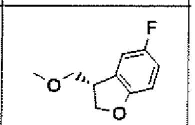 |
| 21<br>22<br>23<br>24<br>25 | H<br>F<br>Cl<br>$CH_3$<br>$CH_3O$ | 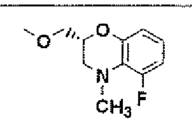 | 71<br>72<br>73<br>74<br>75 | H<br>F<br>Cl<br>$CH_3$<br>$CH_3O$ | 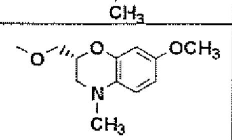 | 121<br>122<br>123<br>124<br>125 | H<br>F<br>Cl<br>$CH_3$<br>$CH_3O$ | 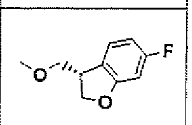 |
| 26<br>27<br>28<br>29<br>30 | H<br>F<br>Cl<br>$CH_3$<br>$CH_3O$ | 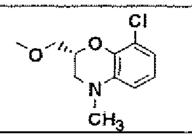 | 76<br>77<br>78<br>79<br>80 | H<br>F<br>Cl<br>$CH_3$<br>$CH_3O$ | 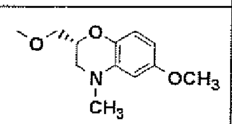 | 126<br>127<br>128<br>129<br>130 | H<br>F<br>Cl<br>$CH_3$<br>$CH_3O$ | 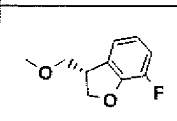 |
| 31<br>32<br>33<br>34<br>35 | H<br>F<br>Cl<br>$CH_3$<br>$CH_3O$ | 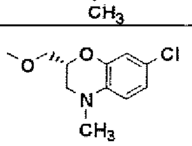 | 81<br>82<br>83<br>84<br>85 | H<br>F<br>Cl<br>$CH_3$<br>$CH_3O$ | 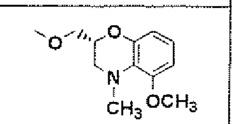 | 131<br>132<br>133<br>134<br>135 | H<br>F<br>Cl<br>$CH_3$<br>$CH_3O$ | 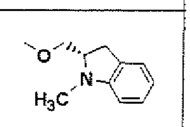 |
| 36<br>37<br>38<br>39<br>40 | H<br>F<br>Cl<br>$CH_3$<br>$CH_3O$ | 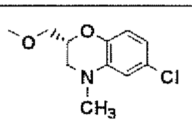 | 86<br>87<br>88<br>89<br>90 | H<br>F<br>Cl<br>$CH_3$<br>$CH_3O$ | 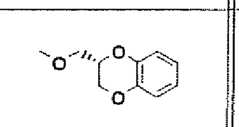 | 136<br>137<br>138<br>139<br>140 | H<br>F<br>Cl<br>$CH_3$<br>$CH_3O$ | 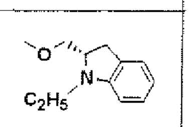 |
| 41<br>42<br>43<br>44<br>45 | H<br>F<br>Cl<br>$CH_3$<br>$CH_3O$ | 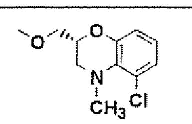 | 91<br>92<br>93<br>94<br>95 | H<br>F<br>Cl<br>$CH_3$<br>$CH_3O$ | 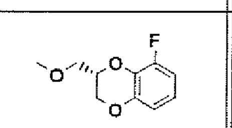 | 141<br>142<br>143<br>144<br>145 | H<br>F<br>Cl<br>$CH_3$<br>$CH_3O$ | 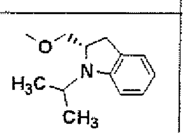 |
| 46<br>47<br>48<br>49<br>50 | H<br>F<br>Cl<br>$CH_3$<br>$CH_3O$ | 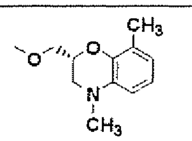 | 96<br>97<br>98<br>99<br>100 | H<br>F<br>Cl<br>$CH_3$<br>$CH_3O$ | 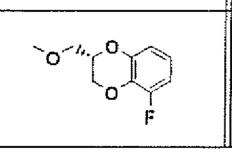 | 146<br>147<br>148<br>149<br>150 | H<br>F<br>Cl<br>$CH_3$<br>$CH_3O$ | 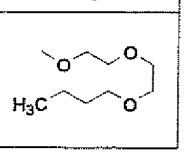 |

Table 37

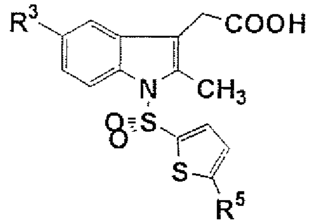

(1-B-4-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 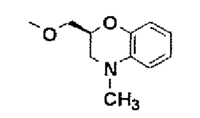 | 51<br>52<br>53<br>54<br>55 | H<br>F<br>Cl<br>$CH_3$<br>$CH_3O$ | 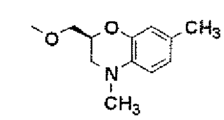 | 101<br>102<br>103<br>104<br>105 | H<br>F<br>Cl<br>$CH_3$<br>$CH_3O$ | 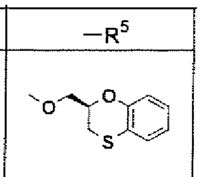 |
| 6<br>7<br>8<br>9<br>10 | H<br>F<br>Cl<br>$CH_3$<br>$CH_3O$ | 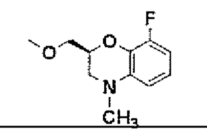 | 56<br>57<br>58<br>59<br>60 | H<br>F<br>Cl<br>$CH_3$<br>$CH_3O$ | 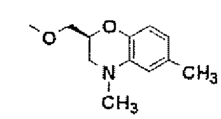 | 106<br>107<br>108<br>109<br>110 | H<br>F<br>Cl<br>$CH_3$<br>$CH_3O$ | 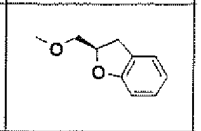 |
| 11<br>12<br>13<br>14<br>15 | H<br>F<br>Cl<br>$CH_3$<br>$CH_3O$ | 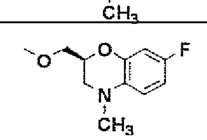 | 61<br>62<br>63<br>64<br>65 | H<br>F<br>Cl<br>$CH_3$<br>$CH_3O$ | 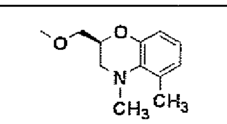 | 111<br>112<br>113<br>114<br>115 | H<br>F<br>Cl<br>$CH_3$<br>$CH_3O$ | 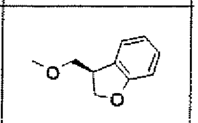 |
| 16<br>17<br>18<br>19<br>20 | H<br>F<br>Cl<br>$CH_3$<br>$CH_3O$ | 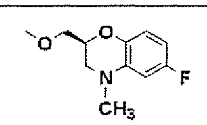 | 66<br>67<br>68<br>69<br>70 | H<br>F<br>Cl<br>$CH_3$<br>$CH_3O$ | 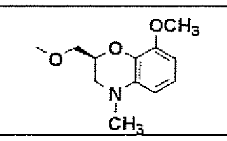 | 116<br>117<br>118<br>119<br>120 | H<br>F<br>Cl<br>$CH_3$<br>$CH_3O$ | 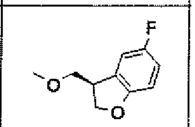 |
| 21<br>22<br>23<br>24<br>25 | H<br>F<br>Cl<br>$CH_3$<br>$CH_3O$ | 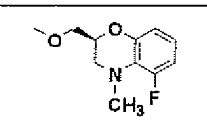 | 71<br>72<br>73<br>74<br>75 | H<br>F<br>Cl<br>$CH_3$<br>$CH_3O$ | 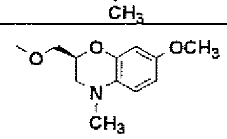 | 121<br>122<br>123<br>124<br>125 | H<br>F<br>Cl<br>$CH_3$<br>$CH_3O$ | 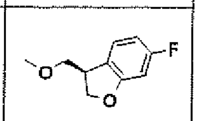 |
| 26<br>27<br>28<br>29<br>30 | H<br>F<br>Cl<br>$CH_3$<br>$CH_3O$ | 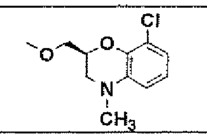 | 76<br>77<br>78<br>79<br>80 | H<br>F<br>Cl<br>$CH_3$<br>$CH_3O$ | 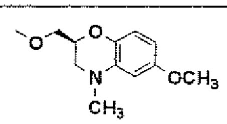 | 126<br>127<br>128<br>129<br>130 | H<br>F<br>Cl<br>$CH_3$<br>$CH_3O$ | 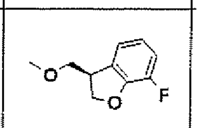 |
| 31<br>32<br>33<br>34<br>35 | H<br>F<br>Cl<br>$CH_3$<br>$CH_3O$ | 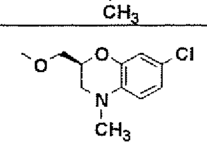 | 81<br>82<br>83<br>84<br>85 | H<br>F<br>Cl<br>$CH_3$<br>$CH_3O$ | 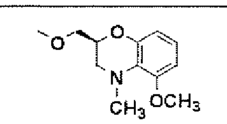 | 131<br>132<br>133<br>134<br>135 | H<br>F<br>Cl<br>$CH_3$<br>$CH_3O$ | 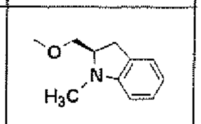 |
| 36<br>37<br>38<br>39<br>40 | H<br>F<br>Cl<br>$CH_3$<br>$CH_3O$ | 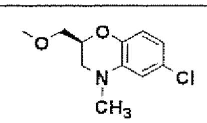 | 86<br>87<br>88<br>89<br>90 | H<br>F<br>Cl<br>$CH_3$<br>$CH_3O$ | 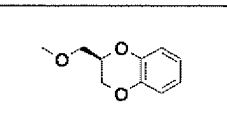 | 136<br>137<br>138<br>139<br>140 | H<br>F<br>Cl<br>$CH_3$<br>$CH_3O$ | 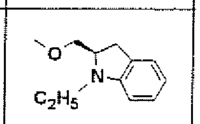 |
| 41<br>42<br>43<br>44<br>45 | H<br>F<br>Cl<br>$CH_3$<br>$CH_3O$ | 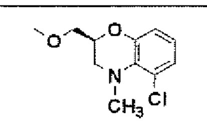 | 91<br>92<br>93<br>94<br>95 | H<br>F<br>Cl<br>$CH_3$<br>$CH_3O$ | 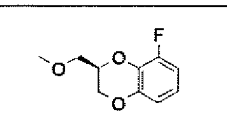 | 141<br>142<br>143<br>144<br>145 | H<br>F<br>Cl<br>$CH_3$<br>$CH_3O$ | 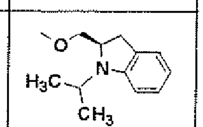 |
| 46<br>47<br>48<br>49<br>50 | H<br>F<br>Cl<br>$CH_3$<br>$CH_3O$ | 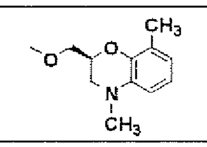 | 96<br>97<br>98<br>99<br>100 | H<br>F<br>Cl<br>$CH_3$<br>$CH_3O$ | 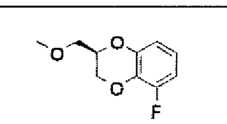 | 146<br>147<br>148<br>149<br>150 | H<br>F<br>Cl<br>$CH_3$<br>$CH_3O$ | 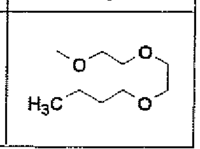 |

Table 38

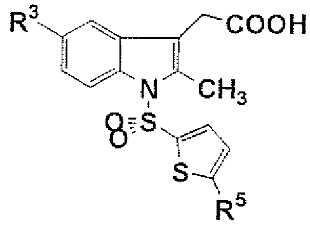

(I－B－4－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 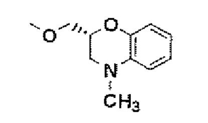 | 51<br>52<br>53<br>54<br>55 | H<br>F<br>Cl<br>$CH_3$<br>$CH_3O$ | 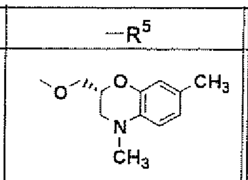 | 101<br>102<br>103<br>104<br>105 | H<br>F<br>Cl<br>$CH_3$<br>$CH_3O$ | 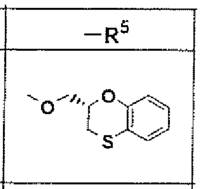 |
| 6<br>7<br>8<br>9<br>10 | H<br>F<br>Cl<br>$CH_3$<br>$CH_3O$ | 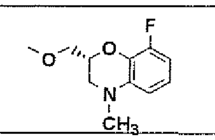 | 56<br>57<br>58<br>59<br>60 | H<br>F<br>Cl<br>$CH_3$<br>$CH_3O$ | 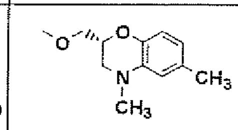 | 106<br>107<br>108<br>109<br>110 | H<br>F<br>Cl<br>$CH_3$<br>$CH_3O$ | 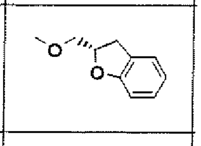 |
| 11<br>12<br>13<br>14<br>15 | H<br>F<br>Cl<br>$CH_3$<br>$CH_3O$ | 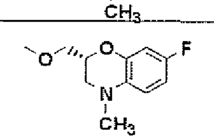 | 61<br>62<br>63<br>64<br>65 | H<br>F<br>Cl<br>$CH_3$<br>$CH_3O$ | 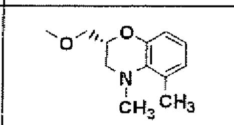 | 111<br>112<br>113<br>114<br>115 | H<br>F<br>Cl<br>$CH_3$<br>$CH_3O$ | 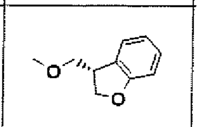 |
| 16<br>17<br>18<br>19<br>20 | H<br>F<br>Cl<br>$CH_3$<br>$CH_3O$ | 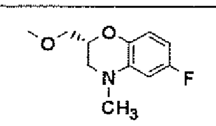 | 66<br>67<br>68<br>69<br>70 | H<br>F<br>Cl<br>$CH_3$<br>$CH_3O$ | 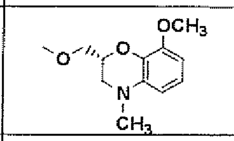 | 116<br>117<br>118<br>119<br>120 | H<br>F<br>Cl<br>$CH_3$<br>$CH_3O$ | 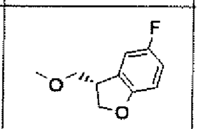 |
| 21<br>22<br>23<br>24<br>25 | H<br>F<br>Cl<br>$CH_3$<br>$CH_3O$ | 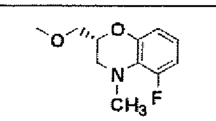 | 71<br>72<br>73<br>74<br>75 | H<br>F<br>Cl<br>$CH_3$<br>$CH_3O$ | 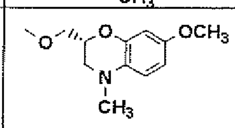 | 121<br>122<br>123<br>124<br>125 | H<br>F<br>Cl<br>$CH_3$<br>$CH_3O$ | 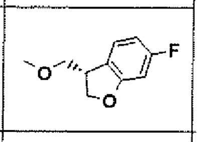 |
| 26<br>27<br>28<br>29<br>30 | H<br>F<br>Cl<br>$CH_3$<br>$CH_3O$ | 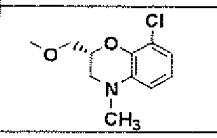 | 76<br>77<br>78<br>79<br>80 | H<br>F<br>Cl<br>$CH_3$<br>$CH_3O$ | 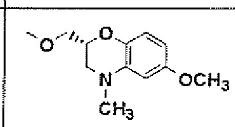 | 126<br>127<br>128<br>129<br>130 | H<br>F<br>Cl<br>$CH_3$<br>$CH_3O$ | 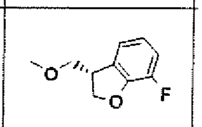 |
| 31<br>32<br>33<br>34<br>35 | H<br>F<br>Cl<br>$CH_3$<br>$CH_3O$ | 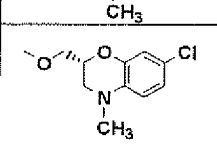 | 81<br>82<br>83<br>84<br>85 | H<br>F<br>Cl<br>$CH_3$<br>$CH_3O$ | 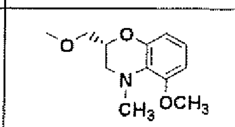 | 131<br>132<br>133<br>134<br>135 | H<br>F<br>Cl<br>$CH_3$<br>$CH_3O$ | 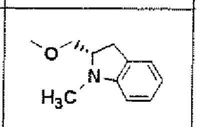 |
| 36<br>37<br>38<br>39<br>40 | H<br>F<br>Cl<br>$CH_3$<br>$CH_3O$ | 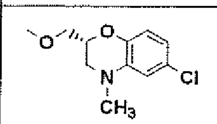 | 86<br>87<br>88<br>89<br>90 | H<br>F<br>Cl<br>$CH_3$<br>$CH_3O$ | 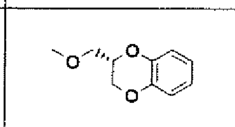 | 136<br>137<br>138<br>139<br>140 | H<br>F<br>Cl<br>$CH_3$<br>$CH_3O$ | 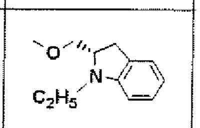 |
| 41<br>42<br>43<br>44<br>45 | H<br>F<br>Cl<br>$CH_3$<br>$CH_3O$ | 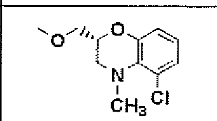 | 91<br>92<br>93<br>94<br>95 | H<br>F<br>Cl<br>$CH_3$<br>$CH_3O$ | 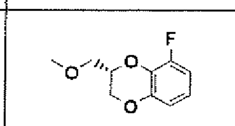 | 141<br>142<br>143<br>144<br>145 | H<br>F<br>Cl<br>$CH_3$<br>$CH_3O$ | 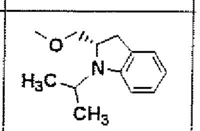 |
| 46<br>47<br>48<br>49<br>50 | H<br>F<br>Cl<br>$CH_3$<br>$CH_3O$ | 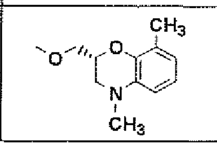 | 96<br>97<br>98<br>99<br>100 | H<br>F<br>Cl<br>$CH_3$<br>$CH_3O$ | 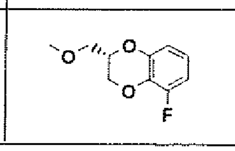 | 146<br>147<br>148<br>149<br>150 | H<br>F<br>Cl<br>$CH_3$<br>$CH_3O$ | 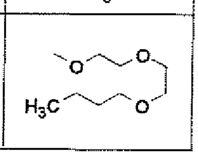 |

Table 39

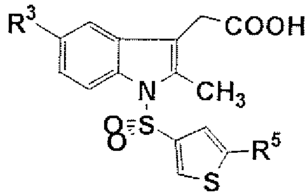

(I-B-5-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 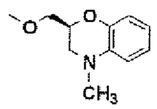 | 51<br>52<br>53<br>54<br>55 | H<br>F<br>Cl<br>$CH_3$<br>$CH_3O$ | 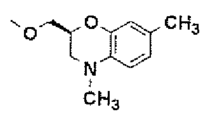 | 101<br>102<br>103<br>104<br>105 | H<br>F<br>Cl<br>$CH_3$<br>$CH_3O$ | 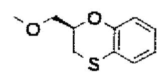 |
| 6<br>7<br>8<br>9<br>10 | H<br>F<br>Cl<br>$CH_3$<br>$CH_3O$ | 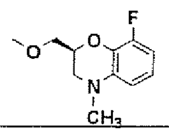 | 56<br>57<br>58<br>59<br>60 | H<br>F<br>Cl<br>$CH_3$<br>$CH_3O$ | 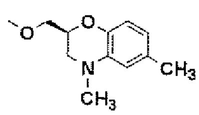 | 106<br>107<br>108<br>109<br>110 | H<br>F<br>Cl<br>$CH_3$<br>$CH_3O$ | 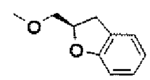 |
| 11<br>12<br>13<br>14<br>15 | H<br>F<br>Cl<br>$CH_3$<br>$CH_3O$ | 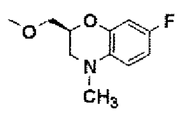 | 61<br>62<br>63<br>64<br>65 | H<br>F<br>Cl<br>$CH_3$<br>$CH_3O$ | 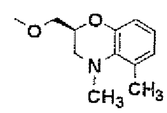 | 111<br>112<br>113<br>114<br>115 | H<br>F<br>Cl<br>$CH_3$<br>$CH_3O$ | 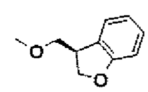 |
| 16<br>17<br>18<br>19<br>20 | H<br>F<br>Cl<br>$CH_3$<br>$CH_3O$ | 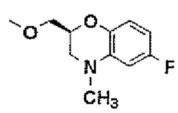 | 66<br>67<br>68<br>69<br>70 | H<br>F<br>Cl<br>$CH_3$<br>$CH_3O$ | 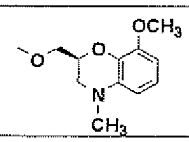 | 116<br>117<br>118<br>119<br>120 | H<br>F<br>Cl<br>$CH_3$<br>$CH_3O$ | 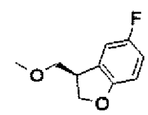 |
| 21<br>22<br>23<br>24<br>25 | H<br>F<br>Cl<br>$CH_3$<br>$CH_3O$ | 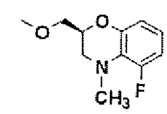 | 71<br>72<br>73<br>74<br>75 | H<br>F<br>Cl<br>$CH_3$<br>$CH_3O$ | 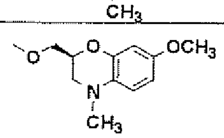 | 121<br>122<br>123<br>124<br>125 | H<br>F<br>Cl<br>$CH_3$<br>$CH_3O$ | 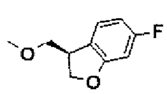 |
| 26<br>27<br>28<br>29<br>30 | H<br>F<br>Cl<br>$CH_3$<br>$CH_3O$ | 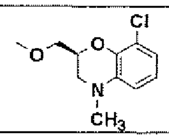 | 76<br>77<br>78<br>79<br>80 | H<br>F<br>Cl<br>$CH_3$<br>$CH_3O$ | 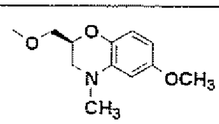 | 126<br>127<br>128<br>129<br>130 | H<br>F<br>Cl<br>$CH_3$<br>$CH_3O$ | 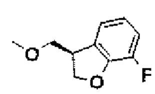 |
| 31<br>32<br>33<br>34<br>35 | H<br>F<br>Cl<br>$CH_3$<br>$CH_3O$ | 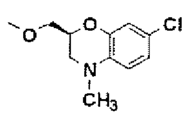 | 81<br>82<br>83<br>84<br>85 | H<br>F<br>Cl<br>$CH_3$<br>$CH_3O$ | 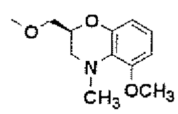 | 131<br>132<br>133<br>134<br>135 | H<br>F<br>Cl<br>$CH_3$<br>$CH_3O$ | 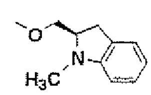 |
| 36<br>37<br>38<br>39<br>40 | H<br>F<br>Cl<br>$CH_3$<br>$CH_3O$ | 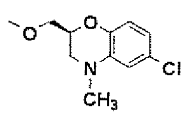 | 86<br>87<br>88<br>89<br>90 | H<br>F<br>Cl<br>$CH_3$<br>$CH_3O$ | 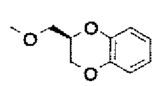 | 136<br>137<br>138<br>139<br>140 | H<br>F<br>Cl<br>$CH_3$<br>$CH_3O$ | 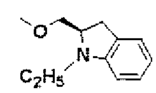 |
| 41<br>42<br>43<br>44<br>45 | H<br>F<br>Cl<br>$CH_3$<br>$CH_3O$ | 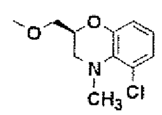 | 91<br>92<br>93<br>94<br>95 | H<br>F<br>Cl<br>$CH_3$<br>$CH_3O$ | 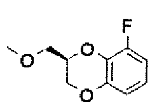 | 141<br>142<br>143<br>144<br>145 | H<br>F<br>Cl<br>$CH_3$<br>$CH_3O$ | 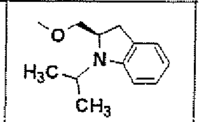 |
| 46<br>47<br>48<br>49<br>50 | H<br>F<br>Cl<br>$CH_3$<br>$CH_3O$ | 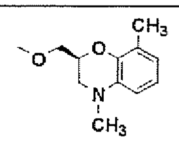 | 96<br>97<br>98<br>99<br>100 | H<br>F<br>Cl<br>$CH_3$<br>$CH_3O$ | 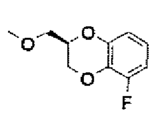 | 146<br>147<br>148<br>149<br>150 | H<br>F<br>Cl<br>$CH_3$<br>$CH_3O$ | 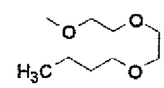 |

Table 40

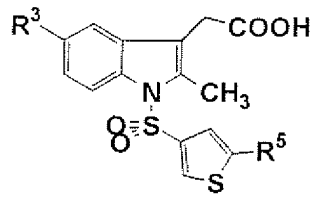

(1-B-5-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 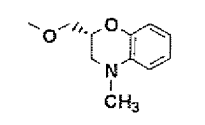 | 51<br>52<br>53<br>54<br>55 | H<br>F<br>Cl<br>$CH_3$<br>$CH_3O$ | 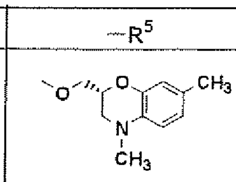 | 101<br>102<br>103<br>104<br>105 | H<br>F<br>Cl<br>$CH_3$<br>$CH_3O$ | 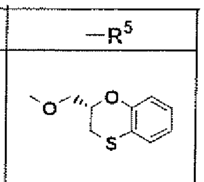 |
| 6<br>7<br>8<br>9<br>10 | H<br>F<br>Cl<br>$CH_3$<br>$CH_3O$ | 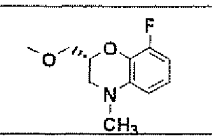 | 56<br>57<br>58<br>59<br>60 | H<br>F<br>Cl<br>$CH_3$<br>$CH_3O$ | 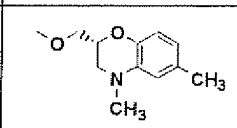 | 106<br>107<br>108<br>109<br>110 | H<br>F<br>Cl<br>$CH_3$<br>$CH_3O$ | 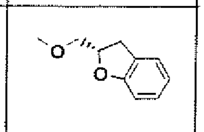 |
| 11<br>12<br>13<br>14<br>15 | H<br>F<br>Cl<br>$CH_3$<br>$CH_3O$ | 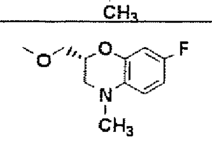 | 61<br>62<br>63<br>64<br>65 | H<br>F<br>Cl<br>$CH_3$<br>$CH_3O$ | 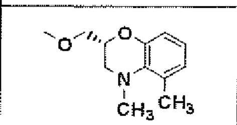 | 111<br>112<br>113<br>114<br>115 | H<br>F<br>Cl<br>$CH_3$<br>$CH_3O$ | 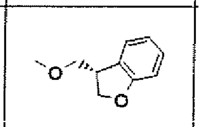 |
| 16<br>17<br>18<br>19<br>20 | H<br>F<br>Cl<br>$CH_3$<br>$CH_3O$ | 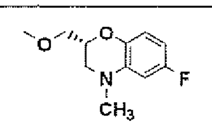 | 66<br>67<br>68<br>69<br>70 | H<br>F<br>Cl<br>$CH_3$<br>$CH_3O$ | 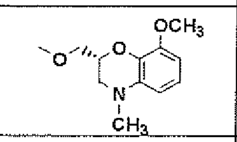 | 116<br>117<br>118<br>119<br>120 | H<br>F<br>Cl<br>$CH_3$<br>$CH_3O$ | 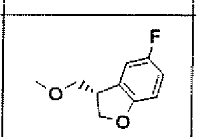 |
| 21<br>22<br>23<br>24<br>25 | H<br>F<br>Cl<br>$CH_3$<br>$CH_3O$ | 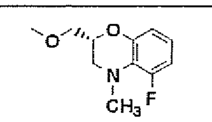 | 71<br>72<br>73<br>74<br>75 | H<br>F<br>Cl<br>$CH_3$<br>$CH_3O$ | 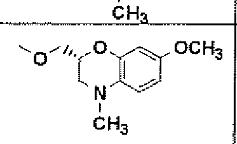 | 121<br>122<br>123<br>124<br>125 | H<br>F<br>Cl<br>$CH_3$<br>$CH_3O$ | 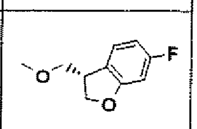 |
| 26<br>27<br>28<br>29<br>30 | H<br>F<br>Cl<br>$CH_3$<br>$CH_3O$ | 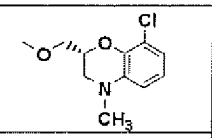 | 76<br>77<br>78<br>79<br>80 | H<br>F<br>Cl<br>$CH_3$<br>$CH_3O$ | 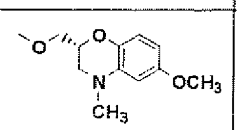 | 126<br>127<br>128<br>129<br>130 | H<br>F<br>Cl<br>$CH_3$<br>$CH_3O$ | 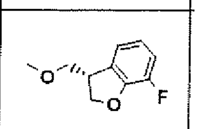 |
| 31<br>32<br>33<br>34<br>35 | H<br>F<br>Cl<br>$CH_3$<br>$CH_3O$ | 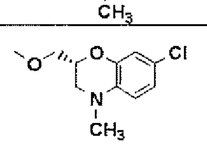 | 81<br>82<br>83<br>84<br>85 | H<br>F<br>Cl<br>$CH_3$<br>$CH_3O$ | 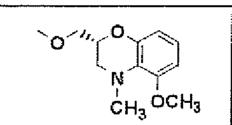 | 131<br>132<br>133<br>134<br>135 | H<br>F<br>Cl<br>$CH_3$<br>$CH_3O$ | 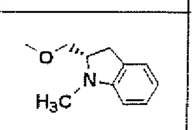 |
| 36<br>37<br>38<br>39<br>40 | H<br>F<br>Cl<br>$CH_3$<br>$CH_3O$ | 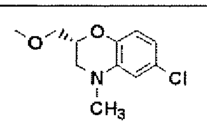 | 86<br>87<br>88<br>89<br>90 | H<br>F<br>Cl<br>$CH_3$<br>$CH_3O$ | 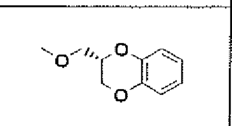 | 136<br>137<br>138<br>139<br>140 | H<br>F<br>Cl<br>$CH_3$<br>$CH_3O$ | 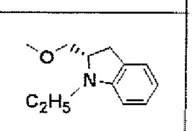 |
| 41<br>42<br>43<br>44<br>45 | H<br>F<br>Cl<br>$CH_3$<br>$CH_3O$ | 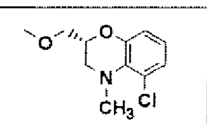 | 91<br>92<br>93<br>94<br>95 | H<br>F<br>Cl<br>$CH_3$<br>$CH_3O$ | 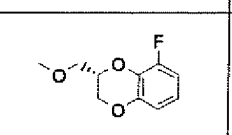 | 141<br>142<br>143<br>144<br>145 | H<br>F<br>Cl<br>$CH_3$<br>$CH_3O$ | 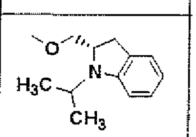 |
| 46<br>47<br>48<br>49<br>50 | H<br>F<br>Cl<br>$CH_3$<br>$CH_3O$ | 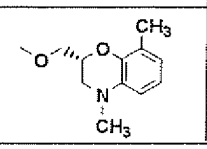 | 96<br>97<br>98<br>99<br>100 | H<br>F<br>Cl<br>$CH_3$<br>$CH_3O$ | 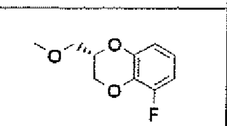 | 146<br>147<br>148<br>149<br>150 | H<br>F<br>Cl<br>$CH_3$<br>$CH_3O$ | 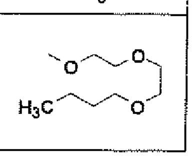 |

Table 41

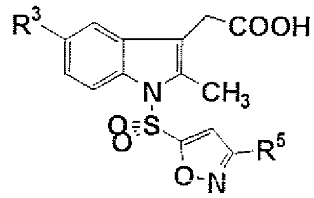

(I-B-6-1)

| No. | $R^3$ | $-R^5$ | No. | $R^3$ | $-R^5$ | No. | $R^3$ | $-R^5$ |
|---|---|---|---|---|---|---|---|---|
| 1 | H | 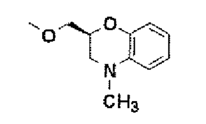 | 51 | H | 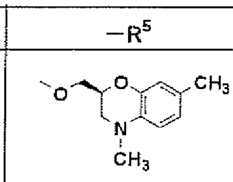 | 101 | H | 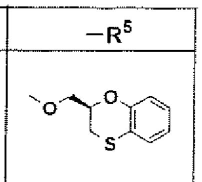 |
| 2 | F | | 52 | F | | 102 | F | |
| 3 | Cl | | 53 | Cl | | 103 | Cl | |
| 4 | $CH_3$ | | 54 | $CH_3$ | | 104 | $CH_3$ | |
| 5 | $CH_3O$ | | 55 | $CH_3O$ | | 105 | $CH_3O$ | |
| 6 | H | 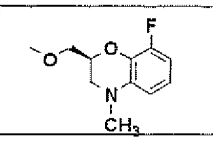 | 56 | H | 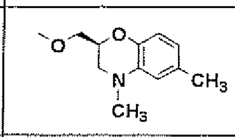 | 106 | H | 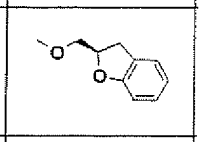 |
| 7 | F | | 57 | F | | 107 | F | |
| 8 | Cl | | 58 | Cl | | 108 | Cl | |
| 9 | $CH_3$ | | 59 | $CH_3$ | | 109 | $CH_3$ | |
| 10 | $CH_3O$ | | 60 | $CH_3O$ | | 110 | $CH_3O$ | |
| 11 | H | 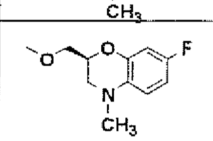 | 61 | H | 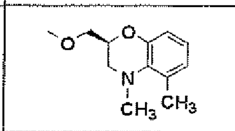 | 111 | H | 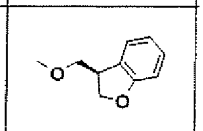 |
| 12 | F | | 62 | F | | 112 | F | |
| 13 | Cl | | 63 | Cl | | 113 | Cl | |
| 14 | $CH_3$ | | 64 | $CH_3$ | | 114 | $CH_3$ | |
| 15 | $CH_3O$ | | 65 | $CH_3O$ | | 115 | $CH_3O$ | |
| 16 | H | 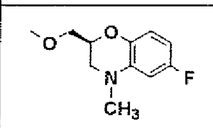 | 66 | H | 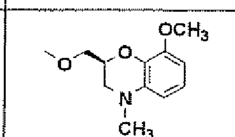 | 116 | H | 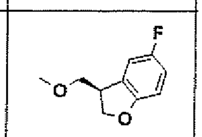 |
| 17 | F | | 67 | F | | 117 | F | |
| 18 | Cl | | 68 | Cl | | 118 | Cl | |
| 19 | $CH_3$ | | 69 | $CH_3$ | | 119 | $CH_3$ | |
| 20 | $CH_3O$ | | 70 | $CH_3O$ | | 120 | $CH_3O$ | |
| 21 | H | 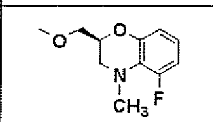 | 71 | H | 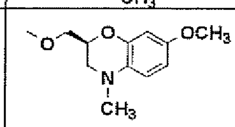 | 121 | H | 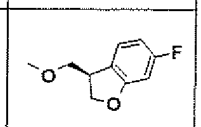 |
| 22 | F | | 72 | F | | 122 | F | |
| 23 | Cl | | 73 | Cl | | 123 | Cl | |
| 24 | $CH_3$ | | 74 | $CH_3$ | | 124 | $CH_3$ | |
| 25 | $CH_3O$ | | 75 | $CH_3O$ | | 125 | $CH_3O$ | |
| 26 | H | 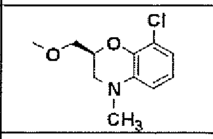 | 76 | H | 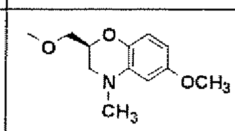 | 126 | H | 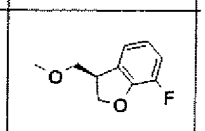 |
| 27 | F | | 77 | F | | 127 | F | |
| 28 | Cl | | 78 | Cl | | 128 | Cl | |
| 29 | $CH_3$ | | 79 | $CH_3$ | | 129 | $CH_3$ | |
| 30 | $CH_3O$ | | 80 | $CH_3O$ | | 130 | $CH_3O$ | |
| 31 | H | 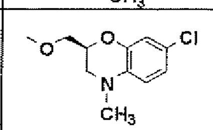 | 81 | H | 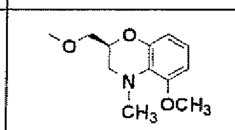 | 131 | H | 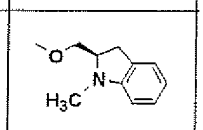 |
| 32 | F | | 82 | F | | 132 | F | |
| 33 | Cl | | 83 | Cl | | 133 | Cl | |
| 34 | $CH_3$ | | 84 | $CH_3$ | | 134 | $CH_3$ | |
| 35 | $CH_3O$ | | 85 | $CH_3O$ | | 135 | $CH_3O$ | |
| 36 | H | 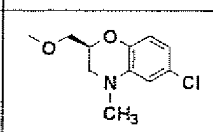 | 86 | H | 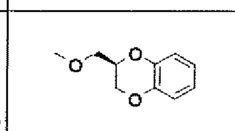 | 136 | H | 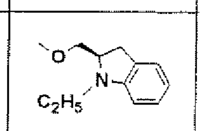 |
| 37 | F | | 87 | F | | 137 | F | |
| 38 | Cl | | 88 | Cl | | 138 | Cl | |
| 39 | $CH_3$ | | 89 | $CH_3$ | | 139 | $CH_3$ | |
| 40 | $CH_3O$ | | 90 | $CH_3O$ | | 140 | $CH_3O$ | |
| 41 | H | 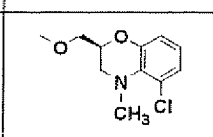 | 91 | H | 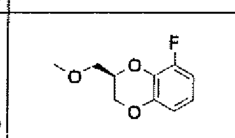 | 141 | H | 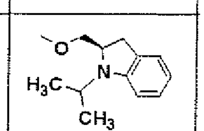 |
| 42 | F | | 92 | F | | 142 | F | |
| 43 | Cl | | 93 | Cl | | 143 | Cl | |
| 44 | $CH_3$ | | 94 | $CH_3$ | | 144 | $CH_3$ | |
| 45 | $CH_3O$ | | 95 | $CH_3O$ | | 145 | $CH_3O$ | |
| 46 | H | 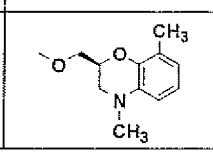 | 96 | H | 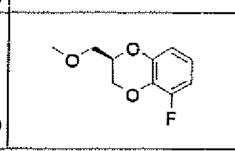 | 146 | H | 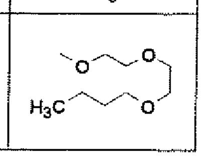 |
| 47 | F | | 97 | F | | 147 | F | |
| 48 | Cl | | 98 | Cl | | 148 | Cl | |
| 49 | $CH_3$ | | 99 | $CH_3$ | | 149 | $CH_3$ | |
| 50 | $CH_3O$ | | 100 | $CH_3O$ | | 150 | $CH_3O$ | |

Table 42

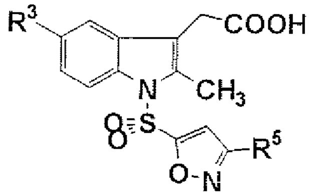

(I－B－6－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 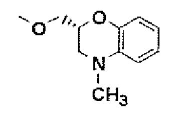 | 51<br>52<br>53<br>54<br>55 | H<br>F<br>Cl<br>$CH_3$<br>$CH_3O$ | 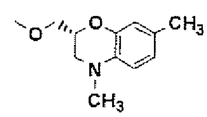 | 101<br>102<br>103<br>104<br>105 | H<br>F<br>Cl<br>$CH_3$<br>$CH_3O$ | 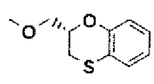 |
| 6<br>7<br>8<br>9<br>10 | H<br>F<br>Cl<br>$CH_3$<br>$CH_3O$ | 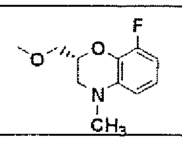 | 56<br>57<br>58<br>59<br>60 | H<br>F<br>Cl<br>$CH_3$<br>$CH_3O$ | 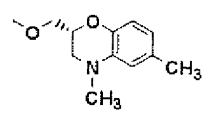 | 106<br>107<br>108<br>109<br>110 | H<br>F<br>Cl<br>$CH_3$<br>$CH_3O$ | 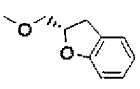 |
| 11<br>12<br>13<br>14<br>15 | H<br>F<br>Cl<br>$CH_3$<br>$CH_3O$ | 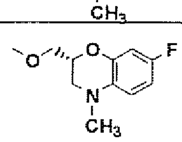 | 61<br>62<br>63<br>64<br>65 | H<br>F<br>Cl<br>$CH_3$<br>$CH_3O$ | 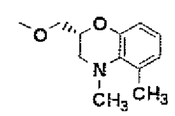 | 111<br>112<br>113<br>114<br>115 | H<br>F<br>Cl<br>$CH_3$<br>$CH_3O$ | 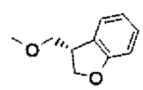 |
| 16<br>17<br>18<br>19<br>20 | H<br>F<br>Cl<br>$CH_3$<br>$CH_3O$ | 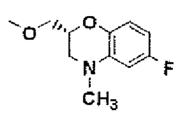 | 66<br>67<br>68<br>69<br>70 | H<br>F<br>Cl<br>$CH_3$<br>$CH_3O$ | 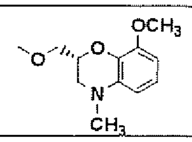 | 116<br>117<br>118<br>119<br>120 | H<br>F<br>Cl<br>$CH_3$<br>$CH_3O$ | 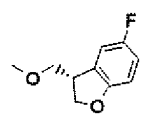 |
| 21<br>22<br>23<br>24<br>25 | H<br>F<br>Cl<br>$CH_3$<br>$CH_3O$ | 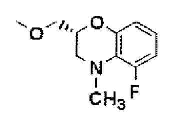 | 71<br>72<br>73<br>74<br>75 | H<br>F<br>Cl<br>$CH_3$<br>$CH_3O$ | 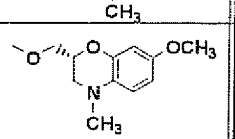 | 121<br>122<br>123<br>124<br>125 | H<br>F<br>Cl<br>$CH_3$<br>$CH_3O$ | 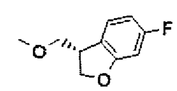 |
| 26<br>27<br>28<br>29<br>30 | H<br>F<br>Cl<br>$CH_3$<br>$CH_3O$ | 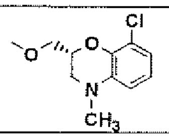 | 76<br>77<br>78<br>79<br>80 | H<br>F<br>Cl<br>$CH_3$<br>$CH_3O$ | 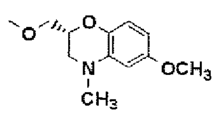 | 126<br>127<br>128<br>129<br>130 | H<br>F<br>Cl<br>$CH_3$<br>$CH_3O$ | 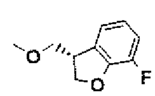 |
| 31<br>32<br>33<br>34<br>35 | H<br>F<br>Cl<br>$CH_3$<br>$CH_3O$ | 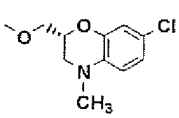 | 81<br>82<br>83<br>84<br>85 | H<br>F<br>Cl<br>$CH_3$<br>$CH_3O$ | 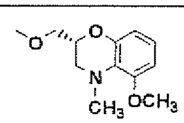 | 131<br>132<br>133<br>134<br>135 | H<br>F<br>Cl<br>$CH_3$<br>$CH_3O$ | 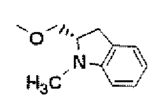 |
| 36<br>37<br>38<br>39<br>40 | H<br>F<br>Cl<br>$CH_3$<br>$CH_3O$ | 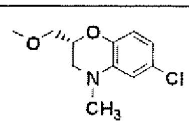 | 86<br>87<br>88<br>89<br>90 | H<br>F<br>Cl<br>$CH_3$<br>$CH_3O$ | 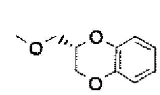 | 136<br>137<br>138<br>139<br>140 | H<br>F<br>Cl<br>$CH_3$<br>$CH_3O$ | 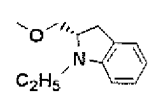 |
| 41<br>42<br>43<br>44<br>45 | H<br>F<br>Cl<br>$CH_3$<br>$CH_3O$ | 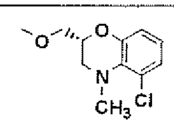 | 91<br>92<br>93<br>94<br>95 | H<br>F<br>Cl<br>$CH_3$<br>$CH_3O$ | 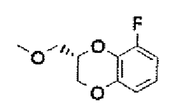 | 141<br>142<br>143<br>144<br>145 | H<br>F<br>Cl<br>$CH_3$<br>$CH_3O$ | 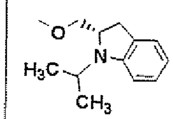 |
| 46<br>47<br>48<br>49<br>50 | H<br>F<br>Cl<br>$CH_3$<br>$CH_3O$ | 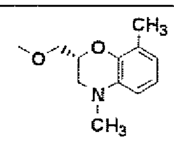 | 96<br>97<br>98<br>99<br>100 | H<br>F<br>Cl<br>$CH_3$<br>$CH_3O$ | 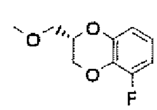 | 146<br>147<br>148<br>149<br>150 | H<br>F<br>Cl<br>$CH_3$<br>$CH_3O$ | 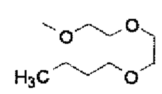 |

Table 43

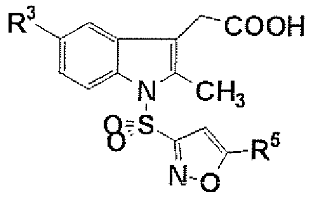

(I-B-7-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 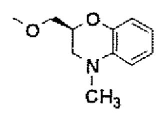 | 51<br>52<br>53<br>54<br>55 | H<br>F<br>Cl<br>$CH_3$<br>$CH_3O$ | 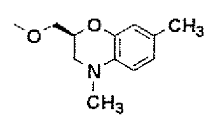 | 101<br>102<br>103<br>104<br>105 | H<br>F<br>Cl<br>$CH_3$<br>$CH_3O$ | 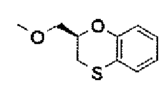 |
| 6<br>7<br>8<br>9<br>10 | H<br>F<br>Cl<br>$CH_3$<br>$CH_3O$ | 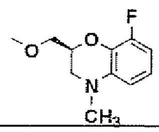 | 56<br>57<br>58<br>59<br>60 | H<br>F<br>Cl<br>$CH_3$<br>$CH_3O$ | 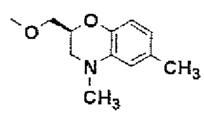 | 106<br>107<br>108<br>109<br>110 | H<br>F<br>Cl<br>$CH_3$<br>$CH_3O$ | 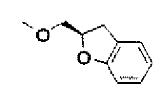 |
| 11<br>12<br>13<br>14<br>15 | H<br>F<br>Cl<br>$CH_3$<br>$CH_3O$ | 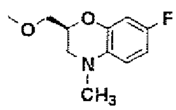 | 61<br>62<br>63<br>64<br>65 | H<br>F<br>Cl<br>$CH_3$<br>$CH_3O$ | 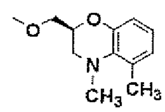 | 111<br>112<br>113<br>114<br>115 | H<br>F<br>Cl<br>$CH_3$<br>$CH_3O$ | 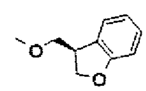 |
| 16<br>17<br>18<br>19<br>20 | H<br>F<br>Cl<br>$CH_3$<br>$CH_3O$ | 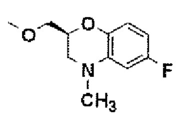 | 66<br>67<br>68<br>69<br>70 | H<br>F<br>Cl<br>$CH_3$<br>$CH_3O$ | 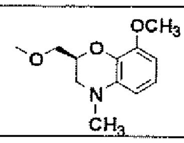 | 116<br>117<br>118<br>119<br>120 | H<br>F<br>Cl<br>$CH_3$<br>$CH_3O$ | 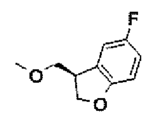 |
| 21<br>22<br>23<br>24<br>25 | H<br>F<br>Cl<br>$CH_3$<br>$CH_3O$ | 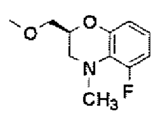 | 71<br>72<br>73<br>74<br>75 | H<br>F<br>Cl<br>$CH_3$<br>$CH_3O$ | 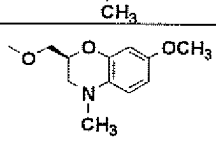 | 121<br>122<br>123<br>124<br>125 | H<br>F<br>Cl<br>$CH_3$<br>$CH_3O$ | 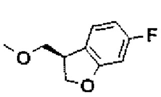 |
| 26<br>27<br>28<br>29<br>30 | H<br>F<br>Cl<br>$CH_3$<br>$CH_3O$ | 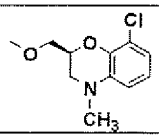 | 76<br>77<br>78<br>79<br>80 | H<br>F<br>Cl<br>$CH_3$<br>$CH_3O$ | 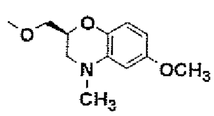 | 126<br>127<br>128<br>129<br>130 | H<br>F<br>Cl<br>$CH_3$<br>$CH_3O$ | 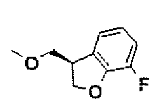 |
| 31<br>32<br>33<br>34<br>35 | H<br>F<br>Cl<br>$CH_3$<br>$CH_3O$ | 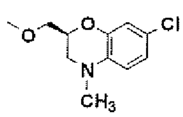 | 81<br>82<br>83<br>84<br>85 | H<br>F<br>Cl<br>$CH_3$<br>$CH_3O$ | 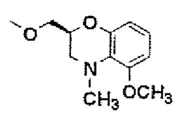 | 131<br>132<br>133<br>134<br>135 | H<br>F<br>Cl<br>$CH_3$<br>$CH_3O$ | 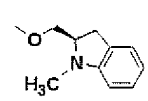 |
| 36<br>37<br>38<br>39<br>40 | H<br>F<br>Cl<br>$CH_3$<br>$CH_3O$ | 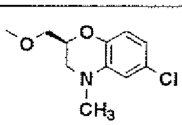 | 86<br>87<br>88<br>89<br>90 | H<br>F<br>Cl<br>$CH_3$<br>$CH_3O$ | 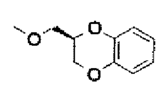 | 136<br>137<br>138<br>139<br>140 | H<br>F<br>Cl<br>$CH_3$<br>$CH_3O$ | 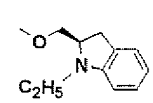 |
| 41<br>42<br>43<br>44<br>45 | H<br>F<br>Cl<br>$CH_3$<br>$CH_3O$ | 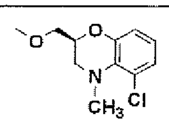 | 91<br>92<br>93<br>94<br>95 | H<br>F<br>Cl<br>$CH_3$<br>$CH_3O$ | 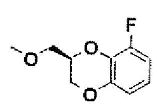 | 141<br>142<br>143<br>144<br>145 | H<br>F<br>Cl<br>$CH_3$<br>$CH_3O$ | 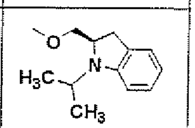 |
| 46<br>47<br>48<br>49<br>50 | H<br>F<br>Cl<br>$CH_3$<br>$CH_3O$ | 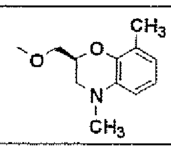 | 96<br>97<br>98<br>99<br>100 | H<br>F<br>Cl<br>$CH_3$<br>$CH_3O$ | 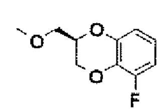 | 146<br>147<br>148<br>149<br>150 | H<br>F<br>Cl<br>$CH_3$<br>$CH_3O$ | 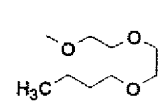 |

Table 44

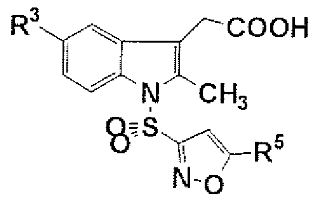

(I－B－7－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 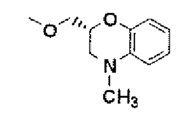 | 51<br>52<br>53<br>54<br>55 | H<br>F<br>Cl<br>$CH_3$<br>$CH_3O$ | 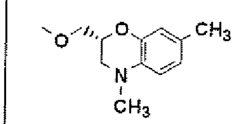 | 101<br>102<br>103<br>104<br>105 | H<br>F<br>Cl<br>$CH_3$<br>$CH_3O$ | 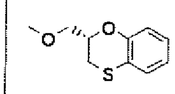 |
| 6<br>7<br>8<br>9<br>10 | H<br>F<br>Cl<br>$CH_3$<br>$CH_3O$ | 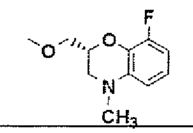 | 56<br>57<br>58<br>59<br>60 | H<br>F<br>Cl<br>$CH_3$<br>$CH_3O$ | 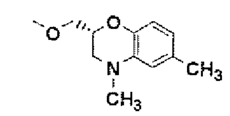 | 106<br>107<br>108<br>109<br>110 | H<br>F<br>Cl<br>$CH_3$<br>$CH_3O$ | 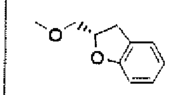 |
| 11<br>12<br>13<br>14<br>15 | H<br>F<br>Cl<br>$CH_3$<br>$CH_3O$ | 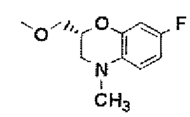 | 61<br>62<br>63<br>64<br>65 | H<br>F<br>Cl<br>$CH_3$<br>$CH_3O$ | 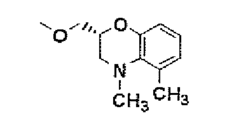 | 111<br>112<br>113<br>114<br>115 | H<br>F<br>Cl<br>$CH_3$<br>$CH_3O$ | 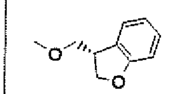 |
| 16<br>17<br>18<br>19<br>20 | H<br>F<br>Cl<br>$CH_3$<br>$CH_3O$ | 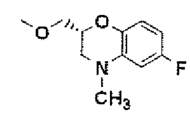 | 66<br>67<br>68<br>69<br>70 | H<br>F<br>Cl<br>$CH_3$<br>$CH_3O$ | 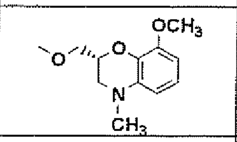 | 116<br>117<br>118<br>119<br>120 | H<br>F<br>Cl<br>$CH_3$<br>$CH_3O$ | 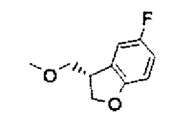 |
| 21<br>22<br>23<br>24<br>25 | H<br>F<br>Cl<br>$CH_3$<br>$CH_3O$ | 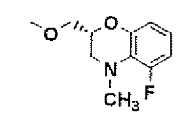 | 71<br>72<br>73<br>74<br>75 | H<br>F<br>Cl<br>$CH_3$<br>$CH_3O$ | 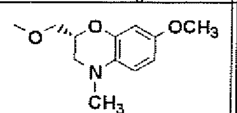 | 121<br>122<br>123<br>124<br>125 | H<br>F<br>Cl<br>$CH_3$<br>$CH_3O$ | 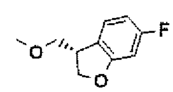 |
| 26<br>27<br>28<br>29<br>30 | H<br>F<br>Cl<br>$CH_3$<br>$CH_3O$ | 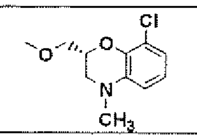 | 76<br>77<br>78<br>79<br>80 | H<br>F<br>Cl<br>$CH_3$<br>$CH_3O$ | 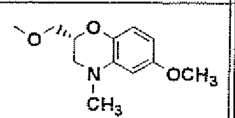 | 126<br>127<br>128<br>129<br>130 | H<br>F<br>Cl<br>$CH_3$<br>$CH_3O$ | 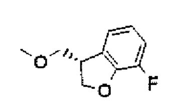 |
| 31<br>32<br>33<br>34<br>35 | H<br>F<br>Cl<br>$CH_3$<br>$CH_3O$ | 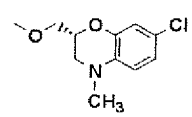 | 81<br>82<br>83<br>84<br>85 | H<br>F<br>Cl<br>$CH_3$<br>$CH_3O$ | 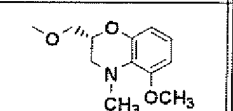 | 131<br>132<br>133<br>134<br>135 | H<br>F<br>Cl<br>$CH_3$<br>$CH_3O$ | 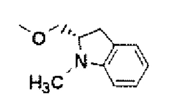 |
| 36<br>37<br>38<br>39<br>40 | H<br>F<br>Cl<br>$CH_3$<br>$CH_3O$ | 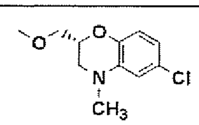 | 86<br>87<br>88<br>89<br>90 | H<br>F<br>Cl<br>$CH_3$<br>$CH_3O$ | 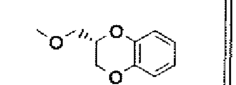 | 136<br>137<br>138<br>139<br>140 | H<br>F<br>Cl<br>$CH_3$<br>$CH_3O$ | 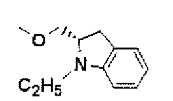 |
| 41<br>42<br>43<br>44<br>45 | H<br>F<br>Cl<br>$CH_3$<br>$CH_3O$ | 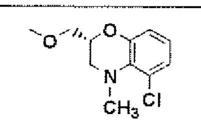 | 91<br>92<br>93<br>94<br>95 | H<br>F<br>Cl<br>$CH_3$<br>$CH_3O$ | 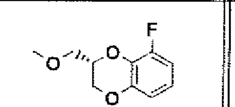 | 141<br>142<br>143<br>144<br>145 | H<br>F<br>Cl<br>$CH_3$<br>$CH_3O$ | 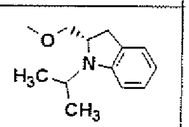 |
| 46<br>47<br>48<br>49<br>50 | H<br>F<br>Cl<br>$CH_3$<br>$CH_3O$ | 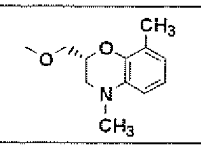 | 96<br>97<br>98<br>99<br>100 | H<br>F<br>Cl<br>$CH_3$<br>$CH_3O$ | 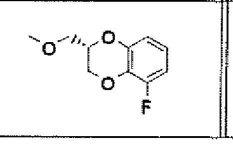 | 146<br>147<br>148<br>149<br>150 | H<br>F<br>Cl<br>$CH_3$<br>$CH_3O$ | 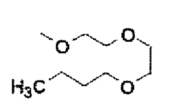 |

Table 45

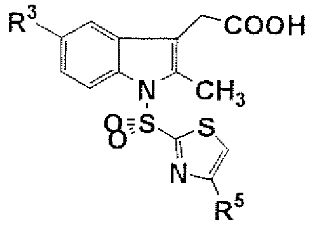

(I－B－8－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 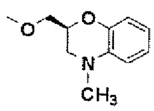 | 51<br>52<br>53<br>54<br>55 | H<br>F<br>Cl<br>$CH_3$<br>$CH_3O$ | 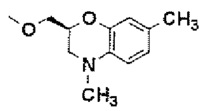 | 101<br>102<br>103<br>104<br>105 | H<br>F<br>Cl<br>$CH_3$<br>$CH_3O$ | 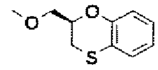 |
| 6<br>7<br>8<br>9<br>10 | H<br>F<br>Cl<br>$CH_3$<br>$CH_3O$ | 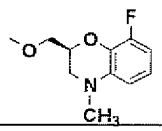 | 56<br>57<br>58<br>59<br>60 | H<br>F<br>Cl<br>$CH_3$<br>$CH_3O$ | 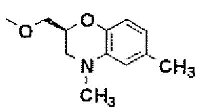 | 106<br>107<br>108<br>109<br>110 | H<br>F<br>Cl<br>$CH_3$<br>$CH_3O$ | 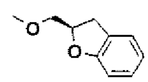 |
| 11<br>12<br>13<br>14<br>15 | H<br>F<br>Cl<br>$CH_3$<br>$CH_3O$ | 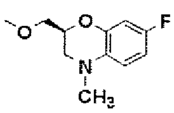 | 61<br>62<br>63<br>64<br>65 | H<br>F<br>Cl<br>$CH_3$<br>$CH_3O$ | 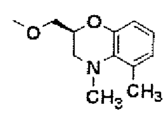 | 111<br>112<br>113<br>114<br>115 | H<br>F<br>Cl<br>$CH_3$<br>$CH_3O$ | 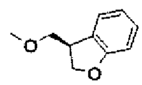 |
| 16<br>17<br>18<br>19<br>20 | H<br>F<br>Cl<br>$CH_3$<br>$CH_3O$ | 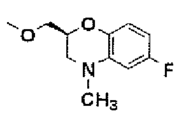 | 66<br>67<br>68<br>69<br>70 | H<br>F<br>Cl<br>$CH_3$<br>$CH_3O$ | 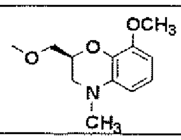 | 116<br>117<br>118<br>119<br>120 | H<br>F<br>Cl<br>$CH_3$<br>$CH_3O$ | 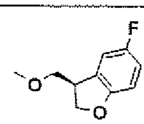 |
| 21<br>22<br>23<br>24<br>25 | H<br>F<br>Cl<br>$CH_3$<br>$CH_3O$ | 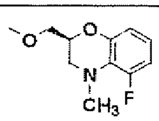 | 71<br>72<br>73<br>74<br>75 | H<br>F<br>Cl<br>$CH_3$<br>$CH_3O$ | 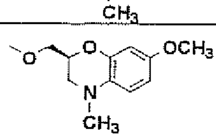 | 121<br>122<br>123<br>124<br>125 | H<br>F<br>Cl<br>$CH_3$<br>$CH_3O$ | 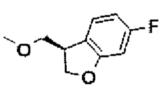 |
| 26<br>27<br>28<br>29<br>30 | H<br>F<br>Cl<br>$CH_3$<br>$CH_3O$ | 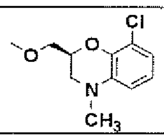 | 76<br>77<br>78<br>79<br>80 | H<br>F<br>Cl<br>$CH_3$<br>$CH_3O$ | 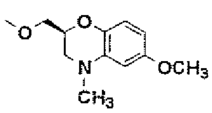 | 126<br>127<br>128<br>129<br>130 | H<br>F<br>Cl<br>$CH_3$<br>$CH_3O$ | 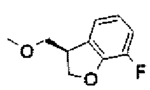 |
| 31<br>32<br>33<br>34<br>35 | H<br>F<br>Cl<br>$CH_3$<br>$CH_3O$ | 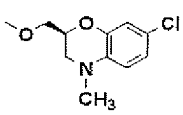 | 81<br>82<br>83<br>84<br>85 | H<br>F<br>Cl<br>$CH_3$<br>$CH_3O$ | 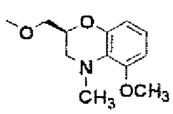 | 131<br>132<br>133<br>134<br>135 | H<br>F<br>Cl<br>$CH_3$<br>$CH_3O$ | 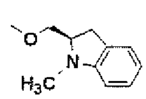 |
| 36<br>37<br>38<br>39<br>40 | H<br>F<br>Cl<br>$CH_3$<br>$CH_3O$ | 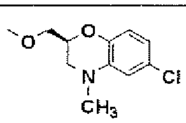 | 86<br>87<br>88<br>89<br>90 | H<br>F<br>Cl<br>$CH_3$<br>$CH_3O$ | 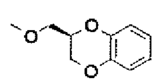 | 136<br>137<br>138<br>139<br>140 | H<br>F<br>Cl<br>$CH_3$<br>$CH_3O$ | 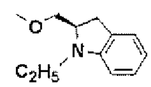 |
| 41<br>42<br>43<br>44<br>45 | H<br>F<br>Cl<br>$CH_3$<br>$CH_3O$ | 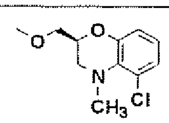 | 91<br>92<br>93<br>94<br>95 | H<br>F<br>Cl<br>$CH_3$<br>$CH_3O$ | 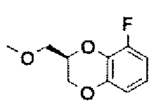 | 141<br>142<br>143<br>144<br>145 | H<br>F<br>Cl<br>$CH_3$<br>$CH_3O$ | 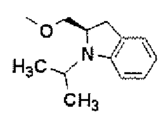 |
| 46<br>47<br>48<br>49<br>50 | H<br>F<br>Cl<br>$CH_3$<br>$CH_3O$ | 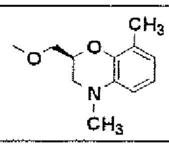 | 96<br>97<br>98<br>99<br>100 | H<br>F<br>Cl<br>$CH_3$<br>$CH_3O$ | 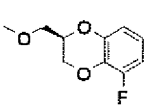 | 146<br>147<br>148<br>149<br>150 | H<br>F<br>Cl<br>$CH_3$<br>$CH_3O$ | 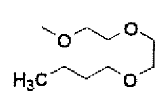 |

Table 46

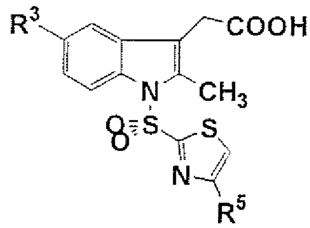

(1-B-8-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 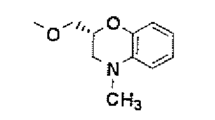 | 51<br>52<br>53<br>54<br>55 | H<br>F<br>Cl<br>$CH_3$<br>$CH_3O$ | 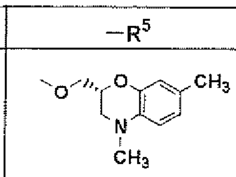 | 101<br>102<br>103<br>104<br>105 | H<br>F<br>Cl<br>$CH_3$<br>$CH_3O$ | 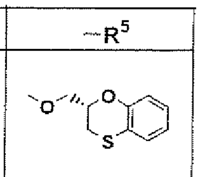 |
| 6<br>7<br>8<br>9<br>10 | H<br>F<br>Cl<br>$CH_3$<br>$CH_3O$ | 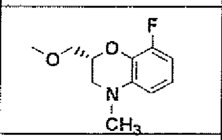 | 56<br>57<br>58<br>59<br>60 | H<br>F<br>Cl<br>$CH_3$<br>$CH_3O$ | 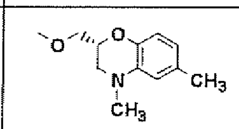 | 106<br>107<br>108<br>109<br>110 | H<br>F<br>Cl<br>$CH_3$<br>$CH_3O$ | 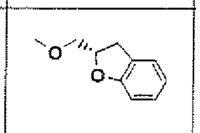 |
| 11<br>12<br>13<br>14<br>15 | H<br>F<br>Cl<br>$CH_3$<br>$CH_3O$ | 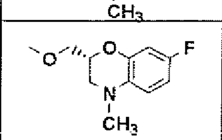 | 61<br>62<br>63<br>64<br>65 | H<br>F<br>Cl<br>$CH_3$<br>$CH_3O$ | 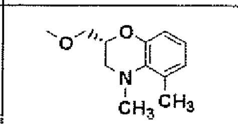 | 111<br>112<br>113<br>114<br>115 | H<br>F<br>Cl<br>$CH_3$<br>$CH_3O$ | 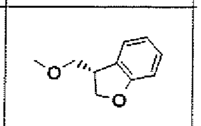 |
| 16<br>17<br>18<br>19<br>20 | H<br>F<br>Cl<br>$CH_3$<br>$CH_3O$ | 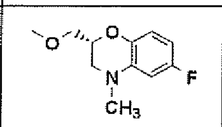 | 66<br>67<br>68<br>69<br>70 | H<br>F<br>Cl<br>$CH_3$<br>$CH_3O$ | 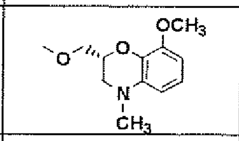 | 116<br>117<br>118<br>119<br>120 | H<br>F<br>Cl<br>$CH_3$<br>$CH_3O$ | 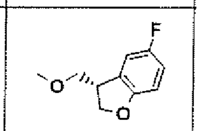 |
| 21<br>22<br>23<br>24<br>25 | H<br>F<br>Cl<br>$CH_3$<br>$CH_3O$ | 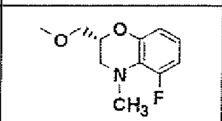 | 71<br>72<br>73<br>74<br>75 | H<br>F<br>Cl<br>$CH_3$<br>$CH_3O$ | 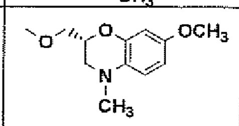 | 121<br>122<br>123<br>124<br>125 | H<br>F<br>Cl<br>$CH_3$<br>$CH_3O$ | 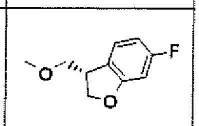 |
| 26<br>27<br>28<br>29<br>30 | H<br>F<br>Cl<br>$CH_3$<br>$CH_3O$ | 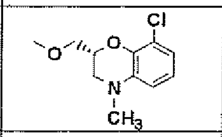 | 76<br>77<br>78<br>79<br>80 | H<br>F<br>Cl<br>$CH_3$<br>$CH_3O$ | 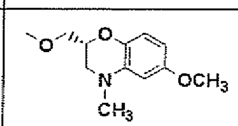 | 126<br>127<br>128<br>129<br>130 | H<br>F<br>Cl<br>$CH_3$<br>$CH_3O$ | 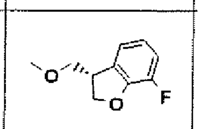 |
| 31<br>32<br>33<br>34<br>35 | H<br>F<br>Cl<br>$CH_3$<br>$CH_3O$ | 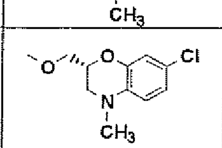 | 81<br>82<br>83<br>84<br>85 | H<br>F<br>Cl<br>$CH_3$<br>$CH_3O$ | 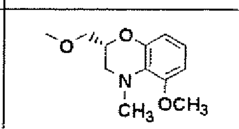 | 131<br>132<br>133<br>134<br>135 | H<br>F<br>Cl<br>$CH_3$<br>$CH_3O$ | 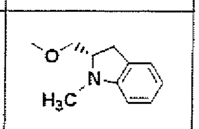 |
| 36<br>37<br>38<br>39<br>40 | H<br>F<br>Cl<br>$CH_3$<br>$CH_3O$ | 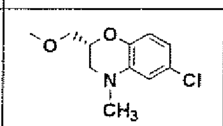 | 86<br>87<br>88<br>89<br>90 | H<br>F<br>Cl<br>$CH_3$<br>$CH_3O$ | 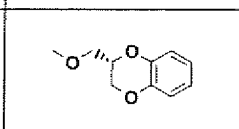 | 136<br>137<br>138<br>139<br>140 | H<br>F<br>Cl<br>$CH_3$<br>$CH_3O$ | 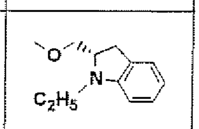 |
| 41<br>42<br>43<br>44<br>45 | H<br>F<br>Cl<br>$CH_3$<br>$CH_3O$ | 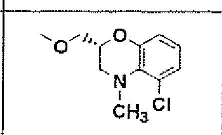 | 91<br>92<br>93<br>94<br>95 | H<br>F<br>Cl<br>$CH_3$<br>$CH_3O$ | 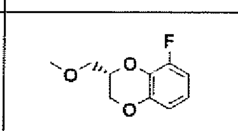 | 141<br>142<br>143<br>144<br>145 | H<br>F<br>Cl<br>$CH_3$<br>$CH_3O$ | 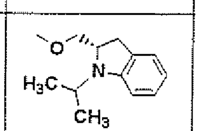 |
| 46<br>47<br>48<br>49<br>50 | H<br>F<br>Cl<br>$CH_3$<br>$CH_3O$ | 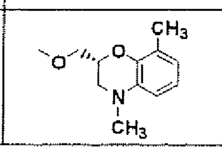 | 96<br>97<br>98<br>99<br>100 | H<br>F<br>Cl<br>$CH_3$<br>$CH_3O$ | 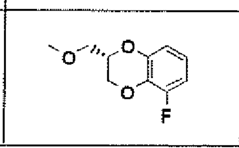 | 146<br>147<br>148<br>149<br>150 | H<br>F<br>Cl<br>$CH_3$<br>$CH_3O$ | 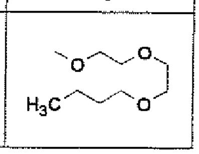 |

Table 47

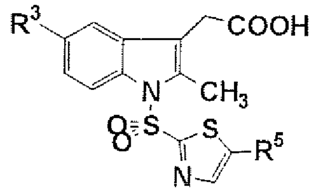

(I－B－8－2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 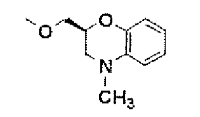 | 51<br>52<br>53<br>54<br>55 | H<br>F<br>Cl<br>$CH_3$<br>$CH_3O$ | 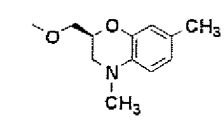 | 101<br>102<br>103<br>104<br>105 | H<br>F<br>Cl<br>$CH_3$<br>$CH_3O$ | 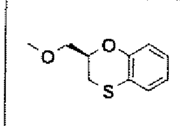 |
| 6<br>7<br>8<br>9<br>10 | H<br>F<br>Cl<br>$CH_3$<br>$CH_3O$ | 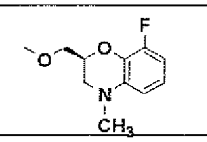 | 56<br>57<br>58<br>59<br>60 | H<br>F<br>Cl<br>$CH_3$<br>$CH_3O$ | 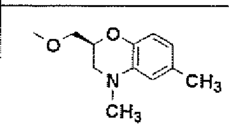 | 106<br>107<br>108<br>109<br>110 | H<br>F<br>Cl<br>$CH_3$<br>$CH_3O$ | 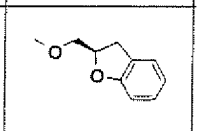 |
| 11<br>12<br>13<br>14<br>15 | H<br>F<br>Cl<br>$CH_3$<br>$CH_3O$ | 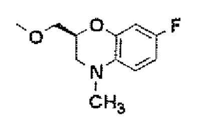 | 61<br>62<br>63<br>64<br>65 | H<br>F<br>Cl<br>$CH_3$<br>$CH_3O$ | 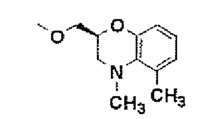 | 111<br>112<br>113<br>114<br>115 | H<br>F<br>Cl<br>$CH_3$<br>$CH_3O$ | 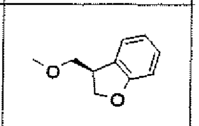 |
| 16<br>17<br>18<br>19<br>20 | H<br>F<br>Cl<br>$CH_3$<br>$CH_3O$ | 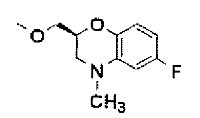 | 66<br>67<br>68<br>69<br>70 | H<br>F<br>Cl<br>$CH_3$<br>$CH_3O$ | 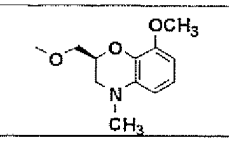 | 116<br>117<br>118<br>119<br>120 | H<br>F<br>Cl<br>$CH_3$<br>$CH_3O$ | 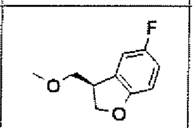 |
| 21<br>22<br>23<br>24<br>25 | H<br>F<br>Cl<br>$CH_3$<br>$CH_3O$ | 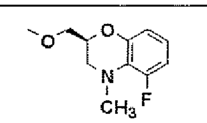 | 71<br>72<br>73<br>74<br>75 | H<br>F<br>Cl<br>$CH_3$<br>$CH_3O$ | 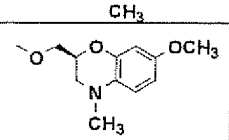 | 121<br>122<br>123<br>124<br>125 | H<br>F<br>Cl<br>$CH_3$<br>$CH_3O$ | 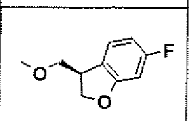 |
| 26<br>27<br>28<br>29<br>30 | H<br>F<br>Cl<br>$CH_3$<br>$CH_3O$ | 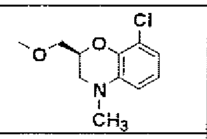 | 76<br>77<br>78<br>79<br>80 | H<br>F<br>Cl<br>$CH_3$<br>$CH_3O$ | 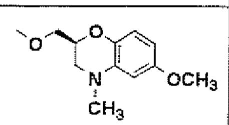 | 126<br>127<br>128<br>129<br>130 | H<br>F<br>Cl<br>$CH_3$<br>$CH_3O$ | 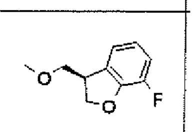 |
| 31<br>32<br>33<br>34<br>35 | H<br>F<br>Cl<br>$CH_3$<br>$CH_3O$ | 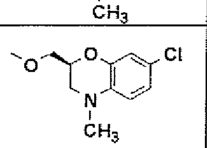 | 81<br>82<br>83<br>84<br>85 | H<br>F<br>Cl<br>$CH_3$<br>$CH_3O$ | 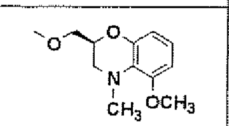 | 131<br>132<br>133<br>134<br>135 | H<br>F<br>Cl<br>$CH_3$<br>$CH_3O$ | 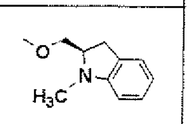 |
| 36<br>37<br>38<br>39<br>40 | H<br>F<br>Cl<br>$CH_3$<br>$CH_3O$ | 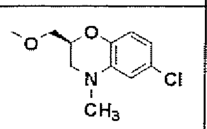 | 86<br>87<br>88<br>89<br>90 | H<br>F<br>Cl<br>$CH_3$<br>$CH_3O$ | 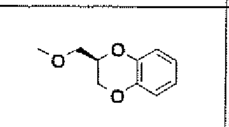 | 136<br>137<br>138<br>139<br>140 | H<br>F<br>Cl<br>$CH_3$<br>$CH_3O$ | 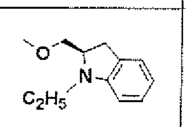 |
| 41<br>42<br>43<br>44<br>45 | H<br>F<br>Cl<br>$CH_3$<br>$CH_3O$ | 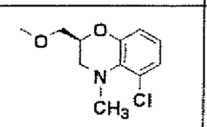 | 91<br>92<br>93<br>94<br>95 | H<br>F<br>Cl<br>$CH_3$<br>$CH_3O$ | 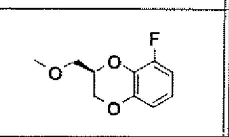 | 141<br>142<br>143<br>144<br>145 | H<br>F<br>Cl<br>$CH_3$<br>$CH_3O$ | 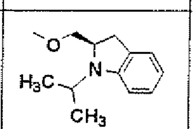 |
| 46<br>47<br>48<br>49<br>50 | H<br>F<br>Cl<br>$CH_3$<br>$CH_3O$ | 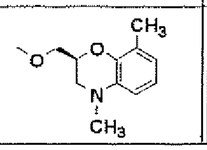 | 96<br>97<br>98<br>99<br>100 | H<br>F<br>Cl<br>$CH_3$<br>$CH_3O$ | 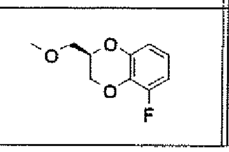 | 146<br>147<br>148<br>149<br>150 | H<br>F<br>Cl<br>$CH_3$<br>$CH_3O$ | 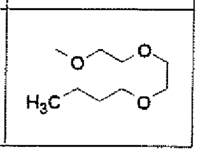 |

Table 48

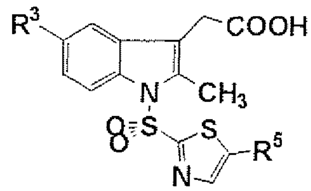

(I－B－8－2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 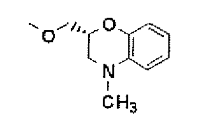 | 51<br>52<br>53<br>54<br>55 | H<br>F<br>Cl<br>$CH_3$<br>$CH_3O$ | 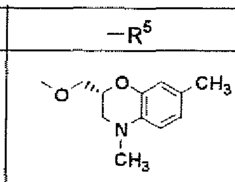 | 101<br>102<br>103<br>104<br>105 | H<br>F<br>Cl<br>$CH_3$<br>$CH_3O$ | 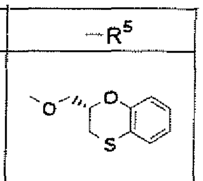 |
| 6<br>7<br>8<br>9<br>10 | H<br>F<br>Cl<br>$CH_3$<br>$CH_3O$ | 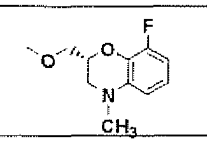 | 56<br>57<br>58<br>59<br>60 | H<br>F<br>Cl<br>$CH_3$<br>$CH_3O$ | 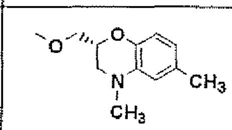 | 106<br>107<br>108<br>109<br>110 | H<br>F<br>Cl<br>$CH_3$<br>$CH_3O$ | 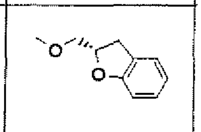 |
| 11<br>12<br>13<br>14<br>15 | H<br>F<br>Cl<br>$CH_3$<br>$CH_3O$ | 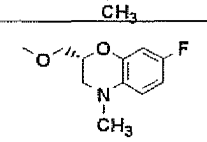 | 61<br>62<br>63<br>64<br>65 | H<br>F<br>Cl<br>$CH_3$<br>$CH_3O$ | 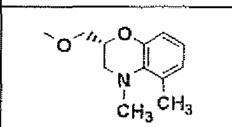 | 111<br>112<br>113<br>114<br>115 | H<br>F<br>Cl<br>$CH_3$<br>$CH_3O$ | 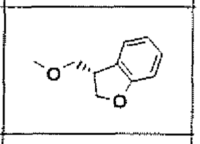 |
| 16<br>17<br>18<br>19<br>20 | H<br>F<br>Cl<br>$CH_3$<br>$CH_3O$ | 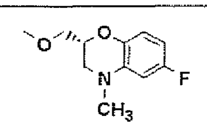 | 66<br>67<br>68<br>69<br>70 | H<br>F<br>Cl<br>$CH_3$<br>$CH_3O$ | 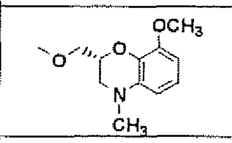 | 116<br>117<br>118<br>119<br>120 | H<br>F<br>Cl<br>$CH_3$<br>$CH_3O$ | 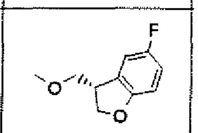 |
| 21<br>22<br>23<br>24<br>25 | H<br>F<br>Cl<br>$CH_3$<br>$CH_3O$ | 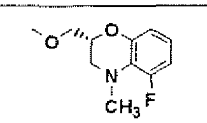 | 71<br>72<br>73<br>74<br>75 | H<br>F<br>Cl<br>$CH_3$<br>$CH_3O$ | 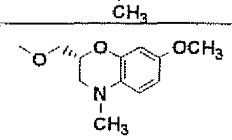 | 121<br>122<br>123<br>124<br>125 | H<br>F<br>Cl<br>$CH_3$<br>$CH_3O$ | 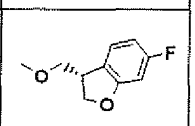 |
| 26<br>27<br>28<br>29<br>30 | H<br>F<br>Cl<br>$CH_3$<br>$CH_3O$ | 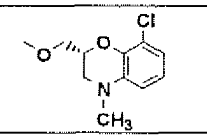 | 76<br>77<br>78<br>79<br>80 | H<br>F<br>Cl<br>$CH_3$<br>$CH_3O$ | 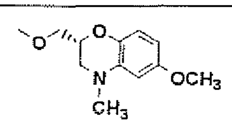 | 126<br>127<br>128<br>129<br>130 | H<br>F<br>Cl<br>$CH_3$<br>$CH_3O$ | 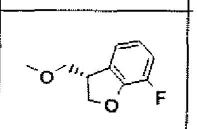 |
| 31<br>32<br>33<br>34<br>35 | H<br>F<br>Cl<br>$CH_3$<br>$CH_3O$ | 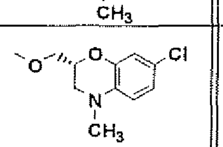 | 81<br>82<br>83<br>84<br>85 | H<br>F<br>Cl<br>$CH_3$<br>$CH_3O$ | 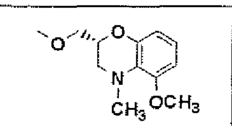 | 131<br>132<br>133<br>134<br>135 | H<br>F<br>Cl<br>$CH_3$<br>$CH_3O$ | 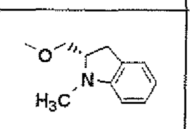 |
| 36<br>37<br>38<br>39<br>40 | H<br>F<br>Cl<br>$CH_3$<br>$CH_3O$ | 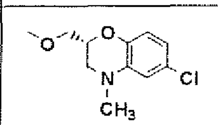 | 86<br>87<br>88<br>89<br>90 | H<br>F<br>Cl<br>$CH_3$<br>$CH_3O$ | 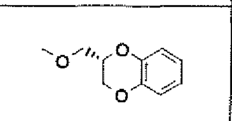 | 136<br>137<br>138<br>139<br>140 | H<br>F<br>Cl<br>$CH_3$<br>$CH_3O$ | 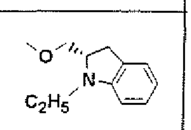 |
| 41<br>42<br>43<br>44<br>45 | H<br>F<br>Cl<br>$CH_3$<br>$CH_3O$ | 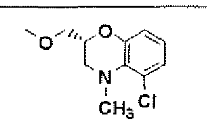 | 91<br>92<br>93<br>94<br>95 | H<br>F<br>Cl<br>$CH_3$<br>$CH_3O$ | 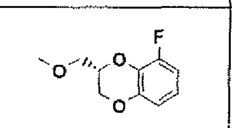 | 141<br>142<br>143<br>144<br>145 | H<br>F<br>Cl<br>$CH_3$<br>$CH_3O$ | 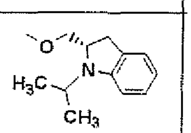 |
| 46<br>47<br>48<br>49<br>50 | H<br>F<br>Cl<br>$CH_3$<br>$CH_3O$ | 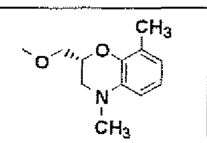 | 96<br>97<br>98<br>99<br>100 | H<br>F<br>Cl<br>$CH_3$<br>$CH_3O$ | 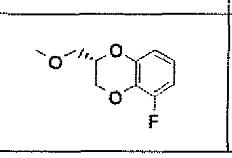 | 146<br>147<br>148<br>149<br>150 | H<br>F<br>Cl<br>$CH_3$<br>$CH_3O$ | 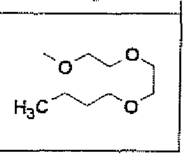 |

Table 49

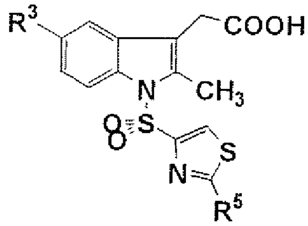

(I-B-9-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 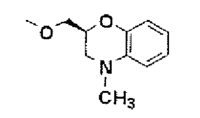 | 51<br>52<br>53<br>54<br>55 | H<br>F<br>Cl<br>$CH_3$<br>$CH_3O$ | 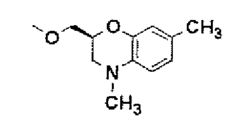 | 101<br>102<br>103<br>104<br>105 | H<br>F<br>Cl<br>$CH_3$<br>$CH_3O$ | 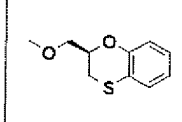 |
| 6<br>7<br>8<br>9<br>10 | H<br>F<br>Cl<br>$CH_3$<br>$CH_3O$ | 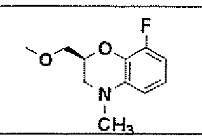 | 56<br>57<br>58<br>59<br>60 | H<br>F<br>Cl<br>$CH_3$<br>$CH_3O$ | 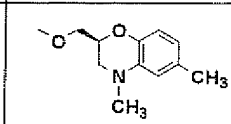 | 106<br>107<br>108<br>109<br>110 | H<br>F<br>Cl<br>$CH_3$<br>$CH_3O$ | 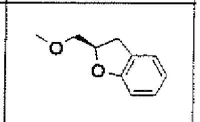 |
| 11<br>12<br>13<br>14<br>15 | H<br>F<br>Cl<br>$CH_3$<br>$CH_3O$ | 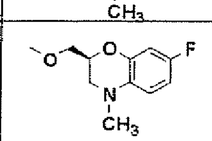 | 61<br>62<br>63<br>64<br>65 | H<br>F<br>Cl<br>$CH_3$<br>$CH_3O$ | 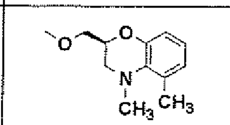 | 111<br>112<br>113<br>114<br>115 | H<br>F<br>Cl<br>$CH_3$<br>$CH_3O$ | 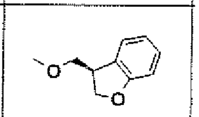 |
| 16<br>17<br>18<br>19<br>20 | H<br>F<br>Cl<br>$CH_3$<br>$CH_3O$ | 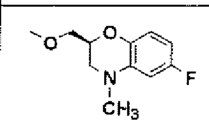 | 66<br>67<br>68<br>69<br>70 | H<br>F<br>Cl<br>$CH_3$<br>$CH_3O$ | 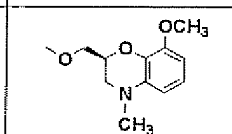 | 116<br>117<br>118<br>119<br>120 | H<br>F<br>Cl<br>$CH_3$<br>$CH_3O$ | 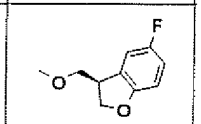 |
| 21<br>22<br>23<br>24<br>25 | H<br>F<br>Cl<br>$CH_3$<br>$CH_3O$ | 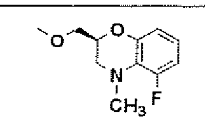 | 71<br>72<br>73<br>74<br>75 | H<br>F<br>Cl<br>$CH_3$<br>$CH_3O$ | 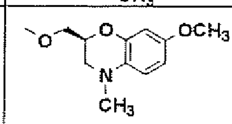 | 121<br>122<br>123<br>124<br>125 | H<br>F<br>Cl<br>$CH_3$<br>$CH_3O$ | 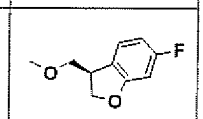 |
| 26<br>27<br>28<br>29<br>30 | H<br>F<br>Cl<br>$CH_3$<br>$CH_3O$ | 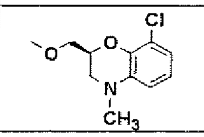 | 76<br>77<br>78<br>79<br>80 | H<br>F<br>Cl<br>$CH_3$<br>$CH_3O$ | 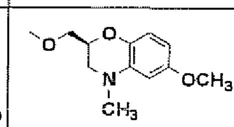 | 126<br>127<br>128<br>129<br>130 | H<br>F<br>Cl<br>$CH_3$<br>$CH_3O$ | 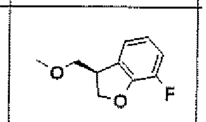 |
| 31<br>32<br>33<br>34<br>35 | H<br>F<br>Cl<br>$CH_3$<br>$CH_3O$ | 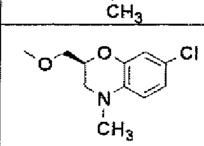 | 81<br>82<br>83<br>84<br>85 | H<br>F<br>Cl<br>$CH_3$<br>$CH_3O$ | 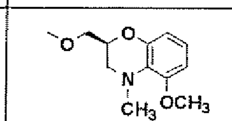 | 131<br>132<br>133<br>134<br>135 | H<br>F<br>Cl<br>$CH_3$<br>$CH_3O$ | 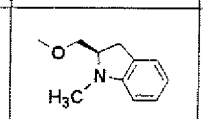 |
| 36<br>37<br>38<br>39<br>40 | H<br>F<br>Cl<br>$CH_3$<br>$CH_3O$ | 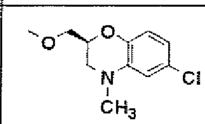 | 86<br>87<br>88<br>89<br>90 | H<br>F<br>Cl<br>$CH_3$<br>$CH_3O$ | 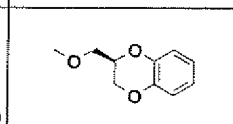 | 136<br>137<br>138<br>139<br>140 | H<br>F<br>Cl<br>$CH_3$<br>$CH_3O$ | 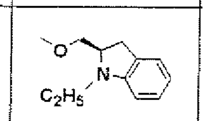 |
| 41<br>42<br>43<br>44<br>45 | H<br>F<br>Cl<br>$CH_3$<br>$CH_3O$ | 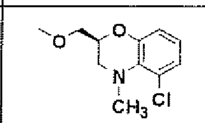 | 91<br>92<br>93<br>94<br>95 | H<br>F<br>Cl<br>$CH_3$<br>$CH_3O$ | 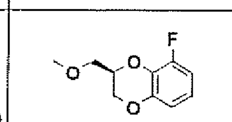 | 141<br>142<br>143<br>144<br>145 | H<br>F<br>Cl<br>$CH_3$<br>$CH_3O$ | 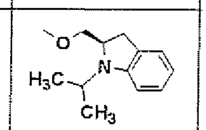 |
| 46<br>47<br>48<br>49<br>50 | H<br>F<br>Cl<br>$CH_3$<br>$CH_3O$ | 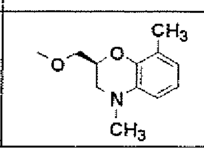 | 96<br>97<br>98<br>99<br>100 | H<br>F<br>Cl<br>$CH_3$<br>$CH_3O$ | 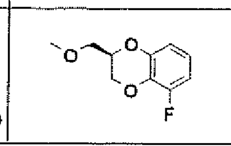 | 146<br>147<br>148<br>149<br>150 | H<br>F<br>Cl<br>$CH_3$<br>$CH_3O$ | 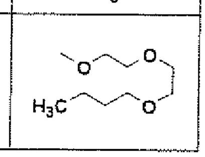 |

Table 50

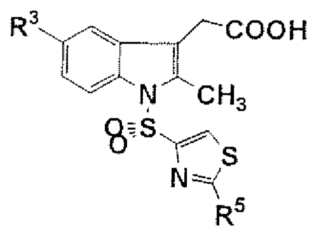

(I−B−9−1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 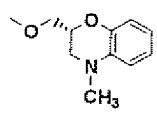 | 51<br>52<br>53<br>54<br>55 | H<br>F<br>Cl<br>$CH_3$<br>$CH_3O$ | 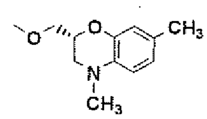 | 101<br>102<br>103<br>104<br>105 | H<br>F<br>Cl<br>$CH_3$<br>$CH_3O$ | 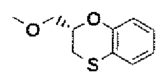 |
| 6<br>7<br>8<br>9<br>10 | H<br>F<br>Cl<br>$CH_3$<br>$CH_3O$ | 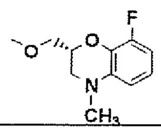 | 56<br>57<br>58<br>59<br>60 | H<br>F<br>Cl<br>$CH_3$<br>$CH_3O$ | 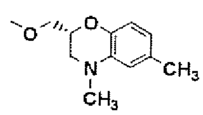 | 106<br>107<br>108<br>109<br>110 | H<br>F<br>Cl<br>$CH_3$<br>$CH_3O$ | 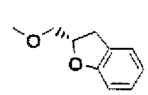 |
| 11<br>12<br>13<br>14<br>15 | H<br>F<br>Cl<br>$CH_3$<br>$CH_3O$ | 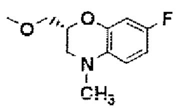 | 61<br>62<br>63<br>64<br>65 | H<br>F<br>Cl<br>$CH_3$<br>$CH_3O$ | 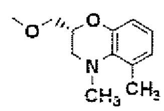 | 111<br>112<br>113<br>114<br>115 | H<br>F<br>Cl<br>$CH_3$<br>$CH_3O$ | 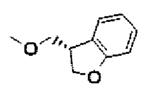 |
| 16<br>17<br>18<br>19<br>20 | H<br>F<br>Cl<br>$CH_3$<br>$CH_3O$ | 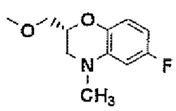 | 66<br>67<br>68<br>69<br>70 | H<br>F<br>Cl<br>$CH_3$<br>$CH_3O$ | 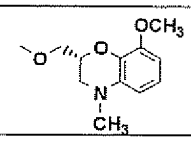 | 116<br>117<br>118<br>119<br>120 | H<br>F<br>Cl<br>$CH_3$<br>$CH_3O$ | 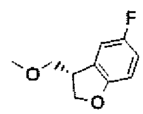 |
| 21<br>22<br>23<br>24<br>25 | H<br>F<br>Cl<br>$CH_3$<br>$CH_3O$ | 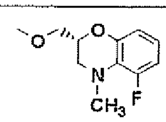 | 71<br>72<br>73<br>74<br>75 | H<br>F<br>Cl<br>$CH_3$<br>$CH_3O$ | 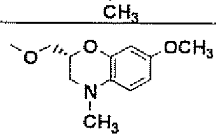 | 121<br>122<br>123<br>124<br>125 | H<br>F<br>Cl<br>$CH_3$<br>$CH_3O$ | 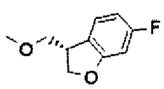 |
| 26<br>27<br>28<br>29<br>30 | H<br>F<br>Cl<br>$CH_3$<br>$CH_3O$ | 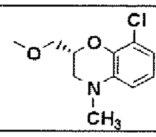 | 76<br>77<br>78<br>79<br>80 | H<br>F<br>Cl<br>$CH_3$<br>$CH_3O$ | 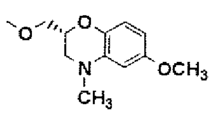 | 126<br>127<br>128<br>129<br>130 | H<br>F<br>Cl<br>$CH_3$<br>$CH_3O$ | 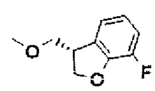 |
| 31<br>32<br>33<br>34<br>35 | H<br>F<br>Cl<br>$CH_3$<br>$CH_3O$ | 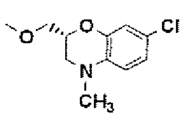 | 81<br>82<br>83<br>84<br>85 | H<br>F<br>Cl<br>$CH_3$<br>$CH_3O$ | 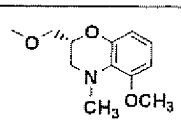 | 131<br>132<br>133<br>134<br>135 | H<br>F<br>Cl<br>$CH_3$<br>$CH_3O$ | 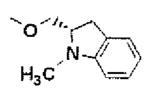 |
| 36<br>37<br>38<br>39<br>40 | H<br>F<br>Cl<br>$CH_3$<br>$CH_3O$ | 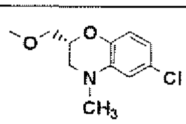 | 86<br>87<br>88<br>89<br>90 | H<br>F<br>Cl<br>$CH_3$<br>$CH_3O$ | 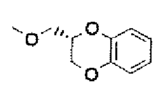 | 136<br>137<br>138<br>139<br>140 | H<br>F<br>Cl<br>$CH_3$<br>$CH_3O$ | 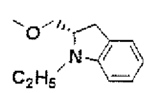 |
| 41<br>42<br>43<br>44<br>45 | H<br>F<br>Cl<br>$CH_3$<br>$CH_3O$ | 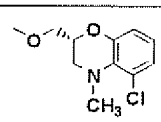 | 91<br>92<br>93<br>94<br>95 | H<br>F<br>Cl<br>$CH_3$<br>$CH_3O$ | 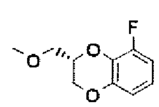 | 141<br>142<br>143<br>144<br>145 | H<br>F<br>Cl<br>$CH_3$<br>$CH_3O$ | 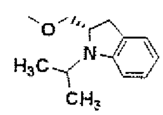 |
| 46<br>47<br>48<br>49<br>50 | H<br>F<br>Cl<br>$CH_3$<br>$CH_3O$ | 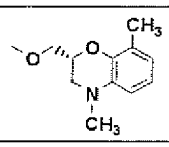 | 96<br>97<br>98<br>99<br>100 | H<br>F<br>Cl<br>$CH_3$<br>$CH_3O$ | 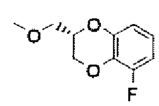 | 146<br>147<br>148<br>149<br>150 | H<br>F<br>Cl<br>$CH_3$<br>$CH_3O$ | 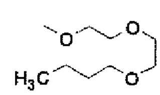 |

Table 51

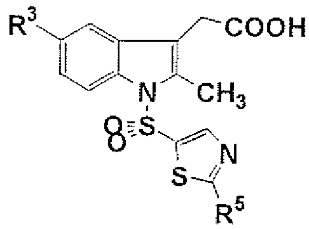

(I-B-10-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 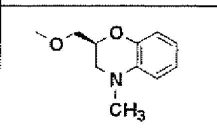 | 51<br>52<br>53<br>54<br>55 | H<br>F<br>Cl<br>$CH_3$<br>$CH_3O$ | 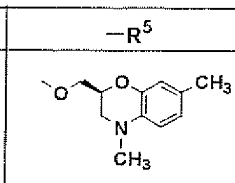 | 101<br>102<br>103<br>104<br>105 | H<br>F<br>Cl<br>$CH_3$<br>$CH_3O$ | 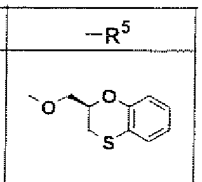 |
| 6<br>7<br>8<br>9<br>10 | H<br>F<br>Cl<br>$CH_3$<br>$CH_3O$ | 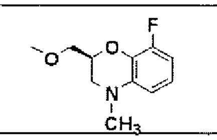 | 56<br>57<br>58<br>59<br>60 | H<br>F<br>Cl<br>$CH_3$<br>$CH_3O$ | 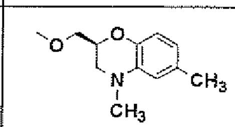 | 106<br>107<br>108<br>109<br>110 | H<br>F<br>Cl<br>$CH_3$<br>$CH_3O$ | 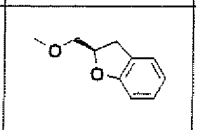 |
| 11<br>12<br>13<br>14<br>15 | H<br>F<br>Cl<br>$CH_3$<br>$CH_3O$ | 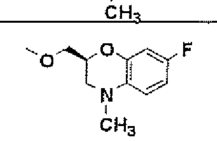 | 61<br>62<br>63<br>64<br>65 | H<br>F<br>Cl<br>$CH_3$<br>$CH_3O$ | 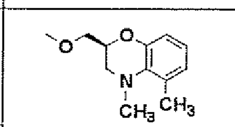 | 111<br>112<br>113<br>114<br>115 | H<br>F<br>Cl<br>$CH_3$<br>$CH_3O$ | 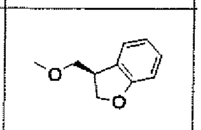 |
| 16<br>17<br>18<br>19<br>20 | H<br>F<br>Cl<br>$CH_3$<br>$CH_3O$ | 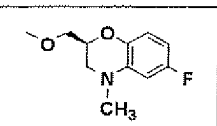 | 66<br>67<br>68<br>69<br>70 | H<br>F<br>Cl<br>$CH_3$<br>$CH_3O$ | 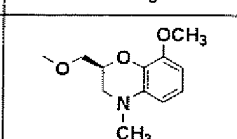 | 116<br>117<br>118<br>119<br>120 | H<br>F<br>Cl<br>$CH_3$<br>$CH_3O$ | 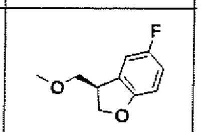 |
| 21<br>22<br>23<br>24<br>25 | H<br>F<br>Cl<br>$CH_3$<br>$CH_3O$ | 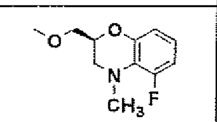 | 71<br>72<br>73<br>74<br>75 | H<br>F<br>Cl<br>$CH_3$<br>$CH_3O$ | 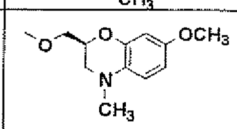 | 121<br>122<br>123<br>124<br>125 | H<br>F<br>Cl<br>$CH_3$<br>$CH_3O$ | 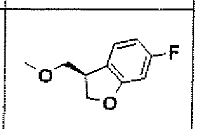 |
| 26<br>27<br>28<br>29<br>30 | H<br>F<br>Cl<br>$CH_3$<br>$CH_3O$ | 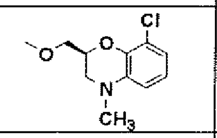 | 76<br>77<br>78<br>79<br>80 | H<br>F<br>Cl<br>$CH_3$<br>$CH_3O$ | 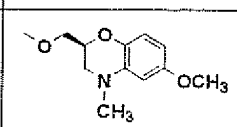 | 126<br>127<br>128<br>129<br>130 | H<br>F<br>Cl<br>$CH_3$<br>$CH_3O$ | 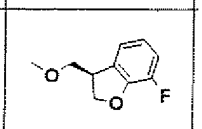 |
| 31<br>32<br>33<br>34<br>35 | H<br>F<br>Cl<br>$CH_3$<br>$CH_3O$ | 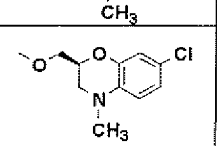 | 81<br>82<br>83<br>84<br>85 | H<br>F<br>Cl<br>$CH_3$<br>$CH_3O$ | 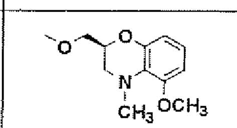 | 131<br>132<br>133<br>134<br>135 | H<br>F<br>Cl<br>$CH_3$<br>$CH_3O$ | 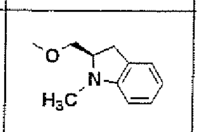 |
| 36<br>37<br>38<br>39<br>40 | H<br>F<br>Cl<br>$CH_3$<br>$CH_3O$ | 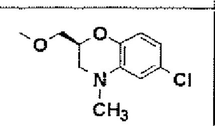 | 86<br>87<br>88<br>89<br>90 | H<br>F<br>Cl<br>$CH_3$<br>$CH_3O$ | 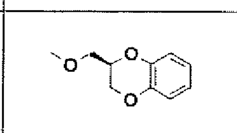 | 136<br>137<br>138<br>139<br>140 | H<br>F<br>Cl<br>$CH_3$<br>$CH_3O$ | 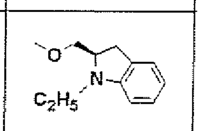 |
| 41<br>42<br>43<br>44<br>45 | H<br>F<br>Cl<br>$CH_3$<br>$CH_3O$ | 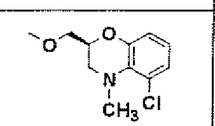 | 91<br>92<br>93<br>94<br>95 | H<br>F<br>Cl<br>$CH_3$<br>$CH_3O$ | 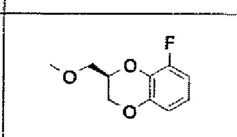 | 141<br>142<br>143<br>144<br>145 | H<br>F<br>Cl<br>$CH_3$<br>$CH_3O$ | 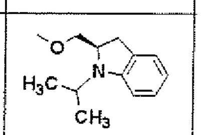 |
| 46<br>47<br>48<br>49<br>50 | H<br>F<br>Cl<br>$CH_3$<br>$CH_3O$ | 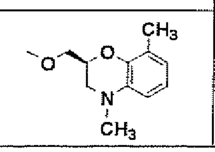 | 96<br>97<br>98<br>99<br>100 | H<br>F<br>Cl<br>$CH_3$<br>$CH_3O$ | 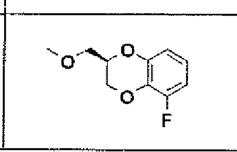 | 146<br>147<br>148<br>149<br>150 | H<br>F<br>Cl<br>$CH_3$<br>$CH_3O$ | 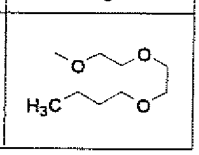 |

Table 52

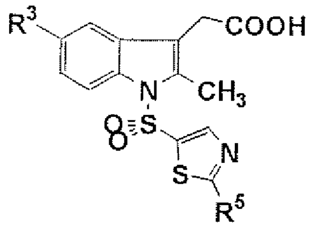

(1-B-10-1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 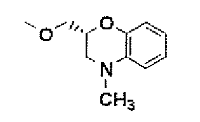 | 51<br>52<br>53<br>54<br>55 | H<br>F<br>Cl<br>$CH_3$<br>$CH_3O$ | 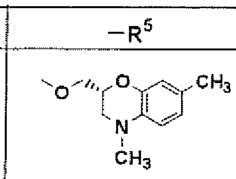 | 101<br>102<br>103<br>104<br>105 | H<br>F<br>Cl<br>$CH_3$<br>$CH_3O$ | 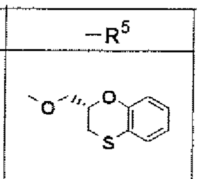 |
| 6<br>7<br>8<br>9<br>10 | H<br>F<br>Cl<br>$CH_3$<br>$CH_3O$ | 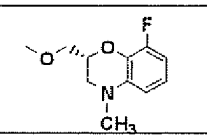 | 56<br>57<br>58<br>59<br>60 | H<br>F<br>Cl<br>$CH_3$<br>$CH_3O$ | 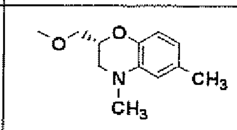 | 106<br>107<br>108<br>109<br>110 | H<br>F<br>Cl<br>$CH_3$<br>$CH_3O$ | 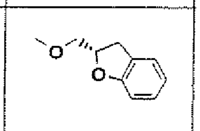 |
| 11<br>12<br>13<br>14<br>15 | H<br>F<br>Cl<br>$CH_3$<br>$CH_3O$ | 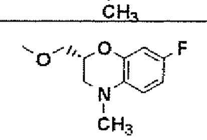 | 61<br>62<br>63<br>64<br>65 | H<br>F<br>Cl<br>$CH_3$<br>$CH_3O$ | 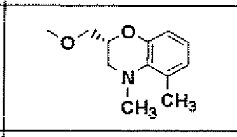 | 111<br>112<br>113<br>114<br>115 | H<br>F<br>Cl<br>$CH_3$<br>$CH_3O$ | 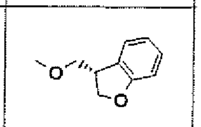 |
| 16<br>17<br>18<br>19<br>20 | H<br>F<br>Cl<br>$CH_3$<br>$CH_3O$ | 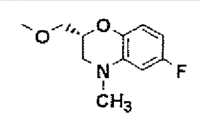 | 66<br>67<br>68<br>69<br>70 | H<br>F<br>Cl<br>$CH_3$<br>$CH_3O$ | 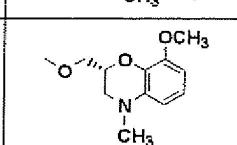 | 116<br>117<br>118<br>119<br>120 | H<br>F<br>Cl<br>$CH_3$<br>$CH_3O$ | 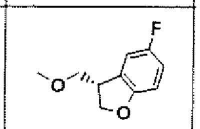 |
| 21<br>22<br>23<br>24<br>25 | H<br>F<br>Cl<br>$CH_3$<br>$CH_3O$ | 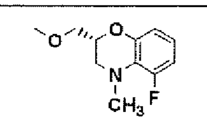 | 71<br>72<br>73<br>74<br>75 | H<br>F<br>Cl<br>$CH_3$<br>$CH_3O$ | 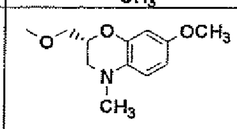 | 121<br>122<br>123<br>124<br>125 | H<br>F<br>Cl<br>$CH_3$<br>$CH_3O$ | 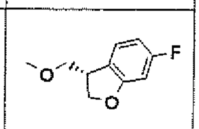 |
| 26<br>27<br>28<br>29<br>30 | H<br>F<br>Cl<br>$CH_3$<br>$CH_3O$ | 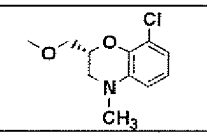 | 76<br>77<br>78<br>79<br>80 | H<br>F<br>Cl<br>$CH_3$<br>$CH_3O$ | 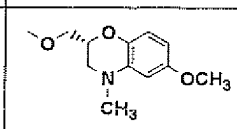 | 126<br>127<br>128<br>129<br>130 | H<br>F<br>Cl<br>$CH_3$<br>$CH_3O$ | 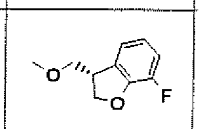 |
| 31<br>32<br>33<br>34<br>35 | H<br>F<br>Cl<br>$CH_3$<br>$CH_3O$ | 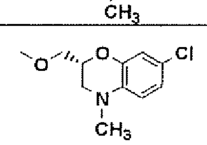 | 81<br>82<br>83<br>84<br>85 | H<br>F<br>Cl<br>$CH_3$<br>$CH_3O$ | 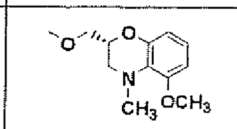 | 131<br>132<br>133<br>134<br>135 | H<br>F<br>Cl<br>$CH_3$<br>$CH_3O$ | 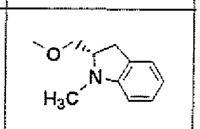 |
| 36<br>37<br>38<br>39<br>40 | H<br>F<br>Cl<br>$CH_3$<br>$CH_3O$ | 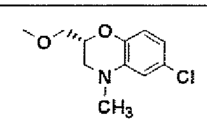 | 86<br>87<br>88<br>89<br>90 | H<br>F<br>Cl<br>$CH_3$<br>$CH_3O$ | 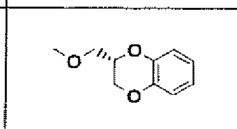 | 136<br>137<br>138<br>139<br>140 | H<br>F<br>Cl<br>$CH_3$<br>$CH_3O$ | 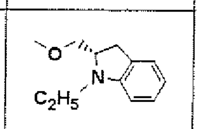 |
| 41<br>42<br>43<br>44<br>45 | H<br>F<br>Cl<br>$CH_3$<br>$CH_3O$ | 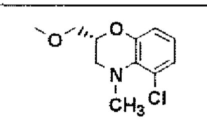 | 91<br>92<br>93<br>94<br>95 | H<br>F<br>Cl<br>$CH_3$<br>$CH_3O$ | 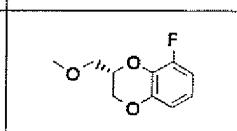 | 141<br>142<br>143<br>144<br>145 | H<br>F<br>Cl<br>$CH_3$<br>$CH_3O$ | 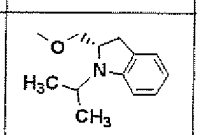 |
| 46<br>47<br>48<br>49<br>50 | H<br>F<br>Cl<br>$CH_3$<br>$CH_3O$ | 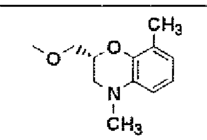 | 96<br>97<br>98<br>99<br>100 | H<br>F<br>Cl<br>$CH_3$<br>$CH_3O$ | 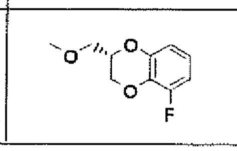 | 146<br>147<br>148<br>149<br>150 | H<br>F<br>Cl<br>$CH_3$<br>$CH_3O$ | 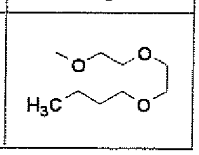 |

Table 53

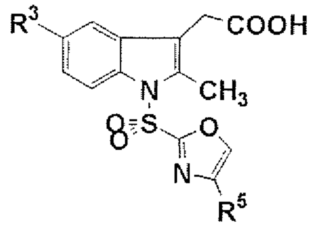

(I－B－11－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 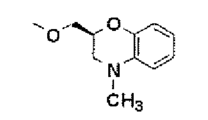 | 51<br>52<br>53<br>54<br>55 | H<br>F<br>Cl<br>$CH_3$<br>$CH_3O$ | 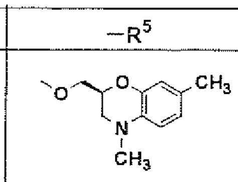 | 101<br>102<br>103<br>104<br>105 | H<br>F<br>Cl<br>$CH_3$<br>$CH_3O$ | 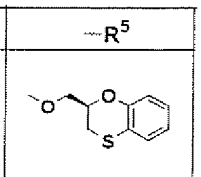 |
| 6<br>7<br>8<br>9<br>10 | H<br>F<br>Cl<br>$CH_3$<br>$CH_3O$ | 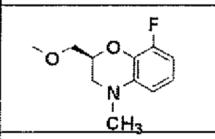 | 56<br>57<br>58<br>59<br>60 | H<br>F<br>Cl<br>$CH_3$<br>$CH_3O$ | 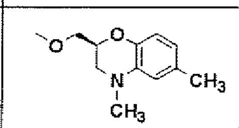 | 106<br>107<br>108<br>109<br>110 | H<br>F<br>Cl<br>$CH_3$<br>$CH_3O$ | 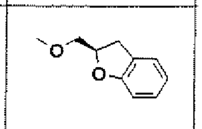 |
| 11<br>12<br>13<br>14<br>15 | H<br>F<br>Cl<br>$CH_3$<br>$CH_3O$ | 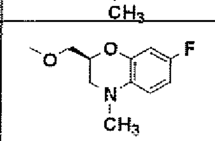 | 61<br>62<br>63<br>64<br>65 | H<br>F<br>Cl<br>$CH_3$<br>$CH_3O$ | 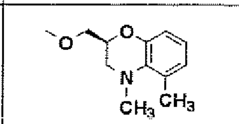 | 111<br>112<br>113<br>114<br>115 | H<br>F<br>Cl<br>$CH_3$<br>$CH_3O$ | 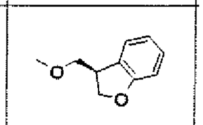 |
| 16<br>17<br>18<br>19<br>20 | H<br>F<br>Cl<br>$CH_3$<br>$CH_3O$ | 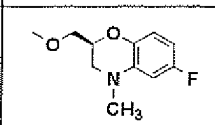 | 66<br>67<br>68<br>69<br>70 | H<br>F<br>Cl<br>$CH_3$<br>$CH_3O$ | 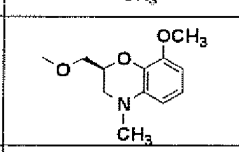 | 116<br>117<br>118<br>119<br>120 | H<br>F<br>Cl<br>$CH_3$<br>$CH_3O$ | 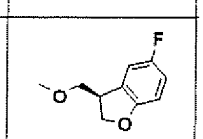 |
| 21<br>22<br>23<br>24<br>25 | H<br>F<br>Cl<br>$CH_3$<br>$CH_3O$ | 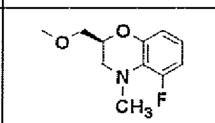 | 71<br>72<br>73<br>74<br>75 | H<br>F<br>Cl<br>$CH_3$<br>$CH_3O$ | 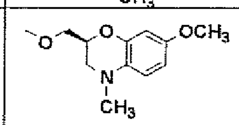 | 121<br>122<br>123<br>124<br>125 | H<br>F<br>Cl<br>$CH_3$<br>$CH_3O$ | 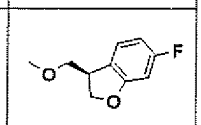 |
| 26<br>27<br>28<br>29<br>30 | H<br>F<br>Cl<br>$CH_3$<br>$CH_3O$ | 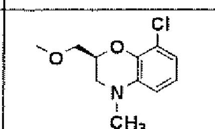 | 76<br>77<br>78<br>79<br>80 | H<br>F<br>Cl<br>$CH_3$<br>$CH_3O$ | 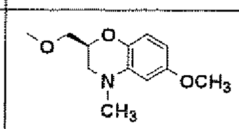 | 126<br>127<br>128<br>129<br>130 | H<br>F<br>Cl<br>$CH_3$<br>$CH_3O$ | 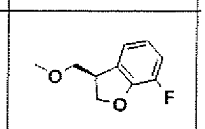 |
| 31<br>32<br>33<br>34<br>35 | H<br>F<br>Cl<br>$CH_3$<br>$CH_3O$ | 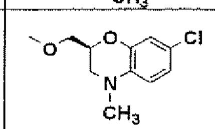 | 81<br>82<br>83<br>84<br>85 | H<br>F<br>Cl<br>$CH_3$<br>$CH_3O$ | 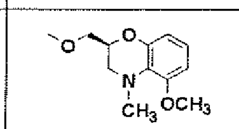 | 131<br>132<br>133<br>134<br>135 | H<br>F<br>Cl<br>$CH_3$<br>$CH_3O$ | 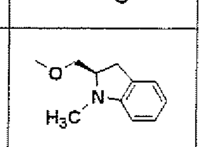 |
| 36<br>37<br>38<br>39<br>40 | H<br>F<br>Cl<br>$CH_3$<br>$CH_3O$ | 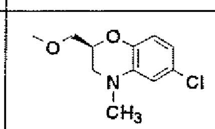 | 86<br>87<br>88<br>89<br>90 | H<br>F<br>Cl<br>$CH_3$<br>$CH_3O$ | 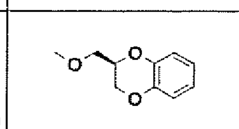 | 136<br>137<br>138<br>139<br>140 | H<br>F<br>Cl<br>$CH_3$<br>$CH_3O$ | 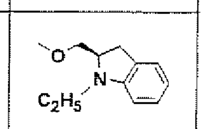 |
| 41<br>42<br>43<br>44<br>45 | H<br>F<br>Cl<br>$CH_3$<br>$CH_3O$ | 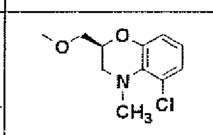 | 91<br>92<br>93<br>94<br>95 | H<br>F<br>Cl<br>$CH_3$<br>$CH_3O$ | 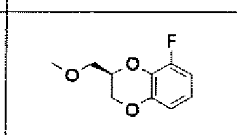 | 141<br>142<br>143<br>144<br>145 | H<br>F<br>Cl<br>$CH_3$<br>$CH_3O$ | 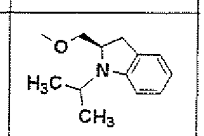 |
| 46<br>47<br>48<br>49<br>50 | H<br>F<br>Cl<br>$CH_3$<br>$CH_3O$ | 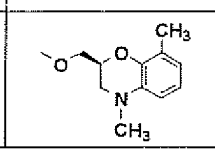 | 96<br>97<br>98<br>99<br>100 | H<br>F<br>Cl<br>$CH_3$<br>$CH_3O$ | 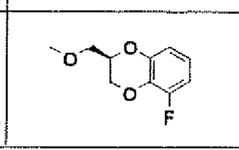 | 146<br>147<br>148<br>149<br>150 | H<br>F<br>Cl<br>$CH_3$<br>$CH_3O$ | 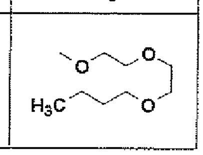 |

Table 54

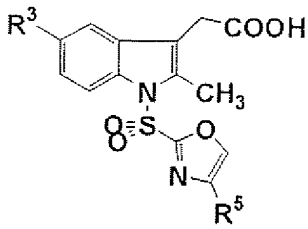

(I−B−11−1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 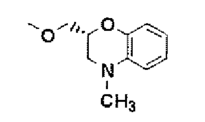 | 51<br>52<br>53<br>54<br>55 | H<br>F<br>Cl<br>$CH_3$<br>$CH_3O$ | 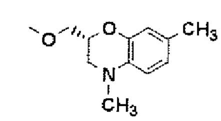 | 101<br>102<br>103<br>104<br>105 | H<br>F<br>Cl<br>$CH_3$<br>$CH_3O$ | 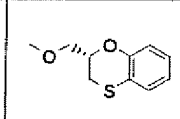 |
| 6<br>7<br>8<br>9<br>10 | H<br>F<br>Cl<br>$CH_3$<br>$CH_3O$ | 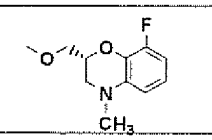 | 56<br>57<br>58<br>59<br>60 | H<br>F<br>Cl<br>$CH_3$<br>$CH_3O$ | 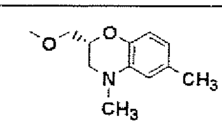 | 106<br>107<br>108<br>109<br>110 | H<br>F<br>Cl<br>$CH_3$<br>$CH_3O$ | 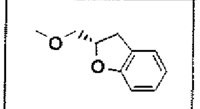 |
| 11<br>12<br>13<br>14<br>15 | H<br>F<br>Cl<br>$CH_3$<br>$CH_3O$ | 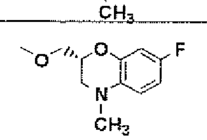 | 61<br>62<br>63<br>64<br>65 | H<br>F<br>Cl<br>$CH_3$<br>$CH_3O$ | 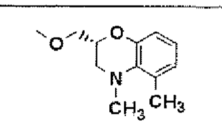 | 111<br>112<br>113<br>114<br>115 | H<br>F<br>Cl<br>$CH_3$<br>$CH_3O$ | 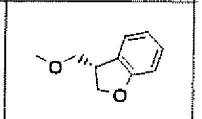 |
| 16<br>17<br>18<br>19<br>20 | H<br>F<br>Cl<br>$CH_3$<br>$CH_3O$ | 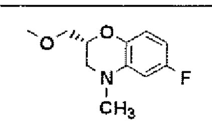 | 66<br>67<br>68<br>69<br>70 | H<br>F<br>Cl<br>$CH_3$<br>$CH_3O$ | 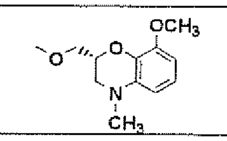 | 116<br>117<br>118<br>119<br>120 | H<br>F<br>Cl<br>$CH_3$<br>$CH_3O$ | 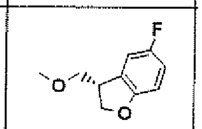 |
| 21<br>22<br>23<br>24<br>25 | H<br>F<br>Cl<br>$CH_3$<br>$CH_3O$ | 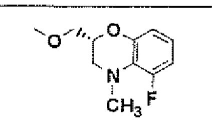 | 71<br>72<br>73<br>74<br>75 | H<br>F<br>Cl<br>$CH_3$<br>$CH_3O$ | 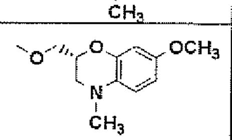 | 121<br>122<br>123<br>124<br>125 | H<br>F<br>Cl<br>$CH_3$<br>$CH_3O$ | 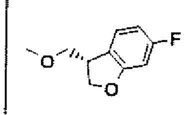 |
| 26<br>27<br>28<br>29<br>30 | H<br>F<br>Cl<br>$CH_3$<br>$CH_3O$ | 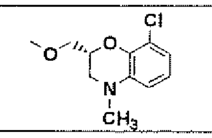 | 76<br>77<br>78<br>79<br>80 | H<br>F<br>Cl<br>$CH_3$<br>$CH_3O$ | 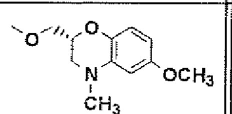 | 126<br>127<br>128<br>129<br>130 | H<br>F<br>Cl<br>$CH_3$<br>$CH_3O$ | 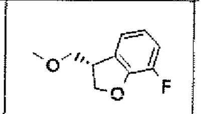 |
| 31<br>32<br>33<br>34<br>35 | H<br>F<br>Cl<br>$CH_3$<br>$CH_3O$ | 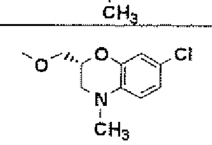 | 81<br>82<br>83<br>84<br>85 | H<br>F<br>Cl<br>$CH_3$<br>$CH_3O$ | 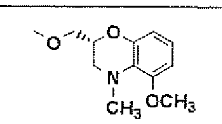 | 131<br>132<br>133<br>134<br>135 | H<br>F<br>Cl<br>$CH_3$<br>$CH_3O$ | 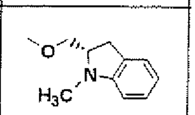 |
| 36<br>37<br>38<br>39<br>40 | H<br>F<br>Cl<br>$CH_3$<br>$CH_3O$ | 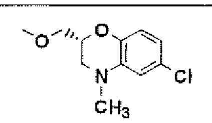 | 86<br>87<br>88<br>89<br>90 | H<br>F<br>Cl<br>$CH_3$<br>$CH_3O$ | 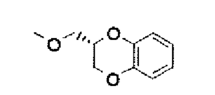 | 136<br>137<br>138<br>139<br>140 | H<br>F<br>Cl<br>$CH_3$<br>$CH_3O$ | 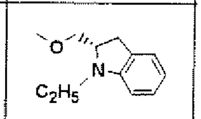 |
| 41<br>42<br>43<br>44<br>45 | H<br>F<br>Cl<br>$CH_3$<br>$CH_3O$ | 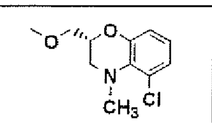 | 91<br>92<br>93<br>94<br>95 | H<br>F<br>Cl<br>$CH_3$<br>$CH_3O$ | 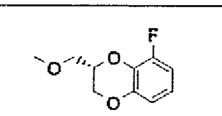 | 141<br>142<br>143<br>144<br>145 | H<br>F<br>Cl<br>$CH_3$<br>$CH_3O$ | 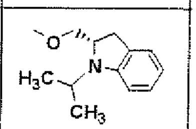 |
| 46<br>47<br>48<br>49<br>50 | H<br>F<br>Cl<br>$CH_3$<br>$CH_3O$ | 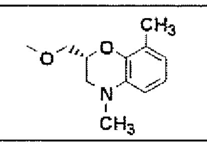 | 96<br>97<br>98<br>99<br>100 | H<br>F<br>Cl<br>$CH_3$<br>$CH_3O$ | 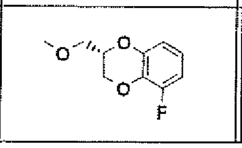 | 146<br>147<br>148<br>149<br>150 | H<br>F<br>Cl<br>$CH_3$<br>$CH_3O$ | 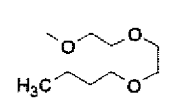 |

Table 55

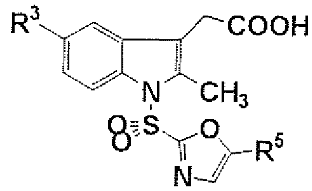

(I-B-11-2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 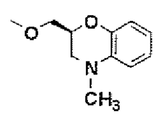 | 51<br>52<br>53<br>54<br>55 | H<br>F<br>Cl<br>$CH_3$<br>$CH_3O$ | 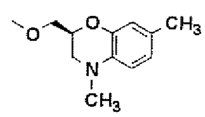 | 101<br>102<br>103<br>104<br>105 | H<br>F<br>Cl<br>$CH_3$<br>$CH_3O$ | 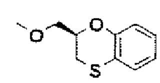 |
| 6<br>7<br>8<br>9<br>10 | H<br>F<br>Cl<br>$CH_3$<br>$CH_3O$ | 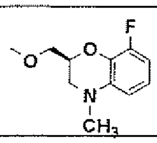 | 56<br>57<br>58<br>59<br>60 | H<br>F<br>Cl<br>$CH_3$<br>$CH_3O$ | 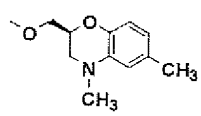 | 106<br>107<br>108<br>109<br>110 | H<br>F<br>Cl<br>$CH_3$<br>$CH_3O$ | 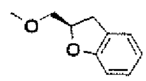 |
| 11<br>12<br>13<br>14<br>15 | H<br>F<br>Cl<br>$CH_3$<br>$CH_3O$ | 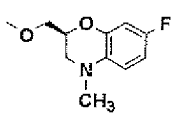 | 61<br>62<br>63<br>64<br>65 | H<br>F<br>Cl<br>$CH_3$<br>$CH_3O$ | 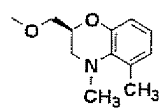 | 111<br>112<br>113<br>114<br>115 | H<br>F<br>Cl<br>$CH_3$<br>$CH_3O$ | 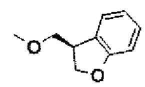 |
| 16<br>17<br>18<br>19<br>20 | H<br>F<br>Cl<br>$CH_3$<br>$CH_3O$ | 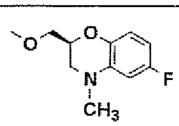 | 66<br>67<br>68<br>69<br>70 | H<br>F<br>Cl<br>$CH_3$<br>$CH_3O$ | 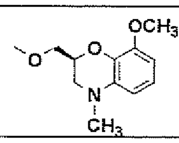 | 116<br>117<br>118<br>119<br>120 | H<br>F<br>Cl<br>$CH_3$<br>$CH_3O$ | 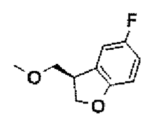 |
| 21<br>22<br>23<br>24<br>25 | H<br>F<br>Cl<br>$CH_3$<br>$CH_3O$ | 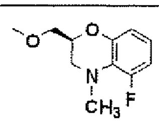 | 71<br>72<br>73<br>74<br>75 | H<br>F<br>Cl<br>$CH_3$<br>$CH_3O$ | 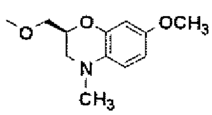 | 121<br>122<br>123<br>124<br>125 | H<br>F<br>Cl<br>$CH_3$<br>$CH_3O$ | 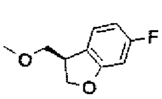 |
| 26<br>27<br>28<br>29<br>30 | H<br>F<br>Cl<br>$CH_3$<br>$CH_3O$ | 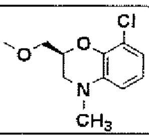 | 76<br>77<br>78<br>79<br>80 | H<br>F<br>Cl<br>$CH_3$<br>$CH_3O$ | 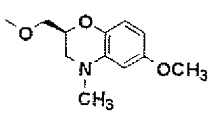 | 126<br>127<br>128<br>129<br>130 | H<br>F<br>Cl<br>$CH_3$<br>$CH_3O$ | 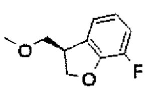 |
| 31<br>32<br>33<br>34<br>35 | H<br>F<br>Cl<br>$CH_3$<br>$CH_3O$ | 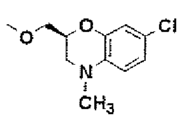 | 81<br>82<br>83<br>84<br>85 | H<br>F<br>Cl<br>$CH_3$<br>$CH_3O$ | 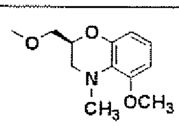 | 131<br>132<br>133<br>134<br>135 | H<br>F<br>Cl<br>$CH_3$<br>$CH_3O$ | 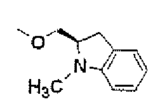 |
| 36<br>37<br>38<br>39<br>40 | H<br>F<br>Cl<br>$CH_3$<br>$CH_3O$ | 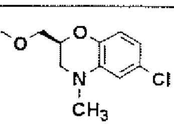 | 86<br>87<br>88<br>89<br>90 | H<br>F<br>Cl<br>$CH_3$<br>$CH_3O$ | 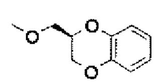 | 136<br>137<br>138<br>139<br>140 | H<br>F<br>Cl<br>$CH_3$<br>$CH_3O$ | 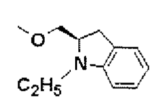 |
| 41<br>42<br>43<br>44<br>45 | H<br>F<br>Cl<br>$CH_3$<br>$CH_3O$ | 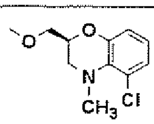 | 91<br>92<br>93<br>94<br>95 | H<br>F<br>Cl<br>$CH_3$<br>$CH_3O$ | 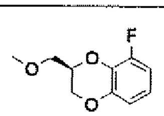 | 141<br>142<br>143<br>144<br>145 | H<br>F<br>Cl<br>$CH_3$<br>$CH_3O$ | 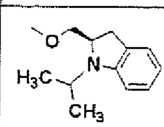 |
| 46<br>47<br>48<br>49<br>50 | H<br>F<br>Cl<br>$CH_3$<br>$CH_3O$ | 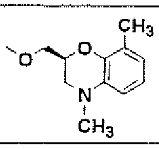 | 96<br>97<br>98<br>99<br>100 | H<br>F<br>Cl<br>$CH_3$<br>$CH_3O$ | 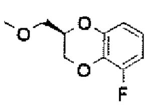 | 146<br>147<br>148<br>149<br>150 | H<br>F<br>Cl<br>$CH_3$<br>$CH_3O$ | 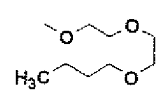 |

Table 56

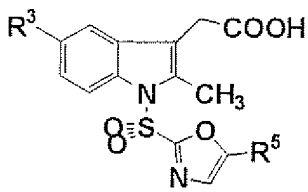

(I-B-11-2)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 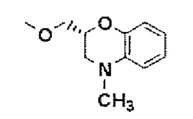 | 51<br>52<br>53<br>54<br>55 | H<br>F<br>Cl<br>$CH_3$<br>$CH_3O$ | 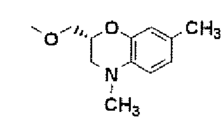 | 101<br>102<br>103<br>104<br>105 | H<br>F<br>Cl<br>$CH_3$<br>$CH_3O$ | 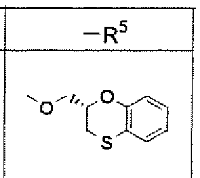 |
| 6<br>7<br>8<br>9<br>10 | H<br>F<br>Cl<br>$CH_3$<br>$CH_3O$ | 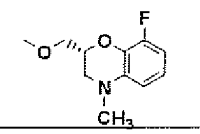 | 56<br>57<br>58<br>59<br>60 | H<br>F<br>Cl<br>$CH_3$<br>$CH_3O$ | 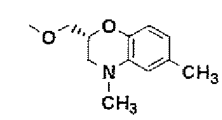 | 106<br>107<br>108<br>109<br>110 | H<br>F<br>Cl<br>$CH_3$<br>$CH_3O$ | 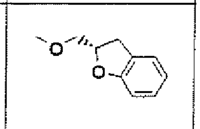 |
| 11<br>12<br>13<br>14<br>15 | H<br>F<br>Cl<br>$CH_3$<br>$CH_3O$ | 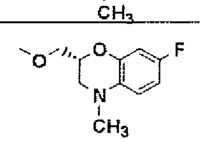 | 61<br>62<br>63<br>64<br>65 | H<br>F<br>Cl<br>$CH_3$<br>$CH_3O$ | 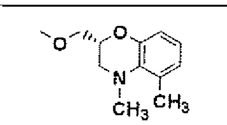 | 111<br>112<br>113<br>114<br>115 | H<br>F<br>Cl<br>$CH_3$<br>$CH_3O$ | 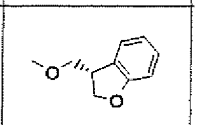 |
| 16<br>17<br>18<br>19<br>20 | H<br>F<br>Cl<br>$CH_3$<br>$CH_3O$ | 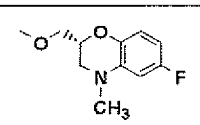 | 66<br>67<br>68<br>69<br>70 | H<br>F<br>Cl<br>$CH_3$<br>$CH_3O$ | 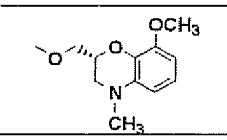 | 116<br>117<br>118<br>119<br>120 | H<br>F<br>Cl<br>$CH_3$<br>$CH_3O$ | 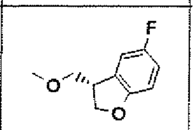 |
| 21<br>22<br>23<br>24<br>25 | H<br>F<br>Cl<br>$CH_3$<br>$CH_3O$ | 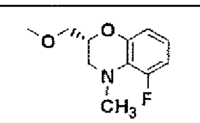 | 71<br>72<br>73<br>74<br>75 | H<br>F<br>Cl<br>$CH_3$<br>$CH_3O$ | 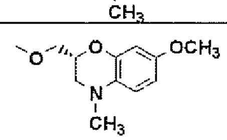 | 121<br>122<br>123<br>124<br>125 | H<br>F<br>Cl<br>$CH_3$<br>$CH_3O$ | 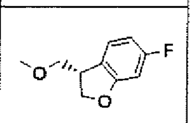 |
| 26<br>27<br>28<br>29<br>30 | H<br>F<br>Cl<br>$CH_3$<br>$CH_3O$ | 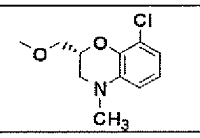 | 76<br>77<br>78<br>79<br>80 | H<br>F<br>Cl<br>$CH_3$<br>$CH_3O$ | 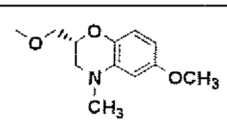 | 126<br>127<br>128<br>129<br>130 | H<br>F<br>Cl<br>$CH_3$<br>$CH_3O$ | 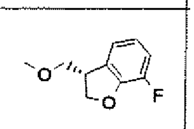 |
| 31<br>32<br>33<br>34<br>35 | H<br>F<br>Cl<br>$CH_3$<br>$CH_3O$ | 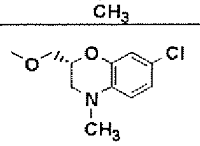 | 81<br>82<br>83<br>84<br>85 | H<br>F<br>Cl<br>$CH_3$<br>$CH_3O$ | 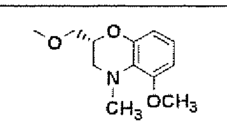 | 131<br>132<br>133<br>134<br>135 | H<br>F<br>Cl<br>$CH_3$<br>$CH_3O$ | 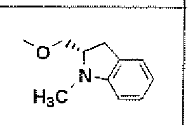 |
| 36<br>37<br>38<br>39<br>40 | H<br>F<br>Cl<br>$CH_3$<br>$CH_3O$ | 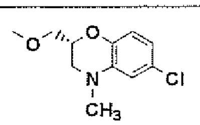 | 86<br>87<br>88<br>89<br>90 | H<br>F<br>Cl<br>$CH_3$<br>$CH_3O$ | 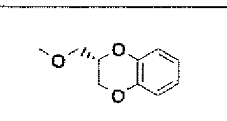 | 136<br>137<br>138<br>139<br>140 | H<br>F<br>Cl<br>$CH_3$<br>$CH_3O$ | 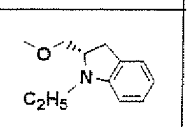 |
| 41<br>42<br>43<br>44<br>45 | H<br>F<br>Cl<br>$CH_3$<br>$CH_3O$ | 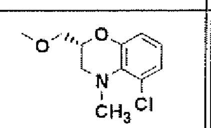 | 91<br>92<br>93<br>94<br>95 | H<br>F<br>Cl<br>$CH_3$<br>$CH_3O$ | 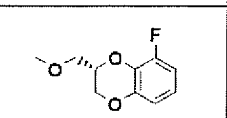 | 141<br>142<br>143<br>144<br>145 | H<br>F<br>Cl<br>$CH_3$<br>$CH_3O$ | 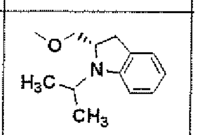 |
| 46<br>47<br>48<br>49<br>50 | H<br>F<br>Cl<br>$CH_3$<br>$CH_3O$ | 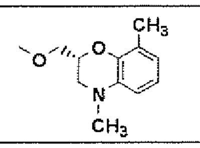 | 96<br>97<br>98<br>99<br>100 | H<br>F<br>Cl<br>$CH_3$<br>$CH_3O$ | 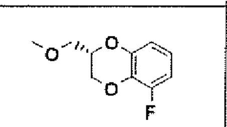 | 146<br>147<br>148<br>149<br>150 | H<br>F<br>Cl<br>$CH_3$<br>$CH_3O$ | 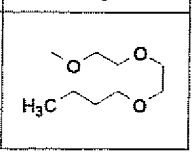 |

Table 57

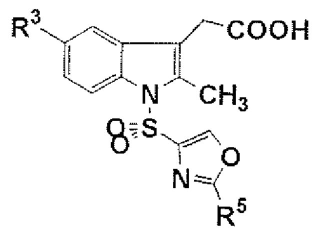

(I－B－12－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 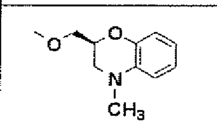 | 51<br>52<br>53<br>54<br>55 | H<br>F<br>Cl<br>$CH_3$<br>$CH_3O$ | 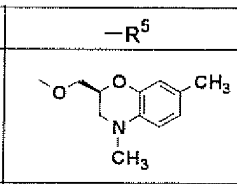 | 101<br>102<br>103<br>104<br>105 | H<br>F<br>Cl<br>$CH_3$<br>$CH_3O$ | 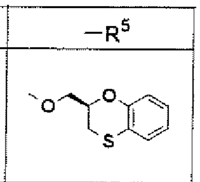 |
| 6<br>7<br>8<br>9<br>10 | H<br>F<br>Cl<br>$CH_3$<br>$CH_3O$ | 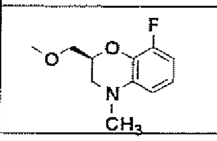 | 56<br>57<br>58<br>59<br>60 | H<br>F<br>Cl<br>$CH_3$<br>$CH_3O$ | 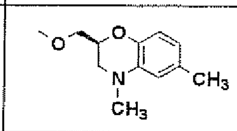 | 106<br>107<br>108<br>109<br>110 | H<br>F<br>Cl<br>$CH_3$<br>$CH_3O$ | 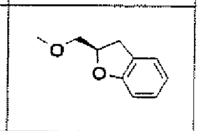 |
| 11<br>12<br>13<br>14<br>15 | H<br>F<br>Cl<br>$CH_3$<br>$CH_3O$ | 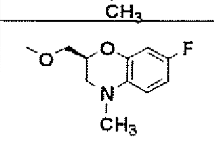 | 61<br>62<br>63<br>64<br>65 | H<br>F<br>Cl<br>$CH_3$<br>$CH_3O$ | 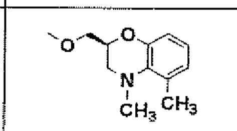 | 111<br>112<br>113<br>114<br>115 | H<br>F<br>Cl<br>$CH_3$<br>$CH_3O$ | 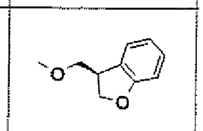 |
| 16<br>17<br>18<br>19<br>20 | H<br>F<br>Cl<br>$CH_3$<br>$CH_3O$ | 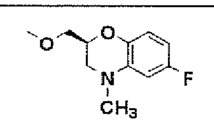 | 66<br>67<br>68<br>69<br>70 | H<br>F<br>Cl<br>$CH_3$<br>$CH_3O$ | 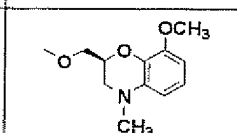 | 116<br>117<br>118<br>119<br>120 | H<br>F<br>Cl<br>$CH_3$<br>$CH_3O$ | 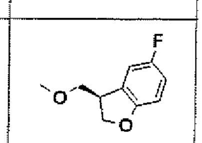 |
| 21<br>22<br>23<br>24<br>25 | H<br>F<br>Cl<br>$CH_3$<br>$CH_3O$ | 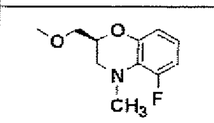 | 71<br>72<br>73<br>74<br>75 | H<br>F<br>Cl<br>$CH_3$<br>$CH_3O$ | 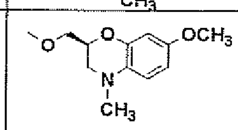 | 121<br>122<br>123<br>124<br>125 | H<br>F<br>Cl<br>$CH_3$<br>$CH_3O$ | 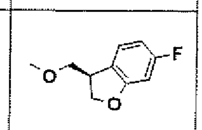 |
| 26<br>27<br>28<br>29<br>30 | H<br>F<br>Cl<br>$CH_3$<br>$CH_3O$ | 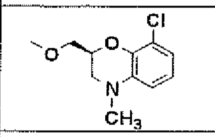 | 76<br>77<br>78<br>79<br>80 | H<br>F<br>Cl<br>$CH_3$<br>$CH_3O$ | 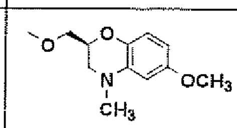 | 126<br>127<br>128<br>129<br>130 | H<br>F<br>Cl<br>$CH_3$<br>$CH_3O$ | 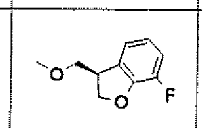 |
| 31<br>32<br>33<br>34<br>35 | H<br>F<br>Cl<br>$CH_3$<br>$CH_3O$ | 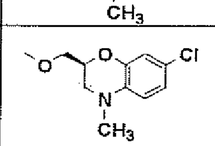 | 81<br>82<br>83<br>84<br>85 | H<br>F<br>Cl<br>$CH_3$<br>$CH_3O$ | 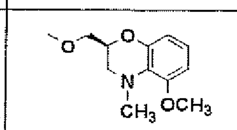 | 131<br>132<br>133<br>134<br>135 | H<br>F<br>Cl<br>$CH_3$<br>$CH_3O$ | 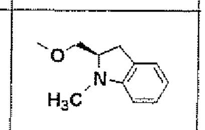 |
| 36<br>37<br>38<br>39<br>40 | H<br>F<br>Cl<br>$CH_3$<br>$CH_3O$ | 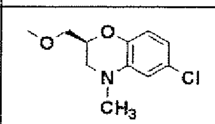 | 86<br>87<br>88<br>89<br>90 | H<br>F<br>Cl<br>$CH_3$<br>$CH_3O$ | 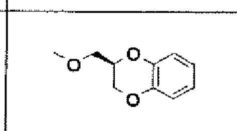 | 136<br>137<br>138<br>139<br>140 | H<br>F<br>Cl<br>$CH_3$<br>$CH_3O$ | 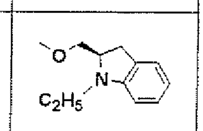 |
| 41<br>42<br>43<br>44<br>45 | H<br>F<br>Cl<br>$CH_3$<br>$CH_3O$ | 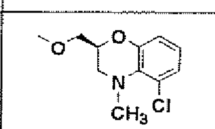 | 91<br>92<br>93<br>94<br>95 | H<br>F<br>Cl<br>$CH_3$<br>$CH_3O$ | 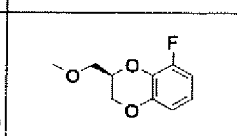 | 141<br>142<br>143<br>144<br>145 | H<br>F<br>Cl<br>$CH_3$<br>$CH_3O$ | 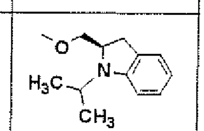 |
| 46<br>47<br>48<br>49<br>50 | H<br>F<br>Cl<br>$CH_3$<br>$CH_3O$ | 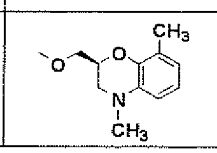 | 96<br>97<br>98<br>99<br>100 | H<br>F<br>Cl<br>$CH_3$<br>$CH_3O$ | 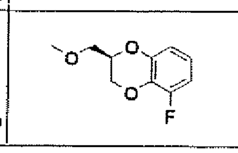 | 146<br>147<br>148<br>149<br>150 | H<br>F<br>Cl<br>$CH_3$<br>$CH_3O$ | 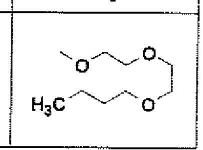 |

Table 58

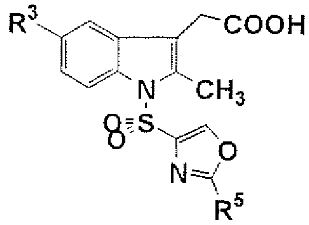

(I−B−12−1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 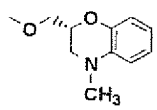 | 51<br>52<br>53<br>54<br>55 | H<br>F<br>Cl<br>$CH_3$<br>$CH_3O$ | 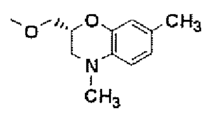 | 101<br>102<br>103<br>104<br>105 | H<br>F<br>Cl<br>$CH_3$<br>$CH_3O$ | 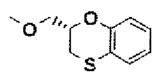 |
| 6<br>7<br>8<br>9<br>10 | H<br>F<br>Cl<br>$CH_3$<br>$CH_3O$ | 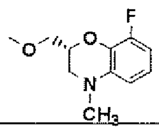 | 56<br>57<br>58<br>59<br>60 | H<br>F<br>Cl<br>$CH_3$<br>$CH_3O$ | 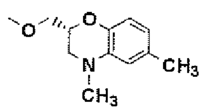 | 106<br>107<br>108<br>109<br>110 | H<br>F<br>Cl<br>$CH_3$<br>$CH_3O$ | 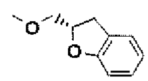 |
| 11<br>12<br>13<br>14<br>15 | H<br>F<br>Cl<br>$CH_3$<br>$CH_3O$ | 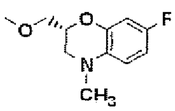 | 61<br>62<br>63<br>64<br>65 | H<br>F<br>Cl<br>$CH_3$<br>$CH_3O$ | 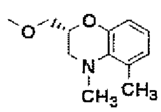 | 111<br>112<br>113<br>114<br>115 | H<br>F<br>Cl<br>$CH_3$<br>$CH_3O$ | 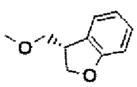 |
| 16<br>17<br>18<br>19<br>20 | H<br>F<br>Cl<br>$CH_3$<br>$CH_3O$ | 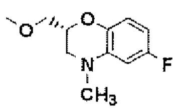 | 66<br>67<br>68<br>69<br>70 | H<br>F<br>Cl<br>$CH_3$<br>$CH_3O$ | 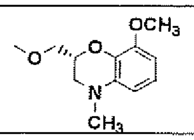 | 116<br>117<br>118<br>119<br>120 | H<br>F<br>Cl<br>$CH_3$<br>$CH_3O$ | 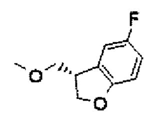 |
| 21<br>22<br>23<br>24<br>25 | H<br>F<br>Cl<br>$CH_3$<br>$CH_3O$ | 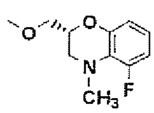 | 71<br>72<br>73<br>74<br>75 | H<br>F<br>Cl<br>$CH_3$<br>$CH_3O$ | 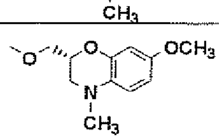 | 121<br>122<br>123<br>124<br>125 | H<br>F<br>Cl<br>$CH_3$<br>$CH_3O$ | 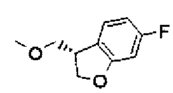 |
| 26<br>27<br>28<br>29<br>30 | H<br>F<br>Cl<br>$CH_3$<br>$CH_3O$ | 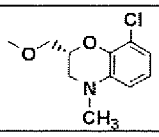 | 76<br>77<br>78<br>79<br>80 | H<br>F<br>Cl<br>$CH_3$<br>$CH_3O$ | 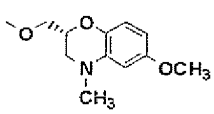 | 126<br>127<br>128<br>129<br>130 | H<br>F<br>Cl<br>$CH_3$<br>$CH_3O$ | 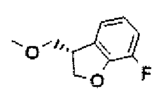 |
| 31<br>32<br>33<br>34<br>35 | H<br>F<br>Cl<br>$CH_3$<br>$CH_3O$ | 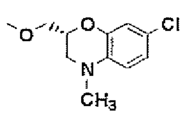 | 81<br>82<br>83<br>84<br>85 | H<br>F<br>Cl<br>$CH_3$<br>$CH_3O$ | 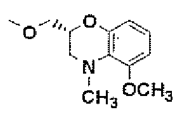 | 131<br>132<br>133<br>134<br>135 | H<br>F<br>Cl<br>$CH_3$<br>$CH_3O$ | 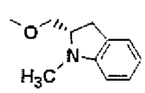 |
| 36<br>37<br>38<br>39<br>40 | H<br>F<br>Cl<br>$CH_3$<br>$CH_3O$ | 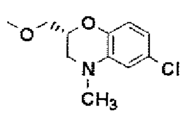 | 86<br>87<br>88<br>89<br>90 | H<br>F<br>Cl<br>$CH_3$<br>$CH_3O$ | 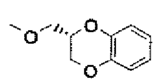 | 136<br>137<br>138<br>139<br>140 | H<br>F<br>Cl<br>$CH_3$<br>$CH_3O$ | 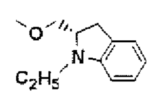 |
| 41<br>42<br>43<br>44<br>45 | H<br>F<br>Cl<br>$CH_3$<br>$CH_3O$ | 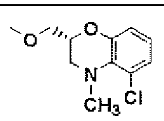 | 91<br>92<br>93<br>94<br>95 | H<br>F<br>Cl<br>$CH_3$<br>$CH_3O$ | 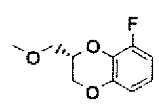 | 141<br>142<br>143<br>144<br>145 | H<br>F<br>Cl<br>$CH_3$<br>$CH_3O$ | 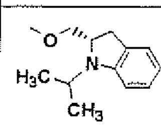 |
| 46<br>47<br>48<br>49<br>50 | H<br>F<br>Cl<br>$CH_3$<br>$CH_3O$ | 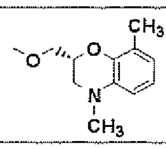 | 96<br>97<br>98<br>99<br>100 | H<br>F<br>Cl<br>$CH_3$<br>$CH_3O$ | 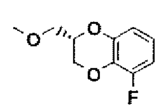 | 146<br>147<br>148<br>149<br>150 | H<br>F<br>Cl<br>$CH_3$<br>$CH_3O$ | 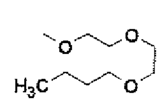 |

Table 59

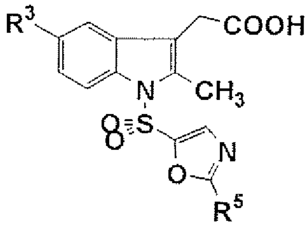

(I−B−13−1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 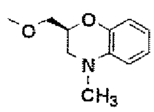 | 51<br>52<br>53<br>54<br>55 | H<br>F<br>Cl<br>$CH_3$<br>$CH_3O$ | 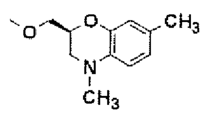 | 101<br>102<br>103<br>104<br>105 | H<br>F<br>Cl<br>$CH_3$<br>$CH_3O$ | 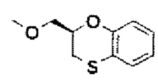 |
| 6<br>7<br>8<br>9<br>10 | H<br>F<br>Cl<br>$CH_3$<br>$CH_3O$ | 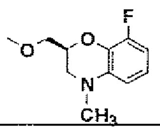 | 56<br>57<br>58<br>59<br>60 | H<br>F<br>Cl<br>$CH_3$<br>$CH_3O$ | 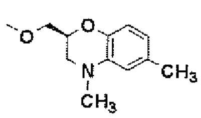 | 106<br>107<br>108<br>109<br>110 | H<br>F<br>Cl<br>$CH_3$<br>$CH_3O$ | 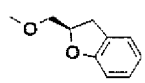 |
| 11<br>12<br>13<br>14<br>15 | H<br>F<br>Cl<br>$CH_3$<br>$CH_3O$ | 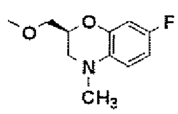 | 61<br>62<br>63<br>64<br>65 | H<br>F<br>Cl<br>$CH_3$<br>$CH_3O$ | 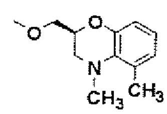 | 111<br>112<br>113<br>114<br>115 | H<br>F<br>Cl<br>$CH_3$<br>$CH_3O$ | 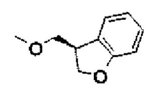 |
| 16<br>17<br>18<br>19<br>20 | H<br>F<br>Cl<br>$CH_3$<br>$CH_3O$ | 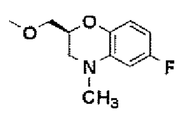 | 66<br>67<br>68<br>69<br>70 | H<br>F<br>Cl<br>$CH_3$<br>$CH_3O$ | 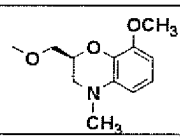 | 116<br>117<br>118<br>119<br>120 | H<br>F<br>Cl<br>$CH_3$<br>$CH_3O$ | 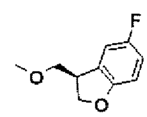 |
| 21<br>22<br>23<br>24<br>25 | H<br>F<br>Cl<br>$CH_3$<br>$CH_3O$ | 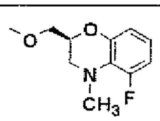 | 71<br>72<br>73<br>74<br>75 | H<br>F<br>Cl<br>$CH_3$<br>$CH_3O$ | 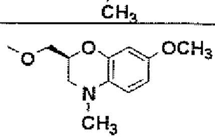 | 121<br>122<br>123<br>124<br>125 | H<br>F<br>Cl<br>$CH_3$<br>$CH_3O$ | 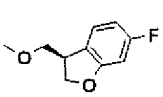 |
| 26<br>27<br>28<br>29<br>30 | H<br>F<br>Cl<br>$CH_3$<br>$CH_3O$ | 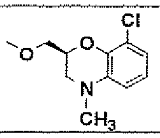 | 76<br>77<br>78<br>79<br>80 | H<br>F<br>Cl<br>$CH_3$<br>$CH_3O$ | 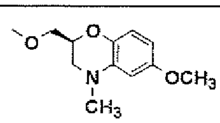 | 126<br>127<br>128<br>129<br>130 | H<br>F<br>Cl<br>$CH_3$<br>$CH_3O$ | 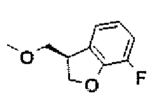 |
| 31<br>32<br>33<br>34<br>35 | H<br>F<br>Cl<br>$CH_3$<br>$CH_3O$ | 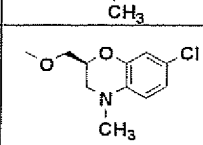 | 81<br>82<br>83<br>84<br>85 | H<br>F<br>Cl<br>$CH_3$<br>$CH_3O$ | 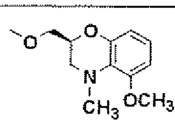 | 131<br>132<br>133<br>134<br>135 | H<br>F<br>Cl<br>$CH_3$<br>$CH_3O$ | 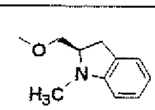 |
| 36<br>37<br>38<br>39<br>40 | H<br>F<br>Cl<br>$CH_3$<br>$CH_3O$ | 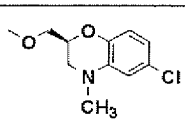 | 86<br>87<br>88<br>89<br>90 | H<br>F<br>Cl<br>$CH_3$<br>$CH_3O$ | 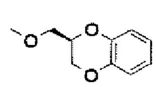 | 136<br>137<br>138<br>139<br>140 | H<br>F<br>Cl<br>$CH_3$<br>$CH_3O$ | 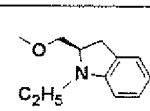 |
| 41<br>42<br>43<br>44<br>45 | H<br>F<br>Cl<br>$CH_3$<br>$CH_3O$ | 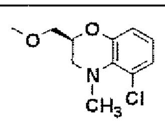 | 91<br>92<br>93<br>94<br>95 | H<br>F<br>Cl<br>$CH_3$<br>$CH_3O$ | 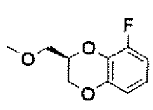 | 141<br>142<br>143<br>144<br>145 | H<br>F<br>Cl<br>$CH_3$<br>$CH_3O$ | 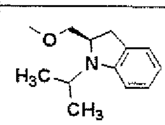 |
| 46<br>47<br>48<br>49<br>50 | H<br>F<br>Cl<br>$CH_3$<br>$CH_3O$ | 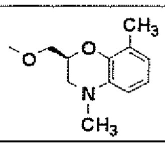 | 96<br>97<br>98<br>99<br>100 | H<br>F<br>Cl<br>$CH_3$<br>$CH_3O$ | 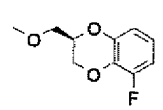 | 146<br>147<br>148<br>149<br>150 | H<br>F<br>Cl<br>$CH_3$<br>$CH_3O$ | 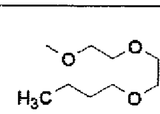 |

Table 60

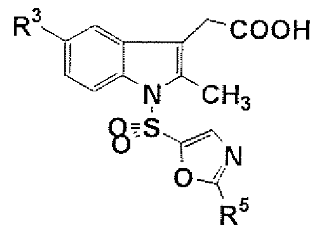

(I－B－1 3－1)

| No. | $R^3$ | $-R^5$ | No. | $R^3$ | $-R^5$ | No. | $R^3$ | $-R^5$ |
|---|---|---|---|---|---|---|---|---|
| 1<br>2<br>3<br>4<br>5 | H<br>F<br>Cl<br>$CH_3$<br>$CH_3O$ | 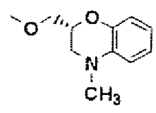 | 51<br>52<br>53<br>54<br>55 | H<br>F<br>Cl<br>$CH_3$<br>$CH_3O$ | 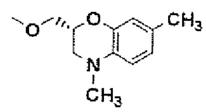 | 101<br>102<br>103<br>104<br>105 | H<br>F<br>Cl<br>$CH_3$<br>$CH_3O$ | 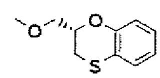 |
| 6<br>7<br>8<br>9<br>10 | H<br>F<br>Cl<br>$CH_3$<br>$CH_3O$ | 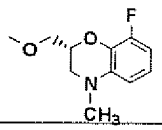 | 56<br>57<br>58<br>59<br>60 | H<br>F<br>Cl<br>$CH_3$<br>$CH_3O$ | 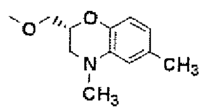 | 106<br>107<br>108<br>109<br>110 | H<br>F<br>Cl<br>$CH_3$<br>$CH_3O$ | 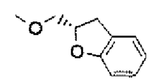 |
| 11<br>12<br>13<br>14<br>15 | H<br>F<br>Cl<br>$CH_3$<br>$CH_3O$ | 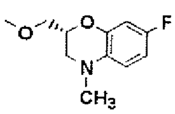 | 61<br>62<br>63<br>64<br>65 | H<br>F<br>Cl<br>$CH_3$<br>$CH_3O$ | 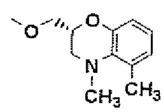 | 111<br>112<br>113<br>114<br>115 | H<br>F<br>Cl<br>$CH_3$<br>$CH_3O$ | 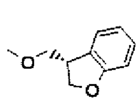 |
| 16<br>17<br>18<br>19<br>20 | H<br>F<br>Cl<br>$CH_3$<br>$CH_3O$ | 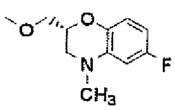 | 66<br>67<br>68<br>69<br>70 | H<br>F<br>Cl<br>$CH_3$<br>$CH_3O$ | 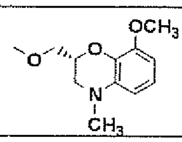 | 116<br>117<br>118<br>119<br>120 | H<br>F<br>Cl<br>$CH_3$<br>$CH_3O$ | 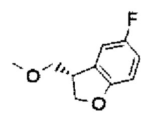 |
| 21<br>22<br>23<br>24<br>25 | H<br>F<br>Cl<br>$CH_3$<br>$CH_3O$ | 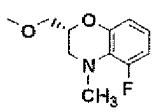 | 71<br>72<br>73<br>74<br>75 | H<br>F<br>Cl<br>$CH_3$<br>$CH_3O$ | 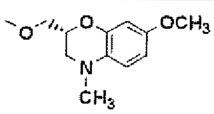 | 121<br>122<br>123<br>124<br>125 | H<br>F<br>Cl<br>$CH_3$<br>$CH_3O$ | 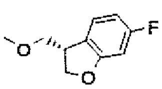 |
| 26<br>27<br>28<br>29<br>30 | H<br>F<br>Cl<br>$CH_3$<br>$CH_3O$ | 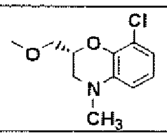 | 76<br>77<br>78<br>79<br>80 | H<br>F<br>Cl<br>$CH_3$<br>$CH_3O$ | 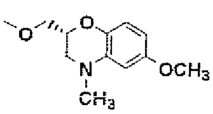 | 126<br>127<br>128<br>129<br>130 | H<br>F<br>Cl<br>$CH_3$<br>$CH_3O$ | 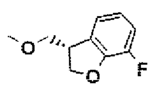 |
| 31<br>32<br>33<br>34<br>35 | H<br>F<br>Cl<br>$CH_3$<br>$CH_3O$ | 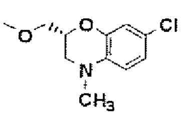 | 81<br>82<br>83<br>84<br>85 | H<br>F<br>Cl<br>$CH_3$<br>$CH_3O$ | 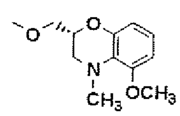 | 131<br>132<br>133<br>134<br>135 | H<br>F<br>Cl<br>$CH_3$<br>$CH_3O$ | 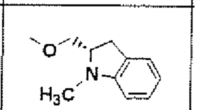 |
| 36<br>37<br>38<br>39<br>40 | H<br>F<br>Cl<br>$CH_3$<br>$CH_3O$ | 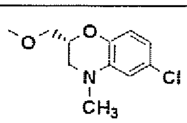 | 86<br>87<br>88<br>89<br>90 | H<br>F<br>Cl<br>$CH_3$<br>$CH_3O$ | 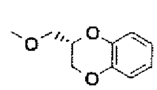 | 136<br>137<br>138<br>139<br>140 | H<br>F<br>Cl<br>$CH_3$<br>$CH_3O$ | 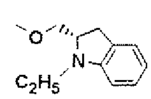 |
| 41<br>42<br>43<br>44<br>45 | H<br>F<br>Cl<br>$CH_3$<br>$CH_3O$ | 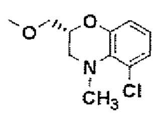 | 91<br>92<br>93<br>94<br>95 | H<br>F<br>Cl<br>$CH_3$<br>$CH_3O$ | 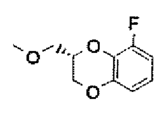 | 141<br>142<br>143<br>144<br>145 | H<br>F<br>Cl<br>$CH_3$<br>$CH_3O$ | 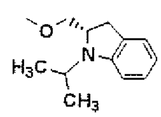 |
| 46<br>47<br>48<br>49<br>50 | H<br>F<br>Cl<br>$CH_3$<br>$CH_3O$ | 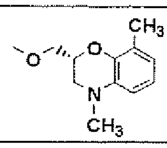 | 96<br>97<br>98<br>99<br>100 | H<br>F<br>Cl<br>$CH_3$<br>$CH_3O$ | 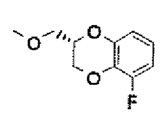 | 146<br>147<br>148<br>149<br>150 | H<br>F<br>Cl<br>$CH_3$<br>$CH_3O$ | 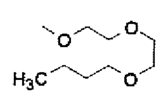 |

The compound of the present invention specifically binds to CRTH2 receptors and/or DP receptors, and has selectivity against prostanoid receptors. Especially, it binds weakly to prostanoid receptors except for $PGD_2$ receptor. In addition, the compounds of the present invention are the compounds having excellent solubility and absorptivity.

Such physical, chemical and pharmacological properties are important for developing as pharmaceuticals and it is believed that the compounds of the present invention have requirements for very useful pharmaceuticals [*The Merck Manual of Diagnosis and Therapy* (17th Ed.), published by Merck & Co.].

Process for Production of the Compounds of the Present Invention:

The compound of the present invention represented by formula (I) are able to be produced by the method as shown below or shown in Examples.

a) Among the compounds represented by formula (I), the compound in which $R^1$ represents $-COR^6$ and $R^6$ represents hydroxy, *i.e.* those represented by formula (IA)

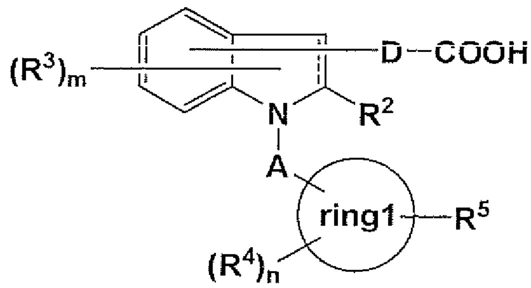

(IA)

(wherein all symbols have the same meanings as described above), is able to be produced according to the process as mentioned below.

The compound represented by formula (IA) can be produced subjecting the compound represented by formula (II)

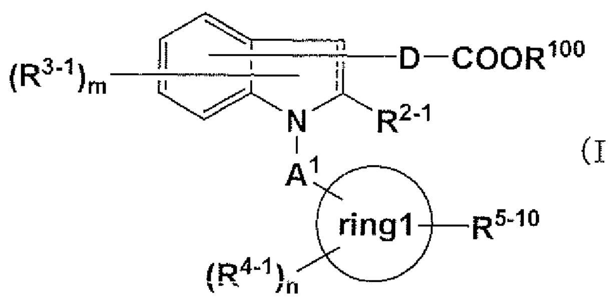

(II)

(wherein $R^{100}$ is a protective group of carboxyl; $R^{2-1}$ , $R^{3-1}$, $R^{4-1}$ , $R^{5-10}$ and $A^1$ are the same meanings as $R^2$, $R^3$, $R^4$, $R^5$ and A respectively, hydroxy or amino in the group represented by $R^{2-1}$, $R^{3-1}$, $R^{4-1}$, $R^{5-10}$ and $A^1$ is protected if necessary; and other symbols have the same meaning as defined above) to deprotection of protective group of carboxyl followed by subjecting to deprotection, if necessary.

Deprotection reaction of a protective group for carboxyl, hydroxyl or amino is known and its examples are as follows.

(1) a hydrolyzing reaction with an alkali;
(2) a deprotection reaction under an acidic condition;
(3) a deprotection reaction by hydrogenolysis;
(4) a deprotection reaction of silyl;
(5) a deprotection reaction using metal; and
(6) a deprotection reaction using an organic metal.

Those methods will be specifically illustrated as follows.

(1) A deprotection reaction using an alkali is carried out, for example, at the temperature of 0 to 40°C using a hydroxide of alkaline metal (such as sodium hydroxide, potassium hydroxide and lithium hydroxide), a hydroxide of alkaline earth metal (such as barium hydroxide and calcium hydroxide), a carbonate (such as sodium carbonate and potassium carbonate), an aqueous solution thereof or a mixture thereof in an organic solvent (such as methanol, tetrahydrofuran and dioxane).

(2) A deprotection reaction under an acidic condition is carried out, for example, at the temperature of 0 to 100°C with or without 2,2,2-trifluoroethanol, in an organic acid (such as acetic acid, trifluoroacetic acid, methanesulfonic acid and p-tosylic acid), an inorganic acid (hydrochloric acid and sulfuric acid) or a mixture thereof (such as hydrogen bromide/acetic acid) in an organic solvent (such as dichloromethane, chloroform, dioxane, ethyl acetate and anisole).

(3) A deprotection reaction by hydrogenolysis is carried out, for example, at the temperature of 0 to 200°C in a hydrogen atmosphere of ordinary pressure or high pressure or in the presence of ammonium formate in the presence of a catalyst [such as palladium-carbon, palladium black, palladium hydroxide, platinum oxide and Raney nickel) in a solvent (such as an ether type (such as tetrahydrofuran, dioxane, dimethoxyethane and diethyl ether), an alcohol type (such as methanol and ethanol), a benzene type (such as benzene and toluene), a ketone type (such as acetone and methyl ethyl ketone), a nitrile type (such as acetonitrile), an amide type (such as dimethylformamide), water, ethyl acetate, acetic acid or a mixed solvent comprising two or more thereof].

(4) A deprotection reaction of silyl is carried out, for example, at the temperature of 0 to 40°C using tetrabutylammonium fluoride in an organic solvent miscible with water (such as tetrahydrofuran and acetonitrile).

(5) A deprotection reaction using metal is carried out, for example, at the temperature of 0 to 40°C with ultrasonic wave, if necessary, in the presence of powdery zinc in an acidic solvent (such as acetic acid, a buffer of pH 4.2 to 7.2 and a mixed solution of a solution thereof with an organic solvent such as tetrahydrofuran).

(6) A deprotection reaction using a metal complex is carried out, for example, at the temperature of 0 to 40°C using a metal complex such as tetrakistriphenylphosphine palladium (0), bis(triphenylphosphine) palladium (II) dichloride, palladium (II) acetate and tris(triphenylphosphine) rhodium (I) chloride) in the presence or absence of a phosphine agent (such as triphenyl phosphine) in the presence of a trap reagent (such as tributyltin hydride, triethylsilane, dimedone, morpholine, diethylamine and pyrrolidine), an organic acid (such as acetic acid, formic acid and 2-ethylhexanoic acid) and/or an organic acid salt (such as sodium 2-ethylhexanoate and potassium 2-ethylhexanoate) in an organic solvent (such as dichloromethane, dimethylformamide, tetrahydrofuran, ethyl acetate, acetonitrile, dioxane and ethanol), water or a mixed solvent thereof.

Besides the above-mentioned method, for example, a deprotection reaction may be carried out by a method mentioned in "T. W. Greene, *Protective Groups in Organic Synthesis,* Wiley, New York, 1999".

The protective group for carboxyl includes such as methyl, ethyl, allyl, t-butyl, trichloroethyl, benzyl (Bn), phenacyl, p-methoxybenzyl, trityl, 2-chlorotrityl, and solid-phase support which those structures linked and the like.

The protective group for hydroxyl includes such as methyl, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc) and 2,2,2-trichloroethoxycarbonyl (Troc).

The protective group of amino includes such as benzyloxycarbonyl, *tert*-butoxycarbonyl, allyloxycarbonyl (Alloc), 1-methyl-1-(4-biphenyl)ethoxycarbonyl (Bpoc), trifluoroacetyl, 9-fluorenylmethoxycarbonyl, benzyl (Bn), p-methoxybenzyl, benzyloxymethyl (BOM) and 2-(trimethylsilyl)ethoxymethyl (SEM) and the like.

With regard to the protective group for carboxyl, for hydroxyl and for amino, there is no particular limitation to the above ones so far as it is a group which is able to be easily and selectively detached. For example, a deprotection reaction may be carried out by a method mentioned in "T. W. Greene, *Protective Groups in Organic Synthesis,* Wiley, New York, 1999".

As persons skilled in the art can easily understand it, the aimed compound of the present invention is able to be easily produced by using appropriate ones among those deprotection reactions.

b) Among the compounds represented by formula (I), the compound in which $R^1$ represents $-COR^6$, and $R^6$ represents C1-6 alkoxy, C1-6 alkoxy substituted with phenyl, or C2-6 alkenyloxy, *i.e*. those represented by formula (IB)

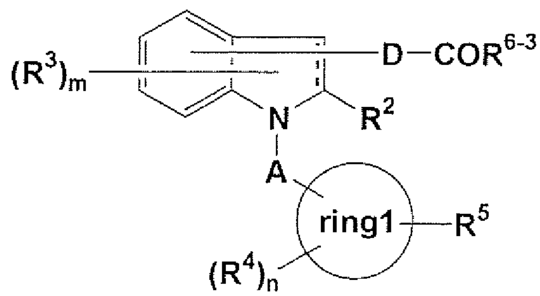

(I B)

(wherein $R^{6-3}$ represents C1-6 alkoxy, C1-6 alkoxy substituted with phenyl, or C2-6 alkenyloxy; other symbols have the same meanings as described above), is able to be produced according to the process as mentioned below.

The compound represented by formula (IB) is able to be produced subjecting the compound represented by formula (III)

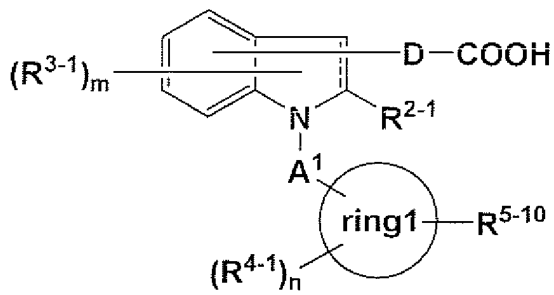

(III)

(wherein all symbols have the same meaning as defined above) to an esterification reaction with formula (IV)

$$R^{200}—OH \qquad (IV)$$

(wherein $R^{200}$ represents C1-6 alkyl, C1-6 alkyl substituted with phenyl, or C2-6 alkenyl) followed, by subjecting to deprotection, if necessary.

Esterification reaction has been known and its examples are

(1) a process using an acid halide,
(2) a process using a mixed acid anhydride and
(3) a process using a condensing agent.

Such processes will be specifically illustrated as follows.

(1) A process using an acid halide is carried out, for example, in such a manner that carboxylic acid reacts with an agent for producing an acid halide (such as oxalyl chloride and thionyl chloride) in an organic solvent (such as chloroform, dichloromethane, diethyl ether and tetrahydrofuran) or without solvent at -20°C to refluxing temperature and the resulting acid halide reacts with an alcohol in the presence of a base (such as pyridine, triethylamine, dimethylaniline, dimethylaminopyridine and diisopropylethylamine) in an inert organic solvent (such as chloroform, dichloromethane, diethyl ether and tetrahydrofuran) at the temperature of 0 to 40°C. It is also possible to conduct the reaction with an acid halide at 0 to 40°C in an organic solvent (such as dioxane and tetrahydrofuran) using an aqueous solution of alkali (such as aqueous solution of sodium bicarbonate and an aqueous solution of sodium hydroxide).

(2) A process using a mixed acid anhydride is carried out, for example, in such a manner that carboxylic acid is made to react with an acid halide (such as pivaloyl chloride, tosyl chloride or mesyl chloride) or with an acid derivative (such as ethyl chloroformate and isobutyl chloroformate) at 0 to 40°C in the presence or absence

of an organic solvent (such as chloroform, dichloromethane, diethyl ether and tetrahydrofuran) or without a solvent in the presence of a base (such as pyridine, triethylamine, dimethylaniline, dimethylaminopyridine and diisopropylethylamine) and the resulting mixed acid anhydride is made to react with an alcohol at 0 to 40°C in an organic solvent (such as chloroform, dichloromethane, diethyl ether and tetrahydrofuran).

(3) A process using a condensing agent is carried out, for example, in such a manner that carboxylic acid and an alcohol are subjected to a reaction at 0 to 40°C with or without 1-hydroxybenztriazole (HOBt) using a condensing agent (such as 1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodide and 1-prapylphosphonic acid cyclic anhydride in the presence or absence of a base (such as pyridine, triethylamine, dimethylanilin and dimethylaminopyridine) in an organic solvent (such as chloroform, dichloromethane, dimethylformamide, diethyl ether and tetrahydrofuran) or without a solvent.

It is preferred that all of the reactions (1), (2) and (3) are carried out in an atmosphere of inert gas (such as argon and nitrogen) under an anhydrous condition.

A deprotection reaction of protection group is able to be carried out by the same methods as those mentioned above.

c) Among the compounds represented by formula (I), the compound in which $R^1$ represents $-COR^6$, and $R^6$ represents $-NR^8R^9$, *i.e.* those represented by formula (IC)

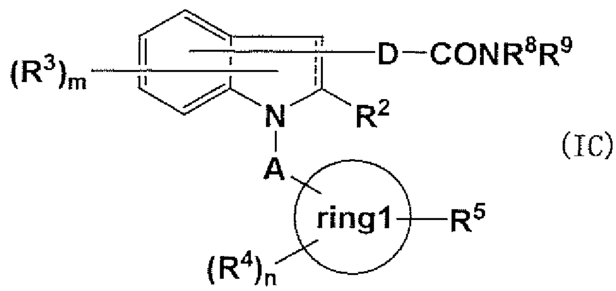

(IC)

(wherein all symbols have the same meanings as described above), is able to be produced according to the process as mentioned below.

The compound represented by formula (IC) is able to be produced subjecting the compound represented by formula (III) to an amidation reaction with formula (V)

$$H—NR^{8-1}R^{9-1} \quad (V)$$

(wherein $R^{8-1}$ and $R^{9-1}$ are the same meanings as $R^8$ and $R^9$ respectively, hydroxy or amino in the group represented by $R^{8-1}$ and $R^{9-1}$ is protected if necessary; and other symbols have the same meaning as defined above) followed, by subjecting to deprotection, if necessary.

Amidation reaction has been known and its examples are

(1) a process using an acid halide,
(2) a process using a mixed acid anhydride and
(3) a process using a condensing agent.

Such processes will be specifically illustrated as follows.

(1) A process using an acid halide is carried out, for example, in such a manner that carboxylic acid reacts with an agent for producing an acid halide (such as oxalyl chloride and thionyl chloride) in an organic solvent (such as chloroform, dichloromethane, diethyl ether, tetrahydrofuran and dimethoxyethane) or without solvent at -20°C to refluxing temperature and the resulting acid halide reacts with an amine in the presence of a base (such as pyridine, triethylamine, dimethylaniline, dimethylaminopyridine and diisopropylethylamine) in an inert organic solvent (such as chloroform, dichloromethane, diethyl ether, tetrahydrofuran, acetonitrile and ethyl acetate) at the temperature of 0 to 40°C. It is also possible to conduct the reaction with an obtained acid halide

at 0 to 40°C in an organic solvent (such as dioxane, tetrahydrofuran and dichloromethane) in the presence or absence of a phase-transfer catalyst (such as a quaternary ammonium salt, *e.g.* tetrabutylammonium chloride, triethylbenzylammonium chloride, tri-n-octylmethylammonium chloride, trimethyldecylammonium chloride and tetramethylammonium bromide) using an aqueous solution of alkali (such as aqueous solution of sodium bicarbonate and an aqueous solution of sodium hydroxide).

(2) A process using a mixed acid anhydride is carried out, for example, in such a manner that carboxylic acid is made to react with an acid halide (such as pivaloyl chloride, tosyl chloride or mesyl chloride) or with an acid derivative (such as ethyl chloroformate and isobutyl chloroformate) at 0 to 40°C in the presence of an organic solvent (such as chloroform, dichloromethane, diethyl ether and tetrahydrofuran) or without a solvent in the presence of a base (such as pyridine, triethylamine, dimethylaniline, dimethylaminopyridine and diisopropylethylamine) and the resulting mixed acid anhydride is made to react with an amine at 0 to 40°C in an organic solvent (such as chloroform, dichloromethane, diethyl ether and tetrahydrofuran).

(3) A process using a condensing agent is carried out, for example, in such a manner that carboxylic acid and an amine are subjected to a reaction at 0 to 40°C with or without 1-hydroxybenztriazole (HOBt) using a condensing agent (such as 1,3-dicyclohexylcarbodiimide (DCC), 1-ethyl-3-[3-(dimethylamino)propyl]carbodiimide (EDC), 1,1'-carbonyldiimidazole (CDI), 2-chloro-1-methylpyridinium iodide and 1-propylphosphonic acid cyclic anhydride in the presence or absence of a base (such as pyridine, triethylamine, dimethylanilin and dimethylaminopyridine) in an organic solvent (such as chloroform, dichloromethane, dimethylformamide, diethyl ether and tetrahydrofuran) or without a solvent.

It is preferred that all of the reactions (1), (2) and (3) are carried out in an atmosphere of inert gas (such as argon and nitrogen) under an anhydrous condition.

A deprotection reaction of protection group is able to be carried out by the same methods as those mentioned above.

d) Among the compounds represented by formula (I), the compound in which $R^1$ represents $-CH_2OR^7$, and $R^7$ represents a hydrogen atom, *i.e.* those represented by formula (ID)

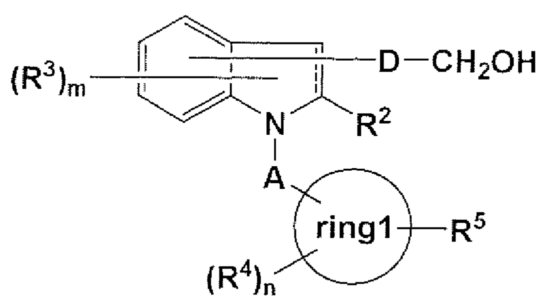

(ID)

(wherein all symbols have the same meanings as described above), is able to be produced according to the process as mentioned below.

The compound represented by formula (ID) can be produced subjecting the compound represented by formula (III) to reduction reaction followed by subjecting to deprotection, if necessary.

The reduction reaction has been known and it is carried out, for example, in such a manner that carboxylic acid is made to react with a borane complex agent (such as boranetetrahydrofuran complex, borane-dimethyl sulfide complex) at 0 to 80°C in an organic solvent (such as tetrahydrofuran) or carboxylic acid is made to react with an acid derivatives (such as ethyl chloroformate, isobutyl chloroformate) at 0 to 40°C in an inert organic solvent (such as chloroform, dichloromethane, diethylether, tetrahydrofuran) or without a solvent in the presence of a tertiary amine (such as pyridine, triethylamine, dimethylaniline, dimethylaminopyridine) and the mixed anhydride is made to react with reducting agent (such as sodium borohydride) at 0 to 40°C in an inert organic solvent (such as chloroform, dichloromethane, diethylether, tetrahydrofuran).

A deprotection reaction of protective group is able to be carried out by the same methods as those mentioned above.

e) Among the compounds represented by formula (I), the compound in which $R^1$ represents $-CH_2OR^7$, and $R^7$ represents C2-6 acyl, *i.e*. those represented by formula (IE)

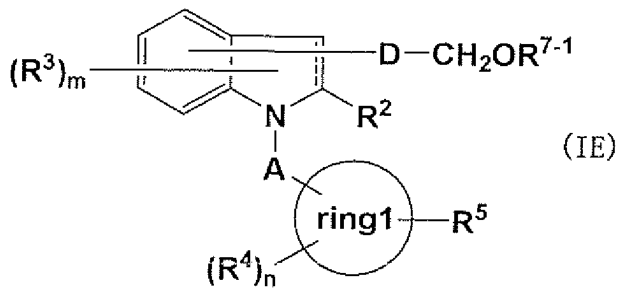

(IE)

(wherein $R^{7-1}$ represents C2-6 acyl; other symbols have the same meanings as described above), is able to be produced according to the process as mentioned below.

The compound represented by formula (IE) is able to be produced subjecting the compound represented by formula (VI)

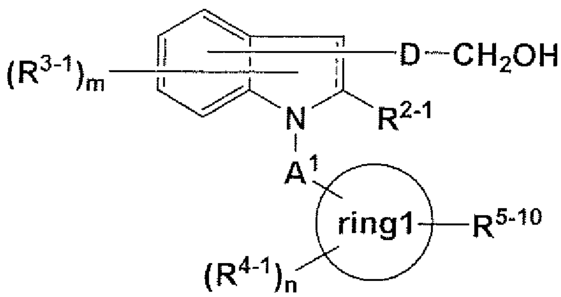

(VI)

(wherein all symbols have the same meaning as defined above) to an esterification reaction with formula (VII)

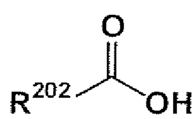

**(VII)**

(wherein $R^{202}$ represents C1-5 alkyl) followed, by subjecting to deprotection, if necessary.

Esterification reaction and deprotection reaction of protection group are able to be carried out by the same methods as those mentioned above.

Among the compounds represented by formula (II), the compound in which A represents carbonyl or $-SO_2-$, *i.e.* those represented by formula (II-1)

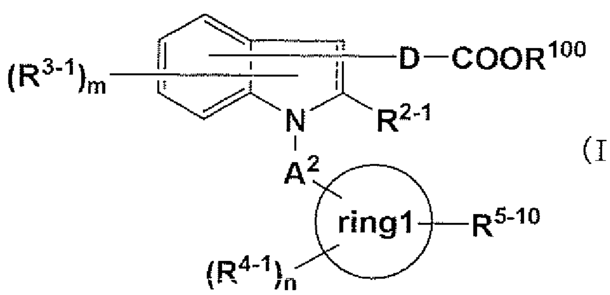

(II-1)

(wherein A represents carbonyl or $-SO_2-$; other symbols have the same meanings as described above), is able to be

produced according to the process as mentioned below.

The compound represented by formula (II-1) is able to be produced subjecting the compound represented by formula (VIII)

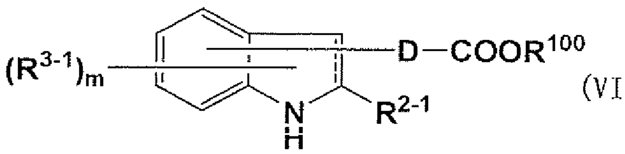

(VIII)

(wherein all symbols have the same meaning as defined above) to an amidation reaction with formula (IX)

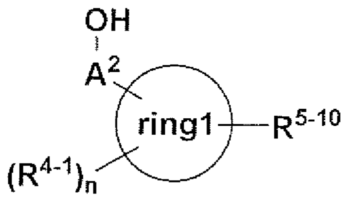

(IX)

(wherein all symbols have the same meaning as defined above) followed, by subjecting to deprotection, if necessary.

Amidation reaction and deprotection reaction of protection group are able to be carried out by the same methods as those mentioned above.

Also, among the compounds represented by formula (II), the compound in which $R^5$ represents

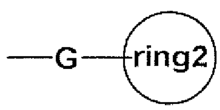

and G represents -O-(C1-5 alkylene)-, *i.e.* those represented by formula (II-2)

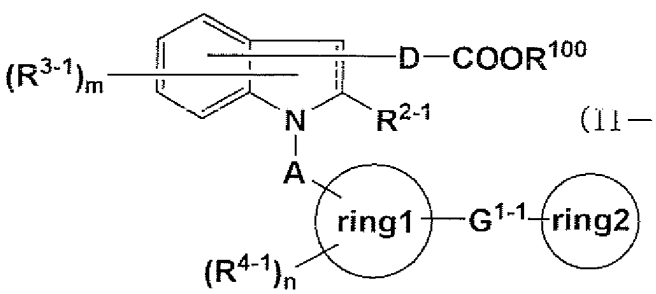

(II-2)

(wherein $G^{1-1}$ represents -O-(C1-5 alkylene)-; other symbols have the same meanings as described above), is able to be produced subjecting the compound represented by formula (X)

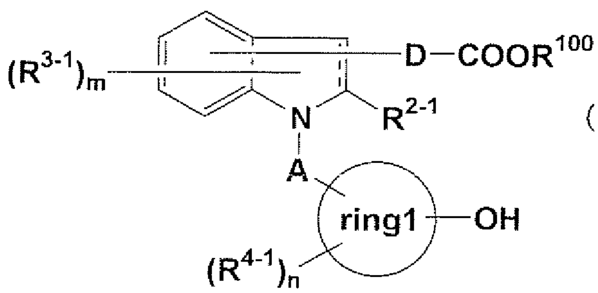

(X)

(wherein all symbols have the same meaning as defined above) to an etherification reaction with formula (XI)

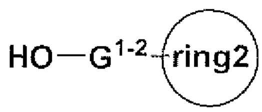

(XI)

(wherein $G^{1-2}$ represents C1-5 alkylene; other symbols have the same meanings as described above) followed, by subjecting to deprotection, if necessary.

An etherification reaction has been known and, it is carried out, for example, at 0 to 60°C with a corresponding alcohol in the presence of an azo compound (such as diethyl azodicarboxylate (DEAD), diisopropyl azodicarboxylate, 1,1'-(azodicarbonyl)-dipyridine and 1,1'-azobis(N,N-dimethylformamide) and a phosphine compound (such as triphenyl phosphine, tributyl phosphine, trimethyl phosphine and polymer-supported triphenyl phosphine) in an organic solvent (such as dichloromethane, diethyl ether, tetrahydrofuran, acetonitrile, benzene and toluene).

A deprotection reaction of protection group are able to be carried out by the same methods as those mentioned above.

Compounds represented by formulae (II), (IV), (V), (VII), (VITI), (IX), (X) and (XI) have been known per se or are able to be easily produced by known methods.

For example, among the compounds represented by formula (II), the compound in which -D-COOR$^{100}$ is substituted at 3-position of indole ring, $R^{3-1}$ is substituted at 4-7 position of indole ring, and A is carbonyl, *i.e.* those represented by formula (II-3)

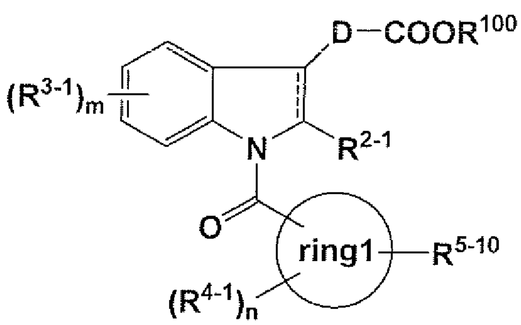

(II-3)

(wherein all symbols have the same meaning as defined above) is able to be produced by the process shown in the following reaction step formula 1 and 2.

In the reaction step formula, $D^1$ represents a single bond or C1-6 alkylene, $D^2$ represents C2-6 alkenylene, $D^3$ represents C1-6 alkylene, $R^{203}$ represents a halogen atom or hydroxy, $R^{204}$ represents protective group for hydroxy and other symbols have the same meanings as those defined above.

reaction step formula 1

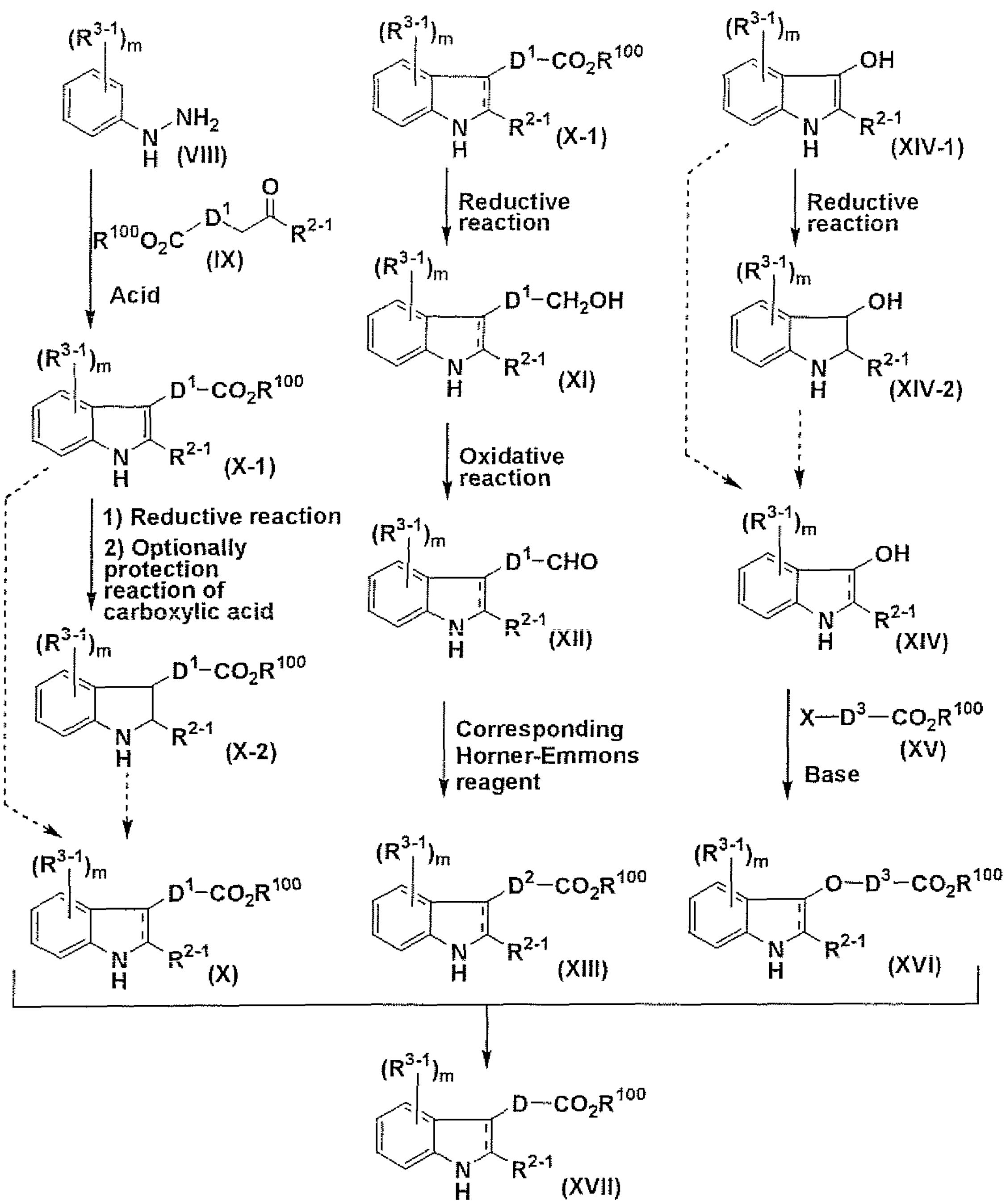

reaction step formula 2

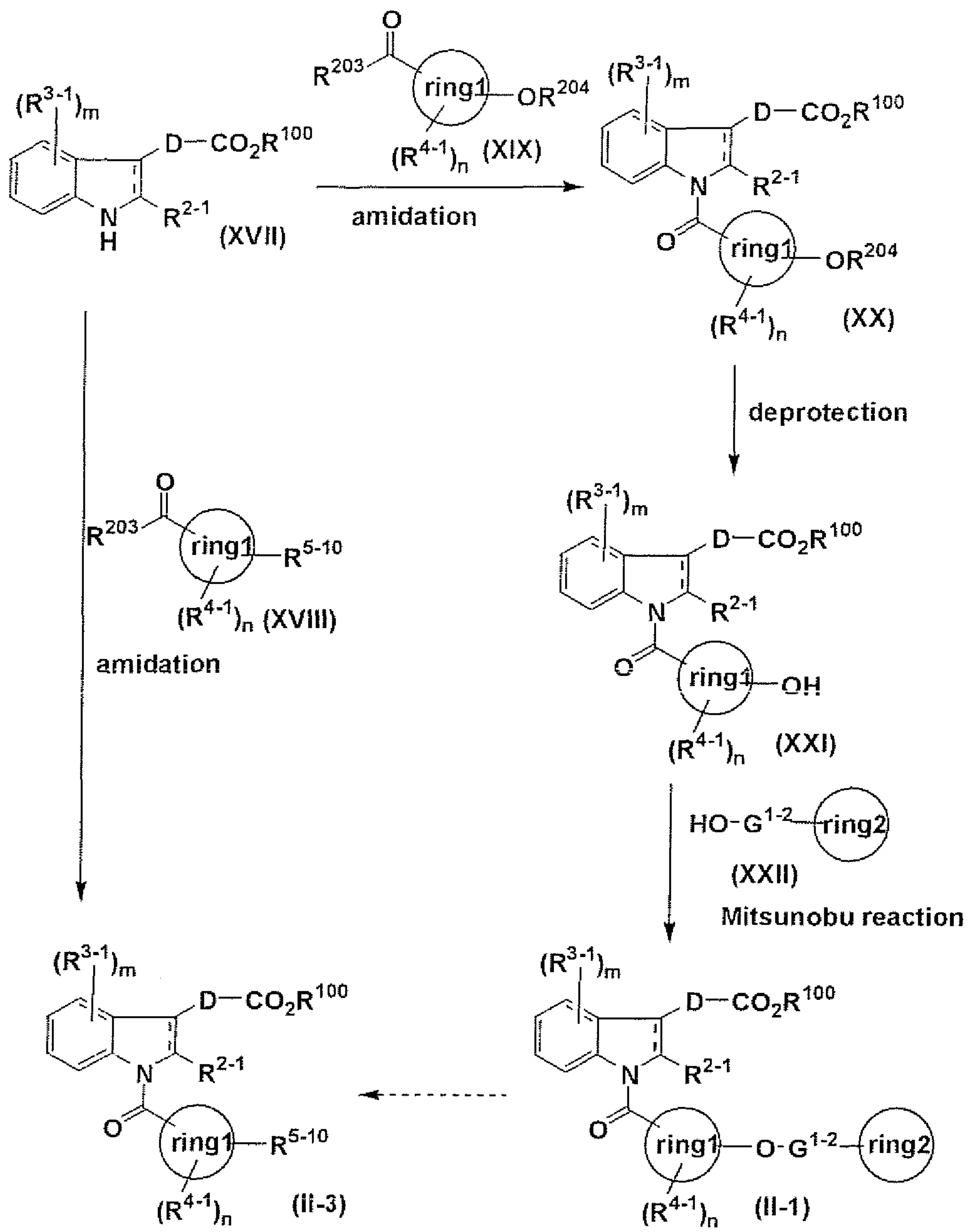


In the above reaction step formula 1 and 2, the compounds represented by formulae (VIII), (IX), (XIV-1), (XV), (XVIII), (XIX) and (XXII) used as starting materials have been known or able to be easily produced by known methods.

For example, the compound represented by formula (XIV-1) may be prepared according to a method described in *Tetrahedron.,* 30, 1445-1455 (1974).

In each of the reactions mentioned in the present specification, the reaction product is able to be purified by a conventional purifying method such as distillation under ordinary pressure, or high performance liquid chromatography, thin-layer chromatography or column chromatography using silica gel or magnesium silicate and recrystallization. Purification may be carried out for each reaction or after completion of some reactions.

Pharmacological activity of the compound of the present invention:

The compound of the present invention represented by formula (I) binds to human CRTH2 receptor strongly and antagonizes. It was ensured by the following receptor binding experiment and receptor antagonism activity measurement experiment. As for the measuring method, there is general description in WO01/14882, JP2002-98702 and the like. In order to measure the activity of the test substances to CRTH2 receptors easily and accurately, the inventors of the present invention made several improvements. Exemplification is shown in the following.

In all of the assays, the effects of the compound of the present inventions were evaluated using Chinese hamster ovary cells stably expressing the human CRTH2 receptor gene (CRTH2-CHO cells).

Example 1

Ligand binding experiment of the human CRTH2 receptor using $[^3H]$-$PGD_2$:

After collection of CRTH2-CHO cells by trypsinization, these cells were suspended in Ham's F-12 (Gibco BRL) containing 10% fatal calf serum (FCS), 100 μg/mL streptomycin (Gibco BRL) and 100 U/mL penicillin (Gibco BRL) at a cell density of $3 \times 10^5$ cells/mL. A 100 μL portion of this suspension was seeded in each well of a 96-well culture plate (Packard) and cultivated for 2 days at 37°C in an atmosphere of 5% $CO_2$. After removal of the culture medium, 150 μL Hank's balanced salt solution (HBSS, Gibco BRL) containing 10 mmol/L HEPES (HEPES/HBSS, pH 7.4) was added to each well (cell rinse). Subsequently, cell rinse by HEPES/HBSS was repeated 2 times. After adding 80 μL of 10 mmol/L HEPES/HBSS to each well, 10 μL of vehicle (10 mmol/L HEPES/HBSS containing 1% dimethyl sulfoxide (DMSO)) or vehicle containing compound of the present invention was further added. In the non-specific binding group, non-labeled $PGD_2$ (final concentration: 10 μmol/L) was added instead of the vehicle or the compound of the present inventions. The reaction was initiated by adding 10 μL of 30 mnol/L $[^3H]$-$PGD_2$ (Amersham) (final concentration of $[^3H]$-$PGD_2$: 3 nmol/L) followed by mixing for 1 min. After incubation for 60 min at ambient temperature, the reaction was terminated by removal of the reaction solution and subsequently the cells were rinsed 2 times with 150 μL of 10 mmol/L HEPES/HBSS containing 0.1% bovine serum albumin (BSA, Sigma). After a 130 μL portion of scintillation cocktail (Microscinti 40, Packard) was added to each well followed by mixing for 15 min, radioactivity in each well was determined by liquid scintillation counter for 96-well plate (TopCount, Packard). Specific binding of $[^3H]$-$PGD_2$ to the human CRTH2 receptor was calculated by subtracting the radioactivity in the non-specific binding group from those in other groups. Inhibition (%) of the specific binding by the compound of the present invention was calculated based upon the specific binding in the vehicle and compound of the present invention groups and subsequently $K_i$ value (dissociation constant of the compound of the present invention) was calculated using estimated $IC_{50}$ value (concentration of the compound of the present invention needed to inhibit the specific binding in the vehicle group by 50%) according to the following formula.

$$K_i = IC_{50}/(1 + ([L]/K_d))$$

[L]: Concentration of $[^3H]$-$PGD_2$ (3 nmol/L)
$K_d$: Dissociation constant of $[^3H]$-$PGD_2$

The $K_d$ value of $[^3H]$-$PGD_2$ was estimated by non-linear regression analysis using specific binding at various concentrations of $[^3H]$-$PGD_2$ in accordance with aforementioned procedure.

From the result of the above measurement, it was found that the compounds of the present invention strongly bound to the human CRTH2 receptor at the Ki value of not more than 10 μmol/L.

Example 2

Calcium assay using CRTH2-CHO cells:

After collection of CRTH2-CHO cells by trypsinization, these cells were suspended in a medium containing calcium indicator ($Ca^{2+}$, $Mg^{2+}$-free HBSS containing 10 μmol/L Fura 2-AM (Dojindo Laboratories), 0.05% pluronic® F-127 (Molecular Probe), 250 μmol/L sulfinpyrazone (Sigma), 0.1% BSA and 10 mmol/L HEPES (Dojindo Laboratories), pH 7.4) at a cell density of $3 \times 10^6$ cells/mL. The cells were incubated for 1 h at 37°C in an atmosphere of 5% $CO_2$ and subsequently centrifuged for 3 min at 800 rpm at room temperature. After the resultant cell pellets were suspended in the assay medium (HBSS (Nissui Pharmaceutical Co., Ltd.) containing 1% BSA, 250 μmol/L sulfinpyrazone and 20 mmol/L HEPES, pH 7.4), the cells were centrifuged for 3 minutes at 800 rpm at room temperature (cell rinse). This manipulation of cell rinse repeated again. The resultant cell pellets were suspended in the assay medium to obtain a cell density at $2 \times 10^6$ cells/mL. A 100 μL portion of this suspension was added to each well of a 96-well microplate (Costar® 3614, Corning Inc.). Fluorescence intensity (FI) was measured by a fluorescence spectrophotometer (FDSS-6000, Hamamatsu Photonics) with dual excitation at 340 and 380 nm and emission at 510 nm, and the ratio of the FI at 510 nm (340 nm/380 nm) was regarded as an indicator of intracellular calcium concentration. Approximately 30

seconds following measurement of the FI, 25 μL of vehicle (5% DMSO diluted with the assay medium) or the compound of the present invention was added to each well. Five minutes later, 25 μL of 60 nmol/L $PGD_2$ (final concentration of $PGD_2$: 10 nmol/L), which was prepared by diluting 6 μmol/L $PGD_2$ in DMSO with the assay medium, was added to each well and the FI was monitored for 90 seconds. Antagonism of the compound of the present invention against the human CRTH2 receptor was evaluated using the $IC_{50}$ value as an indicator, based upon the $PGD_2$-induced increase in the FI in the control and compound of the present invention groups.

From the above-mentioned measuring result, it was found that the compounds of the present invention strongly shows antagonistic activity for human CRTH2 receptors at the $IC_{50}$ value of not more than 10 μmol/L.

The compound of the present invention represented by formula (I) binds to human DP receptor strongly and antagonizes. It was ensured by the following receptor binding experiment and receptor antagonism activity measurement experiment. As for the measuring method, there is general description in WO96/23066. In order to measure the activity of the test substances to human DP receptors easily and accurately, the inventors of the present invention made several improvements. Exemplification is shown in the following.

In all of the assays, the effects of the compound of the present inventions were evaluated using Chinese hamster ovary cells stably expressing the human DP receptor gene (DP-CHO cells).

Example 3

Ligand bonding experiment using cells in which prostanoid DP receptor is expressed:

DP-CHO cells were incubated and, according to a common method, membrane fraction was prepared.

In a tube made of polyethylene, the prepared membrane fraction (50 μL) (membrane protein amount: 30 to 200 μg), 100 μL of an assay buffer (25 mmol/L HEPES-NaOH containing 1 mmol/L of EDTA, 5 mmol/L of $Mg^{2+}$ and 10 mmol/L of $Mn^{2+}$; pH 7.4), 1 μL of a medium (dimethyl sulfoxide; DMSO) or the compound of the present invention (final concentration of DMSO: 0.5%) and 50 μL of 10 nmol/L $[^3H]$-$PGD_2$ (final concentration: 2.5 nmol/L) were placed, and incubated at the room temperature. In a non-specific bonding group, 2 mmol/L of $PGD_2$ was added instead of a medium (final concentration of $PGD_2$: 10 μmol/L). After 20 minutes, 1 mL of ice-cooled buffer for washing (10 mmol/L Tris-HCl buffer containing 0.01% of bovine serum albumin (BSA) and 100 mmol/L of NaCl; pH 7.4) was added to the tube to stop the reaction. Suction in reduced pressure was conducted immediately and the membrane fraction was trapped on a glass fiber filter paper (GF/B). The membrane fraction on the glass fiber filter paper was washed once with about 2 mL of buffer for washing and the glass fiber filter paper was dried. The dried glass fiber filter paper was place in a glass vial, a liquid scintillation cocktail was added thereto and radioactivity was measured by a liquid scintillation counter.

A specific-bonding amount of $[^3H]$-$PGD_2$ to the human DP receptor was calculated by deducting the radioactivity of the non-specific bonding group from the radioactivity of the groups other than the non-specific bonding group. An inhibiting rate by the compound of the present invention was calculated from the specific bonding amounts of $[^3H]$-$PGD_2$ in the medium group and the present invention group and, from the estimated $IC_{50}$ value (concentration of the compound of the present invention for inhibiting the specific bonding amount in the medium group to an extent of 50%), $K_i$ value (dissociation constant of the compound of the present invention) was calculated according to the following formula.

$$K_i = IC_{50}/(1+([L]^*/K_d))$$

$[L]^*$: concentration of $[^3H]$-$PGD_2$ (2.5 nmol/L)

$K_d$: dissociation constant of $[^3H]$-$PGD_2$

Incidentally, the $K_d$ value of $[^3H]$-$PGD_2$ was estimated in accordance with the above-mentioned method from a non-linear regression analysis after calculating the specific bonding amounts upon addition of $[^3H]$-$PGD_2$ in various concentrations.

From the result of the above measurement, it was found that the compounds of the present invention strongly bonded to the DP receptors at the $K_i$ value of not more than 10 μmol/L,

Example 4

cAMP assay using DP-CHO cells:

Incubated DP-CHO cells was suspended in minimum essential medium Eagle alpha modification (Sigma) containing 10% FCS, 100 μg/mL streptomycin , 100U/mL penicillin and 287 μg/mL L-glutamine, sowed on a 24-well

incubation plate in a cell density of 1 × $10^5$ cells/well and incubated at 37°C for 2 days in 5% $CO_2$. Each well was washed with 500 μL of MEM (minimum essential medium), 500 μL of MEM containing 2 μmol/L of diclofenac was added thereto and the mixture was incubated at 37°C for 10 minutes. After the supernatant liquid was removed by suction, 450 μL of an MEM (assay medium) containing 1 mmol/L of 3-isobutyl-1-methylxanthine, 2 μmol/L of diclofenac and 1% BSA was added, followed by incubation at 37°C for 10 minutes. Reaction was started by addition of 50 μL of an assay medium containing $PGD_2$ and medium or an assay medium containing $PGD_2$ and the compound of the present invention was added (final concentration of $PGD_2$: 10 nmol/L) and incubation was carried out at 37°C. After 10 minutes, 500 μL of ice-cooled trichloroacetic acid (TCA) (10% w/v) was added to stop the reaction. After freezing (-80°C) and melting the reaction solution once, the cells were removed therefrom using a scraper followed by centrifugation at 13,000 rpm for 3 minutes. The supernatant liquid was collected and cAMP concentration in the supernatant liquid was measured by a radioimmunoassay using a cAMP assay kit (manufactured by Amersham). The 125 μL of the above-prepared supernatant was moved to polypropylene tube including 200 μL of 0.5 mol/L tri-n-octylamine/chloroform solution (53/239, v/v). After extraction of TCA in a chloroform layer, an aqueous layer was used as a sample for quantifying the amount of cAMP in the sample according to the method mentioned in the cAMP assay kit.

Intensity of the antagonistic activity of the compound of the present invention for human DP receptors was calculated as an $IC_{50}$ value (concentration of the compound of the present invention which is necessary for suppressing the produced amount of cAMP in the absence of the compound of the present invention to an extent of 50%) from a suppressive rate to the production amount of cAMP in 10 nmol/L, in which a submaximum cAMP production activity is shown by $PGD_2$.

From the above-mentioned measuring result, it was found that the compounds of the present invention strongly shows antagonistic activity for DP receptors at the $IC_{50}$ value of not more than 10 μmol/L.

Toxicity:

Toxicity of the compound of the present invention represented by formula (I) is sufficiently low and it was confirmed to be sufficiently safe to be used as pharmaceuticals.

Application to pharmaceuticals:

The compounds of the present invention represented by formula (I) binds $PGD_2$ receptor, *i.e.* CRTH2 receptor and/or DP receptor and shows antagonistic activity.

Since the compounds of the present invention represented by formula (I) binds to CRTH2 receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome. They also participate in sleep and aggregation of platelets and are believed to be useful for those diseases as well.

Also, since the compounds of the present invention represented by formula (I) binds to DP receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome.

Among the compound of the present invention represented by formula (I), since compounds which binds weakly to substances other than $PGD_2$ receptors do not express other activity, they can be pharmaceuticals having little side effects.

The compound of the present invention represented by formula (I) may be administered as a combined preparation by combining with other pharmaceuticals for the purpose of

1) supplementing and/or enhancing of prevention and/or treatment effect of the compound,
2) improvement in pharmacokinetics and absorption and reduction of dose of the compound and/or
3) reduction of side effect of the compound.

The combined preparation of the compound of the present invention represented by formula (I) with other pharmaceuticals may be administered in a form of a compounded agent in which both components are compounded in a preparation or may be in a form in which they are administered by means of separate preparations. The case of administration by means of separate preparations includes a simultaneous administration and administrations with time difference. In the case of administrations with time difference, the compound of the present invention represented by formula (I) may be firstly administered followed by administering the other pharmaceutical or the other pharmaceutical may be administered firstly followed by administering the compound of the present invention represented by formula (I). Methods for each of the administration are the same or different. The each pharmaceutical may be solid composition or liquid composition.

There is no particular limitation for the diseases showing prevention and/or treatment effect by the above-mentioned combined preparation, so far as it is a disease in which the prevention and/or treatment effect of the compound of present invention represented by formula (I) are supplemented and/or enhanced.

The other pharmaceutical for supplementing and/or enhancing the prevention and/or treatment effect of the compound of the present invention represented by formula (I) for allergic rhinitis includes such as antihistaminic agent, suppressor for mediator liberation, inhibitor for thromboxane synthase, antagonist for thromboxane A2 receptor, antagonist for leukotriene receptor, steroid, stimulant for α-adrenaline receptor, xanthine derivative, anticholinergic agent and suppressor for nitrogen monoxide synthase.

The other pharmaceutical for supplementing and/or enhancing the prevention and/or treatment effect of the compound of the present invention represented by formula (I) for allergic conjunctivitis includes such as antagonist to leukotriene receptor, antihistaminic agent, suppressor for mediator liberation, non-steroid anti-inflammatory agent, prostaglandins, steroid and inhibitor for nitrogen monoxide synthase.

The antihistaminic agent includes such as ketotifen fumarate, mequitazine, azelastine hydrochloride, oxatomide, terfenadine, emedastine fumarate, epinastine hydrochloride, astemizole, ebastine, cetirizine hydrochloride, bepotastine, fexofenadine, loratadine, desloratadine, olopatadine hydrochloride, TAK-427, ZCR-2060, NIP-530, mometasone furoate, mizolastine, BP-294, andrast, auranofin and acrivastine.

The suppressor for mediator liberation includes such as tranilast, sodium cromoglicate, amlexanox, repirinast, ibudilast, tazanolast and pemirolast potassium.

Examples of the suppressor for enzymes for synthesis of thromboxane are ozagrel hydrochloride and imitorodast sodium.

The antagonist for thromboxane $A_2$ receptor includes such as seratrodast, ramatroban, domitroban calcium hydrate and KT-2-9G2.

The antagonist for leukotriene receptor includes such as pranlukast hydrate, montelukast, zafirlukast, MCC-847, KCA-757, CS-615, YM-158, L-740515, CP-195494, LM-1484, RS-635, A-93178, S-36496, BIIL-284 and ONO-4057.

The steroid agent, as its external application, includes such as clobetasol propionate, diflorasone acetate, fluocinonide, mometasone furancarboxylate, betamethasone dipropionate, betamethasone butyrate propionate, betamethasone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate propionate, fluocinolone acetonide, beclomethasone propionate, triamcinolone acetonide, flumethasone pivalate, alclometasone propionate, clobetasone valerate, prednisolone, beclomethasone propionate and fludroxycortide.

The agent for oral use and for injection includes such as cortisone acetate, hydrocortisone, hydrocortisone sodium phosphate, hydrocortisone sodium succinate, fludrocortisone acetate, prednisolone, prednisolone acetate, prednisolone sodium succinate, prednisolone butyl acetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate and betamethasone.

The inhalation agent includes such as beclomethasone propionate, fluticasone propionate, budesonide, flunisolide, triamcinolone, ST-126P, ciclesonide, dexamethasone palomithioate, mometasone furancarbonate, prasterone sulfonate, deflazacort, methylprednisolone suleptanate and methylprednisolone sodium succinate.

The xanthine derivative includes such as aminophylline, theophylline, doxophylline, cipamfylline and diprophylline.

The anticholinergic agent includes such as ipratropium bromide, oxitropium bromide, flutropium bromide, cimetropium bromide, temiberin, tiotropium bromide and levatropate (UK-112166).

The non-steroid anti-inflammatory agent includes such as sasapyrine, sodium salicylate, aspirin, aspirin dialuminate compounding, diflunisal, indomethacin, suprofen, ufenamate, dimethylisopropylazulene, bufexamac, felbinac, diclofenac, tolmetin sodium, clinoril, fenbufen, nabumetone, proglumetacin, indomethacin farnesyl, acemetacin, proglumetacin maleate, amfenac sodium, mofezolac, etodolac, ibuprofen, ibuprofen piconol, naproxen, flurbiprofen, flurbiprofen axetil, ketoprofen, fenoprofen calcium, tiaprofen, oxaprozin, pranoprofen, loxoprofen sodium, aluminoprofen, zaltoprofen, mefenamic acid, aluminum mefenamate, tolfenamic acid, floctafenine, ketophenylbutazone, oxyphenbutazone, piroxicam, tenoxicam, ampiroxicam, Napageln ointment, epirizole, tiaramide hydrochloride, tinoridine hydrochloride, emorfazone, sulpyrine, migrenin, salidon, Sedes G, Amipylo-N, Solbon, pyrazolone-type remedy for common cold, acetaminophen, phenacetin, dimethothiazine mesylate, simetride-compounded agent and non-pyrazolone-type remedy for common cold.

The prostaglandins (hereinafter, abbreviated as PG) includes such as a compound which binds PG receptor such as PGE receptors (EPI, EP2, EP3 and EP4), PGF receptor (FP), PGI receptor (IP) and TX receptor (TP) and the like. It is chosen among antagonist or agonist depending on symptom of disease appropriately.

The other PGD receptor antagonist includes such as S-5751 (described in WO97/00853) and a compound described in figure 15 in JP2002-98702 and the like.

There is no particular limitation for the ratio by weight of the compound represented by formula (I) to other pharmaceuticals.

With regard to other pharmaceuticals, any two or more may be compounded and administered.

With regard to other pharmaceuticals which supplement and/or enhance the prevention and/or treatment effect of the compound represented by formula (I), not only that which has been found up to now but also that which will be found in future on the basis of the above-mentioned mechanism are included.

When the compound represented by formula (I) or pharmaceutically acceptable salt thereof used in the present invention or a combined preparation of the compound represented by formula (I) with other pharmaceutical is used for the above-mentioned purpose, it is usually administered systemically or topically in an oral or parenteral form.

Although the dose varies depending upon age, body weight, symptom, therapeutic effect, administering method, treating time and the like, it is usually administered orally within a range of 1 mg to 1,000 mg for one administration to an adult from once to several times a day; parenterally (preferably, as a nasal agent, eye drops or ointment) within a range of 1mg to 100 mg for one administration to an adult from one to several times a day; or intravenously within a range of I to 24 hour(s) a day in a sustained manner.

It goes without saying that the dose varies under various conditions as described above and accordingly that, in some cases, less dose than the above may be sufficient while, in some other cases, more dose than the above range may be necessary.

In administering the compound represented by formula (I) or a pharmaceutically acceptable salts thereof or a combined preparation of the compound represented by formula (I) with other pharmaceutical, it is used as a solid composition, liquid composition and other composition for oral administration or as injection, agent for external application, suppository, and the like for parenteral administration.

The solid composition for oral administration includes such as tablets, pills, capsules, diluted powder and granules.

The capsules include hard capsules and soft capsules.

In such a solid composition, one or more active substance(s) is mixed with at least one inert diluent such as lactose, mannitol, glucose, hydroxypropyl cellulose, microcrystalline cellulose, starch, polyvinylpyrrolidone and magnesium metasilicate aluminate. The composition may contain an additive which is other than the inert diluent by a conventional method such as a lubricant such as magnesium stearate, a disintegrating agent such as calcium cellulose glycolate, a stabilizer such as lactose and a solubilizing agent such as glutamic acid and aspartic acid. Tablet or pill may, if necessary, be coated with film of an intragastrically soluble or enteric substance such as sugar, gelatin, hydroxypropyl cellulose and hydroxypropyl methylcellulose phthalate or may be coated with two or more layers. Capsule of a substance which is able to be absorbed such as gelatin is also included.

Liquid composition for oral administration includes such as pharmaceutically acceptable emulsion/suspension, solution, syrup and elixir. In such a liquid composition, one or more active substance(s) is included in a commonly used inert diluent (such as pure water and ethanol). Besides the inert diluent, the composition may contain an adjuvant such as moisturizer and suspending agent, sweetener, flavor, aromatic agent and antiseptic agent.

Other composition for oral administration includes spray agent which contains one or more active substance (s) and is formulated by a known method *per se.* Besides the inert diluent, the composition may contain a stabilizer such as sodium hydrogen sulfite and a buffer giving isotonicity such as isotonizing agent (such as sodium chloride, sodium citrate and citric acid). Method for the manufacture of spray agents is described, for example, in U. S. Patents

No. 2,868,691 and No. 3,095,355 in detail.

Parenteral injection according to the present invention includes aseptic aqueous and/or non-aqueous solution, suspension and emulsion. Aqueous solution and suspension includes such as distilled water for injection and physiological saline solution. Non-aqueous solution and suspension includes such as propylene glycol, polyethylene glycol, vegetable oil such as olive oil, alcohol such as ethanol and Polysorbate 80 (Registered Trademark). It is also possible that aseptic and aqueous or non-aqueous solution, suspension and emulsion may be mixed and used. Such a composition may further contain adjuvants such as antiseptic, moisturizer, emulsifier, dispersing agent, stabilizer (such as lactose) and solubilizing agent (such as glutamic acid and aspartic acid). They are sterilized by, for example, filtration passing through a bacteria-fixing filter, compounding of a disinfectant or irradiation. They may be also used in such a manner that, an aseptic solid composition is manufactured and, before using as a freeze-dried product for example, they are dissolved in sterilized or aseptic distilled water for injection or in other solvents.

An administration form of eye drop for parenteral administration includes eye drops, eye drops of a suspension type, eye drops of an emulsion type, eye drops which is dissolved upon actual use and eye ointment.

Such eye drops may be manufactured according to a known method. For example, in the case of the eye drops, an isotonizing agent (such as sodium chloride and concentrated glycerol), a buffering agent (such as sodium phosphate and sodium acetate), a surfactant (such as Polysorbate 80 (trade name), polyoxyl stearate 40 and polyoxyethylene hydrogenated castor oil), stabilizer (such as sodium citrate and sodium edetate), antiseptic agent (such as benzalkonium chloride and paraben), and the like are appropriately selected and prepared upon necessity. They are sterilized in the final step or prepared by an aseptic operation.

Inhalation agent for parenteral administration includes aerosol preparation, powder for inhalation, and liquid for inhalation. The liquid for inhalation may be such a form that, in actual use, the ingredient is dissolved or suspended in water or in other appropriate medium.

Those inhalation agents are prepared according to a known method.

For example, in the case of liquid for inhalation, antiseptic agent (such as benzalkonium chloride and paraben), coloring agent, buffer (such as sodium phosphate and sodium acetate), isotonizing agent (such as sodium chloride and concentrated glycerol), thickener (such as carboxyvinyl polymer), absorption promoter, and the like are appropriately selected and prepared upon necessity.

In the case of powder for inhalation, lubricant (such as stearic acid and salt thereof), binder (such as starch and dextrin), excipient (such as lactose and cellulose), coloring agent, antiseptic (such as benzalkonium chloride and paraben), absorption promoter, and the like are appropriately selected and prepared upon necessity.

In the administration of the liquid for inhalation, a spraying device (such as atomizer and nebulizer) are usually used while, in the administration of the powder for inhalation, an administering device for inhalation of powdery pharmaceutical is usually used.

Other composition for parenteral administration includes one or more active substance(s) and outer solution, ointment, liniment, suppository for intrarectal administration, pessary for intravaginal administration, and the like which are formulated by a conventional method.

Effect of the invention:

Since the compounds of the present invention represented by formula (I) binds to CRTH2 receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome. They also participate in sleep and aggregation of platelets and are believed to be useful for those diseases as well.

Since the compounds of the present invention represented by formula (I) binds to DP receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder)

which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome.

Best Mode for Carrying Out the Invention

The following reference examples and examples illustrate the present invention, but do not limit the present invention.

The solvents in the parentheses show the developing or eluting solvents and the ratios of the solvents used are by volume in chromatographic separations or TLC.

The solvents in the parentheses in $^1$H-NMR show the solvents for measurement.

In addition, the compound name shown in reference example and example was named by ACD/Name (version 5.05, Advanced Chemistry Development Inc.).

Reference Example 1

2-fluorophenylformamide

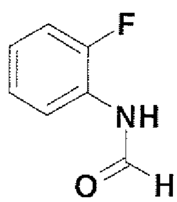

To an acetic anhydride (15.5 mL) was added dropwise formic acid (6.1 mL) at 0°C under an atmosphere of argon. The mixture was stirred at 50°C for 2 hours. After the reaction mixture was cooled to room temperature, it was diluted with tetrahydrofuran (10 mL). To the diluted solution was added 2-fluoroaniline (5.56 g) in THF (20 mL) at room temperature and then the mixture was stirred at room temperature for 1 hour. The reaction mixture was concentrated to give the title compound having the following physical data. The obtained title compound was used to next reaction without further purification.
TLC: Rf 0.70 (hexane : ethyl acetate =2 : 1).

Reference Example 2

N-(2-fluorophenyl)-N-methylamine

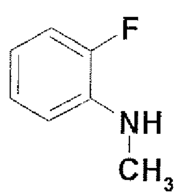

To a solution of the compound prepared in Reference Example 1 in anhydrous tetrahydrofuran (25 mL) was added borane - tetrahydrofuran complex (1M solution in tetrahydrofuran; 125 mL) under an atmosphere of argon, and the mixture was stirred at 50°C for 2 hours. The reaction mixture was cooled to room temperature. To the reaction mixture were added methanol (30 mL) and 4N hydrogen chloride in dioxane (10 mL) under ice cooling and the mixture was stirred at 60°C for 1 hour. The reaction mixture was concentrated, added 2N aqueous solution of sodium hydroxide, and then extracted with ethyl acetate. The organic layer was washed with a saturated aqueous solution of sodium

chloride, dried over anhydrous sodium sulfate. The solution was filtered through Celite (trade mark) and the filtrate was concentrated. To the residue was added mixed solvent (hexane : ethyl acetate = 10 : 1) and then filtered through silica gel. The filtrate was concentrated to give the title compound (6.45g) having the following physical data.
TLC: Rf 0.85 (hexane : ethyl acetate = 5 : 1);
$^1$H-NMR ($CDCl_3$): δ 7.00-6.91, 6.80-6.55, 3.90, 2.82.

Reference Example 3

(2S)-3-((2-fluorophenyl)(methyl)amino)-1,2-propanediol

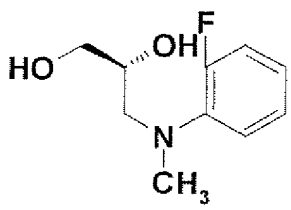

A mixture of the compound prepared in Reference Example 2 (1.24 g), (R)-(+)-glycidol (1.11 g, Aldrich, 98%ee) and ethanol (1 mL) was stirred at 50°C for 12 hours under an atmosphere of argon. The reaction mixture was concentrated to give the title compound having the following physical data. The obtained compound was used to next reaction without further purification.
TLC: Rf 0.40 (hexane : ethyl acetate =1 : 1).

Reference Example 4

((2S)-4-methyl-3,4-dihydro-2H-1,4-benzoxazin-2-yl)methanol

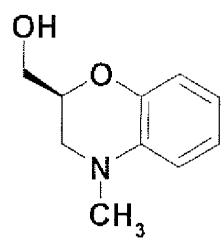

To a solution of the compound prepared in Reference Example 3 in anhydrous dimethylformamide (10 mL) was added potassium t-butoxide (1.68 g) in water bath, the mixture was stirred at 80°C for 3 hours. The reaction mixture was poured into water and extracted with ethyl acetate. The organic layer was washed with a saturated aqueous solution of sodium chloride, dried over anhydrous sodium sulfate, and then concentrated. The residue was purified by column chromatography on silica gel (hexane : ethyl acetate = 3 : 1) to give the title compound (1.55 g, 97.6%ee) having the following physical data.
TLC: Rf 0.35 (hexane : ethyl acetate =2 : 1);
$^1$H-NMR ($CDCl_3$): δ 7.90-6.79, 6.70-6.60, 4.33, 3.82, 3.79, 3.19, 3.17, 2.86.
The optical purity of the title compound was determined by high performance liquid chromatography (HPLC).
Column: CHIRALCEL OD (Daicel Chemical Industries Ltd.), 0.46 cmφ x 25 cm,
Flow rate: 1 mL/minute
Solvent: hexane : 2-propanol =93 : 7,
Detected wave-length: 254 nm,
Retention time: 30.70 minutes,

Temperature: 24°C.

Reference Example 5

((25)-4-methyl-3,4-dihydro-2H-1,4-benzoxazin-2-yl)methyl 4-methylbenzenesulfonate

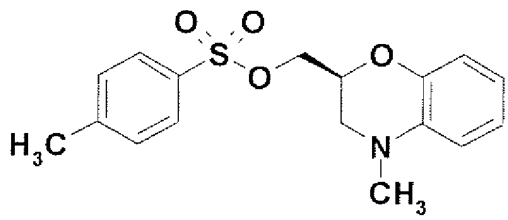

To a solution of the compound prepared in Reference Example 4 (3.06 g) in tetrahydrofuran (9 ml) was added triethylamine (5 ml) under an atmosphere of argon. To the reaction solution was added a solution of p-toluenesulfonic acid chloride (3.42 g) in tetrahydrofuran (9 ml) and N,N-dimethylaminopyridine (209 mg). The mixture was stirred at room temperature for 4 hours. After addition of water to the reaction mixture, it was extracted with t-butyl methyl ether. The organic layer was concentrated. To the obtained residue was added isopropyl alcohol to give a solid. The solid was collected by suction filtration, washed with isopropyl alcohol and dried to give the title compound having the following physical data.
TLC: Rf 0.81 (hexane : ethyl acetate = 1 : 1);
$^1$H-NMR ($CDCl_3$): δ 7.80, 7.34, 7.25-7.15, 6.83, 6.67-6.61, 4.45, 4.19-4.15, 3,24, 3.08, 2.82, 2,45.

Reference Example 6

methyl 6-(((2S)-4-methyl-3,4-dihydro-2H-1,4-benzoxazin-2-yl)methoxy)nicotinate

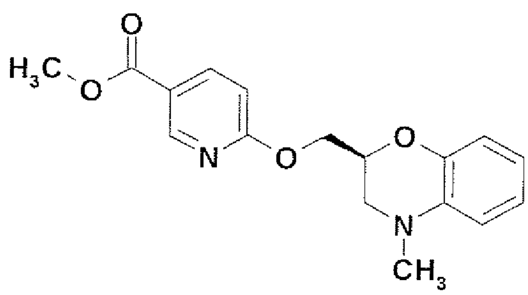

To a solution of methyl 6-hydroxynicotinate (1.0 g) in dimethylformamide (10 mL) was added cesium carbonate (4.7 g) and the compound prepared in Reference Example 5 (2.2 g). The mixture was stirred at 60°C for 6 hours. After addition of water to the reaction mixture, it was extracted with ethyl acetate. The organic layer was washed with water and a saturated aqueous solution of sodium chloride subsequently, and then dried over anhydrous sodium sulfate. The solvent was removed under reduced pressure to give the title compound having the following physical data.
TLC: Rf 0.22 (hexane : ethyl acetate = 1 : 1).

Reference Example 7

6-(((2S)-4-methyl-3,4-dihydro-2H-1,4-benzoxazin-2-yl)methoxy)nicotinic acid

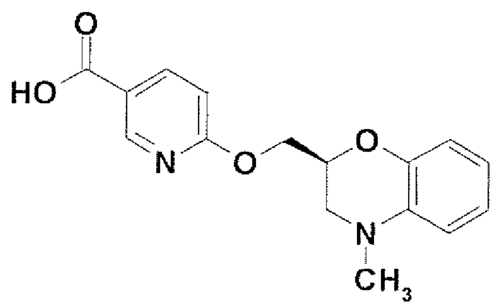

To a solution of the compound prepared in Reference Example 6 in a mixture of methanol (30 mL) - tetrahydrofuran (10 mL) was added 5N aqueous solution of sodium hydroxide (20 mL). The mixture was stirred at room temperature overnight. The reaction mixture was neutralized by adding 2N hydrochloric acid, and the mixture was extracted with ethyl acetate. The organic layer was washed with water and a saturated aqueous solution of sodium chloride, and then dried over anhydrous sodium sulfate. The solvent was removed and the obtained residue was washed with a mixture of ethyl acetate and hexane to give the title compound (1.3 g) having the following physical data.
TLC: Rf 0.43 (chloroform : methanol = 9 : 1);
$^1$H-NMR ($CDCl_3$): δ 8.20, 7.91, 6.94-6.86, 6.85-6.79, 6.75-6.67, 6.59, 4.68-4.58, 4,47, 4.07, 3.40,3.07, 2.89.

Reference Example 8

6-(((2S)-4-methyl-3,4-dihydro-2H-1,4-benzoxazin-2-yl)methoxy)nicotinoyl chloride

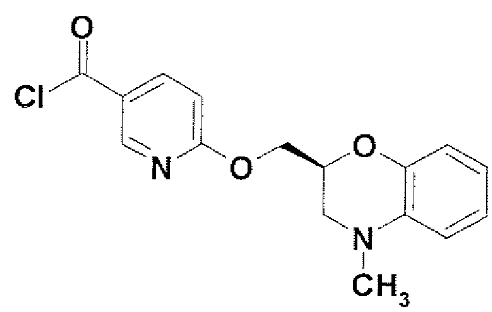

The compound prepared in Reference Example 7 (195 mg) was dissolved in dimethoxyethane (5 mL). To the mixture were added oxalyl chloride (0.13 mL) and dimethylformamide (0.4 μL), and the mixture was stirred at 40°C for 1 hour. The reaction mixture was concentrated *in vacuo* to give the title compound.

Reference Example 9

benzyl (2-methyl-1H-indol-3-yl)acetate

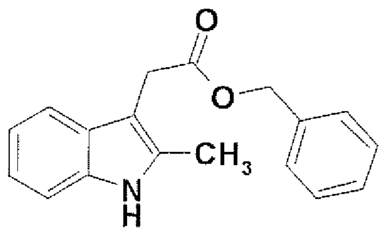

To a solution of 2-(2-methylindol-3-yl)acetic acid (1.73 g) in dimethylformamide (20 mL) were added potassium carbonate (2.52 g) and benzyl bromide (1,2 mL) under an atmosphere of argon and the mixture was stirred at room temperature for 2 hours. The reaction mixture was allowed to cool to room temperature. To the mixture was added water. The mixture was extracted with ethyl acetate. The extraction was washed with water and a saturated aqueous solution of sodium chloride, subsequently, dried over anhydrous sodium sulfate and then concentrated. The residue was purified by column chromatography on silica gel (hexane : ethyl acetate = 4 : 1) to give the title compound (2.63 g) having the following physical data.
TLC: Rf 0.52 (hexane : ethyl acetate = 7 : 3);
$^1$H-NMR ($CDCl_3$):$\delta$ 7.83, 7.55-7.48, 7.37-7.25, 7.16-7.04, 5.11, 3.74, 2.40.

Example 1

benzyl (2-methyl-1-((6-(((2S)-4-methyl-3,4-dihydro-2H-1,4-benzoxazin-2-yl)methoxy)-3-pyridinyl)carbonyl)-1H-indol-3-yl)acetate

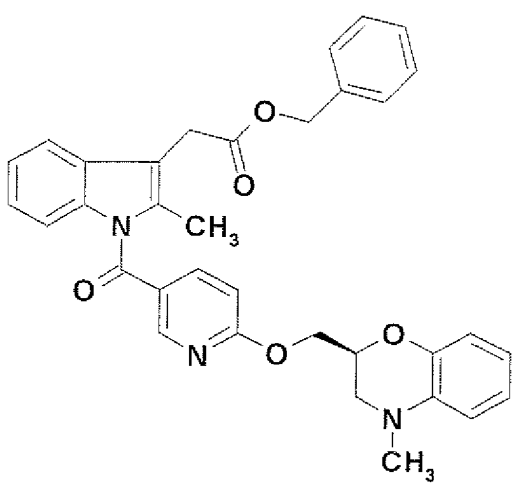

To a solution of the compound prepared in Reference Example 8 (207 mg) and the compound prepared in Reference Example 9 (140 mg) in methylene chloride (5 mL) were added 20N aqueous solution of sodium hydroxide (0.13 mL) and tetrabutyammonium chloride (14 mg) and the mixture was stirred at room temperature for 3 hours. To the reaction mixture was added ethyl acetate and water, and the mixture was extracted with ethyl acetate. The organic layer was washed with water and a saturated aqueous solution of sodium chloride, and dried over anhydrous sodium sulfate. The solvent was removed. The obtained residue was purified by column chromatography on silica gel (hexane : ethyl acetate = 4: 1 $\rightarrow$ 1 : 1) to give the compound (50 mg) of the present invention having the following physical data.

TLC: Rf 0.35 (ethyl acetate : hexane =1 : 1),
$^1$H-NMR ($CDCl_3$): δ 7.98, 7.64, 7.56-7.48, 7.43-7.08, 6.90-6.79, 6.74-6.62, 5.14, 4.67-4.57, 4.49-4.38, 4.08-3.94, 3.76, 3.38, 3.07, 2.85, 2.44.

Example 2

(2-methyl-1-((6-(((2S)-4-methyl-3,4-dihydro-2H-1,4-benzoxazin-2-yl)methoxy)-3-pyridinyl)carbonyl)-1H-indol-3-yl) acetic acid

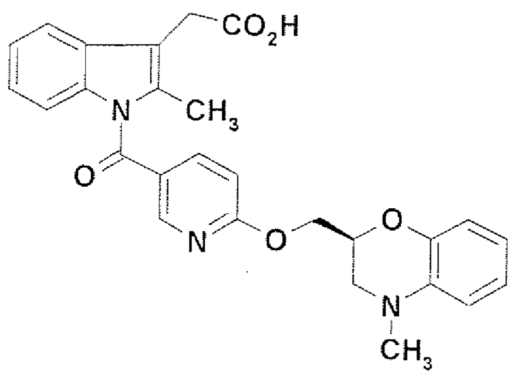

To a solution of the compound prepared in Example 1 (50 mg) in ethyl acetate (5 mL) was added 20% palladium hydroxide on carbon (25 mg) under an atmosphere of argon. The mixture was stirred under an atmosphere of hydrogen for 2 hours. The solution was filtered through cellite (trademark). The filtrate was extracted with ethyl acetate. The organic layer was washed with a saturated aqueous solution of sodium bicarbonate, water, a saturated aqueous solution of ammonium chloride, water, a saturated aqueous solution of sodium chloride, subsequently, and dried over anhydrous sodium sulfate. The solvent was removed to give the compound of the present invention (15 mg) having the following physical data.
TLC: Rf 0.52 (chloroform : methanol = 9 : 1);
$^1$H-NMR ($CDCl_3$): δ 7.96, 7.68, 7.57-7.51, 7.27-7.08, 6.90-6.76, 6.76-6.50, 4.67-4.56, 4.50-4.38, 4.07-3.92, 3.75, 3.37, 3.07, 2.85, 2.46.

Example 3(1)-Example 3(46)

Using 2-(2-methylindol-3-yl)acetic acid or corresponding carboxylic acid derivatives, and the compound prepared in Reference Example 8 or corresponding acid halide derivatives, the following compound of the present invention were obtained by the same procedures as a series of reactions of Reference Example 9 → Example 1 → Example 2.

Example 3(1)

(1-(4-(((2S)-4-methyl-3,4-dihydro-2H-1,4-benzoxazin-2-yl)methoxy)benzoyl)-1H-indol-3-yl)acetic acid

TLC: Rf 0.45 (methanol : chloroform = 1 : 10);
$^1$H-NMR ($CDCl_3$): δ 8.32, 7.73, 7.60, 7.45-7.33, 7.05, 6.95-6.80, 6.72, 4.69, 4.31, 4.21, 3.75, 3.41, 3.28, 2.92.

Example 3(2)

(1-(2-methyl-4-(((2S)-4-methyl-3,4-dihydro-2H-1,4-benzoxazin-2-yl)methoxy)benzoyl)-1H-indol-3-yl)acetic acid

TLC: Rf 0.48 (chloroform : methanol = 9 : 1);
$^1$H-NMR ($CDCl_3$): δ 8.28, 7.58-7.53, 7.42-7.29, 7.12, 6.92-6.82, 6.76-6.66, 4.72-4.62, 4.29, 4.18, 3.71, 3.41,3.27, 2.92, 2.34.

Example 3(3)

(1-(3-methyl-4-(((25)-4-methyl-3,4-dihydro-2H-1,4-benzoxazin-2-yl)methoxy)benzoyl)-1H-indol-3-yl)acetic acid

TLC: Rf 0.44 (chloroform : methanol = 9 : 1);
$^1$H-NMR ($CDCl_3$) : δ 8.30, 7.61-7.52, 7.41-7.27, 6.97-6.81, 6.76-6.66, 4.74-4.65, 4.32, 4.22, 3.74, 3.42, 3.31, 2.93, 2.30.

Example 3(4)

(1-((5-chloro-6-(((2S)-4-methyl-3,4-dihydro-2H-1,4-benzoxazin-2-yl)methoxy)-3-pyridinyl)carbonyl)-2-methyl-1H-indol-3-yl)acetic acid

TLC: Rf 0.40 (chloroform : methanol = 9 : 1);
$^1$H-NMR ($CDCl_3$): δ 7.91, 7.86, 7.59-7.50, 7.30-7.10, 6.87-6.76, 6.68-6.47, 4.68-4.58, 4.54-4.42, 4.14-4.00, 3.75, 3.37, 3.07, 2.84, 2.45.

Example 3(5)

(2-methyl-1-((3-(((2S)-4-methyl-3,4-dihydro-2H-1,4-benzoxazin-2-yl)methoxy)-5-isoxazolyl)carbonyl)-1H-indol-3-yl) acetic acid

TLC: Rf 0.38 (ethyl acetate : hexane : acetic acid = 5 : 5 : 1);
$^1$H-NMR ($CDCl_3$): δ 2.36, 2.91, 3.23, 3.35, 3.69, 4.55, 4.68, 6.64, 6.70, 6.88, 7.23, 7.34, 7.49.

Example 3(6)

(5-fluoro-2-methyl-1-((5-(((2S)-4-methyl-3,4-dihydro-2H-1,4-benzoxazin-2-yl)methoxy)-2-thienyl)carbonyl)-1H-indol-3-yl)acetic acid

TLC: Rf 0.49 (ethyl acetate : hexane : acetic acid = 5 : 5 : 1);
$^1$H-NMR ($CDCl_3$): δ 2.45, 2.91, 3.25, 3.37, 3.70, 4.32, 4.39, 4.69, 6.32, 6.70, 6.86, 7.16, 7.28.

Example 3(7)

(1-((3-(2-ethoxyethoxy)-5-isoxazolyl)carbonyl)-5-fluoro-2-methyl-1H-indol-3-yl)acetic acid

TLC: Rf 0.37 (ethyl acetate : hexane : acetic acid = 5 : 5 : 1);
$^1$H-NMR ($CDCl_3$):δ 1.26, 2.33, 3.61, 3.66, 3.82, 4.49, 6.63, 6.93, 7,15, 7.36.

Example 3(8)

(1-((3-(2-(2-butoxyethoxy)ethoxy)-5-isoxazolyl)carbonyl)-2-methyl-1H-indol-3-yl)acetic acid

TLC: Rf 0.48 (chloroform: methanol = 9 : 1);
$^1$H-NMR ($CDCl_3$): δ 0.92, 1.37, 1.58, 2.37, 3.48, 3.62, 3.72, 3.89, 4.50, 6.61, 7.24, 7.34, 7.49.

Example 3(9)

(1-((3-((2S)-2,3-dihydro-1-benzofuran-2-ylmethoxy)-5-isoxazolyl)carbonyl)-2-methyl-1H-indol-3-yl)acetic acid

TLC: Rf 0.33 (ethyl acetate : hexane : acetic acid = 5 : 5 : 1);
$^1$H-NNM ($CDCl_3$): δ 2.36, 3.09, 3.40, 3.70, 4.54, 5.20, 6.63, 6.88, 7.23, 7.49.

Example 3(10)

(5-chloro-1-((3-(((2S)-2,3-dihydro-1,4-benzodioxin-2-ylmethoxy)-5-isoxazolyl)carbonyl)-2-methyl-1H-indol-3-yl)acetic acid

TLC: Rf 0.55 (methylene chloride : methanol = 9 : 1);
$^1$H-NMR ($CDCl_3$): δ 2.35, 3-68, 4.20, 4.38, 4.61, 6.67, 6.91, 7.18, 7.31, 7.47.

Example 3(11)

(2-methyl-1-(4-(((2S)-4-methyl-3,4-dihydro-2H-1,4-benzoxazin-2-yl)methoxy)-3-(trifluoromethyl)benzoyl)-1H-indol-3-yl)acetic acid

TLC: Rf 0.53 (ethyl acetate : hexane : acetic acid =5 : 5 : 1),
$^1$H-NMR ($CDCl_3$): δ 2.42, 2.91, 3.32, 3.42, 3.75, 4.28, 4.41, 4.72, 6.69, 6.82, 6.89, 6.96, 7.07, 7.20, 7.52, 7.87, 8.08.

Example 3(12)

(5-chloro-2-methyl-1-(4-(((2S)-4-methyl-3,4-dihydro-2H-1,4-benzoxazin-2-yl)methoxy)-3-(trifluoromethyl)benzoyl)-1H-indol-3-yl)acetic acid

TLC: Rf 0.40 (ethyl acetate : hexane : acetic acid = 5 : 5 : 1);
$^1$H-NMR ($CDCl_3$): δ 2.40, 2.91, 3.32, 3.42, 3.71, 4.29, 4.42, 4.73, 6.69, 6.81, 6.88, 7.03, 7.10, 7.49, 7.84, 8.05.

Example 3(13)

(2,5-dimethyl-1-(4-(((2S)-4-methyl-3,4-dihydro-2H-1,4-benzoxazin-2-yl)methoxy)-3-(trifluoromethyl)benzoyl)-1H-indol-3-yl)acetic acid

TLC: Rf 0.43 (ethyl acetate : hexane : acetic acid =5 : 5 : 1);
$^1$H-NMR ($CDCl_3$): δ 2.41, 2.91, 3.32, 3.42, 3.72, 4.28, 4.41, 4.72, 6.69, 6.81, 6.89, 7.08, 7.29, 7.85, 8.06.

Example 3(14)

(1-(2-chloro-4-(((2S)-4,6-dimethyl-3,4-dihydro-2H-1,4-benzoxazin-2-yl)methoxy)benzoyl)-5-fluoro-2-methyl-1H-indol-3-yl)acetic acid

TLC: Rf 0.48 (chloroform: methanol = 10 : 1);
$^1$H-NMR ($CDCl_3$): δ 2.26, 2.28, 2.90, 3.25, 3.38, 3.65, 4.18, 4.27, 4.64, 6.51, 6.73, 6.84, 6.95, 7.04, 7.13, 7.21, 7.43.

Example 3(15)

(5-chloro-1-(2-chloro-4-(((2S)-4,6-dimethyl-3,4-dihydro-2H-1,4-benzoxazin-2-yl)methoxy)benzoyl)-2-methyl-1H-indol-3-yl)acetic acid

TLC: Rf 0.47 (chloroform : methanol = 10 : 1);
$^1$H-NMR ($CDCl_3$): δ 2.26, 2.28, 2.90,3.24, 3.37, 3.64, 4.17, 4.27, 4.64, 6.51, 6.73, 6.94, 7.05, 7.13, 7.42.

Example 3(16)

(1-(2-chloro-4-(((2S)-4,6-dimethyl-3,4-dihydro-2H-1,4-benzoxazin-2-yl)methoxy)benzoyl)-2,5-dimethyl-1H-indol-3-yl) acetic acid

TLC: Rf 0.47 (chloroform : methanol = 10 : 1);
$^1$H-NMR ($CDCl_3$): δ 2.27, 2.29, 2.39, 2.90, 3.24, 3.37, 3.67, 4.17, 4.26, 4.63, 6 .51, 6.72, 6.93, 7.02, 7.24, 7.41.

Example 3(17)

(1-(2-chloro-4-(((2S)-4,6-dimethyl-3,4-dihydro-2H-1,4-benzoxazin-2-yl)methoxy)benzoyl)-2-methyl-1H-indol-3-yl) acetic acid

TLC: Rf 0.46 (chloroform : methanol =10 : 1);
$^1$H-NMR ($CDCl_3$): δ 2.28, 2.30, 2.90, 3.24, 3.37, 3.68, 4.18, 4.27, 4.63, 6.51, 6-73, 6.93, 7.14, 7.44.

Example 3(18)

(1-(4-(((2S)-4,6-dimethyl-3,4-dihydro-2H-1,4-benzoxazin-2-yl)methoxy)benzoyl)-5-fluoro-2-methyl-1H-indol-3-yl) acetic acid

TLC: Rf 0.46 (chloroform : methanol = 10 : 1);
$^1$H-NMR ($CDCl_3$): δ 2.27, 2.32, 2.89, 3.24, 3.37, 3.59, 4.17, 4.27, 4.63, 6.50, 6.72, 6.89, 6.97, 7.10,7.66.

Example 3(19)

(5-chloro-1-(4-(((2S)-4,6-dimethyl-3,4-dihydro-2H-1,4-benzoxazin-2-yl)methoxy)benzoyl)-2-methyl-1H-indol-3-yl) acetic acid

TLC: Rf 0.47 (chloroform : methanol = 10 : 1);
$^1$H-NMR ($CDCl_3$): δ 2.27, 2.32, 2.89, 3.24, 3.37, 3.60, 4.17, 4.27, 4.63, 6.51, 6.72, 6.85, 6.94, 7.42, 7.66.

Example 3(20)

(1-(4-(((2S)-4,6-dimethyl-3,4-dihydro-2H-1,4-benzoxazin-2-yl)methoxy)benzoyl)-2,5-dimethyl-1H-indol-3-yl)acetic acid

TLC: Rf 0.47 (chloroform : methanol = 10 : 1);
$^1$H-NMR ($CDCl_3$): δ 2.28, 2.40, 2.41, 2.90, 3.26, 3.39, 3.71, 4.19, 4.29, 4.65, 6.51, 6.73, 6.84, 6.99, 7.28, 7.71.

Example 3(21)

(1-(4-(((2S)-4,6-dimethyl-3,4-dihydro-2H-1,4-benzoxazin-2-yl)methoxy)-3-methylbenzoyl)-5-fluoro-2-methyl-1H-indol-3-yl)acetic acid

TLC: Rf 0.48 (chloroform : methanol =10 : 1);
$^1$H-NMR ($CDCl_3$): δ 2.25, 2.28, 2.35, 2.90, 3.29, 3.39, 3.64, 4.20, 4.29, 4.64, 6.51, 6.74, 6.85, 6.92, 7.12, 7.50, 7.57.

Example 3(22)

(1-(4-(((2S)-4,6-dimethyl-3,4-dihydro-2H-1,4-benzoxazin-2-yl)methoxy)-3-methylbenzoyl)-2-methyl-1H-indol-3-yl) acetic acid

TLC: Rf 0.48 (chloroform ; methanol = 10 : 1);
$^1$H-NMR ($CDCl_3$): δ 2.26, 2.28, 2.41, 2.91, 3.29, 3.40, 3.73, 4.21, 4.30, 4.66, 6.51, 6.73, 6.87, 7.02, 7.16, 7.50, 7.55, 7.61.

Example 3(23)

(1-(2-chloro-4-(((2S)-6-fluoro-4-methyl-3,4-dihydro-2H-1,4-benzoxazin-2-yl)methoxy)benzoyl)-2-methyl-1H-indol-3-yl)acetic acid

TLC: Rf 0.59 (chloroform: methanol = 9 : 1);
$^1$H-NMR ($CDCl_3$): δ 2.32, 2.91, 3.30, 3.40, 3.71, 4.18, 427, 4.60, 6.38, 6.74, 6.94, 7.14, 7.46.

Example 3(24)

(5-chloro-1-(2-chloro-4-(((2S)-6-fluoro-4-methyl-3,4-dihydro-2H-1,4-benzoxazin-2-yl)methoxy)benzoyl)-2-methyl-1H-indol-3-yl)acetic acid

TLC: Rf 0.59 (chloroform : methanol = 9 : 1);
$^{1}$H-NMR ($CDCl_3$): δ 2.28, 2.91, 3.30, 3.40, 3.66, 4.18, 4.27, 4.61, 6.38, 6.74, 6.95, 7.06, 7.14, 7.44.

Example 3(25)

(1-(2-chloro-4-(((2S)-6-fluoro-4-methyl-3,4-dihydro-2H-1,4-benzoxazin-2-yl)methoxy)benzoyl)-2,5-dimethyl-1H-indol-3-yl)acetic acid

TLC: Rf 0.60 (chloroform: methanol = 9 : 1);
$^{1}$H-NMR ($CDCl_3$): δ 2.30, 2.39, 2.91, 3.29, 3.40, 3.67, 4.17, 4.27, 4.60, 6.38, 6.74, 6.93, 7.02, 7.24, 7.42.

Example 3(26)

(5-fluoro-1-(4-(((2S)-6-fluoro-4-methyl-3,4-dihydro-2H-1,4-benzoxazin-2-yl)methoxy)-3-methylbenzoyl)-2-methyl-1H-indol-3-yl)acetic acid

TLC: Rf 0.66 (chloroform : methanol = 9 : 1);
$^{1}$H-NMR. ($CDCl_3$): δ 2.27, 2.39, 2.91, 3.34, 3.43, 3.70, 4.20, 4.31, 4.63, 6.38, 6.76, 6.88, 6.95, 7.16, 7.56.

Example 3(27)

(5-chloro-1-(4-(((2S)-6-fluoro-4-methyl-3,4-dihydro-2H-1,4-benzoxazin-2-yl)methoxy)-3-methylbenzoyl)-2-methyl-1H-indol-3-yl)acetic acid

TLC: Rf 0.69 (chloroform : methanol = 9 : 1);
$^{1}$H-NMR ($CDCl_3$): δ 2.27, 2.41, 2.91, 3.34, 3.43, 3.71, 4.20, 4.31, 4.63, 6.38, 6.73, 6.89, 6.99, 7.48, 7.56.

Example 3(28)

(1-(4-(((2S)-6-fluoro-4-methyl-3,4-dihydro-2H-1,4-benzoxazin-2-yl)methoxy)-2,5-dimethylbenzoyl)-2-methyl-1H-indol-3-yl)acetic acid

TLC: Rf 0.38 (ethyl acetate : hexane : acetic acid = 5 : 5 : 1);
$^{1}$H-NMR ($CDCl_3$): δ 2.17, 2.26, 2.34, 2.92, 3.34, 3.43, 3.72, 4.18, 4.29, 4.63, 6,38, 6,74, 7.04, 7.18,7.48.

Example 3(29)

(5-fluoro-1-(4-(((2S)-6-fluoro-4-methyl-3,4-dihydro-2H-1,4-benzoxazin-2-yl)methoxy)-2,5-dimethylbenzoyl)-2-methyl-1H-indol-3-yl)acetic acid

TLC: Rf 0.37 (ethyl acetate : hexane : acetic acid = 5 : 5 : 1);
$^{1}$H-NMR ($CDCl_3$): δ 2.17, 2.25, 2.30, 2.92, 3.33, 3.43, 3.67, 4.18, 4.29, 4.63, 6.38, 6.74, 6.80, 7.01, 7.13, 7.16.

Example 3(30)

(1-(4-(((2S)-4,6-dimethyl-3,4-dihydro-2H-1,4-benzoxazin-2-yl)methoxy)-3-methylbenzoyl)-2,5-dimethyl-1H-indol-3-yl) acetic acid

TLC: Rf 0.57 (chloroform: methanol = 9 : 1);
$^{1}$H-NMR ($CDCl_3$): δ 2.25, 2.28, 2.38, 2.91, 3.29, 3.39, 3.67, 4.19, 4.28, 4.66, 6.51, 6.72, 6.84, 7.26, 7.53, 7.58.

Example 3(31)

(5-chloro-1-(4-(((2S)-4,6-dimethyl-3,4-dihydro-2H-1,4-benzoxazin-2-yl)methoxy)-2-methylbenzoyl)-2-methyl-1H-indol-3-yl)acetic acid

TLC: Rf 0.56 (chloroform : methanol = 9 : 1);
$^1$H-NMR ($CDCl_3$): δ 2.27, 2.89, 3.23, 3.37, 3.59, 4.14, 4.25, 4.62, 6.52, 6.74, 6.90, 7.26, 7.39.

Example 3(32)

(1-(4-(((2S)-4,6-dimethyl-3,4-dihydro-2H-1,4-benzoxazin-2-yl)methoxy)-2-methylbenzoyl)-5-fluoro-2-methyl-1H-indol-3-yl)acetic acid

TLC: Rf 0.53 (chloroform : methanol = 9 : 1);
$^1$H-NMR ($CDCl_3$): δ 2.23, 2.27, 2.89, 3.23, 3.37, 3.56, 4.14, 4.25, 4.61, 6.50, 6.73, 6.85, 6.96, 7.07, 7.26.

Example 3(33)

(1-(4-(((2S)-4,6-dimethyl-3,4-dihydro-2H-1,4-benzoxazin-2-yl)methoxy)-2-methylbenzoyl)-2,5-dimethyl-1H-indol-3-yl) acetic acid

TLC: Rf 0.54 (chloroform : methanol = 9 : 1);
$^1$H-NMR ($CDCl_3$): δ 2.27, 2.28, 2.35, 2.90, 3.24, 3.37, 3.62, 4.14, 4.25, 4.62, 6.51, 6.79, 7.21, 7.28.

Example 3 (34)

(5-chloro-1-(4-(((2S)-6-fluoro-4-methyl-3,4-dihydro-2H-1,4-benzoxazin-2-yl)methoxy)-2,5-dimethylbenzoyl)-2-methyl-1H-indol-3-yl)acetic acid

TLC: Rf 0.54 (chloroform : methanol = 9 : 1);
$^1$H-NMR ($CDCl_3$):δ 2.17, 2.25, 2.32, 2.92, 3.33, 3.43, 3.68, 4.13, 4.29, 4.64, 6.39, 6.74, 6.93, 7.00, 7.16, 7.44.

Example 3(35)

(1-(4-(((2S)-6-fluoro-4-methyl-3,4-dihydro-2H-1,4-benzoxazin-2-yl)methoxy)-2,5-dimethylbenzoyl)-2,5-dimethyl-1H-indol-3-yl)acetic acid

TLC: Rf 0.55 (chloroform : methanol = 9 : 1);
$^1$H-NMR ($CDCl_3$): δ 2.17, 2.24, 2.32, 2.39, 2.90, 3.33, 3.43, 3.69, 4,17, 4.29, 4.61, 6.38, 6.74, 6.86, 7.18, 7.26.

Example 3(36)

(1-(4-(((2S)-6-fluoro-4-methyl-3,4-dihydro-2H-1,4-benzoxazin-2-yl)methoxy)benzoyl)-2-methyl-1H-indol-3-yl)acetic acid

TLC: Rf 0.52 (methylene chloride: methanol = 9 : 1);
$^1$H-NMR ($CDCl_3$): δ 2.43, 2.91, 3.31, 3.41, 3.75, 4.19, 4.30, 4.61, 6.37, 6.74, 7.01, 7.17, 7.51, 7.73.

Example 3(37)

(5-fluoro-1-(4-(((2S)-6-fluoro-4-methyl-3,4-dihydro-2H-1,4-benzoxazin-2-yl)methoxy)benzoyl)-2-methyl-1H-indol-3-yl)acetic acid

TLC: Rf 0.48 (methylene chloride : methanol =9 : 1);
$^1$H-NMR ($CDCl_3$): δ 2.40, 2.91, 3.31, 3.42, 3.70, 4.19, 4.30, 4.61, 6.38, 6.76, 6.94, 7.01, 7.16, 7.71.

Example 3(38)

(5-chloro-1-(4-(((2S)-6-fluoro-4-methyl-3,4-dihydro-2H-1,4-benzoxazin-2-yl)methoxy)benzoyl)-2-methyl-1 H-indol-3 -y 1)acetic acid

TLC: Rf 0.52 (methylene chloride : methanol = 9 : 1);
$^1$H-NMR ($CDCl_3$): δ 2.41, 2.91, 3.31, 3.41, 3.71, 4.19, 4.30, 4.61, 6.38, 6.74, 6.91, 7.01, 7.45, 7.71.

Example 3(39)

(5-fluoro-1-(4-(((2S)-6-fluoro-4-methyl-3,4-dihydro-2H-1,4-benzoxazin-2-yl)methoxy)-2-methylbenzoyl)-2-methyl-1H-indol-3-yl)acetic acid

TLC: Rf 0.49 (methylene chloride : methanol =9 : 1);
$^1$H-NMR ($CDCl_3$): δ 2.30, 2.31, 2.91, 3.30, 3.41, 3.67, 4.16, 4.27, 4.60, 6.38, 6.77, 6.87, 7.00, 7.14,7.31.

Example 3(40)

(5-chloro-1-(4-(((2S)-6-fluoro-4-methyl-3,4-dihydro-2H-1,4-benzoxazin-2-yl)methoxy)-2-methylbenzoyl)-2-methyl-1H-indol-3-yl)acetic acid

TLC: Rf 0.48 (methylene chloride : methanol = 9 : 1);
$^1$H-NMR ($CDCl_3$): δ 2.32, 2.91, 3.30, 3.41, 3.68, 4.16, 4.27, 4.60, 6.38, 6.74, 6.79, 6.88, 6.93, 7.02, 7.31, 7.45.

Example 3(41)

(1-(4-(((2S)-6-fluoro-4-fluoro-4-methyl-3,4-dihydro-2H-1,4-benzoxazin-2-yl)-methoxy)-2-methylbenzoyl)-2,5-dimethyl-1H-indol-3-yl)acetic acid

TLC: Rf 0.48 (methylene chloride : methanol = 9 : 1);
$^1$H-NMR ($CDCl_3$): δ 2.30, 2.33, 2.39, 2.91, 3.30, 3.41, 3.69, 4.15, 4.27, 4.59, 6.37, 6,80, 7.25, 7.32.

Example 3(42)

(1-(4-(((2S)-6-fluoro-4-methyl-3,4-dihydro-2H-1,4-benzoxazin-2-yl)methoxy)-3-methylbenzoyl)-2-methyl-1H-indol-3-yl)acetic acid

TLC: Rf 0.54 (chloroform : methanol =9 : 1);
$^1$H-NMR ($CDCl_3$): δ 2.27, 2.42, 2.91, 3.34, 3.43, 3.75, 4.20, 4.31, 4.63, 6.38, 6.73, 6.87, 7.03, 7.16, 7.57.

Example 3(43)

(1-(4-(((2S)-6-fluoro-4-methyl-3,4-dihydro-2H-1,4-benzoxazin-2-yl)methoxy)-3-methylbenzoyl)-2,5-dimethyl-1H-indol-3-yl)acetic acid

TLC: Rf 0.55 (chloroform: methanol =9 : 1);
$^1$H-NMR ($CDCl_3$): δ 2.26, 2.40, 2.41, 2.91, 3.33, 3.43, 3.72, 4.19, 4.30, 4.63,, 6.38, 6.73, 6.85, 7.28, 7.57.

Example 3(44)

(1-(4-(((2S)-4,5-dimethyl-3,4-dihydro-2H-1,4-benzoxazin-2-yl)methoxy)-2,5-dimethylbenzoyl)-5-fluoro-2-methyl-1H-indol-3-yl)acetic acid

TLC: Rf 0.56 (chloroform : methanol =9 : 1);
$^1$H-NMR ($CDCl_3$): δ 2.17, 2.25, 2.28, 2.30, 2.91, 3.29, 3.40, 3.68, 4.19, 4.29, 4.66, 6.51, 6.74, 6.80, 7.00, 7.13.

Example 3(45)

(5-chloro-1-(4-(((2S)-4,6-dimethyl-3,4-dihydro-2H-1,4-benzoxazin-2-yl)methoxy)-2,5-dimethylbenzoyl)-2-methyl-1H-indol-3-yl)acetic acid

TLC: Rf 0.56 (chloroform : methanol =9 : 1);
$^1$H-NMR ($CDCl_3$): δ 2.17, 2.25, 2.28, 2.32, 2.91, 3.29, 3.40, 3.69, 4.19, 4.29, 4.66, 6.53, 6.74, 6.93,7.01, 7.15, 7.45.

Example 3(46)

(1-(4-(((2S)-4,6-dimethyl-3,4-dihydro-2H-1,4-benzoxazin-2-yl)methoxy)-2,5-dimethylbenzoyl)-2-methyl-1H-indol-3-yl) acetic acid

TLC: Rf 0.55 (chloroform : methanol = 9 : 1);
$^1$H-NMR ($CDCl_3$): δ 2.17, 2.26, 2.28, 2.34, 2.91, 3.29, 3.40, 3.72, 4.19, 4.29, 4.66, 6.52, 6,74, 7.05, 7.19, 7.48.

Reference Example 10

benzyl (1-(4-(acetyloxy)benzoyl)-2-methyl-1H-indol-3-yl)acetate

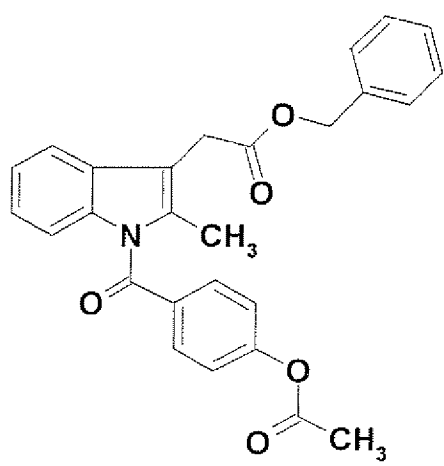

To a solution of the compound prepared in Reference Example 9 (3.45 g) in methylene chloride (100 mL) was added a solution of benzyltriethylammonium chloride (281 mg) and 4-acetyloxybenzoyl chloride (3.68 g) in methylene chloride (24 mL). To the mixture was added sodium hydroxide (2.47 g) and the mixture was stirred at room temperature for 40 minutes. The reaction mixture was filtered through cellite. The filtrate was used for the next reaction.
TLC: Rf 0.49 (hexane : ethyl acetate = 7 : 3).

Reference Example 11

benzyl (1-(4-hydroxybenzoyl)-2-methyl-1H-indol-3-yl)acetate

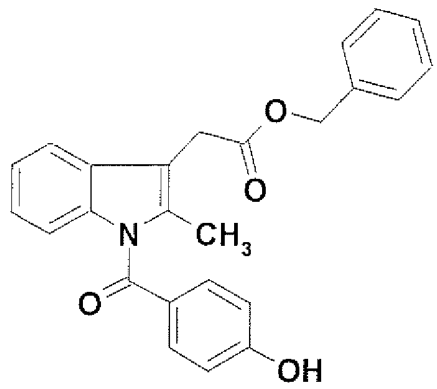

To the filtrate prepared in Reference Example 10 was added piperidine (3.46 mL) at room temperature. The mixture was stirred at room temperature for 1.5 hours. To the reaction mixture was added 2N hydrochloric acid, and then separated. The organic layer was washed with water and a saturated aqueous solution of sodium chloride subsequently, dried over anhydrous sodium sulfate, and concentrated. The residue was purified by column chromatography on silica gel (hexane : ethyl acetate = 7 : 3) to give the title compound (3 g) having the following physical data.
TLC: Rf 0.24 (hexane : ethyl acetate =7 : 3);
$^1$H-NMR($CDCl_3$): δ 7.66, 7.49, 7.38-7.26, 7.15, 7.10-6.97, 6.88, 5.15, 3.76, 2.40.

Example 4

benzyl (1-(4-(2-(2-butoxethoxy)ethoxy)benzoyl)-2-methyl-1H-indol-3-yl)acetate

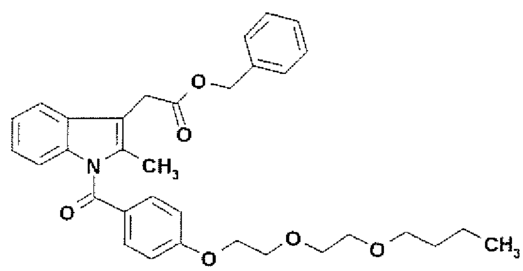

To a solution of the compound prepared in Reference Example 11 (52 mg) and 2-(2-butoxyethoxy)ethanol (57 mg) in tetrahydrofuran (4 mL) were added triphenylphosphine (102 mg) and diethyl azodicarboxylate (40% solution in toluene, 0.2 mL). The mixture was stirred at room temperature for I hour. The solvent was removed from the reaction mixture. The residue was purified by column chromatography on silica gel (ethyl acetate : hexane = 9 : 1 → 4 : 1) to give the compound of the present invention (70 mg) having the following physical data.
TLC: Rf 0.50 (hexane ; ethyl acetate = 7 : 3).

Example 5

(1-(4-(2-(2-butoxyethoxy)ethoxy)benzoyl)-2-methyl-1H-indol-3-yl)acetic acid

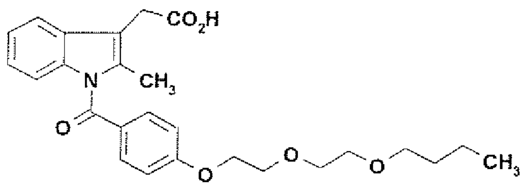

Using the compound prepared in Example 4 instead of the compound prepared in Example 1, the compound of the present invention having the flowing physical data was obtained by the same procedure of Example 2.
TLC: Rf 0.49 (chloroform : methanol = 9 : 1);
$^1$H-NMR ($CDCl_3$): δ 7.71, 7,51, 7.17, 7.04, 7.01-6.40, 4.30-4.10, 3.91, 3.78-3.40, 2.43, 1.70-1.20, 0.91.

Example 6(1)-Example 6(45)

Using the compound prepared in Reference Example 9 or a corresponding indole derivatives, and alcohol derivatives instead of 2-(2-butoxyethoxy)ethanol, the following compounds were obtained by same procedure described in Reference Example 10→Reference Example 11→Example 4→Example 2.

Example 6(1)

(1-(4-(((2S)-2-methoxy-3-(methyl(phenyl)amino)propyl)oxy)benzoyl)-2-methyl-1H-indol-3-yl)acetic acid

TLC: Rf 0.61 (chloroform : methanol = 10 : 1);
$^1$H-NMR ($CDCl_3$): δ 7.71, 7.25-7.23, 7.10-6.90, 6.77-6.70, 6.48, 4.15, 4.07, 3.85, 3.85, 3.66, 3.55, 3.51, 3.01, 2.44.

Example 6(2)

(1-(4-(2-(2,6-dimethylphenyl)ethoxy)-2-methylbenzoyl)-2-methyl-1H-indol-3-yl)acetic acid

TLC: Rf 0.49 (chloroform : methanol =9 : 1);
$^1$H-NMR ($CDCl_3$): δ 7.47, 7.30, 7.17, 7.10-6.95, 6.81, 6.72, 4.10, 3.71, 3.21, 2.41, 2.34, 2.31.

Example 6(3)

(2-methyl-1-(2-methyl-4-(2-(4-methyl-2-pyridinyl)ethoxy)benzoyl)-1H-indol-3-yl)acetic acid

TLC: Rf 0.49 (chloroform : methanol = 9 : 1);
$^1$H-NMR ($CDCl_3$): δ 8.44, 7.71-6.94, 6.75, 6.62, 4.30, 3.71, 3.23, 2.37, 2.32, 2.25.

Example 6(4)

(1-(4-(2-(2,4-dimethoxyphenoxy)ethoxy)-2-methylbenzoyl)-2-methyl-1H-indol-3-yl)acetic acid

TLC: Rf 0.49 (chloroform : methanol = 9 : 1);
$^1$H-NMR ($CDCl_3$): δ 7.48, 7.32, 7.22-7.14, 7.10-6.86, 6.80, 6.53, 6.40, 4.36, 3.8 4, 3.79, 3.72, 2.34, 2.32.

Example 6(5)

(1-(4-(2-(2-methoxyphenoxy)ethoxy)-2-methylbenzoyl)-2-methyl-1H-indol-3-yl)acetic acid

TLC: Rf 0.49 (chloroform : methanol = 9 : 1);
$^1$H-NN4R ($CDCl_3$): δ 7.48, 7.32, 7.22-7.14, 7.10-6.85, 6.80, 4.42, 3.87, 3.72, 2.34, 2.32.

Example 6(6)

(1-(4-(2,3-dihydro-1-benzofuran-3-ylmethoxy)-2-methylbenzoyl)-2-methyl-1H-indol-3-yl)acetic acid

TLC: Rf 0.50 (chloroform : methanol = 9 : 1);
$^1$H-NMR ($CDCl_3$): δ 7.48, 7.37-7.29, 7.25-7.24, 7.10-6.70, 4.73, 4-55, 4.21, 4.18-3.90, 3.72, 2.34,2.32.

Example 6(7)

(1-(4-(1,3-benzodioxol-2-ylmethoxy)-2-methylbenzoyl)-2-methyl-1H-indol-3-yl)acetic acid

TLC: Rf 0.50 (chloroform : methanol =9 : 1);
$^1$H-NMR ($CDCl_3$): δ 7.48, 7.34, 7.24-6.94, 6.92-6.84, 6.79, 6.48, 4.33, 3.72, 2.34, 2.32.

Example 6(8)

(2-methyl-1-(2-methyl-4-((1-methyl-2,3-dihydro-1H-indol-2-yl)methoxy)benzoyl)-1H-indol-3-yl)acetic acid

TLC: Rf 0.50 (chloroform : methanol =9 : 1);
$^1$H-NMR ($CDCl_3$): δ 7.54-6.48, 4.26, 4.21-4.04, 3.90-3.80, 3.72, 3.27, 2.91, 2.34, 2.33.

Example 6(9)

(2-methyl-1-(2-methyl-4-(2-(3-methyl-2-pyridinyl)ethoxy)benzoyl)-1H-indol-3-yl)acetic acid

TLC: Rf 0.44 (chloroform methanol = 9 : 1);
$^1$H-NMR ($CDCl_3$): δ 8.48-8.40, 7.57-7.47, 7.34-6.94, 6.76, 6.64, 4.37, 3.71, 3.30, 2.42, 2.33, 2.26.

Example 6(10)

(2-methyl-1-(2-methyl-4-(2-(6-methyl-2-pyridinyl)ethoxy)benzoyl)-1H-indol-3-yl)acetic acid

TLC: Rf 0.44 (chloroform : methanol =9 : 1);
$^1$H-NMR ($CDCl_3$): δ 7.60-7.47, 7.30-5.96, 6.77, 6.65, 4.31, 3.71, 3.24, 2.56, 2.33, 2.27.

Example 6(11)

(1-(4-(2,3-dihydro-1-benzofuran-2-ylmethoxy)-2-methylbenzoyl)-2-methyl-1H-indol-3-yl)acetic acid

TLC: Rf 0.44 (chloroform : methanol = 9 : 1);
$^1$H-NMR ($CDCl_3$): δ 7.47, 7.32, 7.25-7.09, 7.09-6.94, 6.93-6.76, 5.24-5.13, 4.27, 4.17, 3.72, 3.42, 3.16, 2.34, 2.31.

Example 6(12)

(1-(4-((2R)-2,3-dihydro-1,4-benzodiaxin-2-ylmethoxy)-2-methylbenzoyl)-2-methyl-1H-indol-3-yl)acetic acid

TLC: Rf 0.44 (chloroform methanol = 9 : 1);
$^1$H-NMR ($CDCl_3$): δ 7.48, 7.33, 7.18, 7.10-6.84, 6.79, 4.64-4.56, 4.42, 4.35-4.18, 3.71, 2.34, 2.32.

Example 6(13)

(1-(4-(2-(3,4-dihydro-1(2H)-quinolinyl)ethoxy)-2-methylbenzoyl)-2-methyl-1H-indol-3-yl)acetic acid

TLC: Rf 0.44 (chloroform: methanol = 9 : 1);
$^1$H-NMR ($CDCl_3$): δ 7.47, 7.30, 7.22-6,94, 6.81, 6.73, 6.68-6.56, 4.21, 3.73, 3.71, 3.46, 2.77, 2.33, 2.30, 2.02-1.92.

Example 6(14)

(2-methyl-1-(2-methyl-4-(2-(methyl(3-methylphenyl)amino)ethoxy)benzoyl)-1H-indol-3-yl)acetic acid

TLC: Rf 0.44 (chloroform: methanol = 9 : 1);
$^1$H-NMR ($CDCl_3$): δ 7.47, 7.30, 7.22-7.10, 7.05, 6.97, 6.80, 6.72, 6.63-6.54, 4.19, 3.78, 3.71, 3.06,2.33, 2.32,2.30.

Example 6(15)

(2-methyl-1-(2-methyl-4-(3-(methyl(phenyl)amino)propoxy)benzoyl)-1H-indol-3-yl)acetic acid

TLC: Rf 0.44 (chloroform : methanol = 9 : 1);
$^1$H-NMR ($CDCl_3$) : δ 7.48, 7.31, 7.28-7.14, 7.09-6.97, 6.84, 6.80-6.66, 4.07, 3.72, 3.57, 2.95, 2.34, 2.32, 2.15-2.04.

Example 6(16)

(1-(2-chloro-4-((1-ethyl-2,3-dihydro-1H-indol-5-yl)methoxy)benzotl)-5-fluoro-2methyl-1H-indol-3-yl)acetic acid

TLC: Rf 0.48 (chloroform : methanol =9 : 1);
$^1$H-NMR ($CDCl_3$): δ 7.44, 7.28-6.80, 6.72-6.62, 6.48, 4.24-4.06, 3.66, 3.47-3.23, 2.89, 2.27, 1.17.

Example 6(17)

(1-(2-chloro-4-(((25)-1-ethyl-2,3-dihydro-1H-indol-2-yl)methoxy)benzoyl)-5-fluoro-2-methyl-1H-indol-3-yl)acetic acid

TLC: Rf 0.48 (chloroform : methanol = 9 : 1);
$^1$H-NMR ($CDCl_3$): δ 7.44, 7.28-6.80, 6.72-6.62, 6.48, 4.24-4.06, 3.66, 3.47-3.23, 2.89, 2.27, 1.17.

Example 6(18)

(1-(4-(3,4-dihydro-2H-1,5-benzodioxepin-3-ylmethoxy)-2-methylbenzoyl)-2-methyl-1H-indol-3-yl)acetic acid

TLC: Rf 0.50 (chloroform : methanol = 9 : 1);
$^1$H-NMR ($CDCl_3$): δ 7.48, 7,37-6.74, 4.34, 4.22, 3.72, 2.80-2.68, 2.34, 2.32.

Example 6(19)

(1-(4-(2,3-dihydro-1,4-benzoxathiin-2-ylmethoxy)-2-methylbenzoyl)-2-methyl-1H-indol-3-yl)acetic acid

TLC: Rf 0.50 (chloroform: methanol =9 : 1);
$^1$H-NMR ($CDCl_3$): δ 7.48, 7.38-6.97, 6.94-6,70, 4.72-4.62, 4.35, 4.23, 3.72, 3.28-3.15, 2.35, 2.33.

Example 6(20)

(1-(4-(1-benzothien-2-ylmethoxy)-2-methylbenzoyl)-2-methyl-1H-indol-3-yl)acetic acid

TLC: Rf 0.50 (chloroform: methanol = 9 : 1);
$^1$H-NMR ($CDCl_3$): δ 7.87-7.73, 7.48, 7.42-6.84, 5.38, 3.72, 2.34, 2.33.

Example 6(21)

(1-(2-chloro-4-(2,3-dihydro-1-benzofuran-2-ylmethoxy)benzoyl)-5-fluoro-2-methyl-1H-indol-3-yl)acetic acid

TLC: Rf 0.44 (chloroform ; methanol = 9 : 1);
$^1$H-NMR ($CDCl_3$): δ 7.43, 7.25-7.10, 7.03, 7.00-6.80, 5.25-5.13, 4.27, 4.19, 3.66, 3.43, 3.16, 2.26.

Example 6(22)

(1-(2-chloro-4-((2S)-2,3-dihydro-1-benzofuran-2-ylmethoxy)benzoyl)-5-fluoro-2-methyl-1H-indol-3-yl)acetic acid

TLC: Rf 0.44 (chloroform : methanol = 9 : 1);
$^1$H-NMR ($CDCl_3$): δ 7.43, 7.25-7.10, 7.03, 7.00-6.80, 5.25-5.13, 4.27, 4.19, 3.66, 3.43, 3.16, 2.26.

Example 6(23)

(1-(4-(1,3-benzoxazol-2-ylmethoxy)-2-methylbenzoyl)-2-methyl-1H-indol-3-yl)acetic acid

TLC: Rf 0.55 (chloroform : methanol = 9 : 1);
$^1$H-NMR ($CDCl_3$): δ 7.81-7.74, 7.62-7.55, 7.48, 7.44-7.30, 7,18, 7.07-6.90, 5.39, 3.71, 2.33, 2.32.

Example 6(24)

(1-(4-((2S)-2,3-dihydro-1-benzofuran-2-ylmethoxy)-2-methylbenzoyl)-2-methyl-1H-indol-3-yl)acetic acid

TLC: Rf 0.56 (chloroform : methanol = 9 : 1);
$^1$H-NMR ($CDCl_3$): δ 7.47, 7.32, 7.24-7.10, 7.10-6.94, 6.92-6.75, 5.24-5.12, 4.27, 4.17, 3.71, 3.41,3.16, 2.34,2.31.

Example 6(25)

(1-(4-(((2R)-1-ethyl-2,3-dihydro-1H-indol-2-yl)methoxy)-2-methylbenzoyl)-2-methyl-1H-indol-3-yl)acetic acid

TLC: Rf 0.55 (chloroform : methanol = 9 : 1);
$^1$H-NMR ($CDCl_3$): δ 7.48, 7.33, 7.22-6.96, 6.90-6.74, 6.66, 6.47, 4.26-4.04, 3.72, 3.57-3.16, 2.89, 2.35, 2.33, 1.17.

Example 6(26)

(2-methyl-1-(2-methyl-4-(((2R)-1-propyl-2,3-dihydro-1H-indol-2-yl)methoxy)benzoyl)-1H-indol-3-yl)acetic acid

TLC: Rf 0.59 (chloroform : methanol = 9 : 1);
$^1$H-NMR ($CDCl_3$): δ 7.48, 7.33, 7.22-6.96, 6.84, 6.76, 6.64, 6.45, 4.25-4.02, 3.71, 3.35-3.12, 2.90, 2.34, 2.32, 1.74-4.54, 0.95.

Example 6(27)

(1-(4-(((2R)-1-isopropyl-2,3-dihydro-1H-indol-2-yl)methoxy)-2-methylbenzoyl)-2-methyl-1H-indol-3-yl)acetic acid

TLC: Rf 0.57 (chloroform : methanol = 9 : 1);
$^1$H-NMR ($CDCl_3$): δ 7.48, 7.31, 7.22-6.96, 6.85-6.80, 6.74, 6.67, 6.58, 4.22-4.04, 3.94 -3.75, 3.72, 3.34, 2.94, 2.34, 2.32, 1.30, 1.24.

Example 6(28)

(2-methyl-1-(2-methyl-4-((7-methyl-2,3-dihydro-1-benzofuran-2-yl)methoxy)benzoyl)-1H-indol-3-yl)acetic acid

TLC: Rf0.50 (chloroform : methanol =9 : 1);
$^1$H-NMR ($CDCl_3$): δ 7.48, 7.32, 7.23-7.13, 7.10-6.92, 6.88, 6.84-6.76, 5.21-5.10, 4.28, 4.16, 3.72,3.41, 3.18, 2.34, 2.32, 2.22.

Example 6(29)

(2-methyl-1-(2-methyl-4-(2-(2-pyridinyloxy)ethoxy)benzoyl)-1H-indol-3-yl)acetic acid

TLC: Rf 0.50 (chloroform : methanol = 9 : 1);
$^1$H-NMR ($CDCl_3$): δ 8.20-8.14, 7.64-7.56, 7.48, 7.33, 7.23-7.15, 7.10-6.96, 6.94-6.86, 6.84-6.78, 4.72, 4.40, 3.74, 2.35, 2.32.

Example 6(30)

(2-methyl-1-(2-methyl-4-(2-((2-methyl-3-pyridinyl)oxy)ethoxy)benzoyl)-1H-indol-3-yl)acetic acid

TLC: Rf 0.36 (chloroform : methanol =9 : 1);
$^1$H-NMR ($CDCl_3$): δ 8.14, 7.51, 7.35, 7.24-7.04, 6.88, 6.81, 4.48-4.34, 3.73, 2.48, 2.33.

Example 6(31)

(1-(4-(2-((2-chloro-3-pyridinyl)oxy)ethoxy)-2-methylbenzoyl)-2-methyl-1H-indol-3-yl)acetic acid

TLC: Rf 0.39 (chloroform : methanol = 9 : 1);
$^1$H-NMR ($CDCl_3$): δ 8.06, 7.49, 7.38-6.98, 6.90, 6.82, 4.52-4.40, 3.73, 2.34, 2.33.

Example 6(32)

(1-(4-(2-((5-chloro-3-pyridinyl)oxy)ethoxy)-2-methylbenzoyl)-2-methyl-1H-indol-3-yl)acetic acid

TLC: Rf 0.36 (chloroform : methanol = 9 : 1);
$^1$H-NMR ($CDCl_3$): δ 8.29, 8.24, 7.49, 7.40-6.98, 6.88, 6.79, 4.46-4.38, 3.73, 2.35, 2.33.

Example 6(33)

(1-(4-(2-((6-chloro-2-pyridinyl)oxy)ethoxy)-2-methylbenzoyl)-2-methyl-1H-indol-3-yl)acetic acid

TLC: Rf 0.42 (chloroform: methanol =9 : 1);
$^1$H-NMR ($CDCl_3$): δ 7.55, 7.48, 7.33, 7.10-6.92, 6,89, 6.81, 6.73, 4.71, 4,38, 3.72, 2.34, 2.31.

Example 6(34)

(1-(4-(2-((5-chloro-2-pyridinyl)oxy)ethoxy)-2-methylbenzoyl)-2-methyl-1H-indol-3-yl)acetic acid

TLC: Rf 0.44 (chloroform : methanol =9 : 1);
$^1$H-NMR ($CDCl_3$): δ 8.11, 7.55, 7.48, 7.33, 7.25-6.96, 6.88, 6.84-6.75, 4.68, 4.37, 3.72, 2.35, 2.32.

Example 6(35)

(2-methyl-1-(2-methyl-4-(2-((6-methyl-2-pyridinyl)oxy)ethoxy)benzoyl)-1H-indol-3-yl)acetic acid

TLC: Rf 0.40 (chloroform : methanol = 9 : 1);
$^1$H-NMR ($CDCl_3$): δ 7.48, 7.34-7.12, 7.09-6.95, 6.78, 6.69, 6.51, 6.08, 4.50-4.44, 4.44-4.35, 3.71,2.55,2.31,2.28.

Example 6(36)

(1-(4-(2-(2-butoxyethoxy)ethoxy)benzoyl)-2,5-dimethyl-1H-indol-3-yl)acetic acid

TLC: Rf 0.49 (chloroform : methanol = 9 : 1);
$^1$H-NMR ($CDCl_3$): δ 7.69, 7.30-7.25, 6.97, 6.88-6.78, 4.25-4.15, 3.95-3.88, 3.76-3.70, 3.65-3.59, 3.48, 2.42, 2.40, 1,65-1,50, 1.45-1.30, 0.91.

Example 6(37)

(1-(4-(((2R)-1-ethyl-2,3-dihydro-1H-indol-2-yl)methoxy)benzoyl)-2,5-dimethyl-1H-indol-3-yl)acetic acid

TLC: Rf 0.60 (chloroform : methanol = 9 : 1);
$^1$H-NMR ($CDCl_3$): δ 7.76-7.68, 7.28, 7.12-6.94, 6.87-6.82, 6.66, 6.47, 4.30-4.06, 3.72, 3.46-3.23, 2.90, 2.42, 2.40, 1.16.

Example 6(38)

(1-(4-(((2R)-1-ethyl-2,3-dihydro-1H-indol-2-yl)methoxy)benzoyl)-2-methyl-1H-indol-3-yl)acetic acid

TLC: Rf 0.58 (chloroform : methanol = 9 : 1);
$^1$H-NMR ($CDCl_3$): δ 7.73, 7.51, 7.17, 7.13-6.95, 6.66, 6.47, 4.30-4.06, 3.75, 3.47-3.22, 2.90, 2.43, 1.17.

Example 6(39)

(1-(2-chloro-4-(((2R)-1-ethyl-2)-dihydro-1H-indol-2-yl)methoxy)benzoyl)-5-fluoro-2-methyl-1H-indol-3-yl)acetic acid

TLC: Rf 0.58 (chloroform : methanol =9 : 1);
$^1$H-NMR ($CDCl_3$): δ 7.44, 7.22, 7.17-7.00, 6.93, 6.84, 6.67, 6.48, 4.25 -4.06, 3.67, 3.47-3.22, 2.89, 2.27, 1.17.

Example 6(40)

(1-(2-chloro-4-(((2R)-1-ethyl-2,3-dihydro-1H-indol-2-yl)methoxy)benzoyl)-2-methyl-1H-indol-3-yl)acetic acid

TLC: Rf 0.60 (chloroform : methanol = 9 : 1),
$^1$H-NMR ($CDCl_3$): δ 7.51-7.41, 7.25-7.02, 6.92, 6.67, 6.48, 4.25-4.06, 3.71, 3.47-3.22, 2.89, 2.32, 1.17.

Example 6(41)

(1-(2-chloro-4-(((2S)-6-fluoro-4-methyl-3,4-dihydro-2H-1,4-benzoxazin-2-yl)methoxy)benzoyl)-5-fluoro-2-methyl-1H-indol-3-yl)acetic acid

TLC: Rf 0.60 (chloroform : methanol = 9 : 1);
$^1$H-NMR ($CDCl_3$): δ 2.25, 2.91, 3.30, 3.40, 3.64, 4.17, 4.27, 4.61, 6.38, 6.74, 6.83, 6.95, 7.04, 7.12, 7.22, 7.43.

Example 6(42)

(1-(4-(((2S)-6-fluoro-4-methyl-3,4-dihydro-2H-1,4-benzoxazin-2-yl)methoxy)benzoyl)-2,5-dimethyl-1H-indol-3-yl) acetic acid

TLC: Rf 0.60 (chloroform : methanol = 9 : 1);
$^1$H-NMR ($CDCl_3$): δ 2.40, 2.41, 2.91, 3.30, 3.41, 3.71, 4.18, 4.29, 4.61, 6.37, 6.74, 6.83, 6.98, 7.27, 7.71.

Example 6(43)

(1-((4-(((2S)-4-methyl-3,4-dihydro-2H-1,4-benzoxazin-2-yl)methoxy)phenyl)sulfonyl)-1H-indol-3-yl)acetic acid

TLC: Rf 0.55 (methylene chloride : methanol = 9 : 1);
$^1$H-NMR ($CDCl_3$): δ 7.97, 7.86-7.74, 7.58, 7.50, 7.38-7.22, 6.98-6.63, 4.64-4.53, 4.19, 4.10, 3.73, 3.31, 3.17, 2.85.

Example 6(44)

(2-methyl-1-((4-(((2S)-4-methyl-3,4-dihydro-2H-1,4-benzoxazin-2-yl)methoxy)phenyl)sulfonyl)-1H-indol-3-yl)acetic acid

TLC: Rf 0.55 (methylene chloride : methanol = 9 : 1);
$^1$H-NMR ($CDCl_3$) : δ 8.17, 7.69, 7.42, 7.32-7.19, 6.92-6.75, 6.72-6.64, 4.62-4.34, 4.19, 4.09, 3.62, 3.31, 3.17, 2.86, 2.56.

Example 6(45)

(1-(4-(((2S)-4,6-dimethyl-3,4-dihydro-2H-1,4-benzoxazin-2-yl)methoxy)benzoyl)-2-methyl-1H-indol-3-yl)acetic acid

TLC: Rf 0.27 (hexane : ethyl acetate = 1 : 1);
$^1$H-NMR ($CDCl_3$): δ 7.73, 7.51, 7.17, 7.10-6.92, 6.73, 6.55-6.45, 4.70-4.60, 4.30, 4.20, 3.75, 3.39, 3.26, 2.90, 2.43, 2.28.

Reference Example 12

(2S)-4-methyl-3,4-dihydro-2H-1,4-benzoxazin-2-carbaldehyde

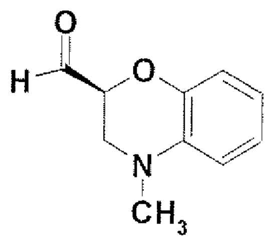

To a solution of the compound prepared in Reference Example 4 (500 mg) in methylene chloride (5 mL) were added triethylamine (1.2 mL) and dimethylsulfoxide (5 mL). To the reaction mixture was added sulfur trioxide pyridine complex (1.4 g). The mixture was stirred at room temperature for 2 hours. To the reaction mixture was added water. The mixture was extracted with ethyl acetate. The organic layer was washed with water and a saturated aqueous solution of sodium chloride, dried over anhydrous sodium sulfate. The solvent was removed and the obtained residue was purified by column chromatography on silica gel (hexane ; ethyl acetate = 4 : 1 → 1 : 1) to give the title compound (220 mg) having the following physical data.
TLC: Rf 0.51 (ethyl acetate : hexane = 1 : 1).

Reference Example 13

benzyl (2-methyl-1-(4-nitrobenzoyl)-1H-indol-3-yl)acetate

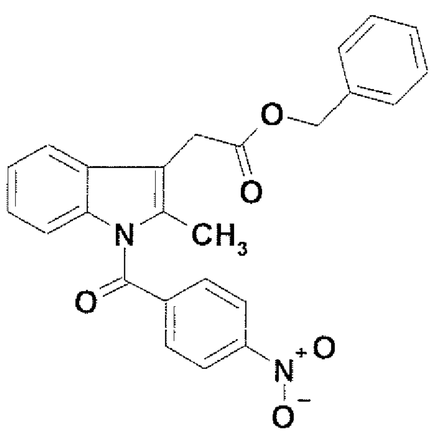

To a solution of the compound prepared in Reference Example 9 (1.1 g) in a mixture of acetonitrile(4 mL) and ethyl acetate (4 mL) were added triethylamine (3.4 mL), 4-dimethylaminopyridine (147 mg) and 4-nitrobenzoylchloride (1.1 g). The mixture was stirred at 40°C for 3 hours. To the reaction mixture were added ethyl acetate and 2N hydrochloric acid. The mixture was extracted with ethyl acetate. The organic layer was washed with water and a saturated

aqueous solution of sodium chloride subsequently, and dried over anhydrous sodium sulfate. The solvent was removed to give the title compound (1.7 g) having the following physical data.
TLC: Rf 0.53 (ethyl acetate : hexane = 3 : 7).

Reference Example 14

benzyl (1-(4-aminobenzoyl)-2-methyl-1H-indol-3-yl)acetate

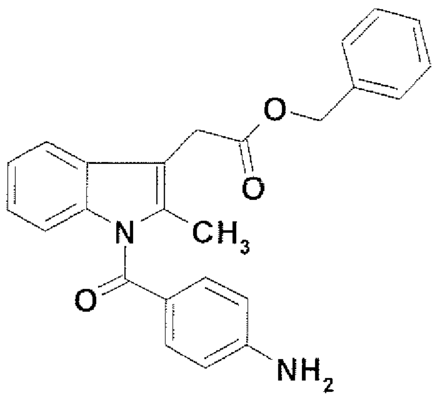

To a solution of the compound prepared in Reference Example 13 (1.7 g) in acetic acid (20 mL) was added iron powder (1.1 g). The mixture was stirred at 60°C for 3 hours. The reaction mixture was diluted with ethyl acetate and then filtered through cellite (trademark). The filtrate was concentrated. The obtained residue was diluted with ethyl acetate, washed with a saturated aqueous solution of sodium bicarbonate, water and a saturated aqueous solution of sodium chloride subsequently, and then dried over anhydrous sodium sulfate. The solvent was removed. The obtained residue was purified by column chromatography on silica gel (hexane : ethyl acetate = 7 : 3) and washed with ethyl acetate - hexane to give the title compound (1.0 g) having the following physical data.
TLC: Rf 0.18 (ethyl acetate : hexane = 3 : 7);
$^1$H-NMR ($CDCl_3$): δ 7.59, 7.50, 7.38-7.26, 7.17-7.00, 6.66, 5.14, 4,25-4.15, 3.76, 2.42.

Example 7

benzyl (2-methyl-1-(4-((((2R)-4-methyl-3,4-dihydro-2H-1,4-benzoxazin-2-yl)methyl)amino)benzoyl)-1H-indol-3-yl) acetate

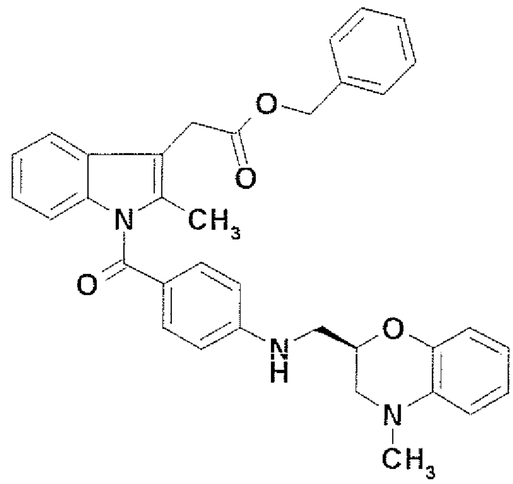

The compound prepared in Reference Example 14 (494 mg) and the compound prepared in Reference Example 12 (220 mg) were dissolved in a mixture of methylene chloride (6 mL) and acetic acid (1 mL). The mixture was stirred at room temperature for 20 minutes. To the reaction mixture was added sodium triacetoxyborohydride (509 mg). The mixture was stirred at room temperature for 30 minutes. To the reaction mixture were added ethyl acetate and water. The mixture was extracted with ethyl acetate. The organic layer was washed with a saturated aqueous solution of sodium bicarbonate, water and a saturated aqueous solution of sodium chloride subsequently, and dried over anhydrous sodium sulfate. The solvent was removed. The obtained residue was purified by column chromatography on silica gel (hexane : ethyl acetate = 4 : 1 → 7 : 3) to give the compound of the present invention (270 mg) having the following physical data.
TLC: Rf 0.63 (ethyl acetate : hexane = 1 : 1);
$^1$H-NMR ($CDCl_3$): δ 7.62, 7.50, 7.38-7.26, 7.17-7.00, 6.93-6.81, 6.71, 6.63, 5.14, 4.78-4.66, 4.56-4.46, 3.77, 3.60-3.44, 3.30, 3.18, 2.90, 2.42.

Example 8

(2-methyl-1-(4-((((2R)-4-methyl-3,4-dihydro-2H-1,4-benzoxazin-2-yl)methyl)amino)benzoyl)-1H-indol-3-yl)acetic acid

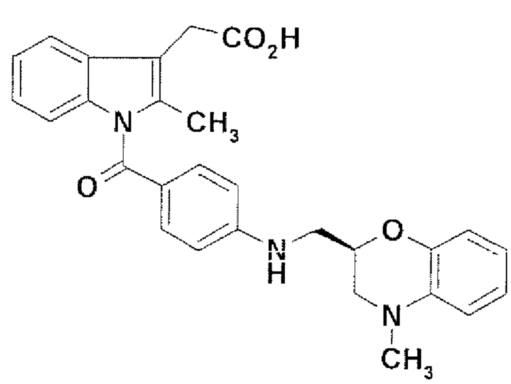

Using the compound prepared in Example 7 instead of the compound prepared in Example 1, the compound of the present invention having the following physical data was obtained by the same procedure of Example 2.
TLC: Rf 0.50 (chloroform : methanol = 9 : 1);
$^1$H-NMR ($CDCl_3$): δ 7.64, 7.51, 7.20-7.10, 7.10-7.00, 6.92-6.78, 6.74-6.58, 4.80-4.66, 4.56-4.43, 3.76, 3.56-3.44, 3.30, 3.18, 2.90, 2.44,

Example 9

(1-(2-chloro-4-((((2R)-4-methyl-3,4-dihydro-2H-1,4-benzoxazin-2-yl)methyl)amino)benzoyl)-2-methyl-1H-indol-3-yl) acetic acid

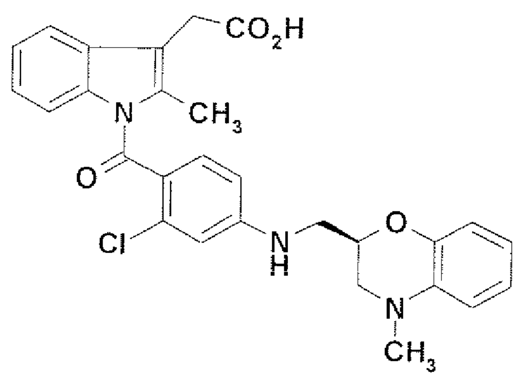

Using a corresponding acid chloride instead of 4-nitrobenzoyl chloride, the compound of the present invention having the following physical data was obtained by the same procedures as a series of reactions of Reference Example 13→Reference Example 14→Example 7→Example 8.
TLC: Rf 0.50 (chloroform : methanol = 9 : 1);
$^1$H-NMR ($CDCl_3$): δ 7.48, 7.34-7.04, 6.93-6.79, 6.75-6.66, 6.56, 4.70-4.56, 4.56-4.45, 3.72, 3.55-3.36, 3.30, 3.17, 2.90, 2.36.

Example 10

benzyl (2-methyl-1-(4-(methyl(((2R)-4-methyl-3,4-dihydro-2H-1,4-benzoxazin-2-yl)methyl)amino)benzoyl)-1H-indol-3-yl)acetate

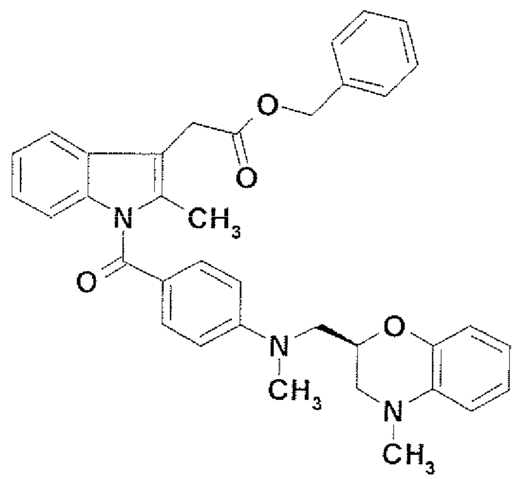

To a solution of the compound prepared in Example 7 (100 mg) in dimethylformamide (3 mL) was added sodium hydride (8 mg). The mixture was stirred at 0°C for 20 minutes. To the reaction mixture was added methyl iodide (0.012 mL). The mixture was stirred at room temperature for 1 hour. To the reaction mixture were added water and ethyl acetate, and then the mixture was extracted with ethyl acetate. The organic layer was washed with water and a saturated aqueous solution of sodium chloride, and dried over anhydrous sodium sulfate. The solvent was removed and the obtained residue was purified by column chromatography on silica gel (hexane : ethyl acetate = 4 : 1 → 7 : 3) to give the compound of the present invention (10 mg).
TLC : Rf 0.35 (hexane : ethyl acetate = 3 : 2).

Example 11

(2-methyl-1-(4-(methyl(((2R)-4-methyl-3,4-dihydro-2H-1,4-benzoxazin-2-yl)methyl)amino)benzoyl)-1H-indol-3-yl) acetic acid

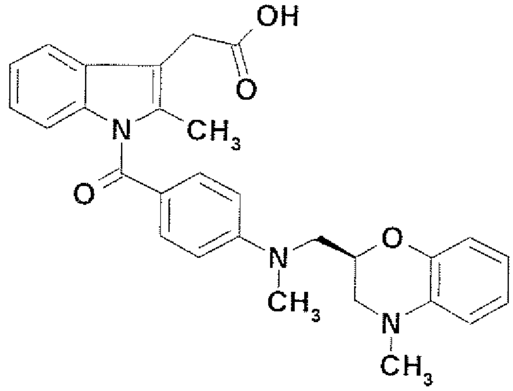

Using the compound prepared in Example 10 instead of the compound prepared in Example 1, the compound of the present invention having the following physical data was obtained by the same procedure of Example 2.

TLC: Rf 0.63 (ethyl acetate : hexane = 1 : 1);
$^1$H-NMR ($CDCl_3$): δ 7.71-7.56, 7.51, 7.19-7.00, 6.92-6.60, 4.60-4.52, 3.75, 3.80-3.62, 3.28, 3.16, 3.06, 2.87, 2.43.

Example 12

[(2S)-6-fluoro-4-methyl-3,4-dihydro-2H-1,4-benzoxazin-2-yl]methyl 4-nitrobenzenesulfonate

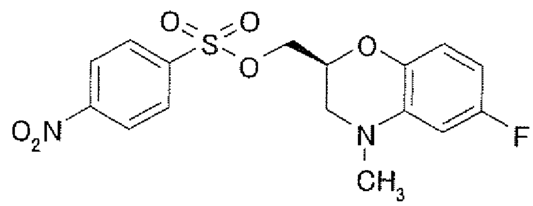

[(2S)-6-fluoro-4-methyl-3,4-dihydro-2H-1,4-benzoxazin-2-yl]methanol (1g; it was prepared by the same procedure as a series of reactions of Reference Example 1→Reference Example 2→Reference Example 3→Reference Example 4, using 2,5-difluoroaniline instead of 2-fluoroaniline.) and triethylamine (1.8 mL) were dissolved in methylene chloride (10 mL) under an atmosphere of argon. To the solution was added 4-nitrobenzenesulfonyl chloride (1.1 g) under ice cooling. The mixture was stirred at room temperature for 1 hour. The reaction mixture was poured into water and then extracted with ethyl acetate. The organic layer was washed with 1N hydrochloric acid, water and a saturated aqueous solution of sodium chloride, dried over anhydrous magnesium sulfate, and concentrated. The obtained solid was washed with the mixed solvent of ethyl acetate and hexane to give the title compound (1.5 g) having the following physical data.
TLC: Rf 0.46 (ethyl acetate : hexane = 1 : 1);
$^1$H-NMR ($CDCl_3$) : δ 8.40, 8.12, 6.51, 6.23-6.40, 4.40-4.49, 4.30, 4.29, 3.27, 3.10-3.18, 2.84.

Example 13

[(2R)-6-fluoro-4-methyl-3,4-dihydro-2H-1,4-benzoxazin-2-yl]acetonitrile

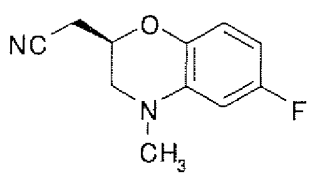

The compound prepared in Example 12 (1.5 g) and potassium cyanide (766 mg) were dissolved in dimethylsulfoxide (20 mL) under an atmosphere of argon and the mixture was stirred at room temperature for 2 hours. The mixture was poured into water and then extracted with ethyl acetate. The organic layer was washed with water and a saturated aqueous solution of sodium chloride, dried over anhydrous magnesium sulfate, and concentrated. The residue was purified by column chromatography on silica gel (ethyl acetate : hexane = 1 : 2) to give the title compound (357 mg) having the following physical data.
TLC: Rf 0.36 (ethyl acetate : hexane = 1 : 1);
$^1$H-NMR ($CDCl_3$): δ 6.72, 6.29-6.46, 4.45-4.58, 3.37, 3.20, 2.90, 2.66-2.86.

Example 14

[(2R)-6-fluoro-4-methyl-3,4-dihydro-2H-1,4-benzoxazin-2-yl]acetoaldehyde

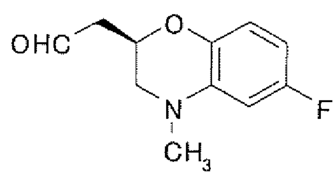

The compound prepared in Example 13 (350 mg) was dissolved in tetrahydrofuran (5 mL) under an atmosphere of argon. To the mixture was added dropwise diisobutylaluminum hydride (0.95M in hexane, 1.97 mL) at -78°C, and the mixture was stirred for 2 hours. To the reaction mixture were added methanol and water at 0°C, and then the mixture was stirred at room temperature for 30 minutes. To the reaction mixture was added 1N hydrochloric acid, the mixture was extracted with ethyl acetate. The organic layer was washed with water and a saturated aqueous solution of sodium chloride and then concentrated to give the mixture of the compound prepared in Example 13 and the title compound (1:2, 290 mg) having the following physical data.
TLC: Rf 0.36 (ethyl acetate : hexane = 1 : 1);
$^1$H-NMR ($CDCl_3$): δ 9.87, 6.67, 6.24-6.45, 4.62-4.78, 3.31, 3.09, 2.87, 2.65-2.88.

Example 15

*tert*-butyl 4-{2-[(2R)-6-fluoro-4-methyl-3,4-dihydro-2H-1,4-benzoxazin-2-yl]ethyl}piperazin-1-carboxylate

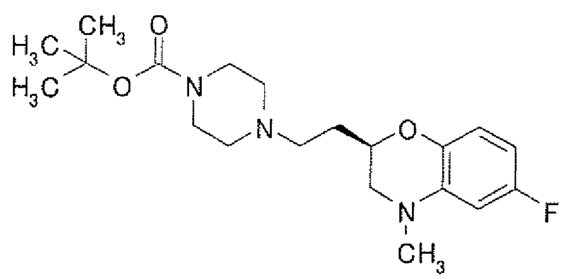

A mixture of the compound prepared in Example 13 and the compound prepared in Example 14 (1 : 2, 290 mg) and *tert*-butyl piperazin-1-carboxylate (172 mg) were dissolved in N,N-dimethylformamide (3 mL) under an atmosphere of argon. To the mixture was added sodium triacetoxyborohydride (391 mg) and the mixture was stirred at room temperature for 3 hours. The mixture was poured into water and then extracted with ethyl acetate. The organic layer was washed with water and a saturated aqueous solution of sodium chloride, dried over an anhydrous magnesium sulfate, and then concentrated. The residue was purified by column chromatography on silica gel (ethyl acetate : hexane = 1 : 2 → methanol : chloroform = 1 : 19) to give the title compound (256 mg) having the following physical data.
TLC: Rf 0.31 (methanol : chloroform = 1 : 19);
$^1$H-NMR ($CDCl_3$): δ 6.66, 6.24-6.40, 4.14-4.25, 3.31-3.57, 3.22, 3.05, 2.86, 2.47-2.66, 2.41, 1.66-1.94, 1.46.

Example 16

(2R)-6-fluoro-4-methyl-2-(2-piperazin-1-ylethyl)-3,4-dihydro-2H-1,4-benzoxazine

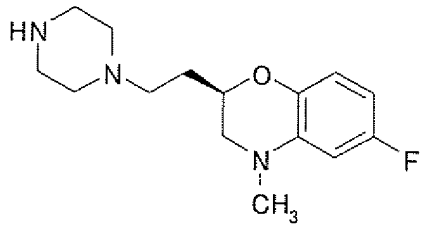

The compound prepared in Example 15 (256 mg) was dissolved in ethyl acetate (2 mL). To the mixture was added 4N hydrogen chloride in ethyl acetate (2 ml), and the mixture was stirred at room temperature for 6 hours. The reaction mixture was stirred at 40°C for 2 hours. To the reaction mixture was added a saturated aqueous solution of sodium bicarbonate, and then the mixture was extracted with chloroform. The organic layer was dried over an anhydrous magnesium sulfate, and concentrated to give the title compound (144 mg) having the following physical data.
$^1$H-NMR ($CDCl_3$): δ 6.66, 6.24-6.40, 4.14-4.24, 3.22, 3.16, 3.05, 2.93-3.01, 2.86, 2.44-2.68, 1.66-1.95.

Example 17

benzyl {1-[(4-{2-[(2R)-6-fluoro-4-methyl-3,4-dihydro-2H-1,4-benzoxazin-2-yl]ethyl}piperazin-1-yl)carbonyl]-2,5-dimethyl-1H-indol-3-yl}acetate

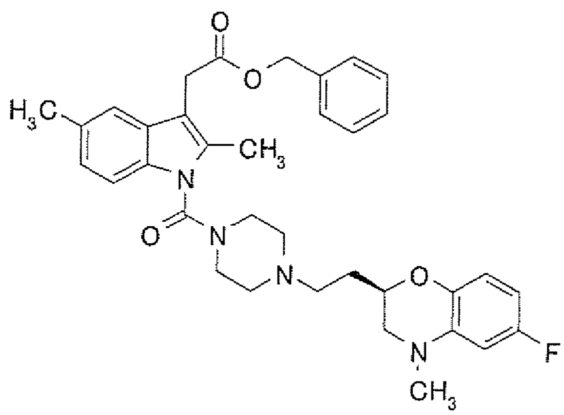

Benzyl (2,5-dimethyl-1H-indol-3-yl)acetate (167 mg; it was prepared by the same procedure of Reference Example 9, using 2-(2,5-dimethylindol-3-yl)acetic acid instead of 2-(2-methylindol-3-yl)acetic acid.) and N,N'-carbonyldiimidazole (97 mg) were dissolved in acetonitrile (2 mL) under an atmosphere of argon. The reaction mixture was stirred at 60°C for 20 hours. To the reaction mixture was added a solution of the compound prepared in Example 16 (144 mg) in acetonitrile (2 mL), and the mixture was stirred at 100°C for 10 hours. The reaction mixture was cooled to at room temperature, diluted with ethyl acetate, washed with water and a saturated aqueous solution of sodium chloride, dried over an anhydrous magnesium sulfate. The organic layer was concentrated, and the obtained residue was purified by column chromatography on silica gel (ethyl acetate) to give the title compound (87 mg) having the following physical data.
TLC: Rf 0.23 (ethyl acetate);
$^1$H-NMR ($CDCl_3$): δ 7.23-7.39, 7.16, 7.01, 6.65, 6.25-6.40, 5.11, 4.14-4.26, 3.70, 3.36-3.65, 3.21, 3.05, 2.86, 2.49-2.66, 2.34-2.49, 2.40, 1.65-1.94.

Example 18

{1-[(4-{2-[(2R)-6-fluoro-4-methyl-3,4-dihydro-2H-1,4-benzoxazin-2-yl]ethyl}-1-piperazinyl)carbonyl]-2,5-dimethyl-1H-indol-3-yl} acetic acid

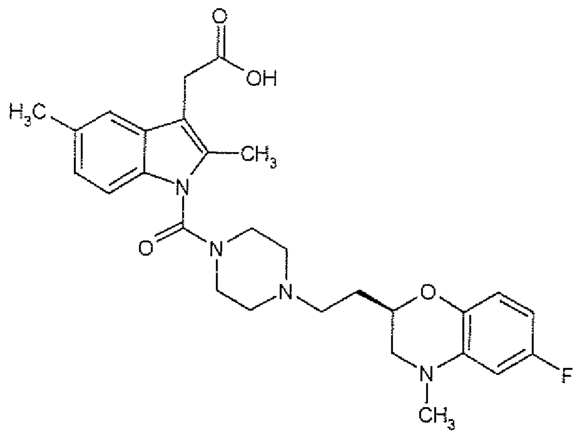

Using the compound prepared in Example 17 instead of the compound prepared in Example 1, the title compound having the following physical data was obtained by the same procedure of Example 2.
TLC: Rf 0.40 (methanol chloroform = 1:9);
$^1$H-NMR ($CDCl_3$): δ 7.30, 7.10, 6.93, 6.64, 6.32, 4.18, 3.65, 3.63, 3.20, 3.03, 2.85, 2.74, 2.40, 2.38, 1.95.

Reference Example 15

4-(chlorosulfonyl)phenyl acetate

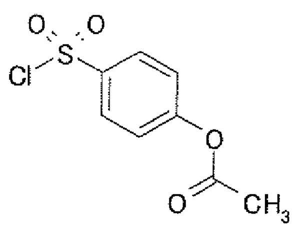

To a solution of 4-hydroxybenzenesulfonic acid (6 g) in pyridine (20 mL) was added acetic anhydride (20 mL) at room temperature, and the reaction mixture was stirred at room temperature overnight. An appeared solid was collected by suction filtration and then washed with hexane. To a solution of the solid in N,N-dimethylformamide (40 mL) was added thionyl chloride (5 mL) at 0°C, and the reaction mixture was stirred at 0°C for 1 hour. To the reaction mixture were added ice-water and ethyl acetate. The mixture was extracted with ethyl acetate (twice). The organic layer was washed with water and a saturated aqueous solution of sodium chloride, dried over anhydrous sodium sulfate, and concentrated *in vacuo* to give the title compound (5.8 g) having the following physical data.
TLC: Rf 0.55 (ethyl acetate : hexane =3:7);
$^1$H-NMR ($CDCl_3$) : δ 8.07, 7.3 7, 2.3 6.

Reference Example 16

benzyl {1-[(4-hydroxyphenyl)sulfonyl]-1H-indol-3-yl}acetate

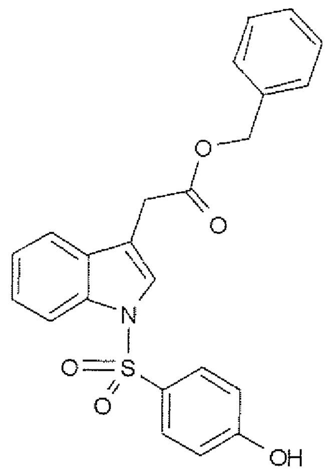

To a solution of the compound prepared in Reference Example 15 (650 mg) and benzyl 1H-indol-3-ylacetate (478 mg; it was prepared by the same procedure of Reference Example 9, using 2-(indol-3-yl)acetic acid instead of 2-(2-methylindol-3-yl)acetic acid) in methylene chloride (4 mL) were added 20N aqueous solution of sodium hydroxide (0.46 mL) and tetrabutyammonium chloride (51 mg), and the mixture was stirred at room temperature for 1 hour. To the reaction mixture were added ethyl acetate and water. The mixture was extracted with ethyl acetate (twice). The organic layer was washed with water and a saturated aqueous solution of sodium chloride, dried over anhydrous sodium sulfate, and then concentrated. The obtained residue was dissolved in methylene chloride (5 mL). To the solution was added piperidine (1.5 mL), and the mixture was stirred at room temperature overnight. To the reaction mixture was added 2N hydrochloric acid, and the mixture was extracted with ethyl acetate (twice). The organic layer was washed with water and a saturated aqueous solution of sodium chloride, dried over anhydrous sodium sulfate, and then concentrated *in vacuo.* The obtained residue was purified by column chromatography on silica gel (hexane : entyl acetate = 9 : 1 → 4 : 1 → 7 : 3) to give the title compound (300 mg) having the following physical data.
TLC: Rf 0.14 (ethyl acetate : hexane = 3 : 7);
$^1$H-NMR ($CDCl_3$): δ 7.96, 7.72, 7.55, 7.46, 7.38-7.27, 7.22, 6.72, 5.58, 5.15, 3.74.

Example 19

benzyl {1-[(4-{[(2R)-1-ethyl-2,3-dihydro-1H-indol-2-yl]methoxy}phenyl)sulfonyl]-1H-indol-3-yl}acetate

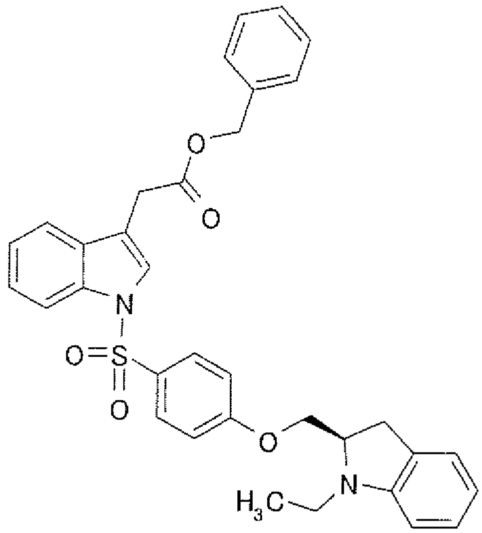

Using the compound prepared in Reference Example 16 instead of the compound prepared in Reference Example 11, and using [(2R)-1-ethyl-2,3-dihydro-1H-indol-2-yl]methanol instead of 2-(2-butoxyethoxy)ethanol, the title compound having the following data was obtained by the same procedure of Example 4.
TLC: Rf 0.53 (ethyl acetate : hexane = 3 : 7).

Example 20

{1-[(4-{[(2R)-1-ethyl-2,3-dihydro-1H-indol-2-yl]methoxy}phenyl)sulfonyl]-1H-indol-3-yl}acetic acid

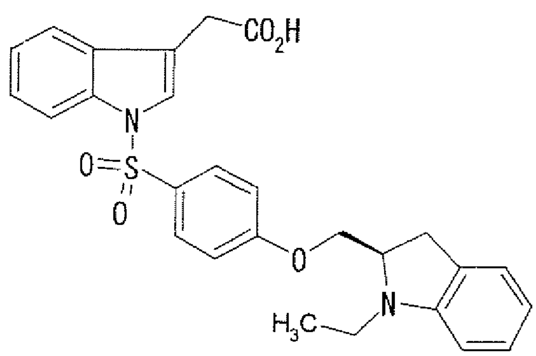

Using the compound prepared in Example 19 instead of Example 1, the title compound having the following data was obtained by the same procedure of Example 2.
TLC: Rf 0.50 (chloroform : methanol = 9 : 1);
$^1$H-NMR ($CDCl_3$): δ 7.97, 7.82, 7.59, 7.50, 7.37-7.21, 7.10-7.00, 6.89, 6.62, 6.43, 4.26-3.96, 3.74, 3.40-3.10, 2.79, 1.10.

Example 21(1)-Example 21(5)

Using 2-(2-methylindol-3-yl)acetic acid or a corresponding carboxylic acid derivatives, and acid halide derivatives, which is corresponding the compound prepared in Reference Example 8, the following compounds were obtained by the same procedures as a series of reactions of Reference Example 9 → Example 1 → Example 2.

Example 21(1)

[2-methyl-1-(2,3,5,6-tetrafluoro-4-{[(2S)-4-methyl-3,4-dihydro-2H-1,4-benzoxazin-2-yl]methoxy}benzoyl)-1H-indol-3-yl]acetic acid

TLC: Rf 0.81 (chloroform : methanol = 9 : 1);
$^1$H-NMR ($CDCl_3$): δ 7.41, 7.19, 6.88, 6.77, 6.69, 4.66, 4.52, 3.66, 3.38, 3.30, 2.91, 2.33.

Example 21(2)

[1-(2-fluoro-5-methyl-4-{[(2S)-4-methyl-3,4-dihydro-2H-1,4-benzoxazin-2-y]methoxy} benzoyl)-2-methyl-1H-indol-3-yl]acetic acid

TLC: Rf 0.46 (chloroform : methanol : acetic acid = 9 : 1 : 0.1);
$^1$H-NMR ($CDCl_3$): δ 7.49, 7.40, 7.16, 6.87, 6.70, 6.62, 4.70, 4.23, 3.73, 3.36, 2.92, 2.40, 2.23.

Example 21(3)

[5-fluoro-1-(2-fluoro-5-methyl-4-{[(2S)-4-methyl-3,4-dihydro-2H-1,4-benzoxazin-2-yl]methoxy}benzoyl)-2-methyl-1H-indol-3-yl]acetic acid

TLC: Rf 0.56 (chloroform : methanol : acetic acid = 9 : 1 : 0.1);
$^1$H-NMR ($CDCl_3$): δ 7.38, 7.15, 6.85, 6.70, 6.62, 4.70, 4.23, 3.67, 3.36, 2.92, 2.35, 2.23.

Example 21(4)

[1-(5-chloro-2-fluoro-4-{[(2S)-4-methyl-3,4-dihydro-2H-1,4-benzoxazin-2-yl]methoxy}benzoyl)-2-methyl-1H-indol-3-yl]acetic acid

TLC: Rf 0.61 (chloroform ; methanol : acetic acid = 9 : 1 : 0.1);
$^1$H-NMR ($CDCl_3$): δ 7.66, 7.50, 7.17, 6.81, 4.75, 4.30, 3.73, 3.39, 2.93, 2,40.

Example 21(5)

[1-(5-chloro-2-fluoro-4-{[(2S)-4-methyl-3,4-dihydro-2H-1,4-benzoxazin-2-yl]methoxy}benzoyl)-5-fluoro-2-methyl-1H-indol-3-yl]acetic acid

TLC: Rf 0.47 (chloroform : methanol : acetic acid = 9 : 1 : 0.1);
$^1$H-NMR ($CDCl_3$): δ 7.65, 7.18, 6.82, 4.74, 4.30, 3.67, 3.39, 2.92, 2.35.

Example 22(1)-Example 22(6)

Using the compound prepared in Reference Example 9 or a corresponding indole derivatives, and alcohol derivatives instead of 2-(2-butoxyethoxy)ethanol, the compounds of the present invention were obtained by the same procedures as a series of reactions of Reference Example 10→Reference Example 11→Example 4→Example 2.

Example 22(1)

[1-(4-{[(2R)-1-ethyl-2,3-dihydro-1H-indol-2-yl]methoxy}-2,5-dimethylbenzoyl)-2-methyl-1H-indol-3-yl]acetic acid

TLC: Rf 0.50 (ethyl acetate : hexane : acetic acid = 5 : 5 : 1);
$^1$H-NMR ($CDCl_3$): δ 7.48, 7.18, 7.05, 6.72, 6.65, 6.47, 4.17, 3.72, 3.38, 2.89, 2.34, 2,27, 2.14, 1.18.

Example 22(2)

[1-(4-{[(2R)-1-ethyl-2,3-dihydro-1H-indol-2-yl]methoxy}-2,5-dimethylbenzoyl)-5-fluoro-2-methyl-1H-indol-3-yl]acetic acid

TLC: Rf 0.50 (ethyl acetate : hexane : acetic acid = 5 : 5 : 1);
$^1$NMR($CDCl_3$): δ 7.07, 6.78, 6.72, 6.66, 6.47, 4.16, 3.67, 3.38, 2.89, 2.30, 2.26, 2.14, 1.18.

Example 22(3)

[1-(4-{[(2R)-1-ethyl-2,3-dihydro-1H-indol-2-yl]methoxy}-3-methylbenzoyl)-2-methyl-1H-indol-3-yl]acetic acid

TLC: Rf 0.41 (chloroform: methanol : acetic acid = 9 : 1 : 0.1);
$^1$H-NMR ($CDCl_3$) : δ 7,56, 7,06, 6.65, 4.92, 4.20, 3.75, 3.40, 2.96, 2.42, 2.23, 1.16.

Example 22(4)

[1-(4-{[(2R)-1-ethyl-2,3-dihydro-1H-indol-2-yl]methoxy}-3-methylbenzoyl)-5-fluoro-2-methyl-1H-indol-3-yl]acetic acid

TLC: Rf 0.52 (chloroform : methanol : acetic acid = 9 : 1 : 0.1);
$^1$H-NMR ($CDCl_3$): δ 7.55, 7.12, 6.96, 6.68, 4.92, 4.25, 3.67, 3,40, 2.96, 2.37, 2.22, 1.16.

Example 22(5)

[1-(4-{[(2R)-1-ethyl-2,3-dihydro-1H-indol-2-yl]methoxy}-2,3-dimethylbenzoyl)-2-methyl-1H-indol-3-yl]acetic acid

TLC: Rf 0.56 (chloroform : methanol : acetic acid =9 : 1 : 0.1);
$^1$H-NMR($CDCl_3$): δ 7.47, 7.10, 6.66, 4.15, 3.70, 3.35, 2.90, 2.31, 2.24, 2.19, 1.16.

Example 22(6)

[1-(4-{[(2R)-1-ethyl-2,3-dihydro-1H-indol-2-yl]methoxy}-2,3-dimethylbenzoyl)-5-fluoro-2-methyl-1H-indol-3-yl]acetic acid

TLC: Rf 0.43 (chloroform methanol : acetic acid = 9 : 1 : 0.1);
$^1$H-NMR ($CDCl_3$): δ 7.17, 6.74, 6.47, 4.15, 3.66, 3.39, 2.90, 2.22, 1.16.

Formulation Example I

The following components were admixed in a conventional method and punched out to obtain 100 tablet of a diameter of 6 mm, thickness 2 mm, 100 mg in weight, each containing 50 mg of the active ingredient.

| | |
|---|---|
| (2-methyl-1-((6-(((2S)-4-methyl-3,4-dihydro-2H-1,4-benzoxazin-2-yl)methoxy)-3-pyridinyl)carbonyl)-1H-indol-3-yl)acetic acid | 5.0g |
| Carboxymethyl cellulose calcium (disintegrating agent) | 0.2 g |
| Magnesium stearate (lubricant) | 0.1 g |
| Microcrystalline cellulose | 4.7 g |

Formulation Example 2

The following components were admixed in a conventional method, and the solution was filtered for dust removal in a conventional method, sterilized by heating or filtration, placed at 5 ml into ampoules and freeze-dried in a conventional method to thereby obtain 100 ampoules each containing 20 mg of the active ingredient.

| | |
|---|---|
| (2-methyl-1-((6-(((2S)-4-methyl-3,4-dihydro-2H-1,4-benzoxazin-2-yl)methoxy)-3-pyridinyl)carbonyl)-1H-indol-3-yl)acetic acid | 2.0g |

(continued)

| | |
|---|---|
| Mannitol | 20 g |
| Distilled water | 1000 ml |

Industrial Applicability

Since the compounds of the present invention represented by formula (I) binds to CRTH2 receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome. They also participate in sleep and aggregation of platelets and are believed to be useful for those diseases as well.

Also, since the compounds of the present invention represented by formula (I) binds to DP receptors and shows antagonistic activity, they are believed to be useful for prevention and/or treatment of diseases such as allergic disease (such as allergic rhinitis, allergic conjunctivitis, atopic dermatitis, bronchial asthma and food allergy), systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch (such as atopic dermatitis, urticaria, allergic conjunctivitis, allergic rhinitis and contact dermatitis), diseases (such as cataract, retinal detachment, inflammation, infection and sleep disorder) which are generated secondarily as a result of behavior accompanied by itch (such as scratching and beating), inflammation, chronic obstructive pulmonary diseases, ischemic reperfusion injury, cerebrovascular accident, autoimmune disease, cerebral lesion, hepatopathy, graft rejection, chronic articular rheumatism, pleuritis, osteoarthritis, Crohn's disease, ulcerative colitis and irritable bowel syndrome.

## Claims

**1.** A indole derivative compound represented by formula (I)

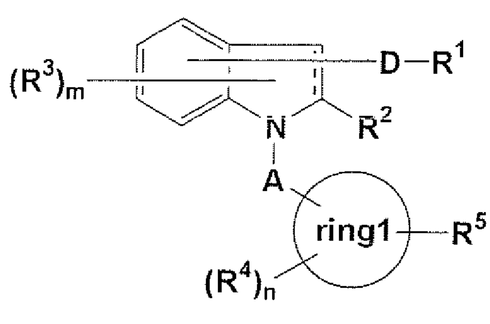

(I)

wherein $R^1$ represents (1) $-COR^6$ or (2) $-CH_2OR^7$;

$R^6$ represents (1) hydroxy, (2) C1-6 alkoxy, (3) $-NR^8R^9$, (4) C1-6 alkoxy substituted with phenyl or (5) C2-6 alkenyloxy;

$R^7$ represents (1) a hydrogen atom or (2) C2-6 acyl;

$R^8$ and $R^9$ each independently represents (1) a hydrogen atom, (2) C1-6 alkyl or (3) - $SO_2R^{10}$;

$R^{10}$ represents (1) C1-6 alkyl, (2) carbocycle-1 or (3) heterocycle-1;

D represents (1) a single bond, (2) C1-6 alkylene, (3) C2-6 alkenylene or (4) -O-(C1-6 alkylene)-;

$R^2$ represents (1) C1-6 alkyl, (2) C1-6 alkoxy, (3) a halogen atom, (4) trihalomethyl, (5) cyano, (6) hydroxy or (7) a hydrogen atom;

$R^3$ and $R^4$ each independently represents (1) a hydrogen atom, (2) C1-6 alkyl, (3) C1-6 alkoxy, (4) C1-6 alkyl substituted with C1-6 alkoxy, (5) a halogen atom, (6) nitro, (7) - $NR^{11}R^{12}$, (8) trihalomethyl, (9) cyano, (10) hydroxy or (11) trihalomethoxy;

$R^{11}$ and $R^{12}$ each independently represents a hydrogen atom or C1-6 alkyl;

m represents an integer of 1 to 3 or 4;

n represents an integer of 1 to 4;

$R^5$ represents $R^{5-1}$,$R^{5-2}$, $R^{5-3}$, $R^{5-4}$, $R^{5-5}$ or $R^{5-6}$;

$R^{5-1}$ represents

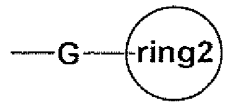 ;

$R^{5-2}$ represents (1) C1-15 alkyl may be substituted with 1-5 of an oxygen atom and/or a sulfur atom, in which the alkyl may be substituted with 1 to 12 substituent(s) selected from C1-6 alkoxy, a halogen atom, hydroxy, cyano, oxo and $NR^{13}R^{14}$, in which $R^{13}$ and $R^{14}$ each independently represents a hydrogen atom, C1-6 alkyl, C2-6 alkenyl, phenyl, benzoyl, naphthyl, phenyl substituted with C1-6 alkyl, or C1-6 alkyl substituted with phenyl or cyano, (2) C2-15 alkenyl may be substituted with 1-5 of an oxygen atom and/or a sulfur atom, in which the alkenyl may be substituted with 1 to 12 substituent(s) selected from C1-6 alkoxy, a halogen atom, hydroxy, cyano, oxo and $NR^{13}R^{14}$, in which $R^{13}$ and $R^{14}$ have the same meanings as described above, or (3) C2-15 alkynyl may be substituted with 1-5 of an oxygen atom and/or a sulfur atom, in which the alkynyl may be substituted with 1 to 12 substituent(s) selected from C1-6 alkoxy, a halogen atom, hydroxy, cyano, oxo and $NR^{13}R^{14}$, in which $R^{13}$ and $R^{14}$ have the same meanings as described above, except a group represented by $R^{5-3}$ and $R^{5-5}$ described below;

$R^{5-3}$ represents (1) C1-6 alkyl substituted with C1-6 alkoxy or (2) C1-6 alkoxy substituted with C1-6 alkoxy;

$R^{5-4}$ represents (1) C1-15 alkyl which is substituted with one nitrogen atom and may be further substituted with 1 to 4 of a nitrogen atom, an oxygen atom and/or a sulfur atom, in which the alkyl may be substituted with 1 to 12 substituent(s) selected from C1-6 alkoxy, a halogen atom, hydroxy, cyano, oxo and $NR^{15}R^{16}$, in which $R^{15}$ and $R^{16}$ each independently represents a hydrogen atom, C1-6 alkyl, C2-6 alkenyl, phenyl, benzoyl, naphthyl, phenyl substituted with C1-6 alkyl, or C1-6 alkyl substituted with phenyl or cyano, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4, (2) C2-15 alkenyl which is substituted with one nitrogen atom and may be further substituted with 1 to 4 of a nitrogen atom, an oxygen atom and/or a sulfur atom, in which the alkenyl may be substituted with 1 to 12 substituent(s) selected from C1-6 alkoxy, a halogen atom, hydroxy, cyano, oxo and $NR^{15}R^{16}$, in which $R^{15}$ and $R^{16}$ have the same meanings as described above, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4) or (3) C2-15 alkynyl which is substituted with one nitrogen atom and may be further substituted with 1 to 4 of a nitrogen atom, an oxygen atom and/or a sulfur atom (in which the alkynyl may be substituted with 1 to 12 substituent(s) selected from C1-6 alkoxy, a halogen atom, hydroxy, cyano, oxo and $NR^{15}R^{16}$, in which $R^{15}$ and $R^{16}$ have the same meanings as described above, and the substituted nitrogen atom may be substituted with (a) C1-6 alkyl, (b) C1-6 alkyl substituted with C1-6 alkoxy, (c) carbocycle-4, (d) heterocycle-4, (e) C1-6 alkyl substituted with carbocycle-4 or (f) C1-6 alkyl substituted with heterocycle-4;

$R^{5-5}$ represents (1) C1-15 alkyl, (2) C1-15 alkoxy, (3) carboxyl, (4) C1-4 alkoxycarbonyl, (5) trihalomethyl or (6) C1-4 alkylthio;

$R^{5-6}$ represents (1) a halogen atom, (2) amino, (3) nitro, (4) cyano or (5) hydroxy;

G represents $G^1$ or $G^2$-,

$G^1$ represents (1) a single bond, (2) C1-6 alkylene may be substituted with 1 to 2 oxygen atom and/or sulfur atom, in which the alkylene may be substituted with hydroxy or C1-4 alkoxy, (3) C2-6 alkenylene may be substituted with 1 to 2 oxygen atom and/or sulfur atom, in which the alkenylene may be substituted with hydroxy or C1-4 alkoxy, (4) -$CONR^{17}$-, (5) -$NR^{18}CO$-, (6) -$SO_2NR^{19}$-, (7) -$NR^{20}SO_2$- or (8) -N=N-;

$G^2$ represents (l) C1-6 alkylene which is substituted with one nitrogen atom and may be further substituted with 1 to 2 of a nitrogen atom, an oxygen atom and/or a sulfur atom, in which the alkylene may be substituted with hydroxy or C1-4 alkoxy, and the substituted nitrogen atom may be substituted with (a) C1-6 alkyl, (b) C1-6 alkyl substituted with C1-6 alkoxy, (c) carbocycle-5, (d) heterocycle-5, (e) C1-6 alkyl substituted with carbocycle-5 or (f) C1-6 alkyl substituted with heterocycle-5, or (2) C2-6 alkenylene which is substituted with one nitrogen atom and may be further substituted with 1 to 2 of a nitrogen atom, an oxygen atom and/or a sulfur atom, in which the alkenylene may be substituted with hydroxy or C1-4 alkoxy, and the substituted nitrogen atom may be substituted

with (a) C1-6 alkyl, (b) C1-6 alkyl substituted with C1-6 alkoxy, (c) carbocycle-5, (d) heterocycle-5, (e) C1-6 alkyl substituted with carbocycle-5 or (f) C1-6 alkyl substituted with heterocycle-5;

$R^{17}$, $R^{18}$, $R^{19}$ and $R^{20}$ each independently represents a hydrogen atom or C1-6 alkyl;

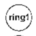 represents (1) carbocycle-2 or (2) heterocycle-2;

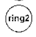 represents (1) carbocycle-3 or (2) heterocycle-3;

carbocycle-1, carbocycle-2, carbocycle-3, carbocycle-4 and carbocyde-5 each independently represents C3-15 mono-, bi- or tricyclic carboaryl which may be partially or fully saturated;

heterocycle-1, heterocycle-2, heterocycle-3, heterocycle-4 and heterocycle-5 each independently represents 3-15 membered mono-, bi- or tricyclic heteroaryl containing 1 to 5 of hetero atom which is selected from an oxygen atom, a nitrogen atom and a sulfur atom, which may be partially or fully saturated;

carbocycle-1, carbocycle-2, carbocycle-3, carbocycle-4, carbocycle-5, heterocycle-1, heterocycle-2, heterocycle-3, heterocycle-4 and heterocycle-5 each independently may be substituted with 1 to 5 of substituent(s) selected from (1) C1-6 alkyl, (2) C1-10 alkoxy, (3) C1-6 alkyl substituted with C1-6 alkoxy, (4) a halogen atom, (5) hydroxy, (6) trihalomethyl, (7) nitro, (8) $-NR^{21}R^{22}$, (9) phenyl, (10) phenoxy, (11) oxo, (12) C2-6 acyl, (13) cyano or (14) $-SO_2R^{23}$;

$R^{21}$ and $R^{22}$ each independently represents a hydrogen atom or C1-6 alkyl;

$R^{23}$ represents C1-6 alkyl;

A represents (1) carbonyl, (2) $-S(O)_p-$, (3)$G^1$ or (4)$G^2$;

p represents 0 or an integer of 1 to 2;

----- represents (1) a single bond or (2) a double bond;

except for compounds of (1) and (2);

(1) 2-(1-(4-benzyloxybenzoyl)-2-methyl-5-methoxyindol-3-yl)acetic acid methyl ester,
(2) 2-(1-(4-phenylbenzoyl)-2-methyl-5-methoxyindol-3-yl)acetic acid methyl ester), a salt thereof, an N-oxide thereof, a solvate thereof or a prodrug thereof

**2.** The indole derivative compound according to claim 1, which is represented by formula (I-1)

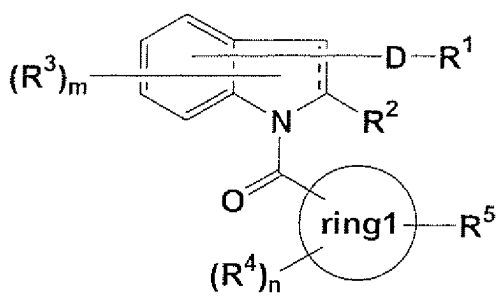

(I − 1)

wherein all symbols have the same meanings as described in claim 1,
a salt thereof, an N-oxide thereof, a solvate thereof or a prodrug thereof.

**3.** The indole derivative compound according to claim 1, which is represented by formula (1-2)

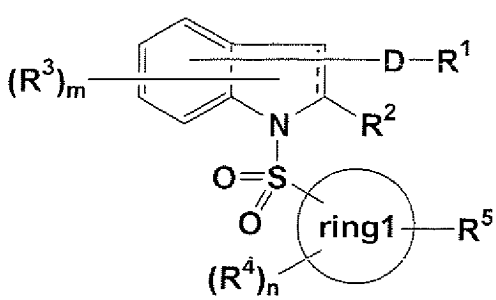

(I − 2)

wherein all symbols have the same meanings as described in claim 1,
a salt thereof, an N-oxide thereof, a solvate thereof or a prodrug thereof.

**4.** The indole derivative compound according to claim 2, which is represented by formula (I-1-1)

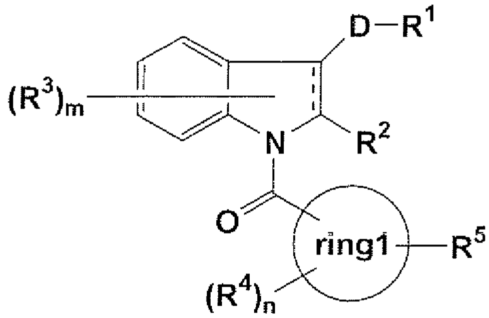

(I－1－1)

wherein all symbols have the same meanings as described in claim 1,
a salt thereof, an N-oxide thereof, a solvate thereof or a prodrug thereof.

**5.** The indole derivative compound according to claim 2, which is represented by formula (I-1-2)

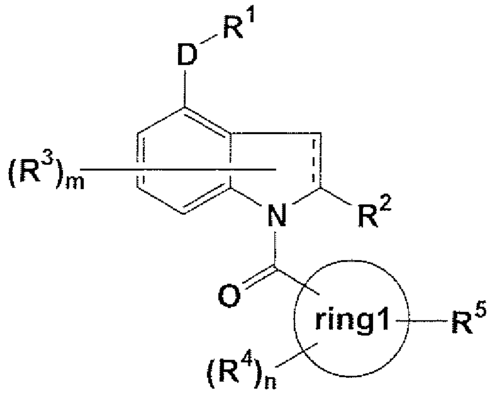

(I－1－2)

wherein all symbols have the same meanings as described in claim 1,
a salt thereof, an N-oxide thereof, a solvate thereof or a prodrug thereof

**6.** The indole derivative compound according to claim 4, wherein, in formula (I-1-1), $R^2$ is (I) C1-6 alkyl, (2) C1-6 alkoxy, (3) a halogen atom, (4) trihalomethyl, (5) cyano or (6) hydroxy, a salt thereof, an N-oxide thereof, a solvate thereof or a prodrug thereof.

**7.** The indole derivative compound according to claim 4, wherein, in formula (I-1-1), $R^2$ is a hydrogen atom, a salt thereof, an N-oxide thereof, a solvate thereof or a prodrug thereof

**8.** The indole derivative compound according to claim 6, wherein 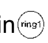 is benzene, a salt thereof, an N-oxide thereof a solvate thereof or a prodrug thereof.

**9.** The indole derivative compound according to claim 6, wherein $R^5$ is $R^{5-1}$ and G is $G^2$, a salt thereof, an N-oxide thereof, a solvate thereof or a prodrug thereof.

**10.** The indole derivative compound according to claim 8, wherein $R^5$ is $R^{5-2}$, a salt thereof, an N-oxide thereof, a solvate thereof or a prodrug thereof

**11.** The indole derivative compound according to claim 8, wherein $R^5$ is $R^{5-4}$, a salt thereof, an N-oxide thereof, a solvate thereof or a prodrug thereof

**12.** The indole derivative compound according to claim 8, wherein (1) $R^5$ is $R^{5-1}$ and G is $G^1$, or (2) $R^5$ is $R^{5-3}$, a salt

thereof, an N-oxide thereof, a solvate thereof or a prodrug thereof.

**13.** The indole derivative compound according to claim 12, which is selected from the group consisting of

(1) (5-chloro-1-(4-(((2S)-4,6-dimethyl-3,4-dihydro-2H-1,4-benzoxazin-2-yl)methoxy)benzoyl)-2-methyl-1H-indol-3-yl)acetic acid,
(2) (1-(4-(((2S)-4,6-dimethyl-3,4-dihydro-2H-1,4-benzoxazin-2-yl)methoxy)benzoyl)-2,5-dimethyl-1H-indol-3-yl)acetic acid,
(3) (1-(4-(((2S)-4,6-dimethyl-3,4-dihydro-2H-1,4-benzoxazin-2-yl)methoxy)-3-methylbenzoyl)-5-fluoro-2-methyl-1H-indol-3-yl)acetic acid,
(4) (1-(2-chloro-4-(((2S)-6-fluoro-4-methyl-3,4-dihydro-2H-1,4-benzoxazin-2-yl)methoxy)benzoyl)-2,5-dimethyl-1H-indol-3-yl)acetic acid,
(5) (5-chloro-1-(4-(((2S)-6-fluoro-4-methyl-3,4-dihydro-2H-1,4-benzoxazin-2-yl)methoxy)-3-methylbenzoyl)-2-methyl-1H-indol-3-yl)acetic acid,
(6) (1-(4-(((2S)-6-fluoro-4-methyl-3,4-dihydro-2H-1,4-benzoxazin-2-yl)methoxy)-2,5-dimethylbenzoyl)-2-methyl-1H-indol-3-yl)acetic acid,
(7) (5-fluoro-1-(4-(((2S)-6-fluoro-4-methyl-3,4-dihydro-2H-1,4-benzoxazin-2-yl)methoxy)-2,5-dimethylbenzoyl)-2-methyl-1H-indol-3-yl)acetic acid,
(8) (1-(4-(((2S)-4,6-dimethyl-3,4-dihydro-2H-1,4-benzoxazin-2-yl)methoxy)-3-methylbenzoyl)-2,5-dimethyl-1H-indol-3-yl)acetic acid,
(9) (1-(4-(((2S)-4,6-dimethyl-3,4-dihydro-2H-1,4-benzoxazin-2-yl)methoxy)-2-methylbenzoyl)-5-fluoro-2-methyl-1H-indol-3-yl)acetic acid,
(10) (5-chloro-1-(4-(((25)-6-fluoro-4-methyl-3,4-dihydro-2H-1,4-benzoxazin-2-yl)methoxy)-2,5-dimethylbenzoyl)-2-methyl-1H-indol-3-yl)acetic acid,
(11) (1-(4-(((2S)-6-fluoro-4-methyl-3,4-dihydro-2H-1,4-benzoxazin-2-yl)methoxy)-2,5-dimethylbenzoyl)-2,5-dimethyl-1H-indol-3-yl)acetic acid,
(12) (1-(4-(((2S)-4,6-dimethyl-3,4-dihydro-2H-1,4-benzoxazin-2-yl)methoxy)-2,5-dimethylbenzoyl)-5-fluoro-2-methyl-1H-indol-3-yl)acetic acid, and
(13) (1-(4-(((2S)-4,6-dimethyl-3,4-dihydro-2H-1,4-benzoxazin-2-yl)methoxy)-2,5-dimethylbenzoyl)-2-methyl-1H-indol-3-yl)acetic acid,

a salt thereof, an N-oxide thereof, a solvate thereof or a prodrug thereof.

**14.** The indole derivative compound according to claim 6, wherein 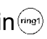 is carbocycle-2, except for benzene, and $R^5$ is $R^{5-1}$, $R^{5-2}$ , $R^{5-3}$ or $R^{5-4}$, a salt thereof, an N-oxide thereof, a solvate thereof or a prodrug thereof.

**15.** The indole derivative compound according to claim 6, wherein 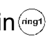 is heterocycle-2 and $R^5$ is $R^{5-1}$, $R^{5-2}$, $R^{5-3}$ or $R^{5-4}$, a salt thereof, an N-oxide thereof, a solvate thereof or a prodrug thereof.

**16.** A CRTH2 receptor antagonist comprising the indole derivative compound according to claim 1, 2-(1-(4-benzyloxybenzoyl)-2-methyl-5-methoxyindol-3-yl)acetic acid methyl ester, or 2-(1-(4-phenylbenzoyl)-2-methyl-5-methoxyindol-3-yl)acetic acid methyl ester, a salt thereof, an N-oxide thereof, a solvate thereof or a prodrug thereof as an active ingredient.

**17.** The CRTH2 receptor antagonist according to claim 16, which comprises the compound according to claim 4 as an active ingredient.

**18.** A DP receptor antagonist comprising the compound according to claim 1, 2-(1-(4-benzyloxybenzoyl)-2-methyl-5-methoxyindol-3-yl)acetic acid methyl ester, or 2-(1-(4-phenylbenzoyl)-2-methyl-5-methoxyindol-3-yl)acetic acid methyl ester, or a pharmaceutically acceptable salt thereof as an active ingredient.

**19.** A pharmaceutical composition comprising the compound according to claim 1 as an active ingredient.

**20.** The pharmaceutical composition according to claim 19, which is an agent for prevention and/or treatment of allergic disease, systemic mastocytosis, systemic mast cell activating disorder, anaphylaxis shock, airway contraction, urticaria, eczema, pimples, allergic bronchial pulmonary aspergillosis, sinusitis, migraine, nasal polypus, anaphylactic vasculitis, eosinophilia, contact dermatitis, diseases accompanied by itch, diseases which is generated secondarily as a result of behavior accompanied by itch, inflammation, chronic obstructive pulmonary diseases, ischemic reperfusion injury, cerebrovascular accident, autoimmune disease, cerebral lesion, hepatopathy, graft

rejection, chronic articular rheumatism, pleuritis, osteoarthritis, Crohn's disease, ulcerative colitis, irritable bowel syndrome, sleep disorder or aggregation of platelets.

**21.** A medicament comprising a combination of the indole derivative compound according to claim 1, a salt thereof, an N-oxide thereof, a solvate thereof or a prodrug thereof, and at least one or more agent(s) selected from DP antagonist, antihistaminic agent, suppressor for mediator liberation, inhibitor for thromboxane synthase, antagonist for thromboxanc A2 receptor, antagonist for leukotriene receptor, steroid, stimulant for α-adrenaline receptor, xanthine derivative, anticholinergic agent and/or suppressor for nitrogen monoxide synthase.

**22.** A method for antagonizing CRTH2 receptor, which comprises administering to a mammal an effective amount of the indole derivative compound represented by formula (I)

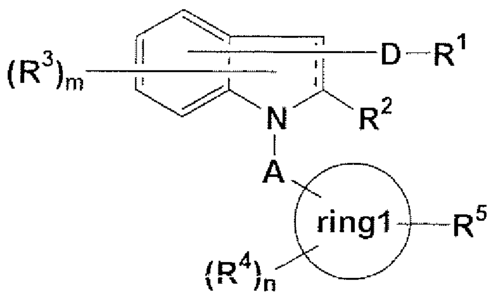

( I )

wherein all symbols have the same meanings as described in claim 1, 2-(1-(4-benzyloxybenzoyl)-2-methyl-5-methoxyindol-3-yl)acetic acid methyl ester, 2-(1-(4-phenylbenzoyl)-2-methyl-5-methoxyindol-3-yl)acetic acid methyl ester, a salt thereof, an N-oxide thereof, a solvate thereof or a prodrug thereof

**23.** Use of the indole derivative compound represented by formula (I)

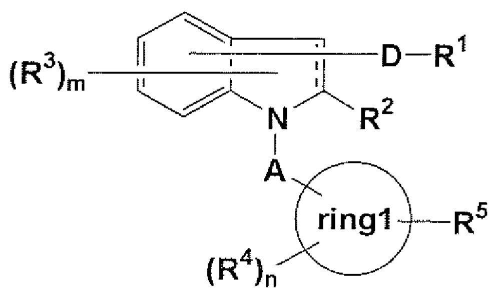

( I )

wherein all symbols have the same meanings as described in claim 1, 2-(1-(4-benzyloxybenzoyl)-2-methyl-5-methoxyindol-3-yl)acetic acid methyl ester, 2-(1-(4-phenylbenzoyl)-2-methyl-5-methoxyindol-3-yl)acetic acid methyl ester, a salt thereof, an N-oxide thereof, a solvate thereof or a prodrug thereof for the manufacture of a CRTH2 receptor antagonist.

**INTERNATIONAL SEARCH REPORT**

International application No.
PCT/JP2004/002813

A. CLASSIFICATION OF SUBJECT MATTER
Int.Cl[7] C07D209/26, 401/12, 403/12, 405/12, 409/12, 411/12, 413/06, 413/12, 413/14, A61K31/405, 31/422, 31/4439, 31/4709, 31/538, 45/06, 31/423, (continue to extra sheet.)
According to International Patent Classification (IPC) or to both national classification and IPC

B. FIELDS SEARCHED
Minimum documentation searched (classification system followed by classification symbols)
Int.Cl[7] C07D209/26, 401/12, 403/12, 405/12, 409/12, 411/12, 413/06, 413/12, 413/14, A61K31/405, 31/422, 31/4439, 31/4709, 31/538, 45/06, 31/423, (continue to extra sheet.)

Documentation searched other than minimum documentation to the extent that such documents are included in the fields searched

Electronic data base consulted during the international search (name of data base and, where practicable, search terms used)
STN/CAS

C. DOCUMENTS CONSIDERED TO BE RELEVANT

| Category* | Citation of document, with indication, where appropriate, of the relevant passages | Relevant to claim No. |
|---|---|---|
| P,X | WO 03/022813 A1 (Ono Pharmaceutical Co., Ltd.), 20 March, 2003 (20.03.03), (Family: none) | 1-21,23 |
| X | WO 01/66520 A1 (Ono Pharmaceutical Co., Ltd.), 13 September, 2001 (13.09.01), & CA 2402174 A & AU 4106801 A & NO 20024281 A & EP 1262475 A | 1-21,23 |
| X | EP 1170594 A2 (Pfizer Products Inc.), 09 January, 2002 (09.01.02), & US 2002/22218 A1 & JP 2002-98702 A | 1-21,23 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 April, 2004 (13.04.04) | Date of mailing of the international search report<br>27 April, 2004 (27.04.04) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2004/002813

**Box No. II Observations where certain claims were found unsearchable (Continuation of item 2 of first sheet)**

This international search report has not been established in respect of certain claims under Article 17(2)(a) for the following reasons:

1. [X] Claims Nos.: 22
because they relate to subject matter not required to be searched by this Authority, namely:
It pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2004/002813

Claim 1 involves a great number of compounds. However, only small part of the claimed compounds are supported by the description in the meaning within PCT Article 6 and disclosed in the meaning within PCT Article 5.

Such being the case, the search was made mainly on the part supported and disclosed in the description, i.e., EXAMPLES.

Continuation of A. CLASSIFICATION OF SUBJECT MATTER
(International Patent Classification (IPC))

Int.Cl$^7$ A61P1/04, 1/16, 7/00, 7/02, 9/00, 11/00, A61P11/00, 11/02, 11/06, 11/08, 17/00, 17/04, 17/10, 19/02, 25/06, 25/20, 27/02, 27/12, 27/14, 27/16, 29/00, 37/02, 37/06, 37/08, 43/00

(According to International Patent Classification (IPC) or to both national classification and IPC)

Continuation of B. FIELDS SEARCHED
Minimum documentation searched (International Patent Classification (IPC))

Int.Cl$^7$ A61P1/04, 1/16, 7/00, 7/02, 9/00, 11/00, A61P11/00, 11/02, 11/06, 11/08, 17/00, 17/04, 17/10, 19/02, 25/06, 25/20, 27/02, 27/12, 27/14, 27/16, 29/00, 37/02, 37/06, 37/08, 43/00

Minimum documentation searched (classification system followed by classification symbols)

Form PCT/ISA/210 (extra sheet) (January 2004)